(19) Europäisches Patentamt
European Patent Office
Office européen des brevets

(11) EP 3 753 956 A1

(12) **EUROPEAN PATENT APPLICATION**
published in accordance with Art. 153(4) EPC

(43) Date of publication:
**23.12.2020 Bulletin 2020/52**

(21) Application number: **19754024.8**

(22) Date of filing: **14.02.2019**

(51) Int Cl.:
*C07K 16/42* (2006.01) *C07K 16/00* (2006.01)
*C07K 16/28* (2006.01) *C12N 15/13* (2006.01)

(86) International application number:
**PCT/JP2019/005258**

(87) International publication number:
**WO 2019/160007 (22.08.2019 Gazette 2019/34)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.02.2018 JP 2018024009**

(71) Applicant: **CHUGAI SEIYAKU KABUSHIKI KAISHA**
**Tokyo 115-8543 (JP)**

(72) Inventors:
- **IGAWA, Tomoyuki**
  **Synapse, 138623 (SG)**
- **HIRONIWA, Naoka**
  **Synapse, 138623 (SG)**
- **KAMIKAWAJI, Shogo**
  **Kamakura-shi, Kanagawa 247-8530 (JP)**
- **KIBAYASHI, Tatsuya**
  **Gotemba-shi, Shizuoka 412-8513 (JP)**
- **SAVORY, Nasa**
  **Gotemba-shi, Shizuoka 412-8513 (JP)**
- **MIMOTO, Futa**
  **Synapse, 138623 (SG)**

(74) Representative: **Vossius & Partner**
**Patentanwälte Rechtsanwälte mbB**
**Siebertstrasse 3**
**81675 München (DE)**

# (54) ANTIGEN-BINDING MOLECULE AND COMBINATION

(57) The present invention relates to a first antigen-binding molecule, a second antigen-binding molecule, and a combination thereof. The second antigen-binding molecule binds to an antigen/antigen-binding molecule complex containing a first antigen and the first antigen-binding molecule, and enhances the binding activity of the first antigen-binding molecule to the first antigen.

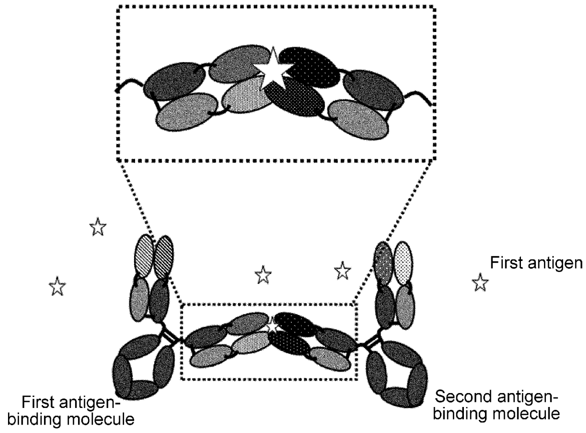


FIG. 1



Printed by Jouve, 75001 PARIS (FR)

SEQUENCE LISTING

<110> CHUGAI SEIYAKU KABUSHIKI KAISHA

<120> ANTIGEN-BINDING MOLECULE AND COMBINATION

<130> C1-A1802P

<150> JP 2018-024009
<151> 2018-02-14

<160> 168

<170> PatentIn version 3.5

<210> 1
<211> 122
<212> PRT
<213> Artificial Sequence

<220>
<223> CE115HA000VH

<400> 1

```
Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asn Ala
            20                  25                  30

Trp Met His Trp Val Arg Gln Ala Pro Gly Lys Gly Leu Glu Trp Val
        35                  40                  45

Ala Gln Ile Lys Ala Lys Ser Asn Asn Tyr Ala Thr Tyr Tyr Ala Glu
    50                  55                  60

Ser Val Lys Gly Arg Phe Thr Ile Ser Arg Asp Asp Ser Lys Asn Ser
65                  70                  75                  80

Leu Tyr Leu Gln Met Asn Ser Leu Lys Thr Glu Asp Thr Ala Val Tyr
                85                  90                  95

Tyr Cys Arg Tyr Val His Tyr Gly Ala Tyr Tyr Gly Val Asp Ala Trp
            100                 105                 110

Gly Gln Gly Thr Thr Val Thr Val Ser Ser
        115                 120
```

<210> 2
<211> 122
<212> PRT
<213> Artificial Sequence

<220>

<223> CE115HA056VH

<400> 2

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Ala His Tyr Gly Ala Tyr Tyr Gly Val Asp Ala Trp
100 105 110

Gly Gln Gly Thr Thr Val Thr Val Ser Ser
115 120

<210> 3
<211> 122
<212> PRT
<213> Artificial Sequence

<220>
<223> CE115HA146VH

<400> 3

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser

65 70 75 80

Leu Tyr Leu Gln Met Asn Ser Leu Lys Thr Glu Asp Thr Ala Val Tyr
85 90 95

Tyr Cys Arg Tyr Val Ala Tyr Gly Ala Tyr Tyr Gly Val Asp Ala Trp
100 105 110

Gly Gln Gly Thr Thr Val Thr Val Ser Ser
115 120

<210> 4
<211> 8
<212> PRT
<213> Artificial Sequence

<220>
<223> GS linker

<400> 4

Gly Gly Gly Ser Gly Gly Gly Ser
1 5

<210> 5
<211> 8
<212> PRT
<213> Homo sapiens

<400> 5

Gln Asp Gly Asn Glu Glu Met Gly
1 5

<210> 6
<211> 463
<212> PRT
<213> Artificial Sequence

<220>
<223> CE115HAPG13rabCH1hG1m

<400> 6

Gln Asp Gly Asn Glu Glu Met Gly Gly Gly Gly Ser Gly Gly Gly Ser
1 5 10 15

Gln Val Gln Leu Val Glu Ser Gly Gly Gly Val Val Gln Pro Gly Arg
20 25 30

Ser Leu Arg Leu Ser Cys Ala Ala Ser Gly Phe Thr Phe Ser Asn Ala
35 40 45

Trp Met His Trp Val Arg Gln Ala Pro Gly Lys Gly Leu Glu Trp Val

50 55 60

Ala Gln Ile Lys Ala Lys Ser Asn Asn Tyr Ala Thr Tyr Tyr Ala Glu
65 70 75 80

Ser Val Lys Gly Arg Phe Thr Ile Ser Arg Asp Asp Ser Lys Asn Ser
85 90 95

Leu Tyr Leu Gln Met Asn Ser Leu Lys Thr Glu Asp Thr Ala Val Tyr
100 105 110

Tyr Cys Arg Tyr Val Ala Tyr Gly Ala Tyr Tyr Gly Val Asp Ala Trp
115 120 125

Gly Gln Gly Thr Thr Val Thr Val Ser Ser Gly Gln Pro Lys Ala Pro
130 135 140

Ser Val Phe Pro Leu Ala Pro Cys Cys Gly Asp Thr Pro Ser Ser Thr
145 150 155 160

Val Thr Leu Gly Cys Leu Val Lys Gly Tyr Leu Pro Glu Pro Val Thr
165 170 175

Val Thr Trp Asn Ser Gly Thr Leu Thr Asn Gly Val Arg Thr Phe Pro
180 185 190

Ser Val Arg Gln Ser Ser Gly Leu Tyr Ser Leu Ser Ser Val Val Ser
195 200 205

Val Thr Ser Ser Ser Gln Pro Val Thr Cys Asn Val Ala His Pro Ala
210 215 220

Thr Asn Thr Lys Val Asp Lys Thr Val Glu Pro Lys Ser Cys Asp Lys
225 230 235 240

Thr His Thr Cys Pro Pro Cys Pro Ala Pro Glu Leu Leu Gly Gly Pro
245 250 255

Ser Val Phe Leu Phe Pro Pro Lys Pro Lys Asp Thr Leu Met Ile Ser
260 265 270

Arg Thr Pro Glu Val Thr Cys Val Val Val Asp Val Ser His Glu Asp
275 280 285

Pro Glu Val Lys Phe Asn Trp Tyr Val Asp Gly Val Glu Val His Asn
290 295 300

Ala Lys Thr Lys Pro Arg Glu Glu Gln Tyr Asn Ser Thr Tyr Arg Val
305                 310                 315                 320

Val Ser Val Leu Thr Val Leu His Gln Asp Trp Leu Asn Gly Lys Glu
                325                 330                 335

Tyr Lys Cys Lys Val Ser Asn Lys Ala Leu Pro Ala Pro Ile Glu Lys
            340                 345                 350

Thr Ile Ser Lys Ala Lys Gly Gln Pro Arg Glu Pro Gln Val Tyr Thr
        355                 360                 365

Leu Pro Pro Ser Arg Glu Glu Met Thr Lys Asn Gln Val Ser Leu Thr
    370                 375                 380

Cys Leu Val Lys Gly Phe Tyr Pro Ser Asp Ile Ala Val Glu Trp Glu
385                 390                 395                 400

Ser Asn Gly Gln Pro Glu Asn Asn Tyr Lys Thr Thr Pro Pro Val Leu
                405                 410                 415

Asp Ser Asp Gly Ser Phe Phe Leu Tyr Ser Lys Leu Thr Val Asp Lys
            420                 425                 430

Ser Arg Trp Gln Gln Gly Asn Val Phe Ser Cys Ser Val Met His Glu
        435                 440                 445

Ala Leu His Asn His Tyr Thr Gln Lys Ser Leu Ser Leu Ser Pro
    450                 455                 460

<210>  7
<211>  216
<212>  PRT
<213>  Artificial Sequence

<220>
<223>  GLS3000-rabk

<400>  7

Asp Ile Val Met Thr Gln Ser Pro Leu Ser Leu Pro Val Thr Pro Gly
1               5                   10                  15

Glu Pro Ala Ser Ile Ser Cys Arg Ser Ser Gln Ser Leu Val His Ser
            20                  25                  30

Asn Arg Asn Thr Tyr Leu His Trp Tyr Gln Gln Lys Pro Gly Gln Ala
        35                  40                  45

Pro Arg Leu Leu Ile Tyr Lys Val Ser Asn Arg Phe Ser Gly Val Pro

50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

Gly Asp Pro Val Ala Pro Thr Val Leu Ile Phe Pro Pro Ala Ala Asp
115 120 125

Gln Val Ala Thr Gly Thr Val Thr Ile Val Cys Val Ala Asn Lys Tyr
130 135 140

Phe Pro Asp Val Thr Val Thr Trp Glu Val Asp Gly Thr Thr Gln Thr
145 150 155 160

Thr Gly Ile Glu Asn Ser Lys Thr Pro Gln Asn Ser Ala Asp Cys Thr
165 170 175

Tyr Asn Leu Ser Ser Thr Leu Thr Leu Thr Ser Thr Gln Tyr Asn Ser
180 185 190

His Lys Glu Tyr Thr Cys Lys Val Thr Gln Gly Thr Thr Ser Val Val
195 200 205

Gln Ser Phe Asn Arg Gly Asp Cys
210 215

<210> 8
<211> 447
<212> PRT
<213> Artificial Sequence

<220>
<223> CE115HA146-rabCH1hG1m

<400> 8

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

```
Ala Gln Ile Lys Ala Lys Ser Asn Asn Tyr Ala Thr Tyr Tyr Ala Glu
    50                  55                  60

Ser Val Lys Gly Arg Phe Thr Ile Ser Arg Asp Asp Ser Lys Asn Ser
65                  70                  75                  80

Leu Tyr Leu Gln Met Asn Ser Leu Lys Thr Glu Asp Thr Ala Val Tyr
                85                  90                  95

Tyr Cys Arg Tyr Val Ala Tyr Gly Ala Tyr Tyr Gly Val Asp Ala Trp
            100                 105                 110

Gly Gln Gly Thr Thr Val Thr Val Ser Ser Gly Gln Pro Lys Ala Pro
        115                 120                 125

Ser Val Phe Pro Leu Ala Pro Cys Cys Gly Asp Thr Pro Ser Ser Thr
    130                 135                 140

Val Thr Leu Gly Cys Leu Val Lys Gly Tyr Leu Pro Glu Pro Val Thr
145                 150                 155                 160

Val Thr Trp Asn Ser Gly Thr Leu Thr Asn Gly Val Arg Thr Phe Pro
                165                 170                 175

Ser Val Arg Gln Ser Ser Gly Leu Tyr Ser Leu Ser Ser Val Val Ser
            180                 185                 190

Val Thr Ser Ser Ser Gln Pro Val Thr Cys Asn Val Ala His Pro Ala
        195                 200                 205

Thr Asn Thr Lys Val Asp Lys Thr Val Glu Pro Lys Ser Cys Asp Lys
    210                 215                 220

Thr His Thr Cys Pro Pro Cys Pro Ala Pro Glu Leu Leu Gly Gly Pro
225                 230                 235                 240

Ser Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Asn Ser Thr Tyr Arg Val
```

```
        290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Glu Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380

Ser Asn Gly Gln Pro Glu Asn Asn Tyr Lys Thr Thr Pro Pro Val Leu
385                 390                 395                 400

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
            420                 425                 430

Ala Leu His Asn His Tyr Thr Gln Lys Ser Leu Ser Leu Ser Pro
        435                 440                 445


<210>  9
<211>  456
<212>  PRT
<213>  Artificial Sequence

<220>
<223>  hGC33VHGP01-rabCH1hG1m

<400>  9

Gln Asp Gly Asn Glu Glu Met Gly Gly Gly Gly Ser Gly Gly Gly Ser
1               5                   10                  15

Gln Val Gln Leu Val Gln Ser Gly Ala Glu Val Lys Lys Pro Gly Ala
            20                  25                  30

Ser Val Lys Val Ser Cys Lys Ala Ser Gly Tyr Thr Phe Thr Asp Tyr
        35                  40                  45

Glu Met His Trp Val Arg Gln Ala Pro Gly Gln Gly Leu Glu Trp Met
    50                  55                  60
```

Gly Ala Leu Asp Pro Lys Thr Gly Asp Thr Ala Tyr Ser Gln Lys Phe
65 70 75 80

Lys Gly Arg Val Thr Leu Thr Ala Asp Lys Ser Thr Ser Thr Ala Tyr
85 90 95

Met Glu Leu Ser Ser Leu Thr Ser Glu Asp Thr Ala Val Tyr Tyr Cys
100 105 110

Thr Arg Phe Tyr Ser Tyr Thr Tyr Trp Gly Gln Gly Thr Leu Val Thr
115 120 125

Val Ser Ser Gly Gln Pro Lys Ala Pro Ser Val Phe Pro Leu Ala Pro
130 135 140

Cys Cys Gly Asp Thr Pro Ser Ser Thr Val Thr Leu Gly Cys Leu Val
145 150 155 160

Lys Gly Tyr Leu Pro Glu Pro Val Thr Val Thr Trp Asn Ser Gly Thr
165 170 175

Leu Thr Asn Gly Val Arg Thr Phe Pro Ser Val Arg Gln Ser Ser Gly
180 185 190

Leu Tyr Ser Leu Ser Ser Val Val Ser Val Thr Ser Ser Ser Gln Pro
195 200 205

Val Thr Cys Asn Val Ala His Pro Ala Thr Asn Thr Lys Val Asp Lys
210 215 220

Thr Val Glu Pro Lys Ser Cys Asp Lys Thr His Thr Cys Pro Pro Cys
225 230 235 240

Pro Ala Pro Glu Leu Leu Gly Gly Pro Ser Val Phe Leu Phe Pro Pro
245 250 255

Lys Pro Lys Asp Thr Leu Met Ile Ser Arg Thr Pro Glu Val Thr Cys
260 265 270

Val Val Val Asp Val Ser His Glu Asp Pro Glu Val Lys Phe Asn Trp
275 280 285

Tyr Val Asp Gly Val Glu Val His Asn Ala Lys Thr Lys Pro Arg Glu
290 295 300

Glu Gln Tyr Asn Ser Thr Tyr Arg Val Val Ser Val Leu Thr Val Leu

305 310 315 320

His Gln Asp Trp Leu Asn Gly Lys Glu Tyr Lys Cys Lys Val Ser Asn
325 330 335

Lys Ala Leu Pro Ala Pro Ile Glu Lys Thr Ile Ser Lys Ala Lys Gly
340 345 350

Gln Pro Arg Glu Pro Gln Val Tyr Thr Leu Pro Pro Ser Arg Glu Glu
355 360 365

Met Thr Lys Asn Gln Val Ser Leu Thr Cys Leu Val Lys Gly Phe Tyr
370 375 380

Pro Ser Asp Ile Ala Val Glu Trp Glu Ser Asn Gly Gln Pro Glu Asn
385 390 395 400

Asn Tyr Lys Thr Thr Pro Pro Val Leu Asp Ser Asp Gly Ser Phe Phe
405 410 415

Leu Tyr Ser Lys Leu Thr Val Asp Lys Ser Arg Trp Gln Gln Gly Asn
420 425 430

Val Phe Ser Cys Ser Val Met His Glu Ala Leu His Asn His Tyr Thr
435 440 445

Gln Lys Ser Leu Ser Leu Ser Pro
450 455

<210> 10
<211> 216
<212> PRT
<213> Artificial Sequence

<220>
<223> hGC33VL-rabk

<400> 10

Asp Val Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Arg Asn Thr Tyr Leu His Trp Tyr Leu Gln Lys Pro Gly Gln Ser
35 40 45

Pro Gln Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Ser Gln Asn
85 90 95

Thr His Val Pro Pro Thr Phe Gly Gln Gly Thr Lys Leu Glu Ile Lys
100 105 110

Gly Asp Pro Val Ala Pro Thr Val Leu Ile Phe Pro Pro Ala Ala Asp
115 120 125

Gln Val Ala Thr Gly Thr Val Thr Ile Val Cys Val Ala Asn Lys Tyr
130 135 140

Phe Pro Asp Val Thr Val Thr Trp Glu Val Asp Gly Thr Thr Gln Thr
145 150 155 160

Thr Gly Ile Glu Asn Ser Lys Thr Pro Gln Asn Ser Ala Asp Cys Thr
165 170 175

Tyr Asn Leu Ser Ser Thr Leu Thr Leu Thr Ser Thr Gln Tyr Asn Ser
180 185 190

His Lys Glu Tyr Thr Cys Lys Val Thr Gln Gly Thr Thr Ser Val Val
195 200 205

Gln Ser Phe Asn Arg Gly Asp Cys
210 215

<210> 11
<211> 17
<212> DNA
<213> Artificial Sequence

<220>
<223> H chain fowrd primer

<400> 11
caccatggag actgggc 17

<210> 12
<211> 15
<212> DNA
<213> Artificial Sequence

<220>
<223> H chain reverse primer

<400> 12

ggaggagacg gtgac 15

<210> 13
<211> 16
<212> DNA
<213> Artificial Sequence

<220>
<223> L chain forward primer

<400> 13
atggacacga gggccc 16

<210> 14
<211> 19
<212> DNA
<213> Artificial Sequence

<220>
<223> L chain reverse primer

<400> 14
tttgaccacc acctcggtc 19

<210> 15
<211> 1039
<212> DNA
<213> Artificial Sequence

<220>
<223> H chain constant region

<400> 15
gaattccacc atggagactg ggtaccgtca ccgtctcctc cgcttccacc aagggcccat 60

cggtcttccc cctggcaccc tcctccaagt ccacctctgg gggcacagcg gccctgggct 120

gcctggtcaa ggactacttc cccgaaccgg tgacggtgtc gtggaactca ggcgccctga 180

cctccggcgt gcacaccttc ccggctgtcc tacagtcctc aggactctac tccctctcct 240

ccgtggtgac cgtgccctcc tcgtccttgg gcacccagac ctacatctgc aacgtgaatc 300

acaagccctc caacaccaag gtggacaaga aagttgagcc caaatcttgt gacaaaactc 360

acacatgccc accgtgccca gcacctgaac tccggggggg accgaaagtc ttcctcttcc 420

ccccaaaacc caaggacacc ctcatgatct cccggacccc tgaggtcaca tgcgtggtgg 480

tggacgtgtc ccacgaagac cctgaggtca agttcaactg gtacgtggac ggcgtggagg 540

tgcataatgc caagacaaag ccgcgggagg agcagtacgc ctccacgtac cgtgtggtct 600

ccgtcctcac cgtcctgcac caggactggc tgaatggcaa ggagtacaag tgcaaggtct 660

ccaacaaagc cctcccagcc cccatcgaga aaaccatctc caaagccaaa gggcagcccc 720

gagaaccaca ggtgtacacc ctgcccccat cccggaagga aatgaccaag aaccaggtct 780

ccctgacctg cctggtcaaa ggcttctatc cctccgacat cgccgtggag tgggagtcca 840

atgggcagcc ggagaacaac tacaagacca cgcctcccta cctggactcc gacggctcct 900

tcttcctcta ctccaagctc accgtggaca agtccaggtg gcagcagggg aacgtcttct 960

catgctccgt gatgcatgag gctctgcaca accactacac gcagaagtcg ctctccctgt 1020

ctccgtgata agcggccgc 1039

<210> 16
<211> 378
<212> DNA
<213> Artificial Sequence

<220>
<223> L chain constant region

<400> 16
gaattccacc atggacacga ggggtaccga ggtggtggtc aaacgtacgg tggctgcacc 60

atctgtcttc atcttcccgc catctgatga gcagttgaaa tctggaactg cctctgttgt 120

gtgcctgctg aataacttct atcccagaga ggccaaagta cagtggaagg tggataacgc 180

cctccaatcg ggtaactccc aggagagtgt cacagagcag gactccaagg actgcaccta 240

ctccctctcc tccaccctga cgctgtccaa agcagactac gagaaacaca aagtctacgc 300

ctgcgaagtc acccatcagg gcctgtcctc gcccgtcaca aagtccttca acaggggaga 360

gtgttgataa gcggccgc 378

<210> 17
<211> 443
<212> PRT
<213> Artificial Sequence

<220>
<223> GCH065-F760mnN17

<400> 17

Gln Val Gln Leu Val Gln Ser Gly Ala Glu Val Lys Lys Pro Gly Ala
1               5                   10                  15

Ser Val Thr Val Ser Cys Lys Ala Ser Gly Tyr Thr Phe Thr Asp Tyr
            20                  25                  30

Glu Met His Trp Ile Arg Gln Pro Pro Gly Glu Gly Leu Glu Trp Ile
        35                  40                  45

Gly Ala Ile Asp Gly Pro Thr Pro Asp Thr Ala Tyr Ser Glu Lys Phe
    50                  55                  60

Lys Gly Arg Val Thr Leu Thr Ala Asp Lys Ser Thr Ser Thr Ala Tyr
65                  70                  75                  80

```
Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Lys Ser Thr Ser Gly Gly Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly
            180                 185                 190

Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Arg Gly Gly Pro Lys Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Ala Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335
```

Ala Lys Gly Gln Pro Arg Glu Pro Gln Val Tyr Thr Leu Pro Pro Ser
340 345 350

Arg Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Tyr Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Glu Ser Leu Ser Leu Ser Pro
435 440

<210> 18
<211> 219
<212> PRT
<213> Artificial Sequence

<220>
<223> L0011-k0

<400> 18

Asp Ile Val Met Thr Gln Ser Pro Leu Ser Leu Pro Val Thr Pro Gly
1 5 10 15

Glu Pro Ala Ser Ile Ser Cys Arg Ser Ser Gln Pro Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 19
<211> 450
<212> PRT
<213> Artificial Sequence

<220>
<223> CE115HA146-F760mnP17

<400> 19

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

```
Leu Tyr Leu Gln Met Asn Ser Leu Lys Thr Glu Asp Thr Ala Val Tyr
                85                  90                  95

Tyr Cys Arg Tyr Val Ala Tyr Gly Ala Tyr Tyr Gly Val Asp Ala Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Arg
225                 230                 235                 240

Gly Gly Pro Lys Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Ala Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335
```

Ile Glu Lys Thr Ile Ser Lys Ala Lys Gly Gln Pro Arg Glu Pro Gln
340 345 350

Val Tyr Thr Leu Pro Pro Ser Arg Lys Glu Met Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Tyr Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
435 440 445

Ser Pro
450

<210> 20
<211> 219
<212> PRT
<213> Artificial Sequence

<220>
<223> GLS3000-k0

<400> 20

Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 21
<211> 6
<212> PRT
<213> Artificial Sequence

<220>
<223> CLA0022_HC_CDR1

<400> 21

Ser Ser Tyr Trp Ile Tyr
1 5

<210> 22
<211> 18
<212> PRT
<213> Artificial Sequence

<220>
<223> CLA0022_HC_CDR2

<400> 22

Cys Ile Tyr Ala Gly Val Asn Asp Ile Thr Tyr Tyr Ala Asn Trp Ala
1 5 10 15

Lys Gly

<210> 23
<211> 9
<212> PRT
<213> Artificial Sequence

<220>
<223> CLA0022_HC_CDR3

<400> 23

Gly Ser Pro Asp Asp Ala Phe His Ser
1               5

<210> 24
<211> 11
<212> PRT
<213> Artificial Sequence

<220>
<223> CLA0022_LC_CDR1

<400> 24

Gln Ala Ser Gln Ser Ile Gly Asn Tyr Leu Ala
1               5                   10

<210> 25
<211> 7
<212> PRT
<213> Artificial Sequence

<220>
<223> CLA0022_LC_CDR2

<400> 25

Tyr Ala Ser Asn Leu Ala Ser
1               5

<210> 26
<211> 11
<212> PRT
<213> Artificial Sequence

<220>
<223> CLA0022_LC_CDR3

<400> 26

Gln Cys Ala Tyr Tyr Asp Ser Val Tyr Val Thr
1               5                   10

<210> 27
<211> 6

<212> PRT
<213> Artificial Sequence

<220>
<223> CLA0028_HC_CDR1

<400> 27

Ser Ser Tyr Trp Ile Tyr
1 5

<210> 28
<211> 18
<212> PRT
<213> Artificial Sequence

<220>
<223> CLA0028_HC_CDR2

<400> 28

Cys Ile Tyr Ala Gly Ser Thr Ser Ser Thr Tyr Tyr Ala Ser Trp Ala
1 5 10 15

Lys Gly

<210> 29
<211> 9
<212> PRT
<213> Artificial Sequence

<220>
<223> CLA0028_HC_CDR3

<400> 29

Gly Gly Pro Asp Asp Ala Phe His Ser
1 5

<210> 30
<211> 11
<212> PRT
<213> Artificial Sequence

<220>
<223> CLA0028_LC_CDR1

<400> 30

Gln Ala Ser Gln Ser Ile Gly Asn Tyr Leu Ala
1 5 10

<210> 31
<211> 7
<212> PRT
<213> Artificial Sequence

<220>
<223> CLA0028_LC_CDR2

<400> 31

Tyr Ala Ser Asn Leu Ala Ser
1               5

<210> 32
<211> 11
<212> PRT
<213> Artificial Sequence

<220>
<223> CLA0028_LC_CDR3

<400> 32

Gln Cys Ala Tyr Tyr Glu Ser Ser Tyr Val Thr
1               5                   10

<210> 33
<211> 6
<212> PRT
<213> Artificial Sequence

<220>
<223> CLA0311_HC_CDR1

<400> 33

Ser Ser Tyr Trp Ile Cys
1               5

<210> 34
<211> 18
<212> PRT
<213> Artificial Sequence

<220>
<223> CLA0311_HC_CDR2

<400> 34

Cys Ile Tyr Thr Gly Ser Gly Gly Ala Thr His Tyr Ala Ser Trp Ala
1               5                   10                  15

Lys Gly

<210> 35
<211> 13
<212> PRT
<213> Artificial Sequence

<220>
<223> CLA0311_HC_CDR3

<400> 35

Ala Glu Ser Gly Tyr Tyr Ala Gly Phe Phe Phe Ala Pro
1 5 10

<210> 36
<211> 11
<212> PRT
<213> Artificial Sequence

<220>
<223> CLA0311_LC_CDR1

<400> 36

Gln Ala Ser Glu Ser Ile Tyr Ser Gly Leu Ala
1 5 10

<210> 37
<211> 7
<212> PRT
<213> Artificial Sequence

<220>
<223> CLA0311_LC_CDR2

<400> 37

Ser Ala Ser Thr Leu Ala Ser
1 5

<210> 38
<211> 13
<212> PRT
<213> Artificial Sequence

<220>
<223> CLA0311_LC_CDR3

<400> 38

Gln Ser Tyr Tyr Gly Gly Ser Val Thr Gly Tyr Asn Thr
1 5 10

<210> 39
<211> 6
<212> PRT
<213> Artificial Sequence

<220>
<223> CLA0334_HC_CDR1

<400> 39

Val Ser Tyr Trp Ile Cys
1 5

<210> 40

<211>  18
<212>  PRT
<213>  Artificial Sequence

<220>
<223>  CLA0334_HC_CDR2

<400>  40

Cys Ile Tyr Ala Gly Ser Thr Gly Ser Thr Trp Tyr Ala Asn Trp Ala
1               5                   10                  15

Lys Gly

<210>  41
<211>  9
<212>  PRT
<213>  Artificial Sequence

<220>
<223>  CLA0334_HC_CDR3

<400>  41

Asp Ser Gly Trp Asn Ala Phe Asp Leu
1               5

<210>  42
<211>  11
<212>  PRT
<213>  Artificial Sequence

<220>
<223>  CLA0334_LC_CDR1

<400>  42

Gln Ala Ser Gln Ser Ile Gly Ser Asn Leu Ala
1               5                   10

<210>  43
<211>  7
<212>  PRT
<213>  Artificial Sequence

<220>
<223>  CLA0334_LC_CDR2

<400>  43

Glu Ala Ser Gly Leu Ala Ser
1               5

<210>  44
<211>  14
<212>  PRT
<213>  Artificial Sequence

<220>
<223> CLA0334_LC_CDR3

<400> 44

Gln Ser Tyr Tyr Tyr Ser Pro Ser Val Ser Val His Tyr Ala
1 5 10

<210> 45
<211> 118
<212> PRT
<213> Artificial Sequence

<220>
<223> CLA0022_HC_VR

<400> 45

Gln Ser Leu Glu Glu Ser Gly Gly Asp Leu Val Lys Pro Gly Ala Ser
1 5 10 15

Leu Thr Leu Thr Cys Thr Ala Ser Gly Phe Ser Phe Ser Ser Ser Tyr
20 25 30

Trp Ile Tyr Trp Val Arg Gln Ala Pro Gly Lys Gly Leu Glu Trp Ile
35 40 45

Ala Cys Ile Tyr Ala Gly Val Asn Asp Ile Thr Tyr Tyr Ala Asn Trp
50 55 60

Ala Lys Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Lys Gly Ser Pro Asp Asp Ala Phe His Ser Trp Gly Pro Gly Thr
100 105 110

Leu Val Thr Val Ser Ser
115

<210> 46
<211> 109
<212> PRT
<213> Artificial Sequence

<220>
<223> CLA0022_LC_VR

<400> 46

Asp Val Val Met Thr Gln Thr Pro Ala Ser Val Ser Glu Pro Val Gly

```
1               5                   10                  15

Gly Thr Val Thr Ile Lys Cys Gln Ala Ser Gln Ser Ile Gly Asn Tyr
            20                  25                  30

Leu Ala Trp Tyr Gln Gln Lys Pro Gly Gln Pro Pro Lys Leu Leu Ile
        35                  40                  45

Tyr Tyr Ala Ser Asn Leu Ala Ser Gly Val Ser Ser Arg Phe Lys Gly
    50                  55                  60

Ser Arg Ser Gly Thr Glu Phe Thr Leu Thr Ile Asn Asp Leu Glu Cys
65                  70                  75                  80

Ala Asp Ala Ala Thr Tyr Tyr Cys Gln Cys Ala Tyr Tyr Asp Ser Val
                85                  90                  95

Tyr Val Thr Phe Gly Gly Gly Thr Glu Val Val Val Lys
            100                 105


<210>  47
<211>  118
<212>  PRT
<213>  Artificial Sequence

<220>
<223>  CLA0028_HC_VR

<400>  47

Gln Ser Leu Glu Glu Ser Gly Gly Asp Leu Val Lys Pro Gly Ala Ser
1               5                   10                  15

Leu Thr Leu Thr Cys Thr Ala Ser Gly Phe Ser Phe Ser Ser Ser Tyr
            20                  25                  30

Trp Ile Tyr Trp Val Arg Gln Ala Pro Gly Lys Gly Leu Glu Trp Ile
        35                  40                  45

Ala Cys Ile Tyr Ala Gly Ser Thr Ser Ser Thr Tyr Tyr Ala Ser Trp
    50                  55                  60

Ala Lys Gly Arg Phe Thr Ile Ser Lys Thr Ser Ser Thr Thr Val Thr
65                  70                  75                  80

Leu Gln Met Thr Ser Leu Thr Ala Ala Asp Thr Ala Thr Tyr Phe Cys
                85                  90                  95

Ala Lys Gly Gly Pro Asp Asp Ala Phe His Ser Trp Gly Pro Gly Thr
            100                 105                 110
```

Leu Val Thr Val Ser Ser
        115

<210>  48
<211>  109
<212>  PRT
<213>  Artificial Sequence

<220>
<223>  CLA0028_LC_VR

<400>  48

Asp Val Val Met Thr Gln Thr Pro Ala Ser Val Ser Glu Pro Val Gly
1               5                   10                  15

Gly Thr Val Thr Ile Lys Cys Gln Ala Ser Gln Ser Ile Gly Asn Tyr
            20                  25                  30

Leu Ala Trp Tyr Gln Gln Lys Pro Gly Gln Pro Pro Lys Leu Leu Ile
        35                  40                  45

Tyr Tyr Ala Ser Asn Leu Ala Ser Gly Val Ser Ser Arg Phe Lys Gly
    50                  55                  60

Ser Arg Ser Gly Thr Glu Phe Thr Leu Thr Ile Ser Asp Leu Glu Cys
65                  70                  75                  80

Ala Asp Ala Ala Thr Tyr Tyr Cys Gln Cys Ala Tyr Tyr Glu Ser Ser
                85                  90                  95

Tyr Val Thr Phe Gly Gly Gly Thr Glu Val Val Val Lys
            100                 105

<210>  49
<211>  122
<212>  PRT
<213>  Artificial Sequence

<220>
<223>  CLA0311_HC_VR

<400>  49

Gln Ser Leu Glu Glu Ser Gly Gly Gly Leu Val Gln Pro Glu Gly Ser
1               5                   10                  15

Leu Thr Leu Thr Cys Thr Ala Ser Gly Phe Ser Leu Ser Ser Ser Tyr
            20                  25                  30

Trp Ile Cys Trp Val Arg Gln Ala Pro Gly Lys Gly Leu Glu Trp Ile

35 40 45

Gly Cys Ile Tyr Thr Gly Ser Gly Gly Ala Thr His Tyr Ala Ser Trp
50 55 60

Ala Lys Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Ser Ala Glu Ser Gly Tyr Tyr Ala Gly Phe Phe Phe Ala Pro Trp
100 105 110

Gly Pro Gly Thr Leu Val Thr Val Ser Ser
115 120

<210> 50
<211> 111
<212> PRT
<213> Artificial Sequence

<220>
<223> CLA0311_LC_VR

<400> 50

Ala Phe Glu Leu Thr Gln Thr Pro Ser Ser Val Glu Ala Ala Val Gly
1 5 10 15

Gly Thr Val Thr Ile Lys Cys Gln Ala Ser Glu Ser Ile Tyr Ser Gly
20 25 30

Leu Ala Trp Tyr Lys Gln Lys Pro Gly Gln Pro Pro Lys Leu Leu Ile
35 40 45

Tyr Ser Ala Ser Thr Leu Ala Ser Gly Val Ser Ser Arg Phe Lys Gly
50 55 60

Ser Gly Ser Gly Thr Gln Phe Thr Leu Thr Ile Ser Asp Leu Glu Cys
65 70 75 80

Ala Asp Ala Ala Thr Tyr Tyr Cys Gln Ser Tyr Tyr Gly Gly Ser Val
85 90 95

Thr Gly Tyr Asn Thr Phe Gly Gly Gly Thr Glu Val Val Val Lys
100 105 110

<210> 51
<211> 119

<212> PRT
<213> Artificial Sequence

<220>
<223> CLA0334_HC_VR

<400> 51

Gln Glu Gln Leu Glu Glu Ser Gly Gly Asp Leu Val Lys Pro Glu Gly
1               5                   10                  15

Ser Leu Thr Leu Thr Cys Thr Ala Ser Gly Phe Ser Leu Ser Val Ser
            20                  25                  30

Tyr Trp Ile Cys Trp Val Arg Gln Ala Pro Gly Lys Gly Leu Glu Trp
        35                  40                  45

Ile Ala Cys Ile Tyr Ala Gly Ser Thr Gly Ser Thr Trp Tyr Ala Asn
    50                  55                  60

Trp Ala Lys Gly Arg Phe Thr Ile Ser Lys Thr Ser Ser Thr Thr Val
65                  70                  75                  80

Thr Leu Gln Met Thr Ser Leu Thr Ala Ala Asp Thr Ala Thr Tyr Phe
                85                  90                  95

Cys Ala Arg Asp Ser Gly Trp Asn Ala Phe Asp Leu Trp Gly Pro Gly
            100                 105                 110

Thr Leu Val Thr Val Ser Ser
        115

<210> 52
<211> 113
<212> PRT
<213> Artificial Sequence

<220>
<223> CLA0334_LC_VR

<400> 52

Ala Asp Val Val Met Thr Gln Thr Pro Ala Ser Val Ser Ala Ala Val
1               5                   10                  15

Gly Gly Thr Val Thr Ile Lys Cys Gln Ala Ser Gln Ser Ile Gly Ser
            20                  25                  30

Asn Leu Ala Trp Tyr Gln Gln Lys Ser Gly His Pro Pro Asn Leu Leu
        35                  40                  45

Ile Tyr Glu Ala Ser Gly Leu Ala Ser Gly Val Pro Leu Arg Phe Ser

50 55 60

Gly Ser Gly Ser Gly Thr Gln Phe Thr Leu Thr Ile Ser Asp Leu Glu
65 70 75 80

Cys Ala Asp Ala Ala Thr Tyr Tyr Cys Gln Ser Tyr Tyr Tyr Ser Pro
85 90 95

Ser Val Ser Val His Tyr Ala Phe Gly Gly Gly Thr Glu Val Val Val
100 105 110

Lys

<210> 53
<211> 446
<212> PRT
<213> Artificial Sequence

<220>
<223> CLA0022VH-F760mnP17

<400> 53

Gln Ser Leu Glu Glu Ser Gly Gly Asp Leu Val Lys Pro Gly Ala Ser
1 5 10 15

Leu Thr Leu Thr Cys Thr Ala Ser Gly Phe Ser Phe Ser Ser Ser Tyr
20 25 30

Trp Ile Tyr Trp Val Arg Gln Ala Pro Gly Lys Gly Leu Glu Trp Ile
35 40 45

Ala Cys Ile Tyr Ala Gly Val Asn Asp Ile Thr Tyr Tyr Ala Asn Trp
50 55 60

Ala Lys Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Lys Gly Ser Pro Asp Asp Ala Phe His Ser Trp Gly Pro Gly Thr
100 105 110

Leu Val Thr Val Ser Ser Ala Ser Thr Lys Gly Pro Ser Val Phe Pro
115 120 125

Leu Ala Pro Ser Ser Lys Ser Thr Ser Gly Gly Thr Ala Ala Leu Gly
130 135 140

```
Cys Leu Val Lys Asp Tyr Phe Pro Glu Pro Val Thr Val Ser Trp Asn
145                 150                 155                 160

Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Gln Thr Tyr Ile Cys Asn Val Asn His Lys Pro Ser
        195                 200                 205

Asn Thr Lys Val Asp Lys Lys Val Glu Pro Lys Ser Cys Asp Lys Thr
    210                 215                 220

His Thr Cys Pro Pro Cys Pro Ala Pro Glu Leu Arg Gly Gly Pro Lys
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser His Glu Asp Pro
            260                 265                 270

Glu Val Lys Phe Asn Trp Tyr Val Asp Gly Val Glu Val His Asn Ala
        275                 280                 285

Lys Thr Lys Pro Arg Glu Glu Gln Tyr Ala Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Ala Leu Pro Ala Pro Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Lys Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Tyr Leu Asp
```

385 390 395 400

Ser Asp Gly Ser Phe Phe Leu Tyr Ser Lys Leu Thr Val Asp Lys Ser
405 410 415

Arg Trp Gln Gln Gly Asn Val Phe Ser Cys Ser Val Met His Glu Ala
420 425 430

Leu His Asn His Tyr Thr Gln Lys Ser Leu Ser Leu Ser Pro
435 440 445

<210> 54
<211> 216
<212> PRT
<213> Artificial Sequence

<220>
<223> CLA0022VL-k0C

<400> 54

Asp Val Val Met Thr Gln Thr Pro Ala Ser Val Ser Glu Pro Val Gly
1 5 10 15

Gly Thr Val Thr Ile Lys Cys Gln Ala Ser Gln Ser Ile Gly Asn Tyr
20 25 30

Leu Ala Trp Tyr Gln Gln Lys Pro Gly Gln Pro Pro Lys Leu Leu Ile
35 40 45

Tyr Tyr Ala Ser Asn Leu Ala Ser Gly Val Ser Ser Arg Phe Lys Gly
50 55 60

Ser Arg Ser Gly Thr Glu Phe Thr Leu Thr Ile Asn Asp Leu Glu Cys
65 70 75 80

Ala Asp Ala Ala Thr Tyr Tyr Cys Gln Cys Ala Tyr Tyr Asp Ser Val
85 90 95

Tyr Val Thr Phe Gly Gly Gly Thr Glu Val Val Val Lys Arg Thr Val
100 105 110

Ala Ala Pro Ser Val Phe Ile Phe Pro Pro Ser Asp Glu Gln Leu Lys
115 120 125

Ser Gly Thr Ala Ser Val Val Cys Leu Leu Asn Asn Phe Tyr Pro Arg
130 135 140

Glu Ala Lys Val Gln Trp Lys Val Asp Asn Ala Leu Gln Ser Gly Asn
145 150 155 160

Ser Gln Glu Ser Val Thr Glu Gln Asp Ser Lys Asp Cys Thr Tyr Ser
165 170 175

Leu Ser Ser Thr Leu Thr Leu Ser Lys Ala Asp Tyr Glu Lys His Lys
180 185 190

Val Tyr Ala Cys Glu Val Thr His Gln Gly Leu Ser Ser Pro Val Thr
195 200 205

Lys Ser Phe Asn Arg Gly Glu Cys
210 215

<210> 55
<211> 446
<212> PRT
<213> Artificial Sequence

<220>
<223> CLA0028VH-F760mnP17

<400> 55

Gln Ser Leu Glu Glu Ser Gly Gly Asp Leu Val Lys Pro Gly Ala Ser
1 5 10 15

Leu Thr Leu Thr Cys Thr Ala Ser Gly Phe Ser Phe Ser Ser Ser Tyr
20 25 30

Trp Ile Tyr Trp Val Arg Gln Ala Pro Gly Lys Gly Leu Glu Trp Ile
35 40 45

Ala Cys Ile Tyr Ala Gly Ser Thr Ser Ser Thr Tyr Tyr Ala Ser Trp
50 55 60

Ala Lys Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Lys Gly Gly Pro Asp Asp Ala Phe His Ser Trp Gly Pro Gly Thr
100 105 110

Leu Val Thr Val Ser Ser Ala Ser Thr Lys Gly Pro Ser Val Phe Pro
115 120 125

Leu Ala Pro Ser Ser Lys Ser Thr Ser Gly Gly Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Gln Thr Tyr Ile Cys Asn Val Asn His Lys Pro Ser
195 200 205

Asn Thr Lys Val Asp Lys Lys Val Glu Pro Lys Ser Cys Asp Lys Thr
210 215 220

His Thr Cys Pro Pro Cys Pro Ala Pro Glu Leu Arg Gly Gly Pro Lys
225 230 235 240

Val Phe Leu Phe Pro Pro Lys Pro Lys Asp Thr Leu Met Ile Ser Arg
245 250 255

Thr Pro Glu Val Thr Cys Val Val Val Asp Val Ser His Glu Asp Pro
260 265 270

Glu Val Lys Phe Asn Trp Tyr Val Asp Gly Val Glu Val His Asn Ala
275 280 285

Lys Thr Lys Pro Arg Glu Glu Gln Tyr Ala Ser Thr Tyr Arg Val Val
290 295 300

Ser Val Leu Thr Val Leu His Gln Asp Trp Leu Asn Gly Lys Glu Tyr
305 310 315 320

Lys Cys Lys Val Ser Asn Lys Ala Leu Pro Ala Pro Ile Glu Lys Thr
325 330 335

Ile Ser Lys Ala Lys Gly Gln Pro Arg Glu Pro Gln Val Tyr Thr Leu
340 345 350

Pro Pro Ser Arg Lys Glu Met Thr Lys Asn Gln Val Ser Leu Thr Cys
355 360 365

Leu Val Lys Gly Phe Tyr Pro Ser Asp Ile Ala Val Glu Trp Glu Ser
370 375 380

Asn Gly Gln Pro Glu Asn Asn Tyr Lys Thr Thr Pro Pro Tyr Leu Asp
385 390 395 400

Ser Asp Gly Ser Phe Phe Leu Tyr Ser Lys Leu Thr Val Asp Lys Ser
405 410 415

Arg Trp Gln Gln Gly Asn Val Phe Ser Cys Ser Val Met His Glu Ala
420 425 430

Leu His Asn His Tyr Thr Gln Lys Ser Leu Ser Leu Ser Pro
435 440 445

<210> 56
<211> 216
<212> PRT
<213> Artificial Sequence

<220>
<223> CLA0028VL-k0C

<400> 56

Asp Val Val Met Thr Gln Thr Pro Ala Ser Val Ser Glu Pro Val Gly
1 5 10 15

Gly Thr Val Thr Ile Lys Cys Gln Ala Ser Gln Ser Ile Gly Asn Tyr
20 25 30

Leu Ala Trp Tyr Gln Gln Lys Pro Gly Gln Pro Pro Lys Leu Leu Ile
35 40 45

Tyr Tyr Ala Ser Asn Leu Ala Ser Gly Val Ser Ser Arg Phe Lys Gly
50 55 60

Ser Arg Ser Gly Thr Glu Phe Thr Leu Thr Ile Ser Asp Leu Glu Cys
65 70 75 80

Ala Asp Ala Ala Thr Tyr Tyr Cys Gln Cys Ala Tyr Tyr Glu Ser Ser
85 90 95

Tyr Val Thr Phe Gly Gly Gly Thr Glu Val Val Val Lys Arg Thr Val
100 105 110

Ala Ala Pro Ser Val Phe Ile Phe Pro Pro Ser Asp Glu Gln Leu Lys
115 120 125

Ser Gly Thr Ala Ser Val Val Cys Leu Leu Asn Asn Phe Tyr Pro Arg
130 135 140

Glu Ala Lys Val Gln Trp Lys Val Asp Asn Ala Leu Gln Ser Gly Asn
145 150 155 160

Ser Gln Glu Ser Val Thr Glu Gln Asp Ser Lys Asp Cys Thr Tyr Ser
165 170 175

Leu Ser Ser Thr Leu Thr Leu Ser Lys Ala Asp Tyr Glu Lys His Lys
180 185 190

Val Tyr Ala Cys Glu Val Thr His Gln Gly Leu Ser Ser Pro Val Thr
195 200 205

Lys Ser Phe Asn Arg Gly Glu Cys
210 215

<210> 57
<211> 450
<212> PRT
<213> Artificial Sequence

<220>
<223> CLA0311VH-F760mnP17

<400> 57

Gln Ser Leu Glu Glu Ser Gly Gly Gly Leu Val Gln Pro Glu Gly Ser
1 5 10 15

Leu Thr Leu Thr Cys Thr Ala Ser Gly Phe Ser Leu Ser Ser Ser Tyr
20 25 30

Trp Ile Cys Trp Val Arg Gln Ala Pro Gly Lys Gly Leu Glu Trp Ile
35 40 45

Gly Cys Ile Tyr Thr Gly Ser Gly Gly Ala Thr His Tyr Ala Ser Trp
50 55 60

Ala Lys Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Ser Ala Glu Ser Gly Tyr Tyr Ala Gly Phe Phe Phe Ala Pro Trp
100 105 110

Gly Pro Gly Thr Leu Val Thr Val Ser Ser Ala Ser Thr Lys Gly Pro
115 120 125

Ser Val Phe Pro Leu Ala Pro Ser Ser Lys Ser Thr Ser Gly Gly Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Gln Thr Tyr Ile Cys Asn Val Asn
195 200 205

His Lys Pro Ser Asn Thr Lys Val Asp Lys Lys Val Glu Pro Lys Ser
210 215 220

Cys Asp Lys Thr His Thr Cys Pro Pro Cys Pro Ala Pro Glu Leu Arg
225 230 235 240

Gly Gly Pro Lys Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

His Glu Asp Pro Glu Val Lys Phe Asn Trp Tyr Val Asp Gly Val Glu
275 280 285

Val His Asn Ala Lys Thr Lys Pro Arg Glu Glu Gln Tyr Ala Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Ala Leu Pro Ala Pro
325 330 335

Ile Glu Lys Thr Ile Ser Lys Ala Lys Gly Gln Pro Arg Glu Pro Gln
340 345 350

Val Tyr Thr Leu Pro Pro Ser Arg Lys Glu Met Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Tyr Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
435 440 445

Ser Pro
450

<210> 58
<211> 218
<212> PRT
<213> Artificial Sequence

<220>
<223> CLA0311VL-k0C

<400> 58

Ala Phe Glu Leu Thr Gln Thr Pro Ser Ser Val Glu Ala Ala Val Gly
1 5 10 15

Gly Thr Val Thr Ile Lys Cys Gln Ala Ser Glu Ser Ile Tyr Ser Gly
20 25 30

Leu Ala Trp Tyr Lys Gln Lys Pro Gly Gln Pro Pro Lys Leu Leu Ile
35 40 45

Tyr Ser Ala Ser Thr Leu Ala Ser Gly Val Ser Ser Arg Phe Lys Gly
50 55 60

Ser Gly Ser Gly Thr Gln Phe Thr Leu Thr Ile Ser Asp Leu Glu Cys
65 70 75 80

Ala Asp Ala Ala Thr Tyr Tyr Cys Gln Ser Tyr Tyr Gly Gly Ser Val
85 90 95

Thr Gly Tyr Asn Thr Phe Gly Gly Gly Thr Glu Val Val Val Lys Arg
100 105 110

Thr Val Ala Ala Pro Ser Val Phe Ile Phe Pro Pro Ser Asp Glu Gln
115 120 125

Leu Lys Ser Gly Thr Ala Ser Val Val Cys Leu Leu Asn Asn Phe Tyr
130 135 140

Pro Arg Glu Ala Lys Val Gln Trp Lys Val Asp Asn Ala Leu Gln Ser
145 150 155 160

Gly Asn Ser Gln Glu Ser Val Thr Glu Gln Asp Ser Lys Asp Cys Thr
165 170 175

Tyr Ser Leu Ser Ser Thr Leu Thr Leu Ser Lys Ala Asp Tyr Glu Lys
180 185 190

His Lys Val Tyr Ala Cys Glu Val Thr His Gln Gly Leu Ser Ser Pro
195 200 205

Val Thr Lys Ser Phe Asn Arg Gly Glu Cys
210 215

<210> 59
<211> 447
<212> PRT
<213> Artificial Sequence

<220>
<223> CLA0334VH-F760mnP17

<400> 59

Gln Glu Gln Leu Glu Glu Ser Gly Gly Asp Leu Val Lys Pro Glu Gly
1 5 10 15

Ser Leu Thr Leu Thr Cys Thr Ala Ser Gly Phe Ser Leu Ser Val Ser
20 25 30

Tyr Trp Ile Cys Trp Val Arg Gln Ala Pro Gly Lys Gly Leu Glu Trp
35 40 45

Ile Ala Cys Ile Tyr Ala Gly Ser Thr Gly Ser Thr Trp Tyr Ala Asn
50 55 60

Trp Ala Lys Gly Arg Phe Thr Ile Ser Lys Thr Ser Ser Thr Thr Val
65 70 75 80

Thr Leu Gln Met Thr Ser Leu Thr Ala Ala Asp Thr Ala Thr Tyr Phe
85 90 95

Cys Ala Arg Asp Ser Gly Trp Asn Ala Phe Asp Leu Trp Gly Pro Gly
100 105 110

Thr Leu Val Thr Val Ser Ser Ala Ser Thr Lys Gly Pro Ser Val Phe
115 120 125

Pro Leu Ala Pro Ser Ser Lys Ser Thr Ser Gly Gly Thr Ala Ala Leu

```
    130                 135                 140

Gly Cys Leu Val Lys Asp Tyr Phe Pro Glu Pro Val Thr Val Ser Trp
145                 150                 155                 160

Asn Ser Gly Ala Leu Thr Ser Gly Val His Thr Phe Pro Ala Val Leu
                165                 170                 175

Gln Ser Ser Gly Leu Tyr Ser Leu Ser Ser Val Val Thr Val Pro Ser
            180                 185                 190

Ser Ser Leu Gly Thr Gln Thr Tyr Ile Cys Asn Val Asn His Lys Pro
        195                 200                 205

Ser Asn Thr Lys Val Asp Lys Lys Val Glu Pro Lys Ser Cys Asp Lys
    210                 215                 220

Thr His Thr Cys Pro Pro Cys Pro Ala Pro Glu Leu Arg Gly Gly Pro
225                 230                 235                 240

Lys Val Phe Leu Phe Pro Pro Lys Pro Lys Asp Thr Leu Met Ile Ser
                245                 250                 255

Arg Thr Pro Glu Val Thr Cys Val Val Val Asp Val Ser His Glu Asp
            260                 265                 270

Pro Glu Val Lys Phe Asn Trp Tyr Val Asp Gly Val Glu Val His Asn
        275                 280                 285

Ala Lys Thr Lys Pro Arg Glu Glu Gln Tyr Ala Ser Thr Tyr Arg Val
    290                 295                 300

Val Ser Val Leu Thr Val Leu His Gln Asp Trp Leu Asn Gly Lys Glu
305                 310                 315                 320

Tyr Lys Cys Lys Val Ser Asn Lys Ala Leu Pro Ala Pro Ile Glu Lys
                325                 330                 335

Thr Ile Ser Lys Ala Lys Gly Gln Pro Arg Glu Pro Gln Val Tyr Thr
            340                 345                 350

Leu Pro Pro Ser Arg Lys Glu Met Thr Lys Asn Gln Val Ser Leu Thr
        355                 360                 365

Cys Leu Val Lys Gly Phe Tyr Pro Ser Asp Ile Ala Val Glu Trp Glu
    370                 375                 380
```

Ser Asn Gly Gln Pro Glu Asn Asn Tyr Lys Thr Thr Pro Pro Tyr Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Lys Ser Leu Ser Leu Ser Pro
435 440 445

<210> 60
<211> 220
<212> PRT
<213> Artificial Sequence

<220>
<223> CLA0334VL-k0C

<400> 60

Ala Asp Val Val Met Thr Gln Thr Pro Ala Ser Val Ser Ala Ala Val
1 5 10 15

Gly Gly Thr Val Thr Ile Lys Cys Gln Ala Ser Gln Ser Ile Gly Ser
20 25 30

Asn Leu Ala Trp Tyr Gln Gln Lys Ser Gly His Pro Pro Asn Leu Leu
35 40 45

Ile Tyr Glu Ala Ser Gly Leu Ala Ser Gly Val Pro Leu Arg Phe Ser
50 55 60

Gly Ser Gly Ser Gly Thr Gln Phe Thr Leu Thr Ile Ser Asp Leu Glu
65 70 75 80

Cys Ala Asp Ala Ala Thr Tyr Tyr Cys Gln Ser Tyr Tyr Tyr Ser Pro
85 90 95

Ser Val Ser Val His Tyr Ala Phe Gly Gly Gly Thr Glu Val Val Val
100 105 110

Lys Arg Thr Val Ala Ala Pro Ser Val Phe Ile Phe Pro Pro Ser Asp
115 120 125

Glu Gln Leu Lys Ser Gly Thr Ala Ser Val Val Cys Leu Leu Asn Asn
130 135 140

Phe Tyr Pro Arg Glu Ala Lys Val Gln Trp Lys Val Asp Asn Ala Leu

145 150 155 160

Gln Ser Gly Asn Ser Gln Glu Ser Val Thr Glu Gln Asp Ser Lys Asp
165 170 175

Cys Thr Tyr Ser Leu Ser Ser Thr Leu Thr Leu Ser Lys Ala Asp Tyr
180 185 190

Glu Lys His Lys Val Tyr Ala Cys Glu Val Thr His Gln Gly Leu Ser
195 200 205

Ser Pro Val Thr Lys Ser Phe Asn Arg Gly Glu Cys
210 215 220

<210> 61
<211> 461
<212> PRT
<213> Artificial Sequence

<220>
<223> CE115HAPG13-rabIgG

<400> 61

Gln Asp Gly Asn Glu Glu Met Gly Gly Gly Gly Ser Gly Gly Gly Ser
1 5 10 15

Gln Val Gln Leu Val Glu Ser Gly Gly Gly Val Val Gln Pro Gly Arg
20 25 30

Ser Leu Arg Leu Ser Cys Ala Ala Ser Gly Phe Thr Phe Ser Asn Ala
35 40 45

Trp Met His Trp Val Arg Gln Ala Pro Gly Lys Gly Leu Glu Trp Val
50 55 60

Ala Gln Ile Lys Ala Lys Ser Asn Asn Tyr Ala Thr Tyr Tyr Ala Glu
65 70 75 80

Ser Val Lys Gly Arg Phe Thr Ile Ser Arg Asp Asp Ser Lys Asn Ser
85 90 95

Leu Tyr Leu Gln Met Asn Ser Leu Lys Thr Glu Asp Thr Ala Val Tyr
100 105 110

Tyr Cys Arg Tyr Val Ala Tyr Gly Ala Tyr Tyr Gly Val Asp Ala Trp
115 120 125

Gly Gln Gly Thr Thr Val Thr Val Ser Ser Gly Gln Pro Lys Ala Pro
130 135 140

Ser Val Phe Pro Leu Ala Pro Cys Cys Gly Asp Thr Pro Ser Ser Thr
145 150 155 160

Val Thr Leu Gly Cys Leu Val Lys Gly Tyr Leu Pro Glu Pro Val Thr
165 170 175

Val Thr Trp Asn Ser Gly Thr Leu Thr Asn Gly Val Arg Thr Phe Pro
180 185 190

Ser Val Arg Gln Ser Ser Gly Leu Tyr Ser Leu Ser Ser Val Val Ser
195 200 205

Val Thr Ser Ser Ser Gln Pro Val Thr Cys Asn Val Ala His Pro Ala
210 215 220

Thr Asn Thr Lys Val Asp Lys Thr Val Ala Pro Ser Thr Cys Ser Lys
225 230 235 240

Pro Met Cys Pro Pro Pro Glu Leu Leu Gly Gly Pro Ser Val Phe Ile
245 250 255

Phe Pro Pro Lys Pro Lys Asp Thr Leu Met Ile Ser Arg Thr Pro Glu
260 265 270

Val Thr Cys Val Val Val Asp Val Ser Gln Asp Asp Pro Glu Val Gln
275 280 285

Phe Thr Trp Tyr Ile Asn Asn Glu Gln Val Arg Thr Ala Arg Pro Pro
290 295 300

Leu Arg Glu Gln Gln Phe Asn Ser Thr Ile Arg Val Val Ser Thr Leu
305 310 315 320

Pro Ile Ala His Gln Asp Trp Leu Arg Gly Lys Glu Phe Lys Cys Lys
325 330 335

Val His Asn Lys Ala Leu Pro Ala Pro Ile Glu Lys Thr Ile Ser Lys
340 345 350

Ala Arg Gly Gln Pro Leu Glu Pro Lys Val Tyr Thr Met Gly Pro Pro
355 360 365

Arg Glu Glu Leu Ser Ser Arg Ser Val Ser Leu Thr Cys Met Ile Asn
370 375 380

Gly Phe Tyr Pro Ser Asp Ile Ser Val Glu Trp Glu Lys Asn Gly Lys

385 390 395 400

Ala Glu Asp Asn Tyr Lys Thr Thr Pro Thr Val Leu Asp Ser Asp Gly
405 410 415

Ser Tyr Phe Leu Tyr Ser Lys Leu Ser Val Pro Thr Ser Glu Trp Gln
420 425 430

Arg Gly Asp Val Phe Thr Cys Ser Val Met His Glu Ala Leu His Asn
435 440 445

His Tyr Thr Gln Lys Ser Ile Ser Arg Ser Pro Gly Lys
450 455 460

<210> 62
<211> 461
<212> PRT
<213> Artificial Sequence

<220>
<223> CE115HAPG12-rabIgG

<400> 62

Gln Asp Gly Asn Glu Glu Met Gly Gly Gly Gly Ser Gly Gly Gly Ser
1 5 10 15

Gln Val Gln Leu Val Glu Ser Gly Gly Gly Val Val Gln Pro Gly Arg
20 25 30

Ser Leu Arg Leu Ser Cys Ala Ala Ser Gly Phe Thr Phe Ser Asn Ala
35 40 45

Trp Met His Trp Val Arg Gln Ala Pro Gly Lys Gly Leu Glu Trp Val
50 55 60

Ala Gln Ile Lys Ala Lys Ser Asn Asn Tyr Ala Thr Tyr Tyr Ala Glu
65 70 75 80

Ser Val Lys Gly Arg Phe Thr Ile Ser Arg Asp Asp Ser Lys Asn Ser
85 90 95

Leu Tyr Leu Gln Met Asn Ser Leu Lys Thr Glu Asp Thr Ala Val Tyr
100 105 110

Tyr Cys Arg Tyr Ala His Tyr Gly Ala Tyr Tyr Gly Val Asp Ala Trp
115 120 125

Gly Gln Gly Thr Thr Val Thr Val Ser Ser Gly Gln Pro Lys Ala Pro
130 135 140

Ser Val Phe Pro Leu Ala Pro Cys Cys Gly Asp Thr Pro Ser Ser Thr
145 150 155 160

Val Thr Leu Gly Cys Leu Val Lys Gly Tyr Leu Pro Glu Pro Val Thr
165 170 175

Val Thr Trp Asn Ser Gly Thr Leu Thr Asn Gly Val Arg Thr Phe Pro
180 185 190

Ser Val Arg Gln Ser Ser Gly Leu Tyr Ser Leu Ser Ser Val Val Ser
195 200 205

Val Thr Ser Ser Ser Gln Pro Val Thr Cys Asn Val Ala His Pro Ala
210 215 220

Thr Asn Thr Lys Val Asp Lys Thr Val Ala Pro Ser Thr Cys Ser Lys
225 230 235 240

Pro Met Cys Pro Pro Pro Glu Leu Leu Gly Gly Pro Ser Val Phe Ile
245 250 255

Phe Pro Pro Lys Pro Lys Asp Thr Leu Met Ile Ser Arg Thr Pro Glu
260 265 270

Val Thr Cys Val Val Val Asp Val Ser Gln Asp Asp Pro Glu Val Gln
275 280 285

Phe Thr Trp Tyr Ile Asn Asn Glu Gln Val Arg Thr Ala Arg Pro Pro
290 295 300

Leu Arg Glu Gln Gln Phe Asn Ser Thr Ile Arg Val Val Ser Thr Leu
305 310 315 320

Pro Ile Ala His Gln Asp Trp Leu Arg Gly Lys Glu Phe Lys Cys Lys
325 330 335

Val His Asn Lys Ala Leu Pro Ala Pro Ile Glu Lys Thr Ile Ser Lys
340 345 350

Ala Arg Gly Gln Pro Leu Glu Pro Lys Val Tyr Thr Met Gly Pro Pro
355 360 365

Arg Glu Glu Leu Ser Ser Arg Ser Val Ser Leu Thr Cys Met Ile Asn
370 375 380

Gly Phe Tyr Pro Ser Asp Ile Ser Val Glu Trp Glu Lys Asn Gly Lys

385 390 395 400

Ala Glu Asp Asn Tyr Lys Thr Thr Pro Thr Val Leu Asp Ser Asp Gly
405 410 415

Ser Tyr Phe Leu Tyr Ser Lys Leu Ser Val Pro Thr Ser Glu Trp Gln
420 425 430

Arg Gly Asp Val Phe Thr Cys Ser Val Met His Glu Ala Leu His Asn
435 440 445

His Tyr Thr Gln Lys Ser Ile Ser Arg Ser Pro Gly Lys
450 455 460

<210> 63
<211> 450
<212> PRT
<213> Artificial Sequence

<220>
<223> CE115HA000-F760mnP17

<400> 63

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Val His Tyr Gly Ala Tyr Tyr Gly Val Asp Ala Trp
100 105 110

Gly Gln Gly Thr Thr Val Thr Val Ser Ser Ala Ser Thr Lys Gly Pro
115 120 125

Ser Val Phe Pro Leu Ala Pro Ser Ser Lys Ser Thr Ser Gly Gly Thr
130 135 140

```
Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Leu Arg
225                 230                 235                 240

Gly Gly Pro Lys Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Ala Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Lys Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
```

385 390 395 400

Pro Tyr Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
435 440 445

Ser Pro
450

<210> 64
<211> 450
<212> PRT
<213> Artificial Sequence

<220>
<223> CE115HA056-F760mnP17

<400> 64

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Ala His Tyr Gly Ala Tyr Tyr Gly Val Asp Ala Trp
100 105 110

Gly Gln Gly Thr Thr Val Thr Val Ser Ser Ala Ser Thr Lys Gly Pro
115 120 125

Ser Val Phe Pro Leu Ala Pro Ser Ser Lys Ser Thr Ser Gly Gly Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Gln Thr Tyr Ile Cys Asn Val Asn
195 200 205

His Lys Pro Ser Asn Thr Lys Val Asp Lys Lys Val Glu Pro Lys Ser
210 215 220

Cys Asp Lys Thr His Thr Cys Pro Pro Cys Pro Ala Pro Glu Leu Arg
225 230 235 240

Gly Gly Pro Lys Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

His Glu Asp Pro Glu Val Lys Phe Asn Trp Tyr Val Asp Gly Val Glu
275 280 285

Val His Asn Ala Lys Thr Lys Pro Arg Glu Glu Gln Tyr Ala Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Ala Leu Pro Ala Pro
325 330 335

Ile Glu Lys Thr Ile Ser Lys Ala Lys Gly Gln Pro Arg Glu Pro Gln
340 345 350

Val Tyr Thr Leu Pro Pro Ser Arg Lys Glu Met Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro

385 390 395 400

Pro Tyr Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
435 440 445

Ser Pro
450

<210> 65
<211> 444
<212> PRT
<213> Artificial Sequence

<220>
<223> IC17Hdk-F760mnN17

<400> 65

Gln Val Gln Leu Gln Gln Ser Gly Pro Gln Leu Val Arg Pro Gly Ala
1 5 10 15

Ser Val Lys Ile Ser Cys Lys Ala Ser Gly Tyr Ser Phe Thr Ser Tyr
20 25 30

Trp Met His Trp Val Asn Gln Arg Pro Gly Gln Gly Leu Glu Trp Ile
35 40 45

Gly Met Ile Asp Pro Ser Tyr Ser Glu Thr Arg Leu Asn Gln Lys Phe
50 55 60

Lys Asp Lys Ala Thr Leu Thr Val Asp Lys Ser Ser Ser Thr Ala Tyr
65 70 75 80

Met Gln Leu Ser Ser Pro Thr Ser Glu Asp Ser Ala Val Tyr Tyr Cys
85 90 95

Ala Leu Tyr Gly Asn Tyr Phe Asp Tyr Trp Gly Gln Gly Thr Thr Leu
100 105 110

Thr Val Ser Ser Ala Ser Thr Lys Gly Pro Ser Val Phe Pro Leu Ala
115 120 125

Pro Ser Ser Lys Ser Thr Ser Gly Gly Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Lys Thr His Thr
210 215 220

Cys Pro Pro Cys Pro Ala Pro Glu Leu Arg Gly Gly Pro Lys Val Phe
225 230 235 240

Leu Phe Pro Pro Lys Pro Lys Asp Thr Leu Met Ile Ser Arg Thr Pro
245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Ala Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Pro Ala Pro Ile Glu Lys Thr Ile Ser
325 330 335

Lys Ala Lys Gly Gln Pro Arg Glu Pro Gln Val Tyr Thr Leu Pro Pro
340 345 350

Ser Arg Glu Glu Met Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Tyr Leu Asp Ser Asp

385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Glu Ser Leu Ser Leu Ser Pro
435 440

<210> 66
<211> 214
<212> PRT
<213> Artificial Sequence

<220>
<223> IC17L-k0

<400> 66

Asp Ile Gln Met Thr Gln Ser Ser Ser Ser Phe Ser Val Ser Leu Gly
1 5 10 15

Asp Arg Val Thr Ile Thr Cys Lys Ala Ser Glu Asp Ile Tyr Asn Arg
20 25 30

Leu Ala Trp Tyr Gln Gln Lys Pro Gly Asn Ala Pro Arg Leu Leu Ile
35 40 45

Ser Gly Ala Thr Ser Leu Glu Thr Gly Val Pro Ser Arg Phe Ser Gly
50 55 60

Ser Gly Ser Gly Lys Asp Tyr Thr Leu Ser Ile Thr Ser Leu Gln Thr
65 70 75 80

Glu Asp Val Ala Thr Tyr Tyr Cys Gln Gln Tyr Trp Ser Thr Pro Tyr
85 90 95

Thr Phe Gly Gly Gly Thr Lys Leu Glu Val Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 67
<211> 7
<212> PRT
<213> Artificial Sequence

<220>
<223> TEV protease site

<400> 67

Glu Asn Leu Tyr Phe Gln Gly
1 5

<210> 68
<211> 15
<212> PRT
<213> Artificial Sequence

<220>
<223> Avi tag

<400> 68

Gly Leu Asn Asp Ile Phe Glu Ala Gln Lys Ile Glu Trp His Glu
1 5 10 15

<210> 69
<211> 394
<212> PRT
<213> Artificial Sequence

<220>
<223> Fc-hCD3e

<400> 69

Met Gln Ser Gly Thr His Trp Arg Val Leu Gly Leu Cys Leu Leu Ser
1 5 10 15

Val Gly Val Trp Gly Gln Asp Gly Asn Glu Glu Met Gly Gly Ile Thr
20 25 30

```
Gln Thr Pro Tyr Lys Val Ser Ile Ser Gly Thr Thr Val Ile Leu Thr
        35                  40                  45

Cys Pro Gln Tyr Pro Gly Ser Glu Ile Leu Trp Gln His Asn Asp Lys
    50                  55                  60

Asn Ile Gly Gly Asp Glu Asp Asp Lys Asn Ile Gly Ser Asp Glu Asp
65                  70                  75                  80

His Leu Ser Leu Lys Glu Phe Ser Glu Leu Glu Gln Ser Gly Tyr Tyr
                85                  90                  95

Val Cys Tyr Pro Arg Gly Ser Lys Pro Glu Asp Ala Asn Phe Tyr Leu
            100                 105                 110

Tyr Leu Arg Ala Arg Val Cys Glu Asn Cys Met Glu Met Asp Asp Ile
        115                 120                 125

Glu Gly Arg Met Asp Pro Lys Ser Cys Asp Lys Thr His Thr Cys Pro
    130                 135                 140

Pro Cys Pro Ala Pro Glu Leu Leu Gly Gly Pro Ser Val Phe Leu Phe
145                 150                 155                 160

Pro Pro Lys Pro Lys Asp Thr Leu Met Ile Ser Arg Thr Pro Glu Val
                165                 170                 175

Thr Cys Val Val Val Asp Val Ser His Glu Asp Pro Glu Val Lys Phe
            180                 185                 190

Asn Trp Tyr Val Asp Gly Val Glu Val His Asn Ala Lys Thr Lys Pro
        195                 200                 205

Arg Glu Glu Gln Tyr Asn Ser Thr Tyr Arg Val Val Ser Val Leu Thr
    210                 215                 220

Val Leu His Gln Asp Trp Leu Asn Gly Lys Glu Tyr Lys Cys Lys Val
225                 230                 235                 240

Ser Asn Lys Ala Leu Pro Ala Pro Ile Glu Lys Thr Ile Ser Lys Ala
                245                 250                 255

Lys Gly Gln Pro Arg Glu Pro Gln Val Cys Thr Leu Pro Pro Ser Arg
            260                 265                 270

Asp Glu Leu Thr Lys Asn Gln Val Ser Leu Trp Cys Leu Val Lys Gly
        275                 280                 285
```

Phe Tyr Pro Ser Asp Ile Ala Val Glu Trp Glu Ser Asn Gly Gln Pro
    290                 295                 300

Glu Asn Asn Tyr Lys Thr Thr Pro Pro Val Leu Asp Ser Asp Gly Ser
305                 310                 315                 320

Phe Phe Leu Tyr Ser Lys Leu Thr Val Asp Lys Ser Arg Trp Gln Gln
                325                 330                 335

Gly Asn Val Phe Ser Cys Ser Val Met His Glu Ala Leu His Asn His
            340                 345                 350

Tyr Thr Gln Lys Ser Leu Ser Leu Ser Pro Gly Gly Ser Gly Gly Ser
        355                 360                 365

Gly Glu Asn Leu Tyr Phe Gln Gly Ser Gly Gly Gly Leu Asn Asp Ile
    370                 375                 380

Phe Glu Ala Gln Lys Ile Glu Trp His Glu
385                 390

<210> 70
<211> 8
<212> PRT
<213> Artificial Sequence

<220>
<223> Flag tag

<400> 70

Asp Tyr Lys Asp Asp Asp Asp Lys
1               5

<210> 71
<211> 344
<212> PRT
<213> Artificial Sequence

<220>
<223> Fc-hCD3d

<400> 71

Met Glu His Ser Thr Phe Leu Ser Gly Leu Val Leu Ala Thr Leu Leu
1               5                   10                  15

Ser Gln Val Ser Pro Phe Lys Ile Pro Ile Glu Glu Leu Glu Asp Arg
            20                  25                  30

Val Phe Val Asn Cys Asn Thr Ser Ile Thr Trp Val Glu Gly Thr Val

35 40 45

Gly Thr Leu Leu Ser Asp Ile Thr Arg Leu Asp Leu Gly Lys Arg Ile
50 55 60

Leu Asp Pro Arg Gly Ile Tyr Arg Cys Asn Gly Thr Asp Ile Tyr Lys
65 70 75 80

Asp Lys Glu Ser Thr Val Gln Val His Tyr Arg Met Cys Gln Ser Cys
85 90 95

Val Glu Leu Asp Asp Ile Glu Gly Arg Met Asp Pro Lys Ser Cys Asp
100 105 110

Lys Thr His Thr Cys Pro Pro Cys Pro Ala Pro Glu Leu Leu Gly Gly
115 120 125

Pro Ser Val Phe Leu Phe Pro Pro Lys Pro Lys Asp Thr Leu Met Ile
130 135 140

Ser Arg Thr Pro Glu Val Thr Cys Val Val Val Asp Val Ser His Glu
145 150 155 160

Asp Pro Glu Val Lys Phe Asn Trp Tyr Val Asp Gly Val Glu Val His
165 170 175

Asn Ala Lys Thr Lys Pro Arg Glu Glu Gln Tyr Asn Ser Thr Tyr Arg
180 185 190

Val Val Ser Val Leu Thr Val Leu His Gln Asp Trp Leu Asn Gly Lys
195 200 205

Glu Tyr Lys Cys Lys Val Ser Asn Lys Ala Leu Pro Ala Pro Ile Glu
210 215 220

Lys Thr Ile Ser Lys Ala Lys Gly Gln Pro Arg Glu Pro Gln Val Tyr
225 230 235 240

Thr Leu Pro Pro Ser Arg Cys Glu Leu Thr Lys Asn Gln Val Ser Leu
245 250 255

Ser Cys Ala Val Lys Gly Phe Tyr Pro Ser Asp Ile Ala Val Glu Trp
260 265 270

Glu Ser Asn Gly Gln Pro Glu Asn Asn Tyr Lys Thr Thr Pro Pro Val
275 280 285

Leu Asp Ser Asp Gly Ser Phe Phe Leu Val Ser Lys Leu Thr Val Asp
290 295 300

Lys Ser Arg Trp Gln Gln Gly Asn Val Phe Ser Cys Ser Val Met His
305 310 315 320

Glu Ala Leu His Asn His Tyr Thr Gln Lys Ser Leu Ser Leu Ser Pro
325 330 335

Asp Tyr Lys Asp Asp Asp Asp Lys
340

<210> 72
<211> 347
<212> PRT
<213> Escherichia coli

<400> 72

Met Val Leu Ala Ser Ser Thr Thr Ser Ile His Thr Met Leu Leu Leu
1 5 10 15

Leu Leu Met Leu Ala Gln Pro Ala Met Ala Met Lys Asp Asn Thr Val
20 25 30

Pro Leu Lys Leu Ile Ala Leu Leu Ala Asn Gly Glu Phe His Ser Gly
35 40 45

Glu Gln Leu Gly Glu Thr Leu Gly Met Ser Arg Ala Ala Ile Asn Lys
50 55 60

His Ile Gln Thr Leu Arg Asp Trp Gly Val Asp Val Phe Thr Val Pro
65 70 75 80

Gly Lys Gly Tyr Ser Leu Pro Glu Pro Ile Gln Leu Leu Asn Ala Lys
85 90 95

Gln Ile Leu Gly Gln Leu Asp Gly Gly Ser Val Ala Val Leu Pro Val
100 105 110

Ile Asp Ser Thr Asn Gln Tyr Leu Leu Asp Arg Ile Gly Glu Leu Lys
115 120 125

Ser Gly Asp Ala Cys Ile Ala Glu Tyr Gln Gln Ala Gly Arg Gly Arg
130 135 140

Arg Gly Arg Lys Trp Phe Ser Pro Phe Gly Ala Asn Leu Tyr Leu Ser
145 150 155 160

Met Phe Trp Arg Leu Glu Gln Gly Pro Ala Ala Ala Ile Gly Leu Ser
165 170 175

Leu Val Ile Gly Ile Val Met Ala Glu Val Leu Arg Lys Leu Gly Ala
180 185 190

Asp Lys Val Arg Val Lys Trp Pro Asn Asp Leu Tyr Leu Gln Asp Arg
195 200 205

Lys Leu Ala Gly Ile Leu Val Glu Leu Thr Gly Lys Thr Gly Asp Ala
210 215 220

Ala Gln Ile Val Ile Gly Ala Gly Ile Asn Met Ala Met Arg Arg Val
225 230 235 240

Glu Glu Ser Val Val Asn Gln Gly Trp Ile Thr Leu Gln Glu Ala Gly
245 250 255

Ile Asn Leu Asp Arg Asn Thr Leu Ala Ala Met Leu Ile Arg Glu Leu
260 265 270

Arg Ala Ala Leu Glu Leu Phe Glu Gln Glu Gly Leu Ala Pro Tyr Leu
275 280 285

Ser Arg Trp Glu Lys Leu Asp Asn Phe Ile Asn Arg Pro Val Lys Leu
290 295 300

Ile Ile Gly Asp Lys Glu Ile Phe Gly Ile Ser Arg Gly Ile Asp Lys
305 310 315 320

Gln Gly Ala Leu Leu Leu Glu Gln Asp Gly Ile Ile Lys Pro Trp Met
325 330 335

Gly Gly Glu Ile Ser Leu Arg Ser Ala Glu Lys
340 345

<210> 73
<211> 458
<212> PRT
<213> Artificial Sequence

<220>
<223> CE115HA000-pE22Hh

<400> 73

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala

```
                20                  25                  30

Trp Met His Trp Val Arg Gln Ala Pro Gly Lys Gly Leu Glu Trp Val
        35                  40                  45

Ala Gln Ile Lys Ala Lys Ser Asn Asn Tyr Ala Thr Tyr Tyr Ala Glu
    50                  55                  60

Ser Val Lys Gly Arg Phe Thr Ile Ser Arg Asp Asp Ser Lys Asn Ser
65                  70                  75                  80

Leu Tyr Leu Gln Met Asn Ser Leu Lys Thr Glu Asp Thr Ala Val Tyr
                85                  90                  95

Tyr Cys Arg Tyr Val His Tyr Gly Ala Tyr Tyr Gly Val Asp Ala Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Ser Ser Lys Ser Thr Ser Gly Gly Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Gln Thr Tyr Ile Cys Asn Val Asn
        195                 200                 205

His Lys Pro Ser Asn Thr Lys Val Asp Lys Lys Val Glu Pro Lys Ser
    210                 215                 220

Cys Asp Lys Thr His Thr Cys Pro Pro Cys Pro Ala Pro Glu Ala Ala
225                 230                 235                 240

Gly Gly Pro Ser Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270
```

```
His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Ala Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Cys Glu Leu Thr Lys Asn Gln Val
        355                 360                 365

Ser Leu Ser Cys Ala Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Val Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Asp Tyr Lys Asp Asp Asp Asp Lys
    450                 455


<210>  74
<211>  232
<212>  PRT
<213>  Artificial Sequence

<220>
<223>  Kn010

<400>  74

Glu Pro Lys Ser Ser Asp Lys Thr His Thr Cys Pro Pro Cys Pro Ala
1               5                   10                  15

Pro Glu Leu Leu Gly Gly Pro Ser Val Phe Leu Phe Pro Pro Lys Pro
```

```
            20                  25                  30

Lys Asp Thr Leu Met Ile Ser Arg Thr Pro Glu Val Thr Cys Val Val
        35                  40                  45

Val Asp Val Ser His Glu Asp Pro Glu Val Lys Phe Asn Trp Tyr Val
    50                  55                  60

Asp Gly Val Glu Val His Asn Ala Lys Thr Lys Pro Arg Glu Glu Gln
65                  70                  75                  80

Tyr Asn Ser Thr Tyr Arg Val Val Ser Val Leu Thr Val Leu His Gln
                85                  90                  95

Asp Trp Leu Asn Gly Lys Glu Tyr Lys Cys Lys Val Ser Asn Lys Ala
            100                 105                 110

Leu Pro Ala Pro Ile Glu Lys Thr Ile Ser Lys Ala Lys Gly Gln Pro
        115                 120                 125

Arg Glu Pro Gln Val Cys Thr Leu Pro Pro Ser Arg Asp Glu Leu Thr
    130                 135                 140

Lys Asn Gln Val Ser Leu Trp Cys Leu Val Lys Gly Phe Tyr Pro Ser
145                 150                 155                 160

Asp Ile Ala Val Glu Trp Glu Ser Asn Gly Gln Pro Glu Asn Asn Tyr
                165                 170                 175

Lys Thr Thr Pro Pro Val Leu Asp Ser Asp Gly Ser Phe Phe Leu Tyr
            180                 185                 190

Ser Lys Leu Thr Val Asp Lys Ser Arg Trp Gln Gln Gly Asn Val Phe
        195                 200                 205

Ser Cys Ser Val Met His Glu Ala Leu His Asn His Tyr Thr Gln Lys
    210                 215                 220

Ser Leu Ser Leu Ser Pro Gly Lys
225                 230

<210>  75
<211>  458
<212>  PRT
<213>  Artificial Sequence

<220>
<223>  CE115HA056-pE22Hh
```

<400> 75

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Ala His Tyr Gly Ala Tyr Tyr Gly Val Asp Ala Trp
100 105 110

Gly Gln Gly Thr Thr Val Thr Val Ser Ser Ala Ser Thr Lys Gly Pro
115 120 125

Ser Val Phe Pro Leu Ala Pro Ser Ser Lys Ser Thr Ser Gly Gly Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Gln Thr Tyr Ile Cys Asn Val Asn
195 200 205

His Lys Pro Ser Asn Thr Lys Val Asp Lys Lys Val Glu Pro Lys Ser
210 215 220

Cys Asp Lys Thr His Thr Cys Pro Pro Cys Pro Ala Pro Glu Ala Ala
225 230 235 240

Gly Gly Pro Ser Val Phe Leu Phe Pro Pro Lys Pro Lys Asp Thr Leu

```
                245                 250                 255

Met Ile Ser Arg Thr Pro Glu Val Thr Cys Val Val Val Asp Val Ser
            260                 265                 270

His Glu Asp Pro Glu Val Lys Phe Asn Trp Tyr Val Asp Gly Val Glu
        275                 280                 285

Val His Asn Ala Lys Thr Lys Pro Arg Glu Glu Gln Tyr Ala Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Ala Leu Pro Ala Pro
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Cys Glu Leu Thr Lys Asn Gln Val
        355                 360                 365

Ser Leu Ser Cys Ala Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Val Leu Asp Ser Asp Gly Ser Phe Phe Leu Val Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Asp Tyr Lys Asp Asp Asp Asp Lys
    450                 455
```

<210> 76
<211> 458
<212> PRT
<213> Artificial Sequence

<220>
<223> CE115HA146-pE22Hh

<400> 76

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Val Ala Tyr Gly Ala Tyr Tyr Gly Val Asp Ala Trp
100 105 110

Gly Gln Gly Thr Thr Val Thr Val Ser Ser Ala Ser Thr Lys Gly Pro
115 120 125

Ser Val Phe Pro Leu Ala Pro Ser Ser Lys Ser Thr Ser Gly Gly Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Gln Thr Tyr Ile Cys Asn Val Asn
195 200 205

His Lys Pro Ser Asn Thr Lys Val Asp Lys Lys Val Glu Pro Lys Ser
210 215 220

Cys Asp Lys Thr His Thr Cys Pro Pro Cys Pro Ala Pro Glu Ala Ala
225 230 235 240

Gly Gly Pro Ser Val Phe Leu Phe Pro Pro Lys Pro Lys Asp Thr Leu

245 250 255

Met Ile Ser Arg Thr Pro Glu Val Thr Cys Val Val Val Asp Val Ser
260 265 270

His Glu Asp Pro Glu Val Lys Phe Asn Trp Tyr Val Asp Gly Val Glu
275 280 285

Val His Asn Ala Lys Thr Lys Pro Arg Glu Glu Gln Tyr Ala Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Ala Leu Pro Ala Pro
325 330 335

Ile Glu Lys Thr Ile Ser Lys Ala Lys Gly Gln Pro Arg Glu Pro Gln
340 345 350

Val Tyr Thr Leu Pro Pro Ser Arg Cys Glu Leu Thr Lys Asn Gln Val
355 360 365

Ser Leu Ser Cys Ala Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Val Leu Asp Ser Asp Gly Ser Phe Phe Leu Val Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
435 440 445

Ser Pro Asp Tyr Lys Asp Asp Asp Asp Lys
450 455

<210> 77
<211> 452
<212> PRT
<213> Artificial Sequence

<220>
<223> IC17Hdk-pE22Hh

<400> 77

Gln Val Gln Leu Gln Gln Ser Gly Pro Gln Leu Val Arg Pro Gly Ala
1 5 10 15

Ser Val Lys Ile Ser Cys Lys Ala Ser Gly Tyr Ser Phe Thr Ser Tyr
20 25 30

Trp Met His Trp Val Asn Gln Arg Pro Gly Gln Gly Leu Glu Trp Ile
35 40 45

Gly Met Ile Asp Pro Ser Tyr Ser Glu Thr Arg Leu Asn Gln Lys Phe
50 55 60

Lys Asp Lys Ala Thr Leu Thr Val Asp Lys Ser Ser Ser Thr Ala Tyr
65 70 75 80

Met Gln Leu Ser Ser Pro Thr Ser Glu Asp Ser Ala Val Tyr Tyr Cys
85 90 95

Ala Leu Tyr Gly Asn Tyr Phe Asp Tyr Trp Gly Gln Gly Thr Thr Leu
100 105 110

Thr Val Ser Ser Ala Ser Thr Lys Gly Pro Ser Val Phe Pro Leu Ala
115 120 125

Pro Ser Ser Lys Ser Thr Ser Gly Gly Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Lys Thr His Thr
210 215 220

Cys Pro Pro Cys Pro Ala Pro Glu Ala Ala Gly Gly Pro Ser Val Phe
225 230 235 240

Leu Phe Pro Pro Lys Pro Lys Asp Thr Leu Met Ile Ser Arg Thr Pro

```
                245                 250                 255

Glu Val Thr Cys Val Val Val Asp Val Ser His Glu Asp Pro Glu Val
            260                 265                 270

Lys Phe Asn Trp Tyr Val Asp Gly Val Glu Val His Asn Ala Lys Thr
        275                 280                 285

Lys Pro Arg Glu Glu Gln Tyr Ala Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Ala Leu Pro Ala Pro Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Cys Glu Leu Thr Lys Asn Gln Val Ser Leu Ser Cys Ala Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Val Leu Asp Ser Asp
385                 390                 395                 400

Gly Ser Phe Phe Leu Val Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Asp Tyr Lys Asp
        435                 440                 445

Asp Asp Asp Lys
    450


<210>  78
<211>  444
<212>  PRT
<213>  Artificial Sequence

<220>
<223>  EGLVH-F760mnN17
```

<400> 78

Gln Asp Gln Leu Val Gln Ser Gly Ala Glu Val Lys Lys Pro Gly Ala
1 5 10 15

Ser Val Lys Val Ser Cys Lys Ala Ser Gly Phe Asn Ile Lys Asp Thr
20 25 30

Tyr Ile Gln Trp Val Arg Gln Ala Pro Gly Gln Gly Leu Glu Trp Met
35 40 45

Gly Arg Ile Asp Pro Leu Arg Lys Gln Thr Lys Tyr Arg Glu Lys Phe
50 55 60

Glu Gly Arg Val Thr Ile Thr Ala Asp Thr Ser Thr Asn Thr Ala Tyr
65 70 75 80

Met Glu Leu Ser Ser Leu Arg Ser Glu Asp Thr Ala Val Tyr Tyr Cys
85 90 95

Val Arg Ser Gly Arg Glu Phe Asp Tyr Trp Gly Gln Gly Thr Leu Val
100 105 110

Thr Val Ser Ser Ala Ser Thr Lys Gly Pro Ser Val Phe Pro Leu Ala
115 120 125

Pro Ser Ser Lys Ser Thr Ser Gly Gly Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Lys Thr His Thr
210 215 220

Cys Pro Pro Cys Pro Ala Pro Glu Leu Arg Gly Gly Pro Lys Val Phe
225 230 235 240

Leu Phe Pro Pro Lys Pro Lys Asp Thr Leu Met Ile Ser Arg Thr Pro

245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Ala Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Pro Ala Pro Ile Glu Lys Thr Ile Ser
325 330 335

Lys Ala Lys Gly Gln Pro Arg Glu Pro Gln Val Tyr Thr Leu Pro Pro
340 345 350

Ser Arg Glu Glu Met Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Tyr Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Glu Ser Leu Ser Leu Ser Pro
435 440

<210> 79
<211> 213
<212> PRT
<213> Artificial Sequence

<220>
<223> EGLVL-KT0

<400> 79

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Ile His Lys Tyr
20 25 30

Ile Ala Trp Tyr Gln Gln Lys Pro Gly Gln Ala Pro Arg Leu Leu Ile
35 40 45

Gln Tyr Thr Ser Thr Leu Gln Pro Gly Val Pro Ser Arg Phe Ser Gly
50 55 60

Ser Gly Ser Gly Thr Asp Tyr Thr Phe Thr Ile Ser Ser Leu Gln Pro
65 70 75 80

Glu Asp Ile Ala Thr Tyr Tyr Cys Leu Gln Tyr Glu Gln Leu Arg Thr
85 90 95

Phe Gly Gly Gly Thr Lys Val Glu Ile Lys Arg Thr Val Ala Ala Pro
100 105 110

Ser Val Phe Ile Phe Pro Pro Ser Asp Glu Gln Leu Lys Ser Gly Thr
115 120 125

Ala Ser Val Val Cys Leu Leu Asn Asn Phe Tyr Pro Arg Glu Ala Lys
130 135 140

Val Gln Trp Lys Val Asp Asn Ala Leu Gln Ser Gly Asn Ser Gln Glu
145 150 155 160

Ser Val Thr Glu Gln Asp Ser Lys Asp Ser Thr Tyr Ser Leu Ser Ser
165 170 175

Thr Leu Thr Leu Ser Lys Ala Asp Tyr Glu Lys His Lys Val Tyr Ala
180 185 190

Cys Glu Val Thr His Gln Gly Leu Ser Ser Pro Val Thr Lys Ser Phe
195 200 205

Asn Arg Gly Glu Cys
210

<210> 80
<211> 448
<212> PRT
<213> Artificial Sequence

<220>
<223> SMB0002hH-G1m3

<400> 80

```
Gln Val Gln Leu Val Gln Ser Gly Ala Glu Val Lys Lys Pro Gly Ala
1               5                   10                  15

Ser Val Lys Val Ser Cys Lys Val Ser Gly Ile Asp Leu Thr Asn Tyr
            20                  25                  30

Ala Met Gly Trp Val Arg Gln Pro Pro Gly Lys Gly Leu Glu Trp Ile
        35                  40                  45

Gly Ile Ile Gly Ala Asp Ser Ser Thr Trp Tyr Pro Ser Trp Val Lys
    50                  55                  60

Gly Arg Phe Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Arg Gly Arg Phe Val Gly Tyr Thr Asn Ala Phe Asp Pro Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255
```

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

<210> 81
<211> 220
<212> PRT
<213> Artificial Sequence

<220>
<223> SMB0002hL-k0a

<400> 81

Asp Ile Gln Leu Thr Gln Ser Pro Ser Ser Leu Ser Ala Ser Val Gly
1 5 10 15

Asp Arg Val Thr Ile Thr Cys Gln Ser Ser Gln Ser Val Trp Asn Asn
20 25 30

Asn Tyr Leu Ser Trp Tyr Gln Gln Lys Pro Gly Gln Pro Pro Lys Leu
35 40 45

Leu Ile Tyr Asp Ala Ser Thr Leu Ala Ser Gly Val Pro Ser Arg Phe
50 55 60

Ser Gly Ser Gly Ser Gly Thr Asp Phe Thr Leu Thr Ile Asn Ser Leu
65 70 75 80

Glu Ala Glu Asp Ala Ala Thr Tyr Tyr Cys His Gly Ser Tyr Ala Asn
85 90 95

Ser Gly Trp Tyr Asp Asn Ala Phe Gly Gly Gly Thr Glu Val Val Val
100 105 110

Lys Arg Thr Val Ala Ala Pro Ser Val Phe Ile Phe Pro Pro Ser Asp
115 120 125

Glu Gln Leu Lys Ser Gly Thr Ala Ser Val Val Cys Leu Leu Asn Asn
130 135 140

Phe Tyr Pro Arg Glu Ala Lys Val Gln Trp Lys Val Asp Asn Ala Leu
145 150 155 160

Gln Ser Gly Asn Ser Gln Glu Ser Val Thr Glu Gln Asp Ser Lys Asp
165 170 175

Ser Thr Tyr Ser Leu Ser Ser Thr Leu Thr Leu Ser Lys Ala Asp Tyr
180 185 190

Glu Lys His Lys Val Tyr Ala Cys Glu Val Thr His Gln Gly Leu Ser
195 200 205

Ser Pro Val Thr Lys Ser Phe Asn Arg Gly Glu Cys
210 215 220

<210> 82
<211> 448
<212> PRT
<213> Artificial Sequence

<220>
<223> SMBh068-G1m3

<400> 82

```
Gln Val Gln Leu Val Gln Ser Gly Ala Glu Val Lys Lys Pro Gly Ala
1               5                   10                  15

Ser Val Lys Val Ser Cys Lys Val Ser Gly Ile Asp Leu Thr Asn Tyr
            20                  25                  30

Ala Met Gly Trp Val Arg Gln Pro Pro Gly Lys Gly Leu Glu Trp Ile
        35                  40                  45

Gly Ile Ile Gly Ala Asp Ser Ser Thr Trp Tyr Pro Ser Trp Val Lys
    50                  55                  60

Gly Arg Phe Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Arg Gly Arg Phe Val Gly Ala Thr Asn Ala Phe Asp Pro Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255
```

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

<210> 83
<211> 448
<212> PRT
<213> Artificial Sequence

<220>
<223> SMBh508-G1m3

<400> 83

Gln Val Gln Leu Val Gln Ser Gly Ala Glu Val Lys Lys Pro Gly Ala
1 5 10 15

```
Ser Val Lys Val Ser Cys Lys Val Ser Gly Ile Asp Leu Thr Asn Tyr
            20                  25                  30

Ala Met Gly Trp Val Arg Gln Pro Pro Gly Lys Gly Leu Glu Trp Ile
        35                  40                  45

Gly Ala Ile Gly Ala Asp Ser Ser Thr Trp Tyr Pro Ser Trp Val Lys
    50                  55                  60

Gly Arg Phe Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Arg Gly Arg Phe Val Gly Tyr Thr Asn Ala Phe Asp Pro Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270
```

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

<210> 84
<211> 448
<212> PRT
<213> Artificial Sequence

<220>
<223> SMBh606-G1m3

<400> 84

Gln Val Gln Leu Val Gln Ser Gly Ala Glu Val Lys Lys Pro Gly Ala
1 5 10 15

Ser Val Lys Val Ser Cys Lys Val Ser Gly Ile Asp Leu Thr Asn Tyr
20 25 30

Ala Met Gly Trp Val Arg Gln Pro Pro Gly Lys Gly Leu Glu Trp Ile
35 40 45

Gly Ile Ile Gly Ala Asp Ser Ser Thr Trp Tyr Pro Ser Trp Val Lys
50 55 60

Gly Arg Phe Thr Ile Ser Lys Asp Thr Ser Lys Asn Gln Val Val Leu
65 70 75 80

Thr Met Thr Asn Met Asp Pro Val Asp Thr Ala Thr Tyr Tyr Cys Ala
85 90 95

Arg Gly Arg Phe Val Gly Tyr Thr Asn Ala Ala Asp Pro Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Lys Ser Thr Ser Gly Gly Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Leu Gly Gly
225 230 235 240

Pro Ser Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Asn Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Val
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

<210> 85
<211> 220
<212> PRT
<213> Artificial Sequence

<220>
<223> SMB1234-k0a

<400> 85

Asp Ile Gln Leu Thr Gln Ser Pro Ser Ser Leu Ser Ala Ser Val Gly
1 5 10 15

Asp Arg Val Thr Ile Thr Cys Gln Ser Ser Gln Ser Val Trp Asn Asn
20 25 30

Asn Tyr Leu Ser Trp Tyr Gln Gln Lys Pro Gly Gln Pro Pro Lys Leu
35 40 45

Leu Ile Tyr Asp Ala Ser Thr Leu Ala Ser Gly Val Pro Ser Arg Phe
    50                  55                  60

Ser Gly Ser Gly Ser Gly Thr Asp Phe Thr Leu Thr Ile Asn Ser Leu
65                  70                  75                  80

Glu Ala Glu Asp Ala Ala Thr Tyr Tyr Cys His Gly Ser Tyr Ala Asn
                85                  90                  95

Ser Gly Trp Tyr Asp Ala Ala Phe Gly Gly Gly Thr Glu Val Val Val
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220

<210> 86
<211> 220
<212> PRT
<213> Artificial Sequence

<220>
<223> SMB1255-k0a

<400> 86

Asp Ile Gln Leu Thr Gln Ser Pro Ser Ser Leu Ser Ala Ser Val Gly
1               5                   10                  15

Asp Arg Val Thr Ile Thr Cys Gln Ser Ser Gln Ser Val Trp Asn Asn
            20                  25                  30

Asn Tyr Leu Ser Trp Tyr Gln Gln Lys Pro Gly Gln Pro Pro Lys Leu

```
        35                  40                  45

Leu Ile Tyr Asp Ala Ser Thr Leu Ala Ser Gly Val Pro Ser Arg Phe
    50                  55                  60

Ser Gly Ser Gly Ser Gly Thr Asp Phe Thr Leu Thr Ile Asn Ser Leu
65                  70                  75                  80

Glu Ala Glu Asp Ala Ala Thr Tyr Tyr Cys His Gly Ser Tyr Ala Asn
                85                  90                  95

Ser Gly Trp Tyr Asp Asn Ala Ala Gly Gly Gly Thr Glu Val Val Val
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys
    210                 215                 220


<210>  87
<211>  249
<212>  PRT
<213>  Artificial Sequence

<220>
<223>  SMB0002hL-k0aTEVBAP

<400>  87

Asp Ile Gln Leu Thr Gln Ser Pro Ser Ser Leu Ser Ala Ser Val Gly
1               5                   10                  15

Asp Arg Val Thr Ile Thr Cys Gln Ser Ser Gln Ser Val Trp Asn Asn
            20                  25                  30
```

Asn Tyr Leu Ser Trp Tyr Gln Gln Lys Pro Gly Gln Pro Pro Lys Leu
        35                  40                  45

Leu Ile Tyr Asp Ala Ser Thr Leu Ala Ser Gly Val Pro Ser Arg Phe
    50                  55                  60

Ser Gly Ser Gly Ser Gly Thr Asp Phe Thr Leu Thr Ile Asn Ser Leu
65                  70                  75                  80

Glu Ala Glu Asp Ala Ala Thr Tyr Tyr Cys His Gly Ser Tyr Ala Asn
                85                  90                  95

Ser Gly Trp Tyr Asp Asn Ala Phe Gly Gly Gly Thr Glu Val Val Val
            100                 105                 110

Lys Arg Thr Val Ala Ala Pro Ser Val Phe Ile Phe Pro Pro Ser Asp
        115                 120                 125

Glu Gln Leu Lys Ser Gly Thr Ala Ser Val Val Cys Leu Leu Asn Asn
    130                 135                 140

Phe Tyr Pro Arg Glu Ala Lys Val Gln Trp Lys Val Asp Asn Ala Leu
145                 150                 155                 160

Gln Ser Gly Asn Ser Gln Glu Ser Val Thr Glu Gln Asp Ser Lys Asp
                165                 170                 175

Ser Thr Tyr Ser Leu Ser Ser Thr Leu Thr Leu Ser Lys Ala Asp Tyr
            180                 185                 190

Glu Lys His Lys Val Tyr Ala Cys Glu Val Thr His Gln Gly Leu Ser
        195                 200                 205

Ser Pro Val Thr Lys Ser Phe Asn Arg Gly Glu Cys Gly Gly Ser Ser
    210                 215                 220

Glu Asn Leu Tyr Phe Gln Gly Ser Gly Gly Gly Leu Asn Asp Ile Phe
225                 230                 235                 240

Glu Ala Gln Lys Ile Glu Trp His Glu
                245

<210> 88
<211> 453
<212> PRT
<213> Artificial Sequence

<220>
<223> SC001H-G1T3L

<400> 88

Gln Val Gln Leu Val Gln Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Val Tyr Gln Gly Gly Ala Thr Pro Tyr Tyr Tyr Tyr Gly Met
100 105 110

Asp Val Trp Gly Gln Gly Thr Met Val Thr Val Ser Ser Ala Ser Thr
115 120 125

Lys Gly Pro Ser Val Phe Pro Leu Ala Pro Ser Ser Lys Ser Thr Ser
130 135 140

Gly Gly Thr Ala Ala Leu Gly Cys Leu Val Lys Asp Tyr Phe Pro Glu
145 150 155 160

Pro Val Thr Val Ser Trp Asn Ser Gly Ala Leu Thr Ser Gly Val His
165 170 175

Thr Phe Pro Ala Val Leu Gln Ser Ser Gly Leu Tyr Ser Leu Ser Ser
180 185 190

Val Val Thr Val Pro Ser Ser Ser Leu Gly Thr Gln Thr Tyr Ile Cys
195 200 205

Asn Val Asn His Lys Pro Ser Asn Thr Lys Val Asp Lys Arg Val Glu
210 215 220

Pro Lys Ser Cys Asp Lys Thr His Thr Cys Pro Pro Cys Pro Ala Pro
225 230 235 240

Glu Leu Leu Gly Gly Pro Ser Val Phe Leu Phe Pro Pro Lys Pro Lys
245 250 255

Asp Thr Leu Met Ile Ser Arg Thr Pro Glu Val Thr Cys Val Val Val
260 265 270

Asp Val Ser His Glu Asp Pro Glu Val Lys Phe Asn Trp Tyr Val Asp
275 280 285

Gly Val Glu Val His Asn Ala Lys Thr Lys Pro Arg Glu Glu Gln Tyr
290 295 300

Asn Ser Thr Tyr Arg Val Val Ser Val Leu Thr Val Leu His Gln Asp
305 310 315 320

Trp Leu Asn Gly Lys Glu Tyr Lys Cys Lys Val Ser Asn Lys Ala Leu
325 330 335

Pro Ala Pro Ile Glu Lys Thr Ile Ser Lys Ala Lys Gly Gln Pro Arg
340 345 350

Glu Pro Gln Val Tyr Thr Leu Pro Pro Ser Arg Glu Glu Met Thr Lys
355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Lys Leu Thr Val Asp Lys Ser Arg Trp Gln Glu Gly Asn Val Phe Ser
420 425 430

Cys Ser Val Met His Glu Ala Leu His Asn His Tyr Thr Gln Lys Ser
435 440 445

Leu Ser Leu Ser Pro
450

<210> 89
<211> 216
<212> PRT
<213> Artificial Sequence

<220>
<223>  SC001L-SCL1

<400>  89

Ser Tyr Glu Leu Thr Gln Pro Pro Ser Ala Ser Gly Thr Pro Gly Gln
1               5                   10                  15

Arg Val Thr Val Ser Cys Ser Gly Ser Thr Ser Asn Ile Gly Asn His
            20                  25                  30

Ala Val Asn Trp Tyr Gln Gln Leu Pro Gly Thr Ala Pro Lys Leu Leu
        35                  40                  45

Ile Tyr Thr Asn Asp Gln Arg Pro Ser Gly Val Pro Asn Arg Phe Ser
    50                  55                  60

Gly Ser Lys Ser Gly Ile Ser Ala Ser Leu Ala Ile Ser Gly Leu Gln
65                  70                  75                  80

Ser Glu Asp Glu Ala Asp Tyr Tyr Cys Ser Ala Trp Asp Asp Ser Leu
                85                  90                  95

Ile Gly Tyr Val Phe Gly Ala Gly Thr Lys Val Thr Val Leu Gly Gln
            100                 105                 110

Pro Lys Ala Asn Pro Thr Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys
145                 150                 155                 160

Ala Gly Val Glu Thr Thr Lys Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215

<210>  90
<211>  456

<212> PRT
<213> Artificial Sequence

<220>
<223> SC002H-G1T3L

<400> 90

Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Gly Ile Ser Trp Val Arg Gln Ala Pro Gly Gln Gly Leu Glu Trp Met
35 40 45

Gly Trp Ile Ser Ala Tyr Asn Gly Asn Thr Asn Tyr Ala Gln Lys Leu
50 55 60

Gln Gly Arg Val Thr Met Thr Thr Asp Thr Ser Thr Ser Thr Ala Tyr
65 70 75 80

Met Glu Leu Arg Ser Leu Arg Ser Asp Asp Thr Ala Val Tyr Tyr Cys
85 90 95

Ala Arg Val Ser Ser Gly Ser Gln Pro Asp Pro Pro Tyr Tyr Tyr Tyr
100 105 110

Tyr Gly Met Asp Val Trp Gly Gln Gly Thr Met Val Thr Val Ser Ser
115 120 125

Ala Ser Thr Lys Gly Pro Ser Val Phe Pro Leu Ala Pro Ser Ser Lys
130 135 140

Ser Thr Ser Gly Gly Thr Ala Ala Leu Gly Cys Leu Val Lys Asp Tyr
145 150 155 160

Phe Pro Glu Pro Val Thr Val Ser Trp Asn Ser Gly Ala Leu Thr Ser
165 170 175

Gly Val His Thr Phe Pro Ala Val Leu Gln Ser Ser Gly Leu Tyr Ser
180 185 190

Leu Ser Ser Val Val Thr Val Pro Ser Ser Ser Leu Gly Thr Gln Thr
195 200 205

Tyr Ile Cys Asn Val Asn His Lys Pro Ser Asn Thr Lys Val Asp Lys
210 215 220

```
Arg Val Glu Pro Lys Ser Cys Asp Lys Thr His Thr Cys Pro Pro Cys
225                 230                 235                 240

Pro Ala Pro Glu Leu Leu Gly Gly Pro Ser Val Phe Leu Phe Pro Pro
                245                 250                 255

Lys Pro Lys Asp Thr Leu Met Ile Ser Arg Thr Pro Glu Val Thr Cys
            260                 265                 270

Val Val Val Asp Val Ser His Glu Asp Pro Glu Val Lys Phe Asn Trp
        275                 280                 285

Tyr Val Asp Gly Val Glu Val His Asn Ala Lys Thr Lys Pro Arg Glu
    290                 295                 300

Glu Gln Tyr Asn Ser Thr Tyr Arg Val Val Ser Val Leu Thr Val Leu
305                 310                 315                 320

His Gln Asp Trp Leu Asn Gly Lys Glu Tyr Lys Cys Lys Val Ser Asn
                325                 330                 335

Lys Ala Leu Pro Ala Pro Ile Glu Lys Thr Ile Ser Lys Ala Lys Gly
            340                 345                 350

Gln Pro Arg Glu Pro Gln Val Tyr Thr Leu Pro Pro Ser Arg Glu Glu
        355                 360                 365

Met Thr Lys Asn Gln Val Ser Leu Thr Cys Leu Val Lys Gly Phe Tyr
    370                 375                 380

Pro Ser Asp Ile Ala Val Glu Trp Glu Ser Asn Gly Gln Pro Glu Asn
385                 390                 395                 400

Asn Tyr Lys Thr Thr Pro Pro Val Leu Asp Ser Asp Gly Ser Phe Phe
                405                 410                 415

Leu Tyr Ser Lys Leu Thr Val Asp Lys Ser Arg Trp Gln Glu Gly Asn
            420                 425                 430

Val Phe Ser Cys Ser Val Met His Glu Ala Leu His Asn His Tyr Thr
        435                 440                 445

Gln Lys Ser Leu Ser Leu Ser Pro
    450                 455
```

<210>  91
<211>  217

<212> PRT
<213> Artificial Sequence

<220>
<223> SC002L-SCL2

<400> 91

```
Gln Ser Val Leu Thr Gln Pro Ala Ser Val Ser Gly Ser Pro Gly Gln
1               5                   10                  15

Ser Ile Thr Ile Ser Cys Thr Gly Thr Ser Ser Asn Val Glu Ser Tyr
            20                  25                  30

Asn Leu Val Ser Trp Tyr Gln Gln His Pro Gly Lys Ala Pro Lys Phe
        35                  40                  45

Ile Ile Tyr Glu Gly Thr Arg Arg Pro Ser Gly Ile Ser Asn Arg Phe
    50                  55                  60

Ser Gly Ala Asn Ser Gly Asn Ala Ala Ser Leu Thr Ile Ser Gly Leu
65                  70                  75                  80

Gln Ala Glu Asp Glu Ala Asp Tyr Tyr Cys Leu Ser Tyr Val Pro Ser
                85                  90                  95

Arg Arg Arg His Val Phe Gly Thr Gly Thr Lys Val Thr Val Leu Ser
            100                 105                 110

Gln Pro Lys Ala Asn Pro Thr Val Thr Leu Phe Pro Pro Ser Ser Glu
        115                 120                 125

Glu Leu Gln Ala Asn Lys Ala Thr Leu Val Cys Leu Ile Ser Asp Phe
    130                 135                 140

Tyr Pro Gly Ala Val Thr Val Ala Trp Lys Ala Asp Gly Ser Pro Val
145                 150                 155                 160

Lys Ala Gly Val Glu Thr Thr Lys Pro Ser Lys Gln Ser Asn Asn Lys
                165                 170                 175

Tyr Ala Ala Ser Ser Tyr Leu Ser Leu Thr Pro Glu Gln Trp Lys Ser
            180                 185                 190

His Arg Ser Tyr Ser Cys Gln Val Thr His Glu Gly Ser Thr Val Glu
        195                 200                 205

Lys Thr Val Ala Pro Thr Glu Cys Ser
    210                 215
```

<210> 92
<211> 446
<212> PRT
<213> Artificial Sequence

<220>
<223> SC014H-G1T3L

<400> 92

Glu Val Gln Leu Val Gln Ser Gly Gly Gly Leu Val Gln Pro Gly Arg
1 5 10 15

Ser Leu Arg Leu Ser Cys Ala Ala Ser Gly Phe Thr Phe Asp Asp Tyr
20 25 30

Ala Met His Trp Val Arg Gln Ala Pro Gly Lys Gly Leu Glu Trp Val
35 40 45

Ser Gly Ile Ser Trp Asn Ser Gly Ser Ile Gly Tyr Ala Asp Ser Val
50 55 60

Lys Gly Arg Phe Ala Ile Ser Arg Asp Asn Ala Lys Asn Ser Leu Tyr
65 70 75 80

Leu Gln Met Asn Ser Leu Arg Ala Glu Asp Thr Ala Leu Tyr Tyr Cys
85 90 95

Ala Lys Ala Trp Asn Asp Asp Ala Phe Asp Ile Trp Gly Gln Gly Thr
100 105 110

Met Val Thr Val Ser Ser Ala Ser Thr Lys Gly Pro Ser Val Phe Pro
115 120 125

Leu Ala Pro Ser Ser Lys Ser Thr Ser Gly Gly Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Gln Thr Tyr Ile Cys Asn Val Asn His Lys Pro Ser
195 200 205

Asn Thr Lys Val Asp Lys Arg Val Glu Pro Lys Ser Cys Asp Lys Thr
210 215 220

His Thr Cys Pro Pro Cys Pro Ala Pro Glu Leu Leu Gly Gly Pro Ser
225 230 235 240

Val Phe Leu Phe Pro Pro Lys Pro Lys Asp Thr Leu Met Ile Ser Arg
245 250 255

Thr Pro Glu Val Thr Cys Val Val Val Asp Val Ser His Glu Asp Pro
260 265 270

Glu Val Lys Phe Asn Trp Tyr Val Asp Gly Val Glu Val His Asn Ala
275 280 285

Lys Thr Lys Pro Arg Glu Glu Gln Tyr Asn Ser Thr Tyr Arg Val Val
290 295 300

Ser Val Leu Thr Val Leu His Gln Asp Trp Leu Asn Gly Lys Glu Tyr
305 310 315 320

Lys Cys Lys Val Ser Asn Lys Ala Leu Pro Ala Pro Ile Glu Lys Thr
325 330 335

Ile Ser Lys Ala Lys Gly Gln Pro Arg Glu Pro Gln Val Tyr Thr Leu
340 345 350

Pro Pro Ser Arg Glu Glu Met Thr Lys Asn Gln Val Ser Leu Thr Cys
355 360 365

Leu Val Lys Gly Phe Tyr Pro Ser Asp Ile Ala Val Glu Trp Glu Ser
370 375 380

Asn Gly Gln Pro Glu Asn Asn Tyr Lys Thr Thr Pro Pro Val Leu Asp
385 390 395 400

Ser Asp Gly Ser Phe Phe Leu Tyr Ser Lys Leu Thr Val Asp Lys Ser
405 410 415

Arg Trp Gln Glu Gly Asn Val Phe Ser Cys Ser Val Met His Glu Ala
420 425 430

Leu His Asn His Tyr Thr Gln Lys Ser Leu Ser Leu Ser Pro
435 440 445

<210> 93
<211> 219
<212> PRT

<213> Artificial Sequence

<220>
<223> SC014L-SCL7

<400> 93

Gln Ser Val Val Thr Gln Pro Pro Ser Val Ser Gly Ala Pro Gly Gln
1 5 10 15

Arg Val Thr Val Ser Cys Thr Gly Ser Asn Ser Asn Ile Gly Ala Gly
20 25 30

Tyr Asp Val His Trp Tyr Gln Gln Leu Pro Gly Thr Ala Pro Lys Leu
35 40 45

Leu Ile Tyr Gly Ser Asn Asn Arg Pro Ser Gly Val Pro Asp Arg Phe
50 55 60

Ser Ala Ser Arg Ser Gly Thr Ser Ala Ser Leu Ala Ile Thr Gly Leu
65 70 75 80

Gln Ala Glu Asp Glu Ala Glu Tyr Tyr Cys Gln Thr Tyr Asp Thr Gly
85 90 95

Leu Ser Gly Pro Gly Val Val Phe Gly Gly Gly Thr Lys Val Thr Val
100 105 110

Leu Ser Gln Pro Lys Ala Ala Pro Ser Val Thr Leu Phe Pro Pro Ser
115 120 125

Ser Glu Glu Leu Gln Ala Asn Lys Ala Thr Leu Val Cys Leu Ile Ser
130 135 140

Asp Phe Tyr Pro Gly Ala Val Thr Val Ala Trp Lys Ala Asp Ser Ser
145 150 155 160

Pro Val Lys Ala Gly Val Glu Thr Thr Thr Pro Ser Lys Gln Ser Asn
165 170 175

Asn Lys Tyr Ala Ala Ser Ser Tyr Leu Ser Leu Thr Pro Glu Gln Trp
180 185 190

Lys Ser His Arg Ser Tyr Ser Cys Gln Val Thr His Glu Gly Ser Thr
195 200 205

Val Glu Lys Thr Val Ala Pro Thr Glu Cys Ser
210 215

<210> 94
<211> 449
<212> PRT
<213> Artificial Sequence

<220>
<223> SC016H-G1T3L

<400> 94

```
Glu Val Gln Leu Val Gln Ser Gly Gly Gly Leu Val Gln Pro Gly Arg
1               5                   10                  15

Ser Leu Arg Leu Ser Cys Ala Ala Ser Gly Phe Thr Phe Asp Asn His
            20                  25                  30

Ala Met His Trp Val Arg Gln Ala Pro Gly Lys Gly Leu Glu Trp Val
        35                  40                  45

Ser Arg Ile Asn Trp Asn Ser Gly Gly Ile Asp Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Leu Tyr Tyr Cys
                85                  90                  95

Val Lys Asp Arg Gly Ser Gly Trp Tyr Asp Ala Phe Asp Leu Trp Gly
            100                 105                 110

Gln Gly Thr Met Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205

Lys Pro Ser Asn Thr Lys Val Asp Lys Arg Val Glu Pro Lys Ser Cys
```

```
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Glu Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro
```

<210> 95
<211> 214
<212> PRT
<213> Artificial Sequence

<220>
<223> SC016L-hl

<400> 95

Ser Tyr Glu Leu Thr Gln Pro Pro Ser Val Ser Val Ala Pro Gly Gln
1 5 10 15

Thr Thr Arg Ile Ala Cys Gly Gly Asp Ser Val Gly Ser Glu Ser Val
20 25 30

His Trp Tyr Gln Gln Arg Pro Gly Gln Ala Pro Val Leu Val Ile Ser
35 40 45

Tyr Asp Ser Asp Arg Pro Ser Gly Ile Pro Glu Arg Phe Ser Gly Ser
50 55 60

Lys Ser Gly Asn Thr Ala Thr Leu Thr Ile Ser Arg Val Glu Ala Gly
65 70 75 80

Asp Glu Ala Asp Tyr Tyr Cys Gln Val Trp Asn Ser Ala Ser Asp His
85 90 95

Trp Val Phe Gly Gly Gly Thr Lys Leu Thr Val Leu Gly Gln Pro Lys
100 105 110

Ala Ala Pro Ser Val Thr Leu Phe Pro Pro Ser Ser Glu Glu Leu Gln
115 120 125

Ala Asn Lys Ala Thr Leu Val Cys Leu Ile Ser Asp Phe Tyr Pro Gly
130 135 140

Ala Val Thr Val Ala Trp Lys Ala Asp Ser Ser Pro Val Lys Ala Gly
145 150 155 160

Val Glu Thr Thr Thr Pro Ser Lys Gln Ser Asn Asn Lys Tyr Ala Ala
165 170 175

Ser Ser Tyr Leu Ser Leu Thr Pro Glu Gln Trp Lys Ser His Arg Ser
180 185 190

Tyr Ser Cys Gln Val Thr His Glu Gly Ser Thr Val Glu Lys Thr Val
195 200 205

Ala Pro Thr Glu Cys Ser
210

210

<210> 96
<211> 449
<212> PRT
<213> Artificial Sequence

<220>
<223> SC019H-G1T3L

<400> 96

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Asp Tyr
            20                  25                  30

Tyr Met Ser Trp Ile Arg Gln Ala Pro Gly Lys Gly Leu Glu Trp Val
        35                  40                  45

Ser Tyr Ile Ser Ser Ser Gly Ser Thr Ile Tyr Tyr Ala Asp Ser Val
    50                  55                  60

Lys Gly Arg Phe Thr Ile Ser Arg Asp Asn Ala Lys Asn Ser Leu Tyr
65                  70                  75                  80

Leu Gln Met Asn Ser Leu Arg Ala Glu Asp Thr Ala Val Tyr Tyr Cys
                85                  90                  95

Ala Arg Ala Ser Pro Asn Val Asn Glu Gly Ala Phe Asp Ile Trp Gly
            100                 105                 110

Gln Gly Thr Met Val Thr Val Ser Ser Ala Ser Thr Lys Gly Pro Ser
        115                 120                 125

Val Phe Pro Leu Ala Pro Ser Ser Lys Ser Thr Ser Gly Gly Thr Ala
    130                 135                 140

Ala Leu Gly Cys Leu Val Lys Asp Tyr Phe Pro Glu Pro Val Thr Val
145                 150                 155                 160

Ser Trp Asn Ser Gly Ala Leu Thr Ser Gly Val His Thr Phe Pro Ala
                165                 170                 175

Val Leu Gln Ser Ser Gly Leu Tyr Ser Leu Ser Ser Val Val Thr Val
            180                 185                 190

Pro Ser Ser Ser Leu Gly Thr Gln Thr Tyr Ile Cys Asn Val Asn His
        195                 200                 205
```

```
Lys Pro Ser Asn Thr Lys Val Asp Lys Arg Val Glu Pro Lys Ser Cys
    210                 215                 220

Asp Lys Thr His Thr Cys Pro Pro Cys Pro Ala Pro Glu Leu Leu Gly
225                 230                 235                 240

Gly Pro Ser Val Phe Leu Phe Pro Pro Lys Pro Lys Asp Thr Leu Met
                245                 250                 255

Ile Ser Arg Thr Pro Glu Val Thr Cys Val Val Val Asp Val Ser His
            260                 265                 270

Glu Asp Pro Glu Val Lys Phe Asn Trp Tyr Val Asp Gly Val Glu Val
        275                 280                 285

His Asn Ala Lys Thr Lys Pro Arg Glu Glu Gln Tyr Asn Ser Thr Tyr
    290                 295                 300

Arg Val Val Ser Val Leu Thr Val Leu His Gln Asp Trp Leu Asn Gly
305                 310                 315                 320

Lys Glu Tyr Lys Cys Lys Val Ser Asn Lys Ala Leu Pro Ala Pro Ile
                325                 330                 335

Glu Lys Thr Ile Ser Lys Ala Lys Gly Gln Pro Arg Glu Pro Gln Val
            340                 345                 350

Tyr Thr Leu Pro Pro Ser Arg Glu Glu Met Thr Lys Asn Gln Val Ser
        355                 360                 365

Leu Thr Cys Leu Val Lys Gly Phe Tyr Pro Ser Asp Ile Ala Val Glu
    370                 375                 380

Trp Glu Ser Asn Gly Gln Pro Glu Asn Asn Tyr Lys Thr Thr Pro Pro
385                 390                 395                 400

Val Leu Asp Ser Asp Gly Ser Phe Phe Leu Tyr Ser Lys Leu Thr Val
                405                 410                 415

Asp Lys Ser Arg Trp Gln Glu Gly Asn Val Phe Ser Cys Ser Val Met
            420                 425                 430

His Glu Ala Leu His Asn His Tyr Thr Gln Lys Ser Leu Ser Leu Ser
        435                 440                 445

Pro
```

<210> 97
<211> 216
<212> PRT
<213> Artificial Sequence

<220>
<223> SC019L-SCL5

<400> 97

```
Gln Ser Val Leu Thr Gln Pro Pro Ser Met Ser Ala Ala Pro Gly Gln
1               5                   10                  15

Lys Val Thr Ile Ser Cys Ser Gly Ser Gly Ser Asn Ile Gly Ser Ser
            20                  25                  30

Tyr Val Ser Trp Tyr Gln Gln Leu Pro Gly Lys Ala Pro Arg Leu Leu
        35                  40                  45

Ile Tyr Asp Asn Asn Lys Gln Val Ser Trp Val Pro Asp Arg Phe Ser
    50                  55                  60

Gly Ser Lys Ser Gly Thr Ser Ala Ser Leu Val Ile Ser Gly Leu Gln
65                  70                  75                  80

Thr Gly Asp Glu Ala Asp Tyr Tyr Cys Gly Thr Trp Asp Arg Ser Leu
                85                  90                  95

Ser Ala Gly Val Phe Gly Gly Gly Thr Lys Val Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Gly Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205
```

Thr Val Ala Pro Thr Glu Cys Ser
    210                 215

<210>  98
<211>  451
<212>  PRT
<213>  Artificial Sequence

<220>
<223>  SC009H-G1T3L

<400>  98

Gln Val Gln Leu Val Glu Thr Gly Gly Gly Leu Val Lys Pro Gly Gly
1               5                   10                  15

Ser Leu Arg Leu Ser Cys Ala Ala Ser Gly Phe Thr Phe Ser Asn Ala
            20                  25                  30

Trp Met Ser Trp Val Arg Gln Ala Pro Gly Lys Gly Leu Glu Trp Val
        35                  40                  45

Gly Arg Ile Lys Ser Lys Thr Asp Gly Gly Thr Thr Asp Tyr Ala Ala
    50                  55                  60

Pro Val Lys Gly Arg Phe Thr Ile Ser Arg Asp Asp Ser Lys Asn Thr
65                  70                  75                  80

Leu Tyr Leu Gln Met Asn Ser Leu Lys Thr Glu Asp Thr Ala Val Tyr
                85                  90                  95

Tyr Cys Thr Thr Gly Asn Tyr Tyr Tyr Asp Ser Ser Gly Leu Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

```
Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Glu Gly Asn Val Phe Ser Cys Ser
            420                 425                 430

Val Met His Glu Ala Leu His Asn His Tyr Thr Gln Lys Ser Leu Ser
        435                 440                 445
```

Leu Ser Pro
    450

<210> 99
<211> 215
<212> PRT
<213> Artificial Sequence

<220>
<223> SC009K-kp

<400> 99

Asp Ile Gln Leu Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Leu Pro
                85                  90                  95

Leu Thr Phe Gly Gly Gly Thr Lys Val Glu Ile Lys Arg Thr Val Ala
            100                 105                 110

Ala Pro Ser Val Phe Ile Phe Pro Pro Ser Asp Glu Gln Leu Lys Ser
        115                 120                 125

Gly Thr Ala Ser Val Val Cys Leu Leu Asn Asn Phe Tyr Pro Arg Glu
    130                 135                 140

Ala Lys Val Gln Trp Lys Val Asp Asn Ala Leu Gln Ser Gly Asn Ser
145                 150                 155                 160

Gln Glu Ser Val Thr Glu Gln Asp Ser Lys Asp Ser Thr Tyr Ser Leu
                165                 170                 175

Ser Ser Thr Leu Thr Leu Ser Lys Ala Asp Tyr Glu Lys His Lys Val
            180                 185                 190

Tyr Ala Cys Glu Val Thr His Gln Gly Leu Ser Ser Pro Val Thr Lys
        195                 200                 205

Ser Phe Asn Arg Gly Glu Cys
    210                 215

<210>  100
<211>  448
<212>  PRT
<213>  Artificial Sequence

<220>
<223>  SMB0002hH-F760mnP17

<400>  100

Gln Val Gln Leu Val Gln Ser Gly Ala Glu Val Lys Lys Pro Gly Ala
1               5                   10                  15

Ser Val Lys Val Ser Cys Lys Val Ser Gly Ile Asp Leu Thr Asn Tyr
            20                  25                  30

Ala Met Gly Trp Val Arg Gln Pro Pro Gly Lys Gly Leu Glu Trp Ile
        35                  40                  45

Gly Ile Ile Gly Ala Asp Ser Ser Thr Trp Tyr Pro Ser Trp Val Lys
    50                  55                  60

Gly Arg Phe Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Arg Gly Arg Phe Val Gly Tyr Thr Asn Ala Phe Asp Pro Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
180 185 190

Ser Ser Ser Leu Gly Thr Gln Thr Tyr Ile Cys Asn Val Asn His Lys
195 200 205

Pro Ser Asn Thr Lys Val Asp Lys Lys Val Glu Pro Lys Ser Cys Asp
210 215 220

Lys Thr His Thr Cys Pro Pro Cys Pro Ala Pro Glu Leu Arg Gly Gly
225 230 235 240

Pro Lys Val Phe Leu Phe Pro Pro Lys Pro Lys Asp Thr Leu Met Ile
245 250 255

Ser Arg Thr Pro Glu Val Thr Cys Val Val Val Asp Val Ser His Glu
260 265 270

Asp Pro Glu Val Lys Phe Asn Trp Tyr Val Asp Gly Val Glu Val His
275 280 285

Asn Ala Lys Thr Lys Pro Arg Glu Glu Gln Tyr Ala Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Ala Leu Pro Ala Pro Ile Glu
325 330 335

Lys Thr Ile Ser Lys Ala Lys Gly Gln Pro Arg Glu Pro Gln Val Tyr
340 345 350

Thr Leu Pro Pro Ser Arg Lys Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Tyr
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Lys Ser Leu Ser Leu Ser Pro
435 440 445

<210> 101
<211> 453
<212> PRT
<213> Artificial Sequence

<220>
<223> SC001H-F760mnP17

<400> 101

Gln Val Gln Leu Val Gln Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Val Tyr Gln Gly Gly Ala Thr Pro Tyr Tyr Tyr Tyr Gly Met
100 105 110

Asp Val Trp Gly Gln Gly Thr Met Val Thr Val Ser Ser Ala Ser Thr
115 120 125

Lys Gly Pro Ser Val Phe Pro Leu Ala Pro Ser Ser Lys Ser Thr Ser
130 135 140

Gly Gly Thr Ala Ala Leu Gly Cys Leu Val Lys Asp Tyr Phe Pro Glu
145 150 155 160

Pro Val Thr Val Ser Trp Asn Ser Gly Ala Leu Thr Ser Gly Val His
165 170 175

Thr Phe Pro Ala Val Leu Gln Ser Ser Gly Leu Tyr Ser Leu Ser Ser
180 185 190

Val Val Thr Val Pro Ser Ser Ser Leu Gly Thr Gln Thr Tyr Ile Cys
195 200 205

Asn Val Asn His Lys Pro Ser Asn Thr Lys Val Asp Lys Lys Val Glu
210 215 220

Pro Lys Ser Cys Asp Lys Thr His Thr Cys Pro Pro Cys Pro Ala Pro
225 230 235 240

Glu Leu Arg Gly Gly Pro Lys Val Phe Leu Phe Pro Pro Lys Pro Lys
245 250 255

Asp Thr Leu Met Ile Ser Arg Thr Pro Glu Val Thr Cys Val Val Val
260 265 270

Asp Val Ser His Glu Asp Pro Glu Val Lys Phe Asn Trp Tyr Val Asp
275 280 285

Gly Val Glu Val His Asn Ala Lys Thr Lys Pro Arg Glu Glu Gln Tyr
290 295 300

Ala Ser Thr Tyr Arg Val Val Ser Val Leu Thr Val Leu His Gln Asp
305 310 315 320

Trp Leu Asn Gly Lys Glu Tyr Lys Cys Lys Val Ser Asn Lys Ala Leu
325 330 335

Pro Ala Pro Ile Glu Lys Thr Ile Ser Lys Ala Lys Gly Gln Pro Arg
340 345 350

Glu Pro Gln Val Tyr Thr Leu Pro Pro Ser Arg Lys Glu Met Thr Lys
355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Tyr Leu Asp Ser Asp Gly Ser Phe Phe Leu Tyr Ser
405 410 415

Lys Leu Thr Val Asp Lys Ser Arg Trp Gln Gln Gly Asn Val Phe Ser
420 425 430

Cys Ser Val Met His Glu Ala Leu His Asn His Tyr Thr Gln Lys Ser
435 440 445

Leu Ser Leu Ser Pro
    450

<210>  102
<211>  456
<212>  PRT
<213>  Artificial Sequence

<220>
<223>  SC002H-F760mnP17

<400>  102

Glu Val Gln Leu Val Gln Ser Gly Ala Glu Val Lys Lys Pro Gly Ala
1               5                   10                  15

Ser Val Lys Val Ser Cys Lys Ala Ser Gly Tyr Thr Phe Thr Ser Tyr
            20                  25                  30

Gly Ile Ser Trp Val Arg Gln Ala Pro Gly Gln Gly Leu Glu Trp Met
        35                  40                  45

Gly Trp Ile Ser Ala Tyr Asn Gly Asn Thr Asn Tyr Ala Gln Lys Leu
    50                  55                  60

Gln Gly Arg Val Thr Met Thr Thr Asp Thr Ser Thr Ser Thr Ala Tyr
65                  70                  75                  80

Met Glu Leu Arg Ser Leu Arg Ser Asp Asp Thr Ala Val Tyr Tyr Cys
                85                  90                  95

Ala Arg Val Ser Ser Gly Ser Gln Pro Asp Pro Pro Tyr Tyr Tyr Tyr
            100                 105                 110

Tyr Gly Met Asp Val Trp Gly Gln Gly Thr Met Val Thr Val Ser Ser
        115                 120                 125

Ala Ser Thr Lys Gly Pro Ser Val Phe Pro Leu Ala Pro Ser Ser Lys
    130                 135                 140

Ser Thr Ser Gly Gly Thr Ala Ala Leu Gly Cys Leu Val Lys Asp Tyr
145                 150                 155                 160

Phe Pro Glu Pro Val Thr Val Ser Trp Asn Ser Gly Ala Leu Thr Ser
                165                 170                 175

Gly Val His Thr Phe Pro Ala Val Leu Gln Ser Ser Gly Leu Tyr Ser
            180                 185                 190

```
Leu Ser Ser Val Val Thr Val Pro Ser Ser Ser Leu Gly Thr Gln Thr
        195                 200                 205

Tyr Ile Cys Asn Val Asn His Lys Pro Ser Asn Thr Lys Val Asp Lys
    210                 215                 220

Lys Val Glu Pro Lys Ser Cys Asp Lys Thr His Thr Cys Pro Pro Cys
225                 230                 235                 240

Pro Ala Pro Glu Leu Arg Gly Gly Pro Lys Val Phe Leu Phe Pro Pro
                245                 250                 255

Lys Pro Lys Asp Thr Leu Met Ile Ser Arg Thr Pro Glu Val Thr Cys
            260                 265                 270

Val Val Val Asp Val Ser His Glu Asp Pro Glu Val Lys Phe Asn Trp
        275                 280                 285

Tyr Val Asp Gly Val Glu Val His Asn Ala Lys Thr Lys Pro Arg Glu
    290                 295                 300

Glu Gln Tyr Ala Ser Thr Tyr Arg Val Val Ser Val Leu Thr Val Leu
305                 310                 315                 320

His Gln Asp Trp Leu Asn Gly Lys Glu Tyr Lys Cys Lys Val Ser Asn
                325                 330                 335

Lys Ala Leu Pro Ala Pro Ile Glu Lys Thr Ile Ser Lys Ala Lys Gly
            340                 345                 350

Gln Pro Arg Glu Pro Gln Val Tyr Thr Leu Pro Pro Ser Arg Lys Glu
        355                 360                 365

Met Thr Lys Asn Gln Val Ser Leu Thr Cys Leu Val Lys Gly Phe Tyr
    370                 375                 380

Pro Ser Asp Ile Ala Val Glu Trp Glu Ser Asn Gly Gln Pro Glu Asn
385                 390                 395                 400

Asn Tyr Lys Thr Thr Pro Pro Tyr Leu Asp Ser Asp Gly Ser Phe Phe
                405                 410                 415

Leu Tyr Ser Lys Leu Thr Val Asp Lys Ser Arg Trp Gln Gln Gly Asn
            420                 425                 430

Val Phe Ser Cys Ser Val Met His Glu Ala Leu His Asn His Tyr Thr
        435                 440                 445
```

```
Gln Lys Ser Leu Ser Leu Ser Pro
    450                 455


<210>  103
<211>  450
<212>  PRT
<213>  Artificial Sequence

<220>
<223>  TR01H113-F760mnN17

<400>  103

Gln Val Gln Leu Val Glu Ser Gly Gly Gly Val Val Gln Pro Gly Gly
1               5                   10                  15


Ser Leu Arg Leu Ser Cys Ala Ala Ser Gly Phe Thr Phe Ser Asn Ala
            20                  25                  30


Trp Met His Trp Val Arg Gln Ala Pro Gly Lys Gly Leu Glu Trp Val
        35                  40                  45


Ala Gln Ile Lys Asp Lys Ser Gln Asn Tyr Ala Thr Tyr Val Ala Glu
    50                  55                  60


Ser Val Lys Gly Arg Phe Thr Ile Ser Arg Ala Asp Ser Lys Asn Ser
65                  70                  75                  80


Ile Tyr Leu Gln Met Asn Ser Leu Lys Thr Glu Asp Thr Ala Val Tyr
                85                  90                  95


Tyr Cys Arg Tyr Val His Tyr Ala Ala Gly Tyr Gly Val Asp Ile Trp
            100                 105                 110


Gly Gln Gly Thr Thr Val Thr Val Ser Ser Ala Ser Thr Lys Gly Pro
        115                 120                 125


Ser Val Phe Pro Leu Ala Pro Ser Ser Lys Ser Thr Ser Gly Gly Thr
    130                 135                 140


Ala Ala Leu Gly Cys Leu Val Lys Asp Tyr Phe Pro Glu Pro Val Thr
145                 150                 155                 160


Val Ser Trp Asn Ser Gly Ala Leu Thr Ser Gly Val His Thr Phe Pro
                165                 170                 175


Ala Val Leu Gln Ser Ser Gly Leu Tyr Ser Leu Ser Ser Val Val Thr
            180                 185                 190
```

Val Pro Ser Ser Ser Leu Gly Thr Gln Thr Tyr Ile Cys Asn Val Asn
195 200 205

His Lys Pro Ser Asn Thr Lys Val Asp Lys Lys Val Glu Pro Lys Ser
210 215 220

Cys Asp Lys Thr His Thr Cys Pro Pro Cys Pro Ala Pro Glu Leu Arg
225 230 235 240

Gly Gly Pro Lys Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

His Glu Asp Pro Glu Val Lys Phe Asn Trp Tyr Val Asp Gly Val Glu
275 280 285

Val His Asn Ala Lys Thr Lys Pro Arg Glu Glu Gln Tyr Ala Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Ala Leu Pro Ala Pro
325 330 335

Ile Glu Lys Thr Ile Ser Lys Ala Lys Gly Gln Pro Arg Glu Pro Gln
340 345 350

Val Tyr Thr Leu Pro Pro Ser Arg Glu Glu Met Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Tyr Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Glu Ser Leu Ser Leu
435 440 445

Ser Pro
450

<210> 104
<211> 443
<212> PRT
<213> Artificial Sequence

<220>
<223> GCH065-F760mnN17

<400> 104

Gln Val Gln Leu Val Gln Ser Gly Ala Glu Val Lys Lys Pro Gly Ala
1 5 10 15

Ser Val Thr Val Ser Cys Lys Ala Ser Gly Tyr Thr Phe Thr Asp Tyr
20 25 30

Glu Met His Trp Ile Arg Gln Pro Pro Gly Glu Gly Leu Glu Trp Ile
35 40 45

Gly Ala Ile Asp Gly Pro Thr Pro Asp Thr Ala Tyr Ser Glu Lys Phe
50 55 60

Lys Gly Arg Val Thr Leu Thr Ala Asp Lys Ser Thr Ser Thr Ala Tyr
65 70 75 80

Met Glu Leu Ser Ser Leu Thr Ser Glu Asp Thr Ala Val Tyr Tyr Cys
85 90 95

Thr Arg Phe Tyr Ser Tyr Thr Tyr Trp Gly Gln Gly Thr Leu Val Thr
100 105 110

Val Ser Ser Ala Ser Thr Lys Gly Pro Ser Val Phe Pro Leu Ala Pro
115 120 125

Ser Ser Lys Ser Thr Ser Gly Gly Thr Ala Ala Leu Gly Cys Leu Val
130 135 140

Lys Asp Tyr Phe Pro Glu Pro Val Thr Val Ser Trp Asn Ser Gly Ala
145 150 155 160

Leu Thr Ser Gly Val His Thr Phe Pro Ala Val Leu Gln Ser Ser Gly
165 170 175

Leu Tyr Ser Leu Ser Ser Val Val Thr Val Pro Ser Ser Ser Leu Gly
180 185 190

```
Thr Gln Thr Tyr Ile Cys Asn Val Asn His Lys Pro Ser Asn Thr Lys
        195                 200                 205

Val Asp Lys Lys Val Glu Pro Lys Ser Cys Asp Lys Thr His Thr Cys
    210                 215                 220

Pro Pro Cys Pro Ala Pro Glu Leu Arg Gly Gly Pro Lys Val Phe Leu
225                 230                 235                 240

Phe Pro Pro Lys Pro Lys Asp Thr Leu Met Ile Ser Arg Thr Pro Glu
                245                 250                 255

Val Thr Cys Val Val Val Asp Val Ser His Glu Asp Pro Glu Val Lys
            260                 265                 270

Phe Asn Trp Tyr Val Asp Gly Val Glu Val His Asn Ala Lys Thr Lys
        275                 280                 285

Pro Arg Glu Glu Gln Tyr Ala Ser Thr Tyr Arg Val Val Ser Val Leu
    290                 295                 300

Thr Val Leu His Gln Asp Trp Leu Asn Gly Lys Glu Tyr Lys Cys Lys
305                 310                 315                 320

Val Ser Asn Lys Ala Leu Pro Ala Pro Ile Glu Lys Thr Ile Ser Lys
                325                 330                 335

Ala Lys Gly Gln Pro Arg Glu Pro Gln Val Tyr Thr Leu Pro Pro Ser
            340                 345                 350

Arg Glu Glu Met Thr Lys Asn Gln Val Ser Leu Thr Cys Leu Val Lys
        355                 360                 365

Gly Phe Tyr Pro Ser Asp Ile Ala Val Glu Trp Glu Ser Asn Gly Gln
    370                 375                 380

Pro Glu Asn Asn Tyr Lys Thr Thr Pro Pro Tyr Leu Asp Ser Asp Gly
385                 390                 395                 400

Ser Phe Phe Leu Tyr Ser Lys Leu Thr Val Asp Lys Ser Arg Trp Gln
                405                 410                 415

Gln Gly Asn Val Phe Ser Cys Ser Val Met His Glu Ala Leu His Asn
            420                 425                 430

His Tyr Thr Gln Glu Ser Leu Ser Leu Ser Pro
        435                 440
```

<210> 105
<211> 219
<212> PRT
<213> Artificial Sequence

<220>
<223> L0011-k0a

<400> 105

Asp Ile Val Met Thr Gln Ser Pro Leu Ser Leu Pro Val Thr Pro Gly
1 5 10 15

Glu Pro Ala Ser Ile Ser Cys Arg Ser Ser Gln Pro Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
    210                 215

<210>  106
<211>  120
<212>  PRT
<213>  Artificial Sequence

<220>
<223>  SMB0002hH

<400>  106

Gln Val Gln Leu Val Gln Ser Gly Ala Glu Val Lys Lys Pro Gly Ala
1               5                   10                  15

Ser Val Lys Val Ser Cys Lys Val Ser Gly Ile Asp Leu Thr Asn Tyr
            20                  25                  30

Ala Met Gly Trp Val Arg Gln Pro Pro Gly Lys Gly Leu Glu Trp Ile
        35                  40                  45

Gly Ile Ile Gly Ala Asp Ser Ser Thr Trp Tyr Pro Ser Trp Val Lys
    50                  55                  60

Gly Arg Phe Thr Ile Ser Lys Asp Thr Ser Lys Asn Gln Val Val Leu
65                  70                  75                  80

Thr Met Thr Asn Met Asp Pro Val Asp Thr Ala Thr Tyr Tyr Cys Ala
                85                  90                  95

Arg Gly Arg Phe Val Gly Tyr Thr Asn Ala Phe Asp Pro Trp Gly Gln
            100                 105                 110

Gly Thr Leu Val Thr Val Ser Ser
        115                 120

<210>  107
<211>  113
<212>  PRT
<213>  Artificial Sequence

<220>
<223>  SMB0002hL

<400>  107

Asp Ile Gln Leu Thr Gln Ser Pro Ser Ser Leu Ser Ala Ser Val Gly
1               5                   10                  15

Asp Arg Val Thr Ile Thr Cys Gln Ser Ser Gln Ser Val Trp Asn Asn
            20                  25                  30

Asn Tyr Leu Ser Trp Tyr Gln Gln Lys Pro Gly Gln Pro Pro Lys Leu
35 40 45

Leu Ile Tyr Asp Ala Ser Thr Leu Ala Ser Gly Val Pro Ser Arg Phe
50 55 60

Ser Gly Ser Gly Ser Gly Thr Asp Phe Thr Leu Thr Ile Asn Ser Leu
65 70 75 80

Glu Ala Glu Asp Ala Ala Thr Tyr Tyr Cys His Gly Ser Tyr Ala Asn
85 90 95

Ser Gly Trp Tyr Asp Asn Ala Phe Gly Gly Gly Thr Glu Val Val Val
100 105 110

Lys

<210> 108
<211> 117
<212> PRT
<213> Oryctolagus cuniculus

<220>
<223> SMBPH0002

<400> 108

Gln Ser Val Glu Glu Ser Gly Gly Arg Leu Val Thr Pro Gly Thr Pro
1 5 10 15

Leu Thr Leu Thr Cys Thr Val Ser Gly Ile Asp Leu Ser Asn Tyr Ala
20 25 30

Met Gly Trp Val Arg Gln Ala Pro Gly Lys Gly Leu Glu Trp Ile Gly
35 40 45

Ile Ile Gly Ala Asp Ser Ser Thr Trp Tyr Pro Ser Trp Val Lys Gly
50 55 60

Arg Phe Thr Ile Ser Lys Thr Ser Thr Thr Val Asp Leu Lys Met Thr
65 70 75 80

Ser Leu Thr Thr Glu Asp Thr Ala Thr Tyr Phe Cys Ala Arg Gly Arg
85 90 95

Phe Val Gly Tyr Thr Asn Ala Phe Asp Pro Trp Gly Pro Gly Thr Leu
100 105 110

Val Thr Val Ser Ser
        115

<210>  109
<211>  113
<212>  PRT
<213>  Oryctolagus cuniculus

<220>
<223>  SMBPL0002

<400>  109

Ala Gln Val Leu Thr Gln Thr Pro Ser Ser Val Ser Ala Ala Val Gly
1               5                   10                  15

Gly Thr Val Thr Ile Ser Cys Gln Ser Ser Gln Ser Val Trp Asn Asn
            20                  25                  30

Asn Tyr Leu Ser Trp Tyr Gln Gln Lys Pro Gly Gln Pro Pro Lys Leu
        35                  40                  45

Leu Ile Phe Asp Ala Ser Thr Leu Ala Ser Gly Val Pro Ser Arg Phe
    50                  55                  60

Ser Gly Arg Gly Ser Gly Thr Gln Phe Thr Leu Thr Ile Ser Gly Val
65                  70                  75                  80

Gln Cys Glu Asp Ala Ala Thr Tyr Tyr Cys His Gly Ser Tyr Ala Asn
                85                  90                  95

Ser Gly Trp Tyr Asp Asn Ala Phe Gly Gly Gly Thr Glu Val Val Val
            100                 105                 110

Lys

<210>  110
<211>  123
<212>  PRT
<213>  Oryctolagus cuniculus

<220>
<223>  SMBPH0089

<400>  110

Gln Ser Val Glu Glu Ser Gly Gly Arg Leu Val Thr Pro Gly Thr Pro
1               5                   10                  15

Leu Thr Leu Thr Cys Thr Val Ser Gly Phe Ser Leu Ser Ser Tyr Tyr
            20                  25                  30

Met Ser Trp Val Arg Gln Ala Pro Gly Glu Gly Leu Glu Tyr Ile Gly
35 40 45

Phe Ile Asn Thr Gly Gly Ser Ser Tyr Tyr Ala Pro Trp Ala Ile Gly
50 55 60

Arg Leu Thr Ile Ser Lys Thr Ser Thr Thr Val Asp Leu Lys Ile Thr
65 70 75 80

Ser Pro Thr Thr Glu Asp Thr Ala Thr Tyr Phe Cys Ala Arg Val Lys
85 90 95

Ser Tyr Val Asn Ser Asn Gly Tyr Phe Ile Phe Ser Arg Leu Asp Leu
100 105 110

Trp Gly Gln Gly Thr Leu Val Thr Val Ser Ser
115 120

<210> 111
<211> 111
<212> PRT
<213> Oryctolagus cuniculus

<220>
<223> SMBPL0089

<400> 111

Ala Gln Val Leu Thr Gln Thr Ala Ser Ser Val Ser Ala Ala Val Gly
1 5 10 15

Gly Thr Val Thr Ile Ser Cys Gln Ser Ser Lys Ser Val Tyr Asn Asn
20 25 30

Asn Phe Leu Ser Trp Tyr Gln Gln Lys Leu Gly Gln Pro Pro Lys Leu
35 40 45

Leu Ile Tyr Tyr Ala Ser Thr Leu Ala Ser Gly Val Pro Ser Arg Phe
50 55 60

Lys Gly Ser Gly Ser Gly Thr Gln Phe Thr Leu Thr Ile Ser Asp Leu
65 70 75 80

Glu Cys Asp Asp Ala Ala Thr Tyr Tyr Cys Ala Gly Gly Tyr Ser Gly
85 90 95

Ile Pro Ile Asn Ala Phe Gly Gly Gly Thr Glu Val Val Val Lys
100 105 110

<210> 112
<211> 117
<212> PRT
<213> Oryctolagus cuniculus

<220>
<223> SMBPH0104

<400> 112

Gln Ser Val Glu Glu Ser Gly Gly Arg Leu Val Thr Pro Gly Thr Pro
1 5 10 15

Leu Thr Leu Thr Cys Thr Val Ser Gly Ile Asp Leu Ser Ser Asn Ala
20 25 30

Met Ser Trp Val Arg Gln Ala Pro Gly Lys Gly Leu Glu Trp Ile Gly
35 40 45

Ala Ile Gly Gly Ser Gly Asp Thr Gly Tyr Ala Ser Trp Ala Asn Gly
50 55 60

Arg Phe Thr Val Ser Lys Thr Ser Thr Thr Val Asp Leu Lys Met Thr
65 70 75 80

Ser Leu Thr Ala Ala Asp Thr Ala Thr Tyr Phe Cys Val Arg His Ser
85 90 95

Val Gly Ala Ser Trp Trp Val Phe Asn Ile Trp Gly Pro Gly Thr Leu
100 105 110

Val Thr Val Ser Ser
115

<210> 113
<211> 112
<212> PRT
<213> Oryctolagus cuniculus

<220>
<223> SMBPL0104

<400> 113

Ala Gln Val Leu Thr Gln Thr Pro Ser Ser Val Ser Ala Ala Val Gly
1 5 10 15

Gly Thr Val Thr Ile Asn Cys Gln Ser Ser Gln Ser Val Tyr Ser Gly
20 25 30

Asn Phe Phe Ala Trp Phe Gln Gln Lys Pro Gly Gln Pro Pro Lys Leu
35 40 45

Leu Ile Tyr Asp Ala Ser Thr Leu Ala Ser Gly Val Pro Ser Arg Phe
50 55 60

Lys Gly Ser Gly Ser Gly Thr Gln Phe Thr Leu Thr Ile Ser Gly Val
65 70 75 80

Gln Cys Asp Asp Ala Ala Thr Tyr Tyr Cys Gln Gly Thr Tyr Tyr Asn
85 90 95

Ser Gly Trp Ser Asn Val Phe Gly Gly Gly Thr Glu Val Val Val Lys
100 105 110

<210> 114
<211> 125
<212> PRT
<213> Artificial Sequence

<220>
<223> Var_y VH

<400> 114

Glu Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Thr Tyr
20 25 30

Ala Met Asn Trp Val Arg Gln Ala Pro Gly Lys Gly Leu Glu Trp Val
35 40 45

Gly Arg Ile Arg Ser Lys Tyr Asn Asn Tyr Ala Thr Tyr Tyr Ala Asp
50 55 60

Ser Val Lys Asp Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Ala Arg His Gly Asn Phe Gly Asn Ser Tyr Val Ser Tyr Phe
100 105 110

Ala Tyr Trp Gly Gln Gly Thr Leu Val Thr Val Ser Ser
115 120 125

<210> 115
<211> 110
<212> PRT
<213> Artificial Sequence

<220>
<223>  Var_y VL

<400>  115

Asp Ile Gln Met Thr Gln Ser Pro Ser Ser Leu Ser Ala Ser Val Gly
1               5                   10                  15

Asp Arg Val Thr Ile Thr Cys Arg Ser Ser Thr Gly Ala Val Thr Thr
            20                  25                  30

Ser Asn Tyr Ala Asn Trp Val Gln Gln Lys Pro Gly Lys Ala Pro Lys
        35                  40                  45

Ala Leu Ile Gly Gly Thr Asn Lys Arg Ala Pro Gly Val Pro Ser Arg
    50                  55                  60

Phe Ser Gly Ser Leu Ile Gly Asp Lys Ala Thr Leu Thr Ile Ser Ser
65                  70                  75                  80

Leu Gln Pro Glu Asp Phe Ala Thr Tyr Tyr Cys Ala Leu Trp Tyr Ser
                85                  90                  95

Asn Leu Trp Val Phe Gly Gln Gly Thr Lys Val Glu Ile Lys
            100                 105                 110

<210>  116
<211>  125
<212>  PRT
<213>  Artificial Sequence

<220>
<223>  Var_z VH

<400>  116

Glu Val Gln Leu Val Glu Ser Gly Gly Gly Leu Val Gln Pro Gly Gly
1               5                   10                  15

Ser Leu Arg Leu Ser Cys Ala Ala Ser Gly Phe Thr Phe Ser Thr Tyr
            20                  25                  30

Ala Met Asn Trp Val Arg Gln Ala Pro Gly Lys Gly Leu Glu Trp Val
        35                  40                  45

Gly Arg Ile Arg Ser Lys Tyr Asn Asn Tyr Ala Thr Tyr Tyr Ala Asp
    50                  55                  60

Ser Val Lys Asp Arg Phe Thr Ile Ser Arg Asp Asp Ser Lys Asn Ser
65                  70                  75                  80

Leu Tyr Leu Gln Met Asn Ser Leu Lys Thr Glu Asp Thr Ala Val Tyr
85 90 95

Tyr Cys Ala Arg His Gly Asn Phe Gly Asn Ser Tyr Val Ser His Phe
100 105 110

Ala Tyr Trp Gly Gln Gly Thr Leu Val Thr Val Ser Ser
115 120 125

<210> 117
<211> 110
<212> PRT
<213> Artificial Sequence

<220>
<223> Var_zVL

<400> 117

Asp Ile Gln Met Thr Gln Ser Pro Ser Ser Leu Ser Ala Ser Val Gly
1 5 10 15

Asp Arg Val Thr Ile Thr Cys Arg Ser Ser Thr Gly Ala Val Thr Thr
20 25 30

Ser Asn Tyr Ala Asn Trp Val Gln Gln Lys Pro Gly Lys Ala Pro Lys
35 40 45

Gly Leu Ile Gly Gly Thr Asn Lys Arg Ala Pro Gly Val Pro Ser Arg
50 55 60

Phe Ser Gly Ser Leu Ile Gly Asp Lys Ala Thr Leu Thr Ile Ser Ser
65 70 75 80

Leu Gln Pro Glu Asp Phe Ala Thr Tyr Tyr Cys Ala Leu Trp Tyr Ser
85 90 95

Asn Leu Trp Val Phe Gly Gln Gly Thr Lys Val Glu Ile Lys
100 105 110

<210> 118
<211> 125
<212> PRT
<213> Artificial Sequence

<220>
<223> No110 (VH)

<400> 118

Glu Val Gln Leu Leu Glu Ser Gly Gly Gly Leu Val Gln Pro Gly Gly
1 5 10 15

Ser Leu Lys Leu Ser Cys Ala Ala Ser Gly Phe Thr Phe Asn Thr Tyr
20 25 30

Ala Met Asn Trp Val Arg Gln Ala Pro Gly Lys Gly Leu Glu Trp Val
35 40 45

Ala Arg Ile Arg Ser Lys Tyr Asn Asn Tyr Ala Thr Tyr Tyr Ala Asp
50 55 60

Ser Val Lys Asp Arg Phe Thr Ile Ser Arg Asp Asp Ser Lys Asn Thr
65 70 75 80

Ala Tyr Leu Gln Met Asn Asn Leu Lys Thr Glu Asp Thr Ala Val Tyr
85 90 95

Tyr Cys Val Arg His Gly Asn Phe Gly Asn Ser Tyr Val Ser Trp Phe
100 105 110

Ala Tyr Trp Gly Gln Gly Thr Leu Val Thr Val Ser Ser
115 120 125

<210> 119
<211> 109
<212> PRT
<213> Artificial Sequence

<220>
<223> No.168 (VL)

<400> 119

Glu Leu Val Val Thr Gln Glu Pro Ser Leu Thr Val Ser Pro Gly Gly
1 5 10 15

Thr Val Thr Leu Thr Cys Arg Ser Ser Thr Gly Ala Val Thr Thr Ser
20 25 30

Asn Tyr Ala Asn Trp Val Gln Gln Lys Pro Gly Gln Ala Pro Arg Gly
35 40 45

Leu Ile Gly Gly Thr Asn Lys Arg Ala Pro Gly Thr Pro Ala Arg Phe
50 55 60

Ser Gly Ser Leu Leu Gly Gly Lys Ala Ala Leu Thr Leu Ser Gly Val
65 70 75 80

Gln Pro Glu Asp Glu Ala Glu Tyr Tyr Cys Ala Leu Trp Tyr Ser Asn
85 90 95

Leu Trp Val Phe Gly Gly Gly Thr Lys Leu Thr Val Leu
100 105

<210> 120
<211> 125
<212> PRT
<213> Artificial Sequence

<220>
<223> I2CVH

<400> 120

Glu Val Gln Leu Val Glu Ser Gly Gly Gly Leu Val Gln Pro Gly Gly
1 5 10 15

Ser Leu Lys Leu Ser Cys Ala Ala Ser Gly Phe Thr Phe Asn Lys Tyr
20 25 30

Ala Met Asn Trp Val Arg Gln Ala Pro Gly Lys Gly Leu Glu Trp Val
35 40 45

Ala Arg Ile Arg Ser Lys Tyr Asn Asn Tyr Ala Thr Tyr Tyr Ala Asp
50 55 60

Ser Val Lys Asp Arg Phe Thr Ile Ser Arg Asp Asp Ser Lys Asn Thr
65 70 75 80

Ala Tyr Leu Gln Met Asn Asn Leu Lys Thr Glu Asp Thr Ala Val Tyr
85 90 95

Tyr Cys Val Arg His Gly Asn Phe Gly Asn Ser Tyr Ile Ser Tyr Trp
100 105 110

Ala Tyr Trp Gly Gln Gly Thr Leu Val Thr Val Ser Ser
115 120 125

<210> 121
<211> 109
<212> PRT
<213> Artificial Sequence

<220>
<223> I2CVL

<400> 121

Gln Thr Val Val Thr Gln Glu Pro Ser Leu Thr Val Ser Pro Gly Gly
1 5 10 15

Thr Val Thr Leu Thr Cys Gly Ser Ser Thr Gly Ala Val Thr Ser Gly
20 25 30

Asn Tyr Pro Asn Trp Val Gln Gln Lys Pro Gly Gln Ala Pro Arg Gly
35 40 45

Leu Ile Gly Gly Thr Lys Phe Leu Ala Pro Gly Thr Pro Ala Arg Phe
50 55 60

Ser Gly Ser Leu Leu Gly Gly Lys Ala Ala Leu Thr Leu Ser Gly Val
65 70 75 80

Gln Pro Glu Asp Glu Ala Glu Tyr Tyr Cys Val Leu Trp Tyr Ser Asn
85 90 95

Arg Trp Val Phe Gly Gly Gly Thr Lys Leu Thr Val Leu
100 105

<210> 122
<211> 112
<212> PRT
<213> Artificial Sequence

<220>
<223> GLS3000

<400> 122

Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

<210> 123
<211> 453
<212> PRT

<213> Artificial Sequence

<220>
<223> SC003H-G1T3L

<400> 123

Gln Val Gln Leu Val Glu Thr Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Gly Pro Tyr Cys Ser Ser Thr Ser Cys Tyr Leu Ser Pro Phe
100 105 110

Asp Tyr Trp Gly Gln Gly Thr Leu Val Thr Val Ser Ser Ala Ser Thr
115 120 125

Lys Gly Pro Ser Val Phe Pro Leu Ala Pro Ser Ser Lys Ser Thr Ser
130 135 140

Gly Gly Thr Ala Ala Leu Gly Cys Leu Val Lys Asp Tyr Phe Pro Glu
145 150 155 160

Pro Val Thr Val Ser Trp Asn Ser Gly Ala Leu Thr Ser Gly Val His
165 170 175

Thr Phe Pro Ala Val Leu Gln Ser Ser Gly Leu Tyr Ser Leu Ser Ser
180 185 190

Val Val Thr Val Pro Ser Ser Ser Leu Gly Thr Gln Thr Tyr Ile Cys
195 200 205

Asn Val Asn His Lys Pro Ser Asn Thr Lys Val Asp Lys Arg Val Glu
210 215 220

Pro Lys Ser Cys Asp Lys Thr His Thr Cys Pro Pro Cys Pro Ala Pro
225 230 235 240

Glu Leu Leu Gly Gly Pro Ser Val Phe Leu Phe Pro Pro Lys Pro Lys
245 250 255

Asp Thr Leu Met Ile Ser Arg Thr Pro Glu Val Thr Cys Val Val Val
260 265 270

Asp Val Ser His Glu Asp Pro Glu Val Lys Phe Asn Trp Tyr Val Asp
275 280 285

Gly Val Glu Val His Asn Ala Lys Thr Lys Pro Arg Glu Glu Gln Tyr
290 295 300

Asn Ser Thr Tyr Arg Val Val Ser Val Leu Thr Val Leu His Gln Asp
305 310 315 320

Trp Leu Asn Gly Lys Glu Tyr Lys Cys Lys Val Ser Asn Lys Ala Leu
325 330 335

Pro Ala Pro Ile Glu Lys Thr Ile Ser Lys Ala Lys Gly Gln Pro Arg
340 345 350

Glu Pro Gln Val Tyr Thr Leu Pro Pro Ser Arg Glu Glu Met Thr Lys
355 360 365

Asn Gln Val Ser Leu Thr Cys Leu Val Lys Gly Phe Tyr Pro Ser Asp
370 375 380

Ile Ala Val Glu Trp Glu Ser Asn Gly Gln Pro Glu Asn Asn Tyr Lys
385 390 395 400

Thr Thr Pro Pro Val Leu Asp Ser Asp Gly Ser Phe Phe Leu Tyr Ser
405 410 415

Lys Leu Thr Val Asp Lys Ser Arg Trp Gln Glu Gly Asn Val Phe Ser
420 425 430

Cys Ser Val Met His Glu Ala Leu His Asn His Tyr Thr Gln Lys Ser
435 440 445

Leu Ser Leu Ser Pro
450

<210> 124
<211> 216
<212> PRT

<213>  Artificial Sequence

<220>
<223>  SC003L-SCL3

<400>  124

```
Gln Ser Val Leu Thr Gln Pro Pro Ser Val Ser Ala Ala Pro Gly Gln
1               5                   10                  15

Lys Val Thr Ile Ser Cys Ser Gly Asn Thr Ser Asn Ile Gly Asn Asn
            20                  25                  30

Tyr Val Ser Trp Tyr Gln Gln Leu Pro Gly Thr Ala Pro Lys Leu Leu
        35                  40                  45

Ile Tyr Arg Asn Ser Asp Arg Pro Ser Gly Val Pro Asp Arg Phe Ser
    50                  55                  60

Gly Ser Lys Ser Gly Thr Ser Ala Ser Leu Ala Ile Thr Gly Leu Gln
65                  70                  75                  80

Ala Glu Asp Glu Ala Asp Tyr Tyr Cys Gln Ser Tyr Asp Thr Ser Leu
                85                  90                  95

Thr Ala Val Ile Phe Gly Gly Gly Thr Lys Leu Thr Val Leu Gly Gln
            100                 105                 110

Pro Lys Ala Ala Pro Ser Val Thr Leu Phe Pro Pro Ser Ser Glu Glu
        115                 120                 125

Leu Gln Ala Asn Arg Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
        195                 200                 205

Thr Val Ala Pro Thr Glu Cys Ser
    210                 215
```

<210> 125
<211> 455
<212> PRT
<213> Artificial Sequence

<220>
<223> SC032H-G1T3L

<400> 125

Gln Val Gln Leu Gln Gln Ser Gly Ala Glu Val Lys Lys Pro Gly Ser
1 5 10 15

Ser Val Lys Val Ser Cys Lys Ala Ser Gly Gly Thr Phe Ser Ser Tyr
20 25 30

Ala Ile Ser Trp Val Arg Gln Ala Pro Gly Gln Gly Leu Glu Trp Met
35 40 45

Gly Arg Ile Ile Pro Ile Leu Gly Ile Ala Asn Tyr Ala Gln Lys Phe
50 55 60

Gln Gly Arg Val Thr Ile Thr Ala Asp Lys Ser Thr Ser Thr Ala Tyr
65 70 75 80

Met Glu Leu Ser Ser Leu Arg Ser Glu Asp Thr Ala Val Tyr Tyr Cys
85 90 95

Ala Arg Glu Gly Leu Thr Pro Tyr Tyr Tyr Gly Ser Glu Leu Gly Asn
100 105 110

Gly Met Asp Val Trp Gly Gln Gly Thr Thr Val Thr Val Ser Ser Ala
115 120 125

Ser Thr Lys Gly Pro Ser Val Phe Pro Leu Ala Pro Ser Ser Lys Ser
130 135 140

Thr Ser Gly Gly Thr Ala Ala Leu Gly Cys Leu Val Lys Asp Tyr Phe
145 150 155 160

Pro Glu Pro Val Thr Val Ser Trp Asn Ser Gly Ala Leu Thr Ser Gly
165 170 175

Val His Thr Phe Pro Ala Val Leu Gln Ser Ser Gly Leu Tyr Ser Leu
180 185 190

Ser Ser Val Val Thr Val Pro Ser Ser Ser Leu Gly Thr Gln Thr Tyr
195 200 205

Ile Cys Asn Val Asn His Lys Pro Ser Asn Thr Lys Val Asp Lys Arg

```
        210                 215                 220

Val Glu Pro Lys Ser Cys Asp Lys Thr His Thr Cys Pro Pro Cys Pro
225                 230                 235                 240

Ala Pro Glu Leu Leu Gly Gly Pro Ser Val Phe Leu Phe Pro Pro Lys
                245                 250                 255

Pro Lys Asp Thr Leu Met Ile Ser Arg Thr Pro Glu Val Thr Cys Val
            260                 265                 270

Val Val Asp Val Ser His Glu Asp Pro Glu Val Lys Phe Asn Trp Tyr
        275                 280                 285

Val Asp Gly Val Glu Val His Asn Ala Lys Thr Lys Pro Arg Glu Glu
    290                 295                 300

Gln Tyr Asn Ser Thr Tyr Arg Val Val Ser Val Leu Thr Val Leu His
305                 310                 315                 320

Gln Asp Trp Leu Asn Gly Lys Glu Tyr Lys Cys Lys Val Ser Asn Lys
                325                 330                 335

Ala Leu Pro Ala Pro Ile Glu Lys Thr Ile Ser Lys Ala Lys Gly Gln
            340                 345                 350

Pro Arg Glu Pro Gln Val Tyr Thr Leu Pro Pro Ser Arg Glu Glu Met
        355                 360                 365

Thr Lys Asn Gln Val Ser Leu Thr Cys Leu Val Lys Gly Phe Tyr Pro
    370                 375                 380

Ser Asp Ile Ala Val Glu Trp Glu Ser Asn Gly Gln Pro Glu Asn Asn
385                 390                 395                 400

Tyr Lys Thr Thr Pro Pro Val Leu Asp Ser Asp Gly Ser Phe Phe Leu
                405                 410                 415

Tyr Ser Lys Leu Thr Val Asp Lys Ser Arg Trp Gln Glu Gly Asn Val
            420                 425                 430

Phe Ser Cys Ser Val Met His Glu Ala Leu His Asn His Tyr Thr Gln
        435                 440                 445

Lys Ser Leu Ser Leu Ser Pro
    450                 455
```

<210> 126
<211> 214
<212> PRT
<213> Artificial Sequence

<220>
<223> SC032K-kp

<400> 126

Asp Ile Gln Leu Thr Gln Ser Pro Ser Ser Leu Ser Thr Phe Val Gly
1 5 10 15

Asp Arg Val Thr Ile Thr Cys Arg Thr Ser Gln Asn Ile Asn Ser Tyr
20 25 30

Leu Asn Trp Tyr Gln Gln Lys Pro Gly Lys Ala Pro Lys Leu Leu Ile
35 40 45

Ser Thr Thr Ser Asn Leu Gln Ser Gly Val Pro Ser Arg Phe Ser Gly
50 55 60

Ser Gly Ser Gly Thr Asp Phe Thr Leu Thr Ile Asn Asn Leu Gln Pro
65 70 75 80

Glu Asp Phe Ala Ile Tyr Tyr Cys Gln Gln Thr Lys Ser Phe Pro Leu
85 90 95

Thr Phe Gly Gly Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

210

<210> 127
<211> 459
<212> PRT
<213> Artificial Sequence

<220>
<223> SC034H-G1T3L

<400> 127

Gln Val Gln Leu Gln Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Gly Ile Ser Trp Val Arg Gln Ala Pro Gly Gln Gly Leu Glu Trp Met
35 40 45

Gly Trp Ile Ser Ala Tyr Asn Gly Asn Thr Asn Tyr Ala Gln Lys Leu
50 55 60

Gln Gly Arg Val Thr Met Thr Thr Asp Thr Ser Thr Ser Thr Ala Tyr
65 70 75 80

Met Glu Leu Arg Ser Leu Arg Ser Asp Asp Thr Ala Val Tyr Tyr Cys
85 90 95

Ala Arg Asp Tyr His Ser Thr Arg Pro Asp Trp Pro Pro Phe Pro Tyr
100 105 110

Tyr Tyr Tyr Tyr Gly Met Asp Val Trp Gly Gln Gly Thr Thr Val Thr
115 120 125

Val Ser Ser Ala Ser Thr Lys Gly Pro Ser Val Phe Pro Leu Ala Pro
130 135 140

Ser Ser Lys Ser Thr Ser Gly Gly Thr Ala Ala Leu Gly Cys Leu Val
145 150 155 160

Lys Asp Tyr Phe Pro Glu Pro Val Thr Val Ser Trp Asn Ser Gly Ala
165 170 175

Leu Thr Ser Gly Val His Thr Phe Pro Ala Val Leu Gln Ser Ser Gly
180 185 190

Leu Tyr Ser Leu Ser Ser Val Val Thr Val Pro Ser Ser Ser Leu Gly
195 200 205

```
Thr Gln Thr Tyr Ile Cys Asn Val Asn His Lys Pro Ser Asn Thr Lys
    210                 215                 220

Val Asp Lys Arg Val Glu Pro Lys Ser Cys Asp Lys Thr His Thr Cys
225                 230                 235                 240

Pro Pro Cys Pro Ala Pro Glu Leu Leu Gly Gly Pro Ser Val Phe Leu
                245                 250                 255

Phe Pro Pro Lys Pro Lys Asp Thr Leu Met Ile Ser Arg Thr Pro Glu
            260                 265                 270

Val Thr Cys Val Val Val Asp Val Ser His Glu Asp Pro Glu Val Lys
        275                 280                 285

Phe Asn Trp Tyr Val Asp Gly Val Glu Val His Asn Ala Lys Thr Lys
    290                 295                 300

Pro Arg Glu Glu Gln Tyr Asn Ser Thr Tyr Arg Val Val Ser Val Leu
305                 310                 315                 320

Thr Val Leu His Gln Asp Trp Leu Asn Gly Lys Glu Tyr Lys Cys Lys
                325                 330                 335

Val Ser Asn Lys Ala Leu Pro Ala Pro Ile Glu Lys Thr Ile Ser Lys
            340                 345                 350

Ala Lys Gly Gln Pro Arg Glu Pro Gln Val Tyr Thr Leu Pro Pro Ser
        355                 360                 365

Arg Glu Glu Met Thr Lys Asn Gln Val Ser Leu Thr Cys Leu Val Lys
    370                 375                 380

Gly Phe Tyr Pro Ser Asp Ile Ala Val Glu Trp Glu Ser Asn Gly Gln
385                 390                 395                 400

Pro Glu Asn Asn Tyr Lys Thr Thr Pro Pro Val Leu Asp Ser Asp Gly
                405                 410                 415

Ser Phe Phe Leu Tyr Ser Lys Leu Thr Val Asp Lys Ser Arg Trp Gln
            420                 425                 430

Glu Gly Asn Val Phe Ser Cys Ser Val Met His Glu Ala Leu His Asn
        435                 440                 445

His Tyr Thr Gln Lys Ser Leu Ser Leu Ser Pro
```

450 455

<210> 128
<211> 216
<212> PRT
<213> Artificial Sequence

<220>
<223> SC034L-hl

<400> 128

Gln Ser Val Leu Thr Gln Pro His Ser Val Ser Glu Ser Pro Gly Lys
1 5 10 15

Thr Val Thr Ile Ser Cys Thr Arg Ser Ser Gly Arg Ile Ala Ala Asn
20 25 30

Asn Val Gln Trp Tyr Gln Gln Arg Pro Gly Ser Ala Pro Thr Thr Ile
35 40 45

Ile Tyr Glu Asp Asn Arg Arg Pro Ser Gly Val Pro Asp Arg Phe Ser
50 55 60

Gly Ser Ile Asp Gly Ser Ser Asn Ser Ala Ser Leu Thr Ile Ser Gly
65 70 75 80

Leu Lys Pro Glu Asp Glu Ala Asp Tyr Tyr Cys Gln Ser Tyr Val Ser
85 90 95

Thr Thr Val Val Phe Gly Gly Gly Thr Lys Leu Thr Val Leu Gly Gln
100 105 110

Pro Lys Ala Ala Pro Ser Val Thr Leu Phe Pro Pro Ser Ser Glu Glu
115 120 125

Leu Gln Ala Asn Lys Ala Thr Leu Val Cys Leu Ile Ser Asp Phe Tyr
130 135 140

Pro Gly Ala Val Thr Val Ala Trp Lys Ala Asp Ser Ser Pro Val Lys
145 150 155 160

Ala Gly Val Glu Thr Thr Thr Pro Ser Lys Gln Ser Asn Asn Lys Tyr
165 170 175

Ala Ala Ser Ser Tyr Leu Ser Leu Thr Pro Glu Gln Trp Lys Ser His
180 185 190

Arg Ser Tyr Ser Cys Gln Val Thr His Glu Gly Ser Thr Val Glu Lys
195 200 205

Thr Val Ala Pro Thr Glu Cys Ser
210 215

<210> 129
<211> 456
<212> PRT
<213> Artificial Sequence

<220>
<223> SC044H-G1T3L

<400> 129

Gln Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Gly Tyr
20 25 30

Tyr Met His Trp Val Arg Gln Ala Pro Gly Gln Gly Leu Glu Trp Met
35 40 45

Gly Trp Ile Asn Pro Asn Ser Gly Gly Thr Asn Tyr Ala Gln Lys Phe
50 55 60

Gln Gly Arg Val Thr Met Thr Arg Asp Thr Ser Ile Ser Thr Ala Tyr
65 70 75 80

Met Glu Leu Ser Arg Leu Arg Ser Asp Asp Thr Ala Val Tyr Tyr Cys
85 90 95

Ala Arg Ala Ser Tyr Lys Gly Tyr Glu Tyr Ser Val Gln Gly Gly Tyr
100 105 110

Tyr Gly Met Asp Val Trp Gly Gln Gly Thr Thr Val Thr Val Ser Ser
115 120 125

Ala Ser Thr Lys Gly Pro Ser Val Phe Pro Leu Ala Pro Ser Ser Lys
130 135 140

Ser Thr Ser Gly Gly Thr Ala Ala Leu Gly Cys Leu Val Lys Asp Tyr
145 150 155 160

Phe Pro Glu Pro Val Thr Val Ser Trp Asn Ser Gly Ala Leu Thr Ser
165 170 175

Gly Val His Thr Phe Pro Ala Val Leu Gln Ser Ser Gly Leu Tyr Ser
180 185 190

```
Leu Ser Ser Val Val Thr Val Pro Ser Ser Ser Leu Gly Thr Gln Thr
        195                 200                 205

Tyr Ile Cys Asn Val Asn His Lys Pro Ser Asn Thr Lys Val Asp Lys
    210                 215                 220

Arg Val Glu Pro Lys Ser Cys Asp Lys Thr His Thr Cys Pro Pro Cys
225                 230                 235                 240

Pro Ala Pro Glu Leu Leu Gly Gly Pro Ser Val Phe Leu Phe Pro Pro
                245                 250                 255

Lys Pro Lys Asp Thr Leu Met Ile Ser Arg Thr Pro Glu Val Thr Cys
            260                 265                 270

Val Val Val Asp Val Ser His Glu Asp Pro Glu Val Lys Phe Asn Trp
        275                 280                 285

Tyr Val Asp Gly Val Glu Val His Asn Ala Lys Thr Lys Pro Arg Glu
    290                 295                 300

Glu Gln Tyr Asn Ser Thr Tyr Arg Val Val Ser Val Leu Thr Val Leu
305                 310                 315                 320

His Gln Asp Trp Leu Asn Gly Lys Glu Tyr Lys Cys Lys Val Ser Asn
                325                 330                 335

Lys Ala Leu Pro Ala Pro Ile Glu Lys Thr Ile Ser Lys Ala Lys Gly
            340                 345                 350

Gln Pro Arg Glu Pro Gln Val Tyr Thr Leu Pro Pro Ser Arg Glu Glu
        355                 360                 365

Met Thr Lys Asn Gln Val Ser Leu Thr Cys Leu Val Lys Gly Phe Tyr
    370                 375                 380

Pro Ser Asp Ile Ala Val Glu Trp Glu Ser Asn Gly Gln Pro Glu Asn
385                 390                 395                 400

Asn Tyr Lys Thr Thr Pro Pro Val Leu Asp Ser Asp Gly Ser Phe Phe
                405                 410                 415

Leu Tyr Ser Lys Leu Thr Val Asp Lys Ser Arg Trp Gln Glu Gly Asn
            420                 425                 430

Val Phe Ser Cys Ser Val Met His Glu Ala Leu His Asn His Tyr Thr
        435                 440                 445
```

Gln Lys Ser Leu Ser Leu Ser Pro
450 455

<210> 130
<211> 217
<212> PRT
<213> Artificial Sequence

<220>
<223> SC044L-SCL10

<400> 130

Gln Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Asp Val His Trp Tyr Gln Gln Leu Pro Gly Thr Ala Pro Lys Leu
35 40 45

Leu Ile Tyr Gly Asn Ser Asn Arg Pro Ser Gly Val Pro Asp Arg Phe
50 55 60

Ser Gly Ser Lys Ser Gly Thr Ser Ala Ser Leu Ala Ile Thr Gly Leu
65 70 75 80

Gln Ala Glu Asp Glu Ala Asp Tyr Tyr Cys Gln Ser Tyr Asp Asp Ser
85 90 95

Arg Ser Ala Val Val Phe Gly Gly Gly Thr Lys Leu Thr Val Leu Gly
100 105 110

Gln Pro Lys Ala Ala Pro Ser Val Thr Leu Phe Pro Pro Ser Ser Glu
115 120 125

Glu Leu Gln Ala Asn Lys Ala Thr Leu Val Cys Leu Ile Ser Asp Phe
130 135 140

Tyr Pro Gly Ala Val Thr Val Ala Trp Lys Ala Asp Ser Ser Pro Val
145 150 155 160

Lys Ala Gly Val Glu Thr Thr Thr Pro Ser Lys Gln Ser Asn Asn Lys
165 170 175

Tyr Ala Ala Ser Ser Tyr Leu Ser Leu Thr Pro Glu Gln Trp Lys Ser
180 185 190

His Arg Ser Tyr Ser Cys Gln Val Thr His Glu Gly Ser Thr Val Glu
195 200 205

Lys Thr Val Ala Pro Thr Glu Cys Ser
210 215

<210> 131
<211> 451
<212> PRT
<213> Artificial Sequence

<220>
<223> SC045H-G1T3L

<400> 131

Gln Val Gln Leu Val Glu Ser Gly Gly Gly Leu Val Gln Pro Gly Gly
1 5 10 15

Ser Leu Arg Leu Ser Cys Ala Ala Ser Gly Phe Thr Phe Ser Asp His
20 25 30

Tyr Met Asp Trp Val Arg Gln Ala Pro Gly Lys Gly Leu Glu Trp Val
35 40 45

Gly Arg Thr Arg Asn Lys Ala Asn Ser Tyr Thr Thr Glu Tyr Ala Ala
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Ala Arg Gly Pro Thr Leu Ala Tyr Ile Gly Tyr Met Asp Tyr
100 105 110

Trp Gly Gln Gly Thr Leu Val Thr Val Ser Ser Ala Ser Thr Lys Gly
115 120 125

Pro Ser Val Phe Pro Leu Ala Pro Ser Ser Lys Ser Thr Ser Gly Gly
130 135 140

Thr Ala Ala Leu Gly Cys Leu Val Lys Asp Tyr Phe Pro Glu Pro Val
145 150 155 160

Thr Val Ser Trp Asn Ser Gly Ala Leu Thr Ser Gly Val His Thr Phe
165 170 175

```
Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430
```

Val Met His Glu Ala Leu His Asn His Tyr Thr Gln Lys Ser Leu Ser
435 440 445

Leu Ser Pro
450

<210> 132
<211> 217
<212> PRT
<213> Artificial Sequence

<220>
<223> SC045L-SCL10

<400> 132

Gln Ser Val Leu Thr Gln Pro Pro Ser Val Ser Gly Ala Pro Gly Gln
1 5 10 15

Arg Val Thr Ile Ser Cys Thr Gly Ser Ser Ser Asn Ile Gly Ala Gly
20 25 30

Tyr Asp Val His Trp Tyr Gln Gln Leu Pro Gly Thr Ala Pro Lys Leu
35 40 45

Leu Ile Tyr Gly Asn Ser Asn Arg Pro Ser Gly Val Pro Asp Arg Phe
50 55 60

Ser Gly Ser Lys Ser Gly Thr Ser Ala Ser Leu Ala Ile Thr Gly Leu
65 70 75 80

Gln Ala Glu Asp Glu Ala Asp Tyr Tyr Cys Gln Ser Tyr Asp Gly Ser
85 90 95

Leu Ser Val Val Val Phe Gly Gly Gly Thr Lys Leu Thr Val Leu Gly
100 105 110

Gln Pro Lys Ala Ala Pro Ser Val Thr Leu Phe Pro Pro Ser Ser Glu
115 120 125

Glu Leu Gln Ala Asn Lys Ala Thr Leu Val Cys Leu Ile Ser Asp Phe
130 135 140

Tyr Pro Gly Ala Val Thr Val Ala Trp Lys Ala Asp Ser Ser Pro Val
145 150 155 160

Lys Ala Gly Val Glu Thr Thr Thr Pro Ser Lys Gln Ser Asn Asn Lys
165 170 175

Tyr Ala Ala Ser Ser Tyr Leu Ser Leu Thr Pro Glu Gln Trp Lys Ser
180 185 190

His Arg Ser Tyr Ser Cys Gln Val Thr His Glu Gly Ser Thr Val Glu
195 200 205

Lys Thr Val Ala Pro Thr Glu Cys Ser
210 215

<210> 133
<211> 449
<212> PRT
<213> Artificial Sequence

<220>
<223> SC048H-G1T3L

<400> 133

Gln Val Gln Leu Gln Glu Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Lys Gly Tyr Ser Tyr Gly Pro Gly Tyr Phe Phe Asp Tyr Trp Gly
100 105 110

Gln Gly Thr Leu Val Thr Val Ser Ser Ala Ser Thr Lys Gly Pro Ser
115 120 125

Val Phe Pro Leu Ala Pro Ser Ser Lys Ser Thr Ser Gly Gly Thr Ala
130 135 140

Ala Leu Gly Cys Leu Val Lys Asp Tyr Phe Pro Glu Pro Val Thr Val
145 150 155 160

Ser Trp Asn Ser Gly Ala Leu Thr Ser Gly Val His Thr Phe Pro Ala

165 170 175

Val Leu Gln Ser Ser Gly Leu Tyr Ser Leu Ser Ser Val Val Thr Val
180 185 190

Pro Ser Ser Ser Leu Gly Thr Gln Thr Tyr Ile Cys Asn Val Asn His
195 200 205

Lys Pro Ser Asn Thr Lys Val Asp Lys Arg Val Glu Pro Lys Ser Cys
210 215 220

Asp Lys Thr His Thr Cys Pro Pro Cys Pro Ala Pro Glu Leu Leu Gly
225 230 235 240

Gly Pro Ser Val Phe Leu Phe Pro Pro Lys Pro Lys Asp Thr Leu Met
245 250 255

Ile Ser Arg Thr Pro Glu Val Thr Cys Val Val Val Asp Val Ser His
260 265 270

Glu Asp Pro Glu Val Lys Phe Asn Trp Tyr Val Asp Gly Val Glu Val
275 280 285

His Asn Ala Lys Thr Lys Pro Arg Glu Glu Gln Tyr Asn Ser Thr Tyr
290 295 300

Arg Val Val Ser Val Leu Thr Val Leu His Gln Asp Trp Leu Asn Gly
305 310 315 320

Lys Glu Tyr Lys Cys Lys Val Ser Asn Lys Ala Leu Pro Ala Pro Ile
325 330 335

Glu Lys Thr Ile Ser Lys Ala Lys Gly Gln Pro Arg Glu Pro Gln Val
340 345 350

Tyr Thr Leu Pro Pro Ser Arg Glu Glu Met Thr Lys Asn Gln Val Ser
355 360 365

Leu Thr Cys Leu Val Lys Gly Phe Tyr Pro Ser Asp Ile Ala Val Glu
370 375 380

Trp Glu Ser Asn Gly Gln Pro Glu Asn Asn Tyr Lys Thr Thr Pro Pro
385 390 395 400

Val Leu Asp Ser Asp Gly Ser Phe Phe Leu Tyr Ser Lys Leu Thr Val
405 410 415

Asp Lys Ser Arg Trp Gln Glu Gly Asn Val Phe Ser Cys Ser Val Met
420 425 430

His Glu Ala Leu His Asn His Tyr Thr Gln Lys Ser Leu Ser Leu Ser
435 440 445

Pro

<210> 134
<211> 212
<212> PRT
<213> Artificial Sequence

<220>
<223> SC048L-SCL11

<400> 134

Ser Ser Glu Leu Thr Gln Pro Pro Ser Val Ser Val Ser Pro Gly Gln
1 5 10 15

Thr Ala Asn Ile Ser Cys Ser Ala Asp Lys Leu Gly Gly Lys Tyr Val
20 25 30

Ser Trp Tyr Gln Gln Lys Pro Gly Gln Ser Pro Val Leu Val Met Tyr
35 40 45

Gln Asp Lys Lys Arg Pro Ser Gly Ile Pro Glu Arg Leu Ser Gly Ser
50 55 60

Gly Ser Gly Asn Thr Ala Thr Leu Thr Ile Ser Gly Thr Gln Ala Met
65 70 75 80

Asp Glu Ala Thr Tyr Tyr Cys Gln Thr Trp Asp Gly Arg Ser Leu Phe
85 90 95

Phe Gly Gly Gly Thr Arg Leu Thr Val Leu Ser Gln Pro Lys Ala Ala
100 105 110

Pro Ser Val Thr Leu Phe Pro Pro Ser Ser Glu Glu Leu Gln Ala Asn
115 120 125

Lys Ala Thr Leu Val Cys Leu Ile Ser Asp Phe Tyr Pro Gly Ala Val
130 135 140

Thr Val Ala Trp Lys Ala Asp Ser Ser Pro Val Lys Ala Gly Val Glu
145 150 155 160

Thr Thr Thr Pro Ser Lys Gln Ser Asn Asn Lys Tyr Ala Ala Ser Ser

165 170 175

Tyr Leu Ser Leu Thr Pro Glu Gln Trp Lys Ser His Arg Ser Tyr Ser
180 185 190

Cys Gln Val Thr His Glu Gly Ser Thr Val Glu Lys Thr Val Ala Pro
195 200 205

Thr Glu Cys Ser
210

<210> 135
<211> 450
<212> PRT
<213> Artificial Sequence

<220>
<223> CE115HA000-F760mnN17

<400> 135

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Val His Tyr Gly Ala Tyr Tyr Gly Val Asp Ala Trp
100 105 110

Gly Gln Gly Thr Thr Val Thr Val Ser Ser Ala Ser Thr Lys Gly Pro
115 120 125

Ser Val Phe Pro Leu Ala Pro Ser Ser Lys Ser Thr Ser Gly Gly Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Gln Thr Tyr Ile Cys Asn Val Asn
195 200 205

His Lys Pro Ser Asn Thr Lys Val Asp Lys Lys Val Glu Pro Lys Ser
210 215 220

Cys Asp Lys Thr His Thr Cys Pro Pro Cys Pro Ala Pro Glu Leu Arg
225 230 235 240

Gly Gly Pro Lys Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

His Glu Asp Pro Glu Val Lys Phe Asn Trp Tyr Val Asp Gly Val Glu
275 280 285

Val His Asn Ala Lys Thr Lys Pro Arg Glu Glu Gln Tyr Ala Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Ala Leu Pro Ala Pro
325 330 335

Ile Glu Lys Thr Ile Ser Lys Ala Lys Gly Gln Pro Arg Glu Pro Gln
340 345 350

Val Tyr Thr Leu Pro Pro Ser Arg Glu Glu Met Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Tyr Leu Asp Ser Asp Gly Ser Phe Phe Leu Tyr Ser Lys Leu Thr

405 410 415

Val Asp Lys Ser Arg Trp Gln Gln Gly Asn Val Phe Ser Cys Ser Val
420 425 430

Met His Glu Ala Leu His Asn His Tyr Thr Gln Glu Ser Leu Ser Leu
435 440 445

Ser Pro
450

<210> 136
<211> 453
<212> PRT
<213> Artificial Sequence

<220>
<223> SC003H-F760mnP17

<400> 136

Gln Val Gln Leu Val Glu Thr Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Gly Pro Tyr Cys Ser Ser Thr Ser Cys Tyr Leu Ser Pro Phe
100 105 110

Asp Tyr Trp Gly Gln Gly Thr Leu Val Thr Val Ser Ser Ala Ser Thr
115 120 125

Lys Gly Pro Ser Val Phe Pro Leu Ala Pro Ser Ser Lys Ser Thr Ser
130 135 140

Gly Gly Thr Ala Ala Leu Gly Cys Leu Val Lys Asp Tyr Phe Pro Glu
145 150 155 160

```
Pro Val Thr Val Ser Trp Asn Ser Gly Ala Leu Thr Ser Gly Val His
                165                 170                 175

Thr Phe Pro Ala Val Leu Gln Ser Ser Gly Leu Tyr Ser Leu Ser Ser
            180                 185                 190

Val Val Thr Val Pro Ser Ser Ser Leu Gly Thr Gln Thr Tyr Ile Cys
        195                 200                 205

Asn Val Asn His Lys Pro Ser Asn Thr Lys Val Asp Lys Lys Val Glu
    210                 215                 220

Pro Lys Ser Cys Asp Lys Thr His Thr Cys Pro Pro Cys Pro Ala Pro
225                 230                 235                 240

Glu Leu Arg Gly Gly Pro Lys Val Phe Leu Phe Pro Pro Lys Pro Lys
                245                 250                 255

Asp Thr Leu Met Ile Ser Arg Thr Pro Glu Val Thr Cys Val Val Val
            260                 265                 270

Asp Val Ser His Glu Asp Pro Glu Val Lys Phe Asn Trp Tyr Val Asp
        275                 280                 285

Gly Val Glu Val His Asn Ala Lys Thr Lys Pro Arg Glu Glu Gln Tyr
    290                 295                 300

Ala Ser Thr Tyr Arg Val Val Ser Val Leu Thr Val Leu His Gln Asp
305                 310                 315                 320

Trp Leu Asn Gly Lys Glu Tyr Lys Cys Lys Val Ser Asn Lys Ala Leu
                325                 330                 335

Pro Ala Pro Ile Glu Lys Thr Ile Ser Lys Ala Lys Gly Gln Pro Arg
            340                 345                 350

Glu Pro Gln Val Tyr Thr Leu Pro Pro Ser Arg Lys Glu Met Thr Lys
        355                 360                 365

Asn Gln Val Ser Leu Thr Cys Leu Val Lys Gly Phe Tyr Pro Ser Asp
    370                 375                 380

Ile Ala Val Glu Trp Glu Ser Asn Gly Gln Pro Glu Asn Asn Tyr Lys
385                 390                 395                 400

Thr Thr Pro Pro Tyr Leu Asp Ser Asp Gly Ser Phe Phe Leu Tyr Ser
```

405 410 415

Lys Leu Thr Val Asp Lys Ser Arg Trp Gln Gln Gly Asn Val Phe Ser
420 425 430

Cys Ser Val Met His Glu Ala Leu His Asn His Tyr Thr Gln Lys Ser
435 440 445

Leu Ser Leu Ser Pro
450

<210> 137
<211> 444
<212> PRT
<213> Artificial Sequence

<220>
<223> IC17HdK-F760mnN17

<400> 137

Gln Val Gln Leu Gln Gln Ser Gly Pro Gln Leu Val Arg Pro Gly Ala
1 5 10 15

Ser Val Lys Ile Ser Cys Lys Ala Ser Gly Tyr Ser Phe Thr Ser Tyr
20 25 30

Trp Met His Trp Val Asn Gln Arg Pro Gly Gln Gly Leu Glu Trp Ile
35 40 45

Gly Met Ile Asp Pro Ser Tyr Ser Glu Thr Arg Leu Asn Gln Lys Phe
50 55 60

Lys Asp Lys Ala Thr Leu Thr Val Asp Lys Ser Ser Ser Thr Ala Tyr
65 70 75 80

Met Gln Leu Ser Ser Pro Thr Ser Glu Asp Ser Ala Val Tyr Tyr Cys
85 90 95

Ala Leu Tyr Gly Asn Tyr Phe Asp Tyr Trp Gly Gln Gly Thr Thr Leu
100 105 110

Thr Val Ser Ser Ala Ser Thr Lys Gly Pro Ser Val Phe Pro Leu Ala
115 120 125

Pro Ser Ser Lys Ser Thr Ser Gly Gly Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Lys Thr His Thr
210 215 220

Cys Pro Pro Cys Pro Ala Pro Glu Leu Arg Gly Gly Pro Lys Val Phe
225 230 235 240

Leu Phe Pro Pro Lys Pro Lys Asp Thr Leu Met Ile Ser Arg Thr Pro
245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Ala Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Pro Ala Pro Ile Glu Lys Thr Ile Ser
325 330 335

Lys Ala Lys Gly Gln Pro Arg Glu Pro Gln Val Tyr Thr Leu Pro Pro
340 345 350

Ser Arg Glu Glu Met Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Tyr Leu Asp Ser Asp
385 390 395 400

Gly Ser Phe Phe Leu Tyr Ser Lys Leu Thr Val Asp Lys Ser Arg Trp

405 410 415

Gln Gln Gly Asn Val Phe Ser Cys Ser Val Met His Glu Ala Leu His
420 425 430

Asn His Tyr Thr Gln Glu Ser Leu Ser Leu Ser Pro
435 440

<210> 138
<211> 214
<212> PRT
<213> Artificial Sequence

<220>
<223> IC17L-k0a

<400> 138

Asp Ile Gln Met Thr Gln Ser Ser Ser Ser Phe Ser Val Ser Leu Gly
1 5 10 15

Asp Arg Val Thr Ile Thr Cys Lys Ala Ser Glu Asp Ile Tyr Asn Arg
20 25 30

Leu Ala Trp Tyr Gln Gln Lys Pro Gly Asn Ala Pro Arg Leu Leu Ile
35 40 45

Ser Gly Ala Thr Ser Leu Glu Thr Gly Val Pro Ser Arg Phe Ser Gly
50 55 60

Ser Gly Ser Gly Lys Asp Tyr Thr Leu Ser Ile Thr Ser Leu Gln Thr
65 70 75 80

Glu Asp Val Ala Thr Tyr Tyr Cys Gln Gln Tyr Trp Ser Thr Pro Tyr
85 90 95

Thr Phe Gly Gly Gly Thr Lys Leu Glu Val Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 139
<211> 444
<212> PRT
<213> Artificial Sequence

<220>
<223> IC17HdK-F760mnP17

<400> 139

Gln Val Gln Leu Gln Gln Ser Gly Pro Gln Leu Val Arg Pro Gly Ala
1 5 10 15

Ser Val Lys Ile Ser Cys Lys Ala Ser Gly Tyr Ser Phe Thr Ser Tyr
20 25 30

Trp Met His Trp Val Asn Gln Arg Pro Gly Gln Gly Leu Glu Trp Ile
35 40 45

Gly Met Ile Asp Pro Ser Tyr Ser Glu Thr Arg Leu Asn Gln Lys Phe
50 55 60

Lys Asp Lys Ala Thr Leu Thr Val Asp Lys Ser Ser Ser Thr Ala Tyr
65 70 75 80

Met Gln Leu Ser Ser Pro Thr Ser Glu Asp Ser Ala Val Tyr Tyr Cys
85 90 95

Ala Leu Tyr Gly Asn Tyr Phe Asp Tyr Trp Gly Gln Gly Thr Thr Leu
100 105 110

Thr Val Ser Ser Ala Ser Thr Lys Gly Pro Ser Val Phe Pro Leu Ala
115 120 125

Pro Ser Ser Lys Ser Thr Ser Gly Gly Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

Gly Leu Tyr Ser Leu Ser Ser Val Val Thr Val Pro Ser Ser Ser Leu
180 185 190

Gly Thr Gln Thr Tyr Ile Cys Asn Val Asn His Lys Pro Ser Asn Thr
195 200 205

Lys Val Asp Lys Lys Val Glu Pro Lys Ser Cys Asp Lys Thr His Thr
210 215 220

Cys Pro Pro Cys Pro Ala Pro Glu Leu Arg Gly Gly Pro Lys Val Phe
225 230 235 240

Leu Phe Pro Pro Lys Pro Lys Asp Thr Leu Met Ile Ser Arg Thr Pro
245 250 255

Glu Val Thr Cys Val Val Val Asp Val Ser His Glu Asp Pro Glu Val
260 265 270

Lys Phe Asn Trp Tyr Val Asp Gly Val Glu Val His Asn Ala Lys Thr
275 280 285

Lys Pro Arg Glu Glu Gln Tyr Ala Ser Thr Tyr Arg Val Val Ser Val
290 295 300

Leu Thr Val Leu His Gln Asp Trp Leu Asn Gly Lys Glu Tyr Lys Cys
305 310 315 320

Lys Val Ser Asn Lys Ala Leu Pro Ala Pro Ile Glu Lys Thr Ile Ser
325 330 335

Lys Ala Lys Gly Gln Pro Arg Glu Pro Gln Val Tyr Thr Leu Pro Pro
340 345 350

Ser Arg Lys Glu Met Thr Lys Asn Gln Val Ser Leu Thr Cys Leu Val
355 360 365

Lys Gly Phe Tyr Pro Ser Asp Ile Ala Val Glu Trp Glu Ser Asn Gly
370 375 380

Gln Pro Glu Asn Asn Tyr Lys Thr Thr Pro Pro Tyr Leu Asp Ser Asp
385 390 395 400

Gly Ser Phe Phe Leu Tyr Ser Lys Leu Thr Val Asp Lys Ser Arg Trp
405 410 415

Gln Gln Gly Asn Val Phe Ser Cys Ser Val Met His Glu Ala Leu His
420 425 430

Asn His Tyr Thr Gln Lys Ser Leu Ser Leu Ser Pro
435 440

<210> 140
<211> 450
<212> PRT
<213> Artificial Sequence

<220>
<223> CE115HA000-BS03a

<400> 140

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Val His Tyr Gly Ala Tyr Tyr Gly Val Asp Ala Trp
100 105 110

Gly Gln Gly Thr Thr Val Thr Val Ser Ser Ala Ser Thr Lys Gly Pro
115 120 125

Ser Val Phe Pro Leu Ala Pro Ser Ser Arg Ser Thr Ser Glu Ser Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr
180 185 190

Val Pro Ser Ser Ser Leu Gly Thr Lys Thr Tyr Thr Cys Asn Val Asp
195 200 205

His Lys Pro Ser Asn Thr Lys Val Asp Lys Arg Val Glu Pro Lys Ser
210 215 220

Cys Asp Lys Thr His Thr Cys Pro Pro Cys Pro Ala Pro Glu Leu Arg
225 230 235 240

Arg Gly Pro Lys Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

Gln Glu Asp Pro Glu Val Gln Phe Asn Trp Tyr Val Asp Gly Val Glu
275 280 285

Val His Asn Ala Lys Thr Lys Pro Arg Glu Glu Gln Phe Ala Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Gly Leu Pro Ser Ser
325 330 335

Ile Glu Lys Thr Ile Ser Lys Ala Lys Gly Gln Pro Arg Glu Pro Gln
340 345 350

Val Tyr Thr Leu Pro Pro Ser Arg Glu Glu Met Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Tyr Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Glu Ser Leu Ser Leu
435 440 445

Ser Pro
450

<210> 141
<211> 450
<212> PRT
<213> Artificial Sequence

<220>
<223> CE115HA056-BS03a

<400> 141

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Ala His Tyr Gly Ala Tyr Tyr Gly Val Asp Ala Trp
100 105 110

Gly Gln Gly Thr Thr Val Thr Val Ser Ser Ala Ser Thr Lys Gly Pro
115 120 125

Ser Val Phe Pro Leu Ala Pro Ser Ser Arg Ser Thr Ser Glu Ser Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

```
Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Glu Pro Lys Ser
    210                 215                 220

Cys Asp Lys Thr His Thr Cys Pro Pro Cys Pro Ala Pro Glu Leu Arg
225                 230                 235                 240

Arg Gly Pro Lys Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

Gln Glu Asp Pro Glu Val Gln Phe Asn Trp Tyr Val Asp Gly Val Glu
        275                 280                 285

Val His Asn Ala Lys Thr Lys Pro Arg Glu Glu Gln Phe Ala Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gly Leu Pro Ser Ser
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Glu Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Tyr Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430
```

```
Met His Glu Ala Leu His Asn His Tyr Thr Gln Glu Ser Leu Ser Leu
        435                 440                 445

Ser Pro
    450


<210>  142
<211>  450
<212>  PRT
<213>  Artificial Sequence

<220>
<223>  CE115HA146-BS03a

<400>  142

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asn Ala
            20                  25                  30

Trp Met His Trp Val Arg Gln Ala Pro Gly Lys Gly Leu Glu Trp Val
        35                  40                  45

Ala Gln Ile Lys Ala Lys Ser Asn Asn Tyr Ala Thr Tyr Tyr Ala Glu
    50                  55                  60

Ser Val Lys Gly Arg Phe Thr Ile Ser Arg Asp Asp Ser Lys Asn Ser
65                  70                  75                  80

Leu Tyr Leu Gln Met Asn Ser Leu Lys Thr Glu Asp Thr Ala Val Tyr
                85                  90                  95

Tyr Cys Arg Tyr Val Ala Tyr Gly Ala Tyr Tyr Gly Val Asp Ala Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Ser Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175
```

Ala Val Leu Gln Ser Ser Gly Leu Tyr Ser Leu Ser Ser Val Val Thr
180 185 190

Val Pro Ser Ser Ser Leu Gly Thr Lys Thr Tyr Thr Cys Asn Val Asp
195 200 205

His Lys Pro Ser Asn Thr Lys Val Asp Lys Arg Val Glu Pro Lys Ser
210 215 220

Cys Asp Lys Thr His Thr Cys Pro Pro Cys Pro Ala Pro Glu Leu Arg
225 230 235 240

Arg Gly Pro Lys Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

Gln Glu Asp Pro Glu Val Gln Phe Asn Trp Tyr Val Asp Gly Val Glu
275 280 285

Val His Asn Ala Lys Thr Lys Pro Arg Glu Glu Gln Phe Ala Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Gly Leu Pro Ser Ser
325 330 335

Ile Glu Lys Thr Ile Ser Lys Ala Lys Gly Gln Pro Arg Glu Pro Gln
340 345 350

Val Tyr Thr Leu Pro Pro Ser Arg Glu Glu Met Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Tyr Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Glu Ser Leu Ser Leu
435 440 445

Ser Pro
450

<210> 143
<211> 444
<212> PRT
<213> Artificial Sequence

<220>
<223> IC17Hdk-BS03a

<400> 143

Gln Val Gln Leu Gln Gln Ser Gly Pro Gln Leu Val Arg Pro Gly Ala
1 5 10 15

Ser Val Lys Ile Ser Cys Lys Ala Ser Gly Tyr Ser Phe Thr Ser Tyr
20 25 30

Trp Met His Trp Val Asn Gln Arg Pro Gly Gln Gly Leu Glu Trp Ile
35 40 45

Gly Met Ile Asp Pro Ser Tyr Ser Glu Thr Arg Leu Asn Gln Lys Phe
50 55 60

Lys Asp Lys Ala Thr Leu Thr Val Asp Lys Ser Ser Ser Thr Ala Tyr
65 70 75 80

Met Gln Leu Ser Ser Pro Thr Ser Glu Asp Ser Ala Val Tyr Tyr Cys
85 90 95

Ala Leu Tyr Gly Asn Tyr Phe Asp Tyr Trp Gly Gln Gly Thr Thr Leu
100 105 110

Thr Val Ser Ser Ala Ser Thr Lys Gly Pro Ser Val Phe Pro Leu Ala
115 120 125

Pro Ser Ser Arg Ser Thr Ser Glu Ser Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

```
Gly Leu Tyr Ser Leu Ser Ser Val Val Thr Val Pro Ser Ser Ser Leu
            180                 185                 190

Gly Thr Lys Thr Tyr Thr Cys Asn Val Asp His Lys Pro Ser Asn Thr
        195                 200                 205

Lys Val Asp Lys Arg Val Glu Pro Lys Ser Cys Asp Lys Thr His Thr
    210                 215                 220

Cys Pro Pro Cys Pro Ala Pro Glu Leu Arg Arg Gly Pro Lys Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Ala Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Tyr Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430
```

Asn His Tyr Thr Gln Glu Ser Leu Ser Leu Ser Pro
435 440

<210> 144
<211> 446
<212> PRT
<213> Artificial Sequence

<220>
<223> CLA0028VH-BS03b

<400> 144

Gln Ser Leu Glu Glu Ser Gly Gly Asp Leu Val Lys Pro Gly Ala Ser
1 5 10 15

Leu Thr Leu Thr Cys Thr Ala Ser Gly Phe Ser Phe Ser Ser Ser Tyr
20 25 30

Trp Ile Tyr Trp Val Arg Gln Ala Pro Gly Lys Gly Leu Glu Trp Ile
35 40 45

Ala Cys Ile Tyr Ala Gly Ser Thr Ser Ser Thr Tyr Tyr Ala Ser Trp
50 55 60

Ala Lys Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Lys Gly Gly Pro Asp Asp Ala Phe His Ser Trp Gly Pro Gly Thr
100 105 110

Leu Val Thr Val Ser Ser Ala Ser Thr Lys Gly Pro Ser Val Phe Pro
115 120 125

Leu Ala Pro Ser Ser Arg Ser Thr Ser Glu Ser Thr Ala Ala Leu Gly
130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

```
Ser Leu Gly Thr Lys Thr Tyr Thr Cys Asn Val Asp His Lys Pro Ser
        195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Arg Arg Gly Pro Lys
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Ala Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Lys Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Tyr Leu Asp
385                 390                 395                 400

Ser Asp Gly Ser Phe Phe Leu Tyr Ser Lys Leu Thr Val Asp Lys Ser
                405                 410                 415

Arg Trp Gln Gln Gly Asn Val Phe Ser Cys Ser Val Met His Glu Ala
            420                 425                 430

Leu His Asn His Tyr Thr Gln Lys Ser Leu Ser Leu Ser Pro
        435                 440                 445
```

<210> 145
<211> 458
<212> PRT
<213> Artificial Sequence

<220>
<223> CE115H-BS03bFLAG

<400> 145

Gln Val Gln Leu Val Glu Ser Gly Gly Gly Val Val Gln Pro Gly Arg
1               5                   10                  15

Ser Leu Arg Leu Ser Cys Ala Ala Ser Gly Phe Thr Phe Ser Asn Ala
            20                  25                  30

Trp Met His Trp Val Arg Gln Ala Pro Gly Lys Gly Leu Glu Trp Val
        35                  40                  45

Ala Gln Ile Lys Ala Lys Ser Asn Asn Tyr Ala Thr Tyr Tyr Ala Glu
    50                  55                  60

Ser Val Lys Gly Arg Phe Thr Ile Ser Arg Asp Asp Ser Lys Asn Ser
65                  70                  75                  80

Leu Tyr Leu Gln Met Asn Ser Leu Lys Thr Glu Asp Thr Ala Val Tyr
                85                  90                  95

Tyr Cys Arg Tyr Val His Tyr Gly Ala Tyr Tyr Gly Val Asp Ala Trp
            100                 105                 110

Gly Gln Gly Thr Thr Val Thr Val Ser Ser Ala Ser Thr Lys Gly Pro
        115                 120                 125

Ser Val Phe Pro Leu Ala Pro Ser Ser Arg Ser Thr Ser Glu Ser Thr
    130                 135                 140

Ala Ala Leu Gly Cys Leu Val Lys Asp Tyr Phe Pro Glu Pro Val Thr
145                 150                 155                 160

Val Ser Trp Asn Ser Gly Ala Leu Thr Ser Gly Val His Thr Phe Pro
                165                 170                 175

Ala Val Leu Gln Ser Ser Gly Leu Tyr Ser Leu Ser Ser Val Val Thr
            180                 185                 190

Val Pro Ser Ser Ser Leu Gly Thr Lys Thr Tyr Thr Cys Asn Val Asp
        195                 200                 205

His Lys Pro Ser Asn Thr Lys Val Asp Lys Arg Val Glu Pro Lys Ser
    210                 215                 220

Cys Asp Lys Thr His Thr Cys Pro Pro Cys Pro Ala Pro Glu Leu Arg
225                 230                 235                 240

Arg Gly Pro Lys Val Phe Leu Phe Pro Pro Lys Pro Lys Asp Thr Leu
                245                 250                 255

Met Ile Ser Arg Thr Pro Glu Val Thr Cys Val Val Val Asp Val Ser
            260                 265                 270

Gln Glu Asp Pro Glu Val Gln Phe Asn Trp Tyr Val Asp Gly Val Glu
        275                 280                 285

Val His Asn Ala Lys Thr Lys Pro Arg Glu Glu Gln Phe Ala Ser Thr
    290                 295                 300

Tyr Arg Val Val Ser Val Leu Thr Val Leu His Gln Asp Trp Leu Asn
305                 310                 315                 320

Gly Lys Glu Tyr Lys Cys Lys Val Ser Asn Lys Gly Leu Pro Ser Ser
                325                 330                 335

Ile Glu Lys Thr Ile Ser Lys Ala Lys Gly Gln Pro Arg Glu Pro Gln
            340                 345                 350

Val Tyr Thr Leu Pro Pro Ser Arg Lys Glu Met Thr Lys Asn Gln Val
        355                 360                 365

Ser Leu Thr Cys Leu Val Lys Gly Phe Tyr Pro Ser Asp Ile Ala Val
    370                 375                 380

Glu Trp Glu Ser Asn Gly Gln Pro Glu Asn Asn Tyr Lys Thr Thr Pro
385                 390                 395                 400

Pro Tyr Leu Asp Ser Asp Gly Ser Phe Phe Leu Tyr Ser Lys Leu Thr
                405                 410                 415

Val Asp Lys Ser Arg Trp Gln Gln Gly Asn Val Phe Ser Cys Ser Val
            420                 425                 430

Met His Glu Ala Leu His Asn His Tyr Thr Gln Lys Ser Leu Ser Leu
        435                 440                 445

Ser Pro Asp Tyr Lys Asp Asp Asp Asp Lys
    450                 455

<210> 146
<211> 219
<212> PRT
<213> Artificial Sequence

<220>
<223> CE115L-SK1

<400> 146

Asp Ile Val Met Thr Gln Ser Pro Leu Ser Leu Pro Val Thr Pro Gly
1 5 10 15

Glu Pro Ala Ser Ile Ser Cys Arg Ser Ser Gln Ser Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu
180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 147
<211> 455
<212> PRT
<213> Artificial Sequence

<220>
<223> AE3.20H-BS03aHis

<400> 147

Glu Val Lys Leu Val Glu Ser Gly Gly Gly Leu Val Lys Pro Gly Gly
1 5 10 15

Ser Leu Lys Leu Ser Cys Ala Ala Ser Gly Phe Thr Phe Asn Ser Tyr
20 25 30

Thr Met Ser Trp Val Arg Gln Thr Pro Ala Lys Arg Leu Glu Trp Val
35 40 45

Val Thr Ile Ser Ser Gly Gly Gly Arg Thr Tyr Tyr Pro Asp Ser Val
50 55 60

Lys Gly Arg Phe Thr Ile Ser Arg Asp Asn Ala Arg Asn Thr Leu Tyr
65 70 75 80

Leu Gln Met Ser Ser Leu Arg Ser Glu Asp Thr Ala Met Tyr Tyr Cys
85 90 95

Ile Arg Gly Asp Tyr Arg Tyr Asp Gly Phe Ala Tyr Trp Gly Gln Gly
100 105 110

Thr Leu Val Thr Val Ser Thr Ala Ser Thr Lys Gly Pro Ser Val Phe
115 120 125

Pro Leu Ala Pro Ser Ser Arg Ser Thr Ser Glu Ser Thr Ala Ala Leu
130 135 140

Gly Cys Leu Val Lys Asp Tyr Phe Pro Glu Pro Val Thr Val Ser Trp
145 150 155 160

Asn Ser Gly Ala Leu Thr Ser Gly Val His Thr Phe Pro Ala Val Leu
165 170 175

Gln Ser Ser Gly Leu Tyr Ser Leu Ser Ser Val Val Thr Val Pro Ser
180 185 190

Ser Ser Leu Gly Thr Lys Thr Tyr Thr Cys Asn Val Asp His Lys Pro
195 200 205

Ser Asn Thr Lys Val Asp Lys Arg Val Glu Pro Lys Ser Cys Asp Lys
210 215 220

Thr His Thr Cys Pro Pro Cys Pro Ala Pro Glu Leu Arg Arg Gly Pro
225 230 235 240

Lys Val Phe Leu Phe Pro Pro Lys Pro Lys Asp Thr Leu Met Ile Ser
245 250 255

Arg Thr Pro Glu Val Thr Cys Val Val Val Asp Val Ser Gln Glu Asp
260 265 270

Pro Glu Val Gln Phe Asn Trp Tyr Val Asp Gly Val Glu Val His Asn
275 280 285

Ala Lys Thr Lys Pro Arg Glu Glu Gln Phe Ala Ser Thr Tyr Arg Val
290 295 300

Val Ser Val Leu Thr Val Leu His Gln Asp Trp Leu Asn Gly Lys Glu
305 310 315 320

Tyr Lys Cys Lys Val Ser Asn Lys Gly Leu Pro Ser Ser Ile Glu Lys
325 330 335

Thr Ile Ser Lys Ala Lys Gly Gln Pro Arg Glu Pro Gln Val Tyr Thr
340 345 350

Leu Pro Pro Ser Arg Glu Glu Met Thr Lys Asn Gln Val Ser Leu Thr
355 360 365

Cys Leu Val Lys Gly Phe Tyr Pro Ser Asp Ile Ala Val Glu Trp Glu
370 375 380

Ser Asn Gly Gln Pro Glu Asn Asn Tyr Lys Thr Thr Pro Pro Tyr Leu
385 390 395 400

Asp Ser Asp Gly Ser Phe Phe Leu Tyr Ser Lys Leu Thr Val Asp Lys
405 410 415

Ser Arg Trp Gln Gln Gly Asn Val Phe Ser Cys Ser Val Met His Glu
420 425 430

Ala Leu His Asn His Tyr Thr Gln Glu Ser Leu Ser Leu Ser Pro His
435 440 445

His His His His His His His
450 455

<210> 148
<211> 214
<212> PRT
<213> Artificial Sequence

<220>
<223> AE3.20L-SK1

<400> 148

Asp Ile Gln Met Thr Gln Ser Pro Ala Ser Leu Ser Ala Ser Val Gly
1 5 10 15

Glu Thr Val Thr Ile Thr Cys Arg Ala Ser Glu Asn Ile Asp Ser Tyr
20 25 30

Leu Ala Trp Tyr Gln Gln Lys Gln Gly Lys Ser Pro Gln Leu Leu Val
35 40 45

Tyr Ala Ser Thr Leu Leu Val Asp Gly Val Pro Ser Arg Phe Ser Gly
50 55 60

Ser Arg Ser Gly Thr Gln Phe Ser Leu Lys Ile Asn Ser Leu Gln Ser
65 70 75 80

Glu Asp Val Ala Arg Tyr Tyr Cys Gln His Tyr Tyr Ser Ile Pro Tyr
85 90 95

Thr Phe Gly Ser Gly Thr Lys Leu Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 149
<211> 458
<212> PRT
<213> Artificial Sequence

<220>
<223> CE115V95AH-BS03bFLAG

<400> 149

Gln Val Gln Leu Val Glu Ser Gly Gly Gly Val Val Gln Pro Gly Arg
1 5 10 15

Ser Leu Arg Leu Ser Cys Ala Ala Ser Gly Phe Thr Phe Ser Asn Ala
20 25 30

Trp Met His Trp Val Arg Gln Ala Pro Gly Lys Gly Leu Glu Trp Val
35 40 45

Ala Gln Ile Lys Ala Lys Ser Asn Asn Tyr Ala Thr Tyr Tyr Ala Glu
50 55 60

Ser Val Lys Gly Arg Phe Thr Ile Ser Arg Asp Asp Ser Lys Asn Ser
65 70 75 80

Leu Tyr Leu Gln Met Asn Ser Leu Lys Thr Glu Asp Thr Ala Val Tyr
85 90 95

Tyr Cys Arg Tyr Ala His Tyr Gly Ala Tyr Tyr Gly Val Asp Ala Trp
100 105 110

Gly Gln Gly Thr Thr Val Thr Val Ser Ser Ala Ser Thr Lys Gly Pro
115 120 125

Ser Val Phe Pro Leu Ala Pro Ser Ser Arg Ser Thr Ser Glu Ser Thr
130 135 140

Ala Ala Leu Gly Cys Leu Val Lys Asp Tyr Phe Pro Glu Pro Val Thr
145 150 155 160

Val Ser Trp Asn Ser Gly Ala Leu Thr Ser Gly Val His Thr Phe Pro
165 170 175

Ala Val Leu Gln Ser Ser Gly Leu Tyr Ser Leu Ser Ser Val Val Thr

180 185 190

Val Pro Ser Ser Ser Leu Gly Thr Lys Thr Tyr Thr Cys Asn Val Asp
195 200 205

His Lys Pro Ser Asn Thr Lys Val Asp Lys Arg Val Glu Pro Lys Ser
210 215 220

Cys Asp Lys Thr His Thr Cys Pro Pro Cys Pro Ala Pro Glu Leu Arg
225 230 235 240

Arg Gly Pro Lys Val Phe Leu Phe Pro Pro Lys Pro Lys Asp Thr Leu
245 250 255

Met Ile Ser Arg Thr Pro Glu Val Thr Cys Val Val Val Asp Val Ser
260 265 270

Gln Glu Asp Pro Glu Val Gln Phe Asn Trp Tyr Val Asp Gly Val Glu
275 280 285

Val His Asn Ala Lys Thr Lys Pro Arg Glu Glu Gln Phe Ala Ser Thr
290 295 300

Tyr Arg Val Val Ser Val Leu Thr Val Leu His Gln Asp Trp Leu Asn
305 310 315 320

Gly Lys Glu Tyr Lys Cys Lys Val Ser Asn Lys Gly Leu Pro Ser Ser
325 330 335

Ile Glu Lys Thr Ile Ser Lys Ala Lys Gly Gln Pro Arg Glu Pro Gln
340 345 350

Val Tyr Thr Leu Pro Pro Ser Arg Lys Glu Met Thr Lys Asn Gln Val
355 360 365

Ser Leu Thr Cys Leu Val Lys Gly Phe Tyr Pro Ser Asp Ile Ala Val
370 375 380

Glu Trp Glu Ser Asn Gly Gln Pro Glu Asn Asn Tyr Lys Thr Thr Pro
385 390 395 400

Pro Tyr Leu Asp Ser Asp Gly Ser Phe Phe Leu Tyr Ser Lys Leu Thr
405 410 415

Val Asp Lys Ser Arg Trp Gln Gln Gly Asn Val Phe Ser Cys Ser Val
420 425 430

Met His Glu Ala Leu His Asn His Tyr Thr Gln Lys Ser Leu Ser Leu
        435                 440                 445

Ser Pro Asp Tyr Lys Asp Asp Asp Asp Lys
    450                 455

<210>  150
<211>  451
<212>  PRT
<213>  Artificial Sequence

<220>
<223>  GCH065H-BS03aHis

<400>  150

Gln Val Gln Leu Val Gln Ser Gly Ala Glu Val Lys Lys Pro Gly Ala
1               5                   10                  15

Ser Val Thr Val Ser Cys Lys Ala Ser Gly Tyr Thr Phe Thr Asp Tyr
            20                  25                  30

Glu Met His Trp Ile Arg Gln Pro Pro Gly Glu Gly Leu Glu Trp Ile
        35                  40                  45

Gly Ala Ile Asp Gly Pro Thr Pro Asp Thr Ala Tyr Ser Glu Lys Phe
    50                  55                  60

Lys Gly Arg Val Thr Leu Thr Ala Asp Lys Ser Thr Ser Thr Ala Tyr
65                  70                  75                  80

Met Glu Leu Ser Ser Leu Thr Ser Glu Asp Thr Ala Val Tyr Tyr Cys
                85                  90                  95

Thr Arg Phe Tyr Ser Tyr Thr Tyr Trp Gly Gln Gly Thr Leu Val Thr
            100                 105                 110

Val Ser Ser Ala Ser Thr Lys Gly Pro Ser Val Phe Pro Leu Ala Pro
        115                 120                 125

Ser Ser Arg Ser Thr Ser Glu Ser Thr Ala Ala Leu Gly Cys Leu Val
    130                 135                 140

Lys Asp Tyr Phe Pro Glu Pro Val Thr Val Ser Trp Asn Ser Gly Ala
145                 150                 155                 160

Leu Thr Ser Gly Val His Thr Phe Pro Ala Val Leu Gln Ser Ser Gly
                165                 170                 175

Leu Tyr Ser Leu Ser Ser Val Val Thr Val Pro Ser Ser Ser Leu Gly

180 185 190

Thr Lys Thr Tyr Thr Cys Asn Val Asp His Lys Pro Ser Asn Thr Lys
195 200 205

Val Asp Lys Arg Val Glu Pro Lys Ser Cys Asp Lys Thr His Thr Cys
210 215 220

Pro Pro Cys Pro Ala Pro Glu Leu Arg Arg Gly Pro Lys Val Phe Leu
225 230 235 240

Phe Pro Pro Lys Pro Lys Asp Thr Leu Met Ile Ser Arg Thr Pro Glu
245 250 255

Val Thr Cys Val Val Val Asp Val Ser Gln Glu Asp Pro Glu Val Gln
260 265 270

Phe Asn Trp Tyr Val Asp Gly Val Glu Val His Asn Ala Lys Thr Lys
275 280 285

Pro Arg Glu Glu Gln Phe Ala Ser Thr Tyr Arg Val Val Ser Val Leu
290 295 300

Thr Val Leu His Gln Asp Trp Leu Asn Gly Lys Glu Tyr Lys Cys Lys
305 310 315 320

Val Ser Asn Lys Gly Leu Pro Ser Ser Ile Glu Lys Thr Ile Ser Lys
325 330 335

Ala Lys Gly Gln Pro Arg Glu Pro Gln Val Tyr Thr Leu Pro Pro Ser
340 345 350

Arg Glu Glu Met Thr Lys Asn Gln Val Ser Leu Thr Cys Leu Val Lys
355 360 365

Gly Phe Tyr Pro Ser Asp Ile Ala Val Glu Trp Glu Ser Asn Gly Gln
370 375 380

Pro Glu Asn Asn Tyr Lys Thr Thr Pro Pro Tyr Leu Asp Ser Asp Gly
385 390 395 400

Ser Phe Phe Leu Tyr Ser Lys Leu Thr Val Asp Lys Ser Arg Trp Gln
405 410 415

Gln Gly Asn Val Phe Ser Cys Ser Val Met His Glu Ala Leu His Asn
420 425 430

His Tyr Thr Gln Glu Ser Leu Ser Leu Ser Pro His His His His His
435 440 445

His His His
450

<210> 151
<211> 219
<212> PRT
<213> Artificial Sequence

<220>
<223> TR01L0011-SK1

<400> 151

Asp Ile Val Met Thr Gln Ser Pro Leu Ser Leu Pro Val Thr Pro Gly
1 5 10 15

Glu Pro Ala Ser Ile Ser Cys Arg Ser Ser Gln Pro Leu Val His Ser
20 25 30

Asn Arg Asn Thr Tyr Leu His Trp Tyr Gln Gln Lys Pro Gly Gln Ala
35 40 45

Pro Arg Leu Leu Ile Tyr Lys Val Ser Asn Arg Phe Ser Gly Val Pro
50 55 60

Asp Arg Phe Ser Gly Ser Gly Ser Gly Thr Asp Phe Thr Leu Lys Ile
65 70 75 80

Ser Arg Val Glu Ala Glu Asp Val Gly Val Tyr Tyr Cys Gly Gln Gly
85 90 95

Thr Gln Val Pro Tyr Thr Phe Gly Gln Gly Thr Lys Leu Glu Ile Lys
100 105 110

Arg Thr Val Ala Ala Pro Ser Val Phe Ile Phe Pro Pro Ser Asp Glu
115 120 125

Gln Leu Lys Ser Gly Thr Ala Ser Val Val Cys Leu Leu Asn Asn Phe
130 135 140

Tyr Pro Arg Glu Ala Lys Val Gln Trp Lys Val Asp Asn Ala Leu Gln
145 150 155 160

Ser Gly Asn Ser Gln Glu Ser Val Thr Glu Gln Asp Ser Lys Asp Ser
165 170 175

Thr Tyr Ser Leu Ser Ser Thr Leu Thr Leu Ser Lys Ala Asp Tyr Glu

180 185 190

Lys His Lys Val Tyr Ala Cys Glu Val Thr His Gln Gly Leu Ser Ser
195 200 205

Pro Val Thr Lys Ser Phe Asn Arg Gly Glu Cys
210 215

<210> 152
<211> 452
<212> PRT
<213> Artificial Sequence

<220>
<223> cKLHH-BS03aHis

<400> 152

Gln Val Gln Leu Gln Gln Ser Gly Pro Gln Leu Val Arg Pro Gly Ala
1 5 10 15

Ser Val Lys Ile Ser Cys Lys Ala Ser Gly Tyr Ser Phe Thr Ser Tyr
20 25 30

Trp Met His Trp Val Asn Gln Arg Pro Gly Gln Gly Leu Glu Trp Ile
35 40 45

Gly Met Ile Asp Pro Ser Tyr Ser Glu Thr Arg Leu Asn Gln Lys Phe
50 55 60

Lys Asp Lys Ala Thr Leu Thr Val Asp Lys Ser Ser Ser Thr Ala Tyr
65 70 75 80

Met Gln Leu Ser Ser Pro Thr Ser Glu Asp Ser Ala Val Tyr Tyr Cys
85 90 95

Ala Leu Tyr Gly Asn Tyr Phe Asp Tyr Trp Gly Gln Gly Thr Thr Leu
100 105 110

Thr Val Ser Ser Ala Ser Thr Lys Gly Pro Ser Val Phe Pro Leu Ala
115 120 125

Pro Ser Ser Arg Ser Thr Ser Glu Ser Thr Ala Ala Leu Gly Cys Leu
130 135 140

Val Lys Asp Tyr Phe Pro Glu Pro Val Thr Val Ser Trp Asn Ser Gly
145 150 155 160

Ala Leu Thr Ser Gly Val His Thr Phe Pro Ala Val Leu Gln Ser Ser
165 170 175

```
Gly Leu Tyr Ser Leu Ser Ser Val Val Thr Val Pro Ser Ser Ser Leu
            180                 185                 190

Gly Thr Lys Thr Tyr Thr Cys Asn Val Asp His Lys Pro Ser Asn Thr
        195                 200                 205

Lys Val Asp Lys Arg Val Glu Pro Lys Ser Cys Asp Lys Thr His Thr
    210                 215                 220

Cys Pro Pro Cys Pro Ala Pro Glu Leu Arg Arg Gly Pro Lys Val Phe
225                 230                 235                 240

Leu Phe Pro Pro Lys Pro Lys Asp Thr Leu Met Ile Ser Arg Thr Pro
                245                 250                 255

Glu Val Thr Cys Val Val Val Asp Val Ser Gln Glu Asp Pro Glu Val
            260                 265                 270

Gln Phe Asn Trp Tyr Val Asp Gly Val Glu Val His Asn Ala Lys Thr
        275                 280                 285

Lys Pro Arg Glu Glu Gln Phe Ala Ser Thr Tyr Arg Val Val Ser Val
    290                 295                 300

Leu Thr Val Leu His Gln Asp Trp Leu Asn Gly Lys Glu Tyr Lys Cys
305                 310                 315                 320

Lys Val Ser Asn Lys Gly Leu Pro Ser Ser Ile Glu Lys Thr Ile Ser
                325                 330                 335

Lys Ala Lys Gly Gln Pro Arg Glu Pro Gln Val Tyr Thr Leu Pro Pro
            340                 345                 350

Ser Arg Glu Glu Met Thr Lys Asn Gln Val Ser Leu Thr Cys Leu Val
        355                 360                 365

Lys Gly Phe Tyr Pro Ser Asp Ile Ala Val Glu Trp Glu Ser Asn Gly
    370                 375                 380

Gln Pro Glu Asn Asn Tyr Lys Thr Thr Pro Pro Tyr Leu Asp Ser Asp
385                 390                 395                 400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
```

420 425 430

Asn His Tyr Thr Gln Glu Ser Leu Ser Leu Ser Pro His His His His
435 440 445

His His His His
450

<210> 153
<211> 214
<212> PRT
<213> Artificial Sequence

<220>
<223> KLHL-k0

<400> 153

Asp Ile Gln Met Thr Gln Ser Ser Ser Ser Phe Ser Val Ser Leu Gly
1 5 10 15

Asp Arg Val Thr Ile Thr Cys Lys Ala Ser Glu Asp Ile Tyr Asn Arg
20 25 30

Leu Ala Trp Tyr Gln Gln Lys Pro Gly Asn Ala Pro Arg Leu Leu Ile
35 40 45

Ser Gly Ala Thr Ser Leu Glu Thr Gly Val Pro Ser Arg Phe Ser Gly
50 55 60

Ser Gly Ser Gly Lys Asp Tyr Thr Leu Ser Ile Thr Ser Leu Gln Thr
65 70 75 80

Glu Asp Val Ala Thr Tyr Tyr Cys Gln Gln Tyr Trp Ser Thr Pro Tyr
85 90 95

Thr Phe Gly Gly Gly Thr Lys Leu Glu Val Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 154
<211> 454
<212> PRT
<213> Artificial Sequence

<220>
<223> CLA0028H-BS03bFLAG

<400> 154

Gln Ser Leu Glu Glu Ser Gly Gly Asp Leu Val Lys Pro Gly Ala Ser
1 5 10 15

Leu Thr Leu Thr Cys Thr Ala Ser Gly Phe Ser Phe Ser Ser Ser Tyr
20 25 30

Trp Ile Tyr Trp Val Arg Gln Ala Pro Gly Lys Gly Leu Glu Trp Ile
35 40 45

Ala Cys Ile Tyr Ala Gly Ser Thr Ser Ser Thr Tyr Tyr Ala Ser Trp
50 55 60

Ala Lys Gly Arg Phe Thr Ile Ser Lys Thr Ser Ser Thr Thr Val Thr
65 70 75 80

Leu Gln Met Thr Ser Leu Thr Ala Ala Asp Thr Ala Thr Tyr Phe Cys
85 90 95

Ala Lys Gly Gly Pro Asp Asp Ala Phe His Ser Trp Gly Pro Gly Thr
100 105 110

Leu Val Thr Val Ser Ser Ala Ser Thr Lys Gly Pro Ser Val Phe Pro
115 120 125

Leu Ala Pro Ser Ser Arg Ser Thr Ser Glu Ser Thr Ala Ala Leu Gly
130 135 140

Cys Leu Val Lys Asp Tyr Phe Pro Glu Pro Val Thr Val Ser Trp Asn
145 150 155 160

```
Ser Gly Ala Leu Thr Ser Gly Val His Thr Phe Pro Ala Val Leu Gln
                165                 170                 175

Ser Ser Gly Leu Tyr Ser Leu Ser Ser Val Val Thr Val Pro Ser Ser
            180                 185                 190

Ser Leu Gly Thr Lys Thr Tyr Thr Cys Asn Val Asp His Lys Pro Ser
        195                 200                 205

Asn Thr Lys Val Asp Lys Arg Val Glu Pro Lys Ser Cys Asp Lys Thr
    210                 215                 220

His Thr Cys Pro Pro Cys Pro Ala Pro Glu Leu Arg Arg Gly Pro Lys
225                 230                 235                 240

Val Phe Leu Phe Pro Pro Lys Pro Lys Asp Thr Leu Met Ile Ser Arg
                245                 250                 255

Thr Pro Glu Val Thr Cys Val Val Val Asp Val Ser Gln Glu Asp Pro
            260                 265                 270

Glu Val Gln Phe Asn Trp Tyr Val Asp Gly Val Glu Val His Asn Ala
        275                 280                 285

Lys Thr Lys Pro Arg Glu Glu Gln Phe Ala Ser Thr Tyr Arg Val Val
    290                 295                 300

Ser Val Leu Thr Val Leu His Gln Asp Trp Leu Asn Gly Lys Glu Tyr
305                 310                 315                 320

Lys Cys Lys Val Ser Asn Lys Gly Leu Pro Ser Ser Ile Glu Lys Thr
                325                 330                 335

Ile Ser Lys Ala Lys Gly Gln Pro Arg Glu Pro Gln Val Tyr Thr Leu
            340                 345                 350

Pro Pro Ser Arg Lys Glu Met Thr Lys Asn Gln Val Ser Leu Thr Cys
        355                 360                 365

Leu Val Lys Gly Phe Tyr Pro Ser Asp Ile Ala Val Glu Trp Glu Ser
    370                 375                 380

Asn Gly Gln Pro Glu Asn Asn Tyr Lys Thr Thr Pro Pro Tyr Leu Asp
385                 390                 395                 400

Ser Asp Gly Ser Phe Phe Leu Tyr Ser Lys Leu Thr Val Asp Lys Ser
                405                 410                 415
```

Arg Trp Gln Gln Gly Asn Val Phe Ser Cys Ser Val Met His Glu Ala
420 425 430

Leu His Asn His Tyr Thr Gln Lys Ser Leu Ser Leu Ser Pro Asp Tyr
435 440 445

Lys Asp Asp Asp Asp Lys
450

<210> 155
<211> 216
<212> PRT
<213> Artificial Sequence

<220>
<223> CLA0028L-SK1

<400> 155

Asp Val Val Met Thr Gln Thr Pro Ala Ser Val Ser Glu Pro Val Gly
1 5 10 15

Gly Thr Val Thr Ile Lys Cys Gln Ala Ser Gln Ser Ile Gly Asn Tyr
20 25 30

Leu Ala Trp Tyr Gln Gln Lys Pro Gly Gln Pro Pro Lys Leu Leu Ile
35 40 45

Tyr Tyr Ala Ser Asn Leu Ala Ser Gly Val Ser Ser Arg Phe Lys Gly
50 55 60

Ser Arg Ser Gly Thr Glu Phe Thr Leu Thr Ile Ser Asp Leu Glu Cys
65 70 75 80

Ala Asp Ala Ala Thr Tyr Tyr Cys Gln Cys Ala Tyr Tyr Glu Ser Ser
85 90 95

Tyr Val Thr Phe Gly Gly Gly Thr Glu Val Val Val Lys Arg Thr Val
100 105 110

Ala Ala Pro Ser Val Phe Ile Phe Pro Pro Ser Asp Glu Gln Leu Lys
115 120 125

Ser Gly Thr Ala Ser Val Val Cys Leu Leu Asn Asn Phe Tyr Pro Arg
130 135 140

Glu Ala Lys Val Gln Trp Lys Val Asp Asn Ala Leu Gln Ser Gly Asn
145 150 155 160

Ser Gln Glu Ser Val Thr Glu Gln Asp Ser Lys Asp Ser Thr Tyr Ser
165 170 175

Leu Ser Ser Thr Leu Thr Leu Ser Lys Ala Asp Tyr Glu Lys His Lys
180 185 190

Val Tyr Ala Cys Glu Val Thr His Gln Gly Leu Ser Ser Pro Val Thr
195 200 205

Lys Ser Phe Asn Arg Gly Glu Cys
210 215

<210> 156
<211> 456
<212> PRT
<213> Artificial Sequence

<220>
<223> HER2H-BS03aHis

<400> 156

Glu Val Gln Leu Val Glu Ser Gly Gly Gly Leu Val Gln Pro Gly Gly
1 5 10 15

Ser Leu Arg Leu Ser Cys Ala Ala Ser Gly Phe Asn Ile Lys Asp Thr
20 25 30

Tyr Ile His Trp Val Arg Gln Ala Pro Gly Lys Gly Leu Glu Trp Val
35 40 45

Ala Arg Ile Tyr Pro Thr Asn Gly Tyr Thr Arg Tyr Ala Asp Ser Val
50 55 60

Lys Gly Arg Phe Thr Ile Ser Ala Asp Thr Ser Lys Asn Thr Ala Tyr
65 70 75 80

Leu Gln Met Asn Ser Leu Arg Ala Glu Asp Thr Ala Val Tyr Tyr Cys
85 90 95

Ser Arg Trp Gly Gly Asp Gly Phe Tyr Ala Met Asp Tyr Trp Gly Gln
100 105 110

Gly Thr Leu Val Thr Val Ser Ser Ala Ser Thr Lys Gly Pro Ser Val
115 120 125

Phe Pro Leu Ala Pro Ser Ser Arg Ser Thr Ser Glu Ser Thr Ala Ala
130 135 140

Leu Gly Cys Leu Val Lys Asp Tyr Phe Pro Glu Pro Val Thr Val Ser
145 150 155 160

Trp Asn Ser Gly Ala Leu Thr Ser Gly Val His Thr Phe Pro Ala Val
165 170 175

Leu Gln Ser Ser Gly Leu Tyr Ser Leu Ser Ser Val Val Thr Val Pro
180 185 190

Ser Ser Ser Leu Gly Thr Lys Thr Tyr Thr Cys Asn Val Asp His Lys
195 200 205

Pro Ser Asn Thr Lys Val Asp Lys Arg Val Glu Pro Lys Ser Cys Asp
210 215 220

Lys Thr His Thr Cys Pro Pro Cys Pro Ala Pro Glu Leu Arg Arg Gly
225 230 235 240

Pro Lys Val Phe Leu Phe Pro Pro Lys Pro Lys Asp Thr Leu Met Ile
245 250 255

Ser Arg Thr Pro Glu Val Thr Cys Val Val Val Asp Val Ser Gln Glu
260 265 270

Asp Pro Glu Val Gln Phe Asn Trp Tyr Val Asp Gly Val Glu Val His
275 280 285

Asn Ala Lys Thr Lys Pro Arg Glu Glu Gln Phe Ala Ser Thr Tyr Arg
290 295 300

Val Val Ser Val Leu Thr Val Leu His Gln Asp Trp Leu Asn Gly Lys
305 310 315 320

Glu Tyr Lys Cys Lys Val Ser Asn Lys Gly Leu Pro Ser Ser Ile Glu
325 330 335

Lys Thr Ile Ser Lys Ala Lys Gly Gln Pro Arg Glu Pro Gln Val Tyr
340 345 350

Thr Leu Pro Pro Ser Arg Glu Glu Met Thr Lys Asn Gln Val Ser Leu
355 360 365

Thr Cys Leu Val Lys Gly Phe Tyr Pro Ser Asp Ile Ala Val Glu Trp
370 375 380

Glu Ser Asn Gly Gln Pro Glu Asn Asn Tyr Lys Thr Thr Pro Pro Tyr
385 390 395 400

Leu Asp Ser Asp Gly Ser Phe Phe Leu Tyr Ser Lys Leu Thr Val Asp
405 410 415

Lys Ser Arg Trp Gln Gln Gly Asn Val Phe Ser Cys Ser Val Met His
420 425 430

Glu Ala Leu His Asn His Tyr Thr Gln Glu Ser Leu Ser Leu Ser Pro
435 440 445

His His His His His His His His
450 455

<210> 157
<211> 214
<212> PRT
<213> Artificial Sequence

<220>
<223> HER2L-SK1

<400> 157

Asp Ile Gln Met Thr Gln Ser Pro Ser Ser Leu Ser Ala Ser Val Gly
1 5 10 15

Asp Arg Val Thr Ile Thr Cys Arg Ala Ser Gln Asp Val Asn Thr Ala
20 25 30

Val Ala Trp Tyr Gln Gln Lys Pro Gly Lys Ala Pro Lys Leu Leu Ile
35 40 45

Tyr Ser Ala Ser Phe Leu Tyr Ser Gly Val Pro Ser Arg Phe Ser Gly
50 55 60

Ser Arg Ser Gly Thr Asp Phe Thr Leu Thr Ile Ser Ser Leu Gln Pro
65 70 75 80

Glu Asp Phe Ala Thr Tyr Tyr Cys Gln Gln His Tyr Thr Thr Pro Pro
85 90 95

Thr Phe Gly Gln Gly Thr Lys Val Glu Ile Lys Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala
130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 158
<211> 454
<212> PRT
<213> Artificial Sequence

<220>
<223> CD8H-BS03aHis

<400> 158

Glu Val Gln Leu Gln Gln Ser Gly Ala Glu Leu Val Lys Pro Gly Ala
1 5 10 15

Ser Val Lys Leu Ser Cys Thr Ala Ser Gly Phe Asn Ile Lys Asp Thr
20 25 30

Tyr Ile His Phe Val Arg Gln Arg Pro Glu Gln Gly Leu Glu Trp Ile
35 40 45

Gly Arg Ile Asp Pro Ala Asn Asp Asn Thr Leu Tyr Ala Ser Lys Phe
50 55 60

Gln Gly Lys Ala Thr Ile Thr Ala Asp Thr Ser Ser Asn Thr Ala Tyr
65 70 75 80

Met His Leu Cys Ser Leu Thr Ser Gly Asp Thr Ala Val Tyr Tyr Cys
85 90 95

Gly Arg Gly Tyr Gly Tyr Tyr Val Phe Asp His Trp Gly Gln Gly Thr
100 105 110

Thr Leu Thr Val Ser Ser Ala Ser Thr Lys Gly Pro Ser Val Phe Pro
115 120 125

Leu Ala Pro Ser Ser Arg Ser Thr Ser Glu Ser Thr Ala Ala Leu Gly

130 135 140

Cys Leu Val Lys Asp Tyr Phe Pro Glu Pro Val Thr Val Ser Trp Asn
145 150 155 160

Ser Gly Ala Leu Thr Ser Gly Val His Thr Phe Pro Ala Val Leu Gln
165 170 175

Ser Ser Gly Leu Tyr Ser Leu Ser Ser Val Val Thr Val Pro Ser Ser
180 185 190

Ser Leu Gly Thr Lys Thr Tyr Thr Cys Asn Val Asp His Lys Pro Ser
195 200 205

Asn Thr Lys Val Asp Lys Arg Val Glu Pro Lys Ser Cys Asp Lys Thr
210 215 220

His Thr Cys Pro Pro Cys Pro Ala Pro Glu Leu Arg Arg Gly Pro Lys
225 230 235 240

Val Phe Leu Phe Pro Pro Lys Pro Lys Asp Thr Leu Met Ile Ser Arg
245 250 255

Thr Pro Glu Val Thr Cys Val Val Val Asp Val Ser Gln Glu Asp Pro
260 265 270

Glu Val Gln Phe Asn Trp Tyr Val Asp Gly Val Glu Val His Asn Ala
275 280 285

Lys Thr Lys Pro Arg Glu Glu Gln Phe Ala Ser Thr Tyr Arg Val Val
290 295 300

Ser Val Leu Thr Val Leu His Gln Asp Trp Leu Asn Gly Lys Glu Tyr
305 310 315 320

Lys Cys Lys Val Ser Asn Lys Gly Leu Pro Ser Ser Ile Glu Lys Thr
325 330 335

Ile Ser Lys Ala Lys Gly Gln Pro Arg Glu Pro Gln Val Tyr Thr Leu
340 345 350

Pro Pro Ser Arg Glu Glu Met Thr Lys Asn Gln Val Ser Leu Thr Cys
355 360 365

Leu Val Lys Gly Phe Tyr Pro Ser Asp Ile Ala Val Glu Trp Glu Ser
370 375 380

Asn Gly Gln Pro Glu Asn Asn Tyr Lys Thr Thr Pro Pro Tyr Leu Asp
385 390 395 400

Ser Asp Gly Ser Phe Phe Leu Tyr Ser Lys Leu Thr Val Asp Lys Ser
405 410 415

Arg Trp Gln Gln Gly Asn Val Phe Ser Cys Ser Val Met His Glu Ala
420 425 430

Leu His Asn His Tyr Thr Gln Glu Ser Leu Ser Leu Ser Pro His His
435 440 445

His His His His His His
450

<210> 159
<211> 214
<212> PRT
<213> Artificial Sequence

<220>
<223> CD8L-SK1

<400> 159

Asp Val Gln Ile Asn Gln Ser Pro Ser Phe Leu Ala Ala Ser Pro Gly
1 5 10 15

Glu Thr Ile Thr Ile Asn Cys Arg Thr Ser Arg Ser Ile Ser Gln Tyr
20 25 30

Leu Ala Trp Tyr Gln Glu Lys Pro Gly Lys Thr Asn Lys Leu Leu Ile
35 40 45

Tyr Ser Gly Ser Thr Leu Gln Ser Gly Ile Pro Ser Arg Phe Ser Gly
50 55 60

Ser Gly Ser Gly Thr Asp Phe Thr Leu Thr Ile Ser Gly Leu Glu Pro
65 70 75 80

Glu Asp Phe Ala Met Tyr Tyr Cys Gln Gln His Asn Glu Asn Pro Leu
85 90 95

Thr Phe Gly Ala Gly Thr Lys Leu Glu Leu Arg Arg Thr Val Ala Ala
100 105 110

Pro Ser Val Phe Ile Phe Pro Pro Ser Asp Glu Gln Leu Lys Ser Gly
115 120 125

Thr Ala Ser Val Val Cys Leu Leu Asn Asn Phe Tyr Pro Arg Glu Ala

130 135 140

Lys Val Gln Trp Lys Val Asp Asn Ala Leu Gln Ser Gly Asn Ser Gln
145 150 155 160

Glu Ser Val Thr Glu Gln Asp Ser Lys Asp Ser Thr Tyr Ser Leu Ser
165 170 175

Ser Thr Leu Thr Leu Ser Lys Ala Asp Tyr Glu Lys His Lys Val Tyr
180 185 190

Ala Cys Glu Val Thr His Gln Gly Leu Ser Ser Pro Val Thr Lys Ser
195 200 205

Phe Asn Arg Gly Glu Cys
210

<210> 160
<211> 125
<212> PRT
<213> Artificial Sequence

<220>
<223> SC001H

<400> 160

Gln Val Gln Leu Val Gln Ser Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Val Tyr Gln Gly Gly Ala Thr Pro Tyr Tyr Tyr Tyr Gly Met
100 105 110

Asp Val Trp Gly Gln Gly Thr Met Val Thr Val Ser Ser
115 120 125

<210> 161
<211> 110
<212> PRT
<213> Artificial Sequence

<220>
<223> SC001L

<400> 161

Ser Tyr Glu Leu Thr Gln Pro Pro Ser Ala Ser Gly Thr Pro Gly Gln
1 5 10 15

Arg Val Thr Val Ser Cys Ser Gly Ser Thr Ser Asn Ile Gly Asn His
20 25 30

Ala Val Asn Trp Tyr Gln Gln Leu Pro Gly Thr Ala Pro Lys Leu Leu
35 40 45

Ile Tyr Thr Asn Asp Gln Arg Pro Ser Gly Val Pro Asn Arg Phe Ser
50 55 60

Gly Ser Lys Ser Gly Ile Ser Ala Ser Leu Ala Ile Ser Gly Leu Gln
65 70 75 80

Ser Glu Asp Glu Ala Asp Tyr Tyr Cys Ser Ala Trp Asp Asp Ser Leu
85 90 95

Ile Gly Tyr Val Phe Gly Ala Gly Thr Lys Val Thr Val Leu
100 105 110

<210> 162
<211> 128
<212> PRT
<213> Artificial Sequence

<220>
<223> SC002H

<400> 162

Glu Val Gln Leu Val Gln Ser Gly Ala Glu Val Lys Lys Pro Gly Ala
1 5 10 15

Ser Val Lys Val Ser Cys Lys Ala Ser Gly Tyr Thr Phe Thr Ser Tyr
20 25 30

Gly Ile Ser Trp Val Arg Gln Ala Pro Gly Gln Gly Leu Glu Trp Met
35 40 45

Gly Trp Ile Ser Ala Tyr Asn Gly Asn Thr Asn Tyr Ala Gln Lys Leu

50 55 60

Gln Gly Arg Val Thr Met Thr Thr Asp Thr Ser Thr Ser Thr Ala Tyr
65 70 75 80

Met Glu Leu Arg Ser Leu Arg Ser Asp Asp Thr Ala Val Tyr Tyr Cys
85 90 95

Ala Arg Val Ser Ser Gly Ser Gln Pro Asp Pro Pro Tyr Tyr Tyr Tyr
100 105 110

Tyr Gly Met Asp Val Trp Gly Gln Gly Thr Met Val Thr Val Ser Ser
115 120 125

<210> 163
<211> 111
<212> PRT
<213> Artificial Sequence

<220>
<223> SC002L

<400> 163

Gln Ser Val Leu Thr Gln Pro Ala Ser Val Ser Gly Ser Pro Gly Gln
1 5 10 15

Ser Ile Thr Ile Ser Cys Thr Gly Thr Ser Ser Asn Val Glu Ser Tyr
20 25 30

Asn Leu Val Ser Trp Tyr Gln Gln His Pro Gly Lys Ala Pro Lys Phe
35 40 45

Ile Ile Tyr Glu Gly Thr Arg Arg Pro Ser Gly Ile Ser Asn Arg Phe
50 55 60

Ser Gly Ala Asn Ser Gly Asn Ala Ala Ser Leu Thr Ile Ser Gly Leu
65 70 75 80

Gln Ala Glu Asp Glu Ala Asp Tyr Tyr Cys Leu Ser Tyr Val Pro Ser
85 90 95

Arg Arg Arg His Val Phe Gly Thr Gly Thr Lys Val Thr Val Leu
100 105 110

<210> 164
<211> 125
<212> PRT
<213> Artificial Sequence

<220>

<223> SC003H

<400> 164

Gln Val Gln Leu Val Glu Thr Gly Gly Gly Val Val Gln Pro Gly Arg
1 5 10 15

Ser Leu Arg Leu Ser Cys Ala Ala Ser Gly Phe Thr Phe Ser Ser Tyr
20 25 30

Ala Met His Trp Val Arg Gln Ala Pro Gly Lys Gly Leu Glu Trp Val
35 40 45

Ala Val Ile Ser Tyr Asp Gly Ser Asn Lys Tyr Tyr Ala Asp Ser Val
50 55 60

Lys Gly Arg Phe Thr Ile Ser Arg Asp Asn Ser Lys Asn Thr Leu Tyr
65 70 75 80

Leu Gln Met Asn Ser Leu Arg Ala Glu Asp Thr Ala Val Tyr Tyr Cys
85 90 95

Ala Arg Gly Pro Tyr Cys Ser Ser Thr Ser Cys Tyr Leu Ser Pro Phe
100 105 110

Asp Tyr Trp Gly Gln Gly Thr Leu Val Thr Val Ser Ser
115 120 125

<210> 165
<211> 110
<212> PRT
<213> Artificial Sequence

<220>
<223> SC003L

<400> 165

Gln Ser Val Leu Thr Gln Pro Pro Ser Val Ser Ala Ala Pro Gly Gln
1 5 10 15

Lys Val Thr Ile Ser Cys Ser Gly Asn Thr Ser Asn Ile Gly Asn Asn
20 25 30

Tyr Val Ser Trp Tyr Gln Gln Leu Pro Gly Thr Ala Pro Lys Leu Leu
35 40 45

Ile Tyr Arg Asn Ser Asp Arg Pro Ser Gly Val Pro Asp Arg Phe Ser
50 55 60

Gly Ser Lys Ser Gly Thr Ser Ala Ser Leu Ala Ile Thr Gly Leu Gln

65 70 75 80

Ala Glu Asp Glu Ala Asp Tyr Tyr Cys Gln Ser Tyr Asp Thr Ser Leu
85 90 95

Thr Ala Val Ile Phe Gly Gly Gly Thr Lys Leu Thr Val Leu
100 105 110

<210> 166
<211> 6
<212> PRT
<213> Artificial Sequence

<220>
<223> His tag

<400> 166

His His His His His His
1 5

<210> 167
<211> 137
<212> PRT
<213> Artificial Sequence

<220>
<223> hCD3E(ECD)FLAG

<400> 167

Met Gln Ser Gly Thr His Trp Arg Val Leu Gly Leu Cys Leu Leu Ser
1 5 10 15

Val Gly Val Trp Gly Gln Asp Gly Asn Glu Glu Met Gly Gly Ile Thr
20 25 30

Gln Thr Pro Tyr Lys Val Ser Ile Ser Gly Thr Thr Val Ile Leu Thr
35 40 45

Cys Pro Gln Tyr Pro Gly Ser Glu Ile Leu Trp Gln His Asn Asp Lys
50 55 60

Asn Ile Gly Gly Asp Glu Asp Asp Lys Asn Ile Gly Ser Asp Glu Asp
65 70 75 80

His Leu Ser Leu Lys Glu Phe Ser Glu Leu Glu Gln Ser Gly Tyr Tyr
85 90 95

Val Cys Tyr Pro Arg Gly Ser Lys Pro Glu Asp Ala Asn Phe Tyr Leu
100 105 110

```
Tyr Leu Arg Ala Arg Val Cys Glu Asn Cys Met Glu Met Asp Val Met
        115                 120                 125

Ser Asp Tyr Lys Asp Asp Asp Asp Lys
    130                 135

<210>  168
<211>  114
<212>  PRT
<213>  Artificial Sequence

<220>
<223>  hCD3D(ECD)His

<400>  168

Met Glu His Ser Thr Phe Leu Ser Gly Leu Val Leu Ala Thr Leu Leu
1               5                   10                  15

Ser Gln Val Ser Pro Phe Lys Ile Pro Ile Glu Glu Leu Glu Asp Arg
            20                  25                  30

Val Phe Val Asn Cys Asn Thr Ser Ile Thr Trp Val Glu Gly Thr Val
        35                  40                  45

Gly Thr Leu Leu Ser Asp Ile Thr Arg Leu Asp Leu Gly Lys Arg Ile
    50                  55                  60

Leu Asp Pro Arg Gly Ile Tyr Arg Cys Asn Gly Thr Asp Ile Tyr Lys
65                  70                  75                  80

Asp Lys Glu Ser Thr Val Gln Val His Tyr Arg Met Cys Gln Ser Cys
                85                  90                  95

Val Glu Leu Asp Pro Ala Thr Val Ala Gly His His His His His His
            100                 105                 110

His His
```

## Description

[Technical Field]

The present invention relates to antigen-binding molecules and combinations.

[Background Art]

An antibody is a protein that specifically binds to an antigen with high affinity. It is known that various molecules ranging from low-molecular-weight compounds to proteins can be antigens. Since the technique for producing monoclonal antibodies was developed, antibody modification techniques have advanced, making it easier to obtain antibodies that recognize a particular molecule. For example, a domino antibody that recognizes the light chain portion of a first antibody and specifically recognizes the first antibody to which an antigen is bound is used in an immunological assay such as ELISA (PTL 1). Junction epitope antibodies that stabilize protein-protein interactions between IL-6 and gp80 regulate downstream signals (Scientific Reports (2017) 7, 1-15 Ralph, A. *et al.* (NPL 9)).

Antibodies are attracting attention as pharmaceuticals because of their high stability in plasma and few side effects. Antibodies not only have antigen-binding effects, agonistic effects, or antagonistic effects but also induce cytotoxic activities mediated by effector cells (also referred to as effector functions), such as antibody-dependent cytotoxicity (ADCC), antibody-dependent cell phagocytosis (ADCP), and complement-dependent cytotoxicity (CDC). These antibody functions have been taken advantage of to develop pharmaceuticals for cancer, immune diseases, chronic diseases, infections, and such (Paul J. Carter and Greg A. Lazar, "Next generation antibody drugs: pursuit of the 'high-hanging fruit'" [online], December 1, 2017, Nature Reviews Drug Discovery, [retrieved on January 22, 2018], Internet <https://www.nature.com/articles/nrd.2017.227> (NPL 1)).

For example, pharmaceuticals utilizing an agonist antibody against a co-stimulatory molecule that promotes activation of cytotoxic T cells have been developed as anticancer agents (Clinical and Experimental Immunology (2009) 157, 9-19 Peggs, K. S. *et al.* (NPL 2)). In recent years, immune checkpoint-inhibiting antibodies with antagonist activity on co-inhibitory molecules were found to be useful as anticancer agents, and Ipilimumab, Nivolumab , Pembrolizumab, and Atezolizumab, which are antibody drugs that inhibit the interaction of CTLA4/CD80 or PD-1/PD-L1, were put on the market one after another (NPL 1).

Second generation antibody drugs in which the function of a native IgG type antibody is artificially enhanced or added, or attenuated or deleted to enhance or add, or attenuate or delete their functions according to the application of the antibody, have been developed. Examples of second-generation antibody drugs include antibodies with enhanced or deleted effector functions (Current Pharmaceutical Biotechnology (2016) 17, 1298-1314 Mimoto, F. *et al.* (NPL 3)), antibodies binding to antigens in a pH-dependent manner (Nature Biotechnology (2010) 28, 1203-1208 Igawa, T. *et al.* (NPL 4)), and antibodies binding to two or more different antigens per antibody molecule (antibodies binding to two different antigens are generally referred to as "bispecific antibodies") (MAbs. (2012) Mar 1, 4(2) (NPL 5)).

Bispecific antibodies are expected to be more effective pharmaceuticals. For example, antibodies for which one of the antigens is a protein expressed on the cell membrane of T cells and the other is a cancer antigen have been developed, which crosslink cytotoxic T cells with cancer cells and thereby have increased antitumor activity (herein, this antitumor activity is abbreviated as "TDCC activity" (T-cell Dependent Cytotoxicity) and is included in the effector functions) (Journal of Biomolecular Screening (2015) 20, 519-27 Nazarian, A. A. *et al.* (NPL 10)). Bispecific antibodies that have been reported include antibodies whose two Fab regions have different sequences (common light chain bispecific antibodies and hybrid hybridomas), antibodies to which an antigen-binding site is added at the N-terminus or C-terminus (DVD-Ig and scFv-IgG), antibodies in which one Fab region binds to two antigens (Two-in-one IgGs), and antibodies that use the loop portion of the CH3 region as a new antigen-binding site (Fcab) (Nature Review (2010), 10, 301-316 Chan, A. C. and Carter P. J. (NPL 6); and Peds (2010), 23 (4), 289-297 Wozniak-Knopp, G *et al.* (NPL 7)).

On the other hand, antibodies whose effector functions are utilized easily cause side effects by acting even on normal cells that express a target antigen at low levels. Therefore, efforts have been made to allow antibody drugs to exert the effector functions specifically on target tissue. For example, an antibody whose binding ability changes upon binding to a cell metabolite (PTL 2), an antibody that exhibits an antigen-binding ability upon protease cleavage (PTL 3), and technology to control antibody-mediated crosslinking between a chimeric antigen receptor-T cell (CAR-T cells) and a cancer cell by adding a compound (ABT-737) (Nature Chemical Biology (2018) 14, 112-117 Hill Z. B. *et al.* (NPL 8)) have been reported.

[Citation List]

[Patent Literature]

[PTL 1] WO 2009/142221
[PTL 2] WO 2013/180200
[PTL 3] WO 2009/025846

[Non-Patent Literature]

[NPL 1] Paul J. Carter and Greg A. Lazar, Next generation antibody drugs: pursuit of the 'high-hanging fruit', [online], December 1, 2017, Nature Reviews Drug Discovery, [retrieved on January 22, 2017], Internet (https://www.nature.com/articles/nrd.2017.227)
[NPL 2] Clinical and Experimental Immunology (2009) 157, 9-19 Peggs, K. S. et al.
[NPL 3] Current Pharmaceutical Biotechnology (2016) 17, 1298-1314 Mimoto, F. et al.
[NPL 4] Nature Biotechnology (2010) 28, 1203-1208 Igawa, T. et al.
[NPL 5] MAbs (2012) 4, 182-197 Kontermann, R. E.
[NPL 6] Nature Review (2010), 10, 301-316 Chan, A. C. and Carter P. J.
[NPL 7] Peds (2010), 23(4), 289-297 Wozniak-Knopp, G et al.
[NPL 8] Nature Chemical Biology (2018) 14, 112-117 Hill Z. B. et al.
[NPL 9] Scientific Reports (2017) 7, 1-15 Ralph, A. et al.
[NPL 10] Journal of Biomolecular Screening (2015) 20, 519-527 Nazarian, A. A. et al.

[Summary of Invention]

[Technical Problem]

The above-mentioned efforts to allow antibody drugs to exert the effector functions specifically on target tissue are still in progress, and more efforts are desired. Therefore, an objective of the present invention is to provide an antibody modification technique that is useful for allowing an antibody drug to exert the effector functions specifically on target tissue and reducing the side effects of the antibody drug, and that is further applicable to other various kinds of protein engineering.

[Solution to Problem]

As a result of dedicated studies, the present inventors have found the following inventions [1] to [46].

[1] A second antigen-binding molecule, which binds to an antigen/antigen-binding molecule complex comprising a first antigen and a first antigen-binding molecule that binds to the first antigen, and enhances the binding activity of the first antigen-binding molecule to the first antigen.
[2] The second antigen-binding molecule of [1], which has higher binding activity to the first antigen in the presence of the first antigen-binding molecule than in the absence of the first antigen-binding molecule.
[3] The second antigen-binding molecule of [1] or [2], wherein the first antigen is an immune-related molecule or a cellular metabolite.
[4] The second antigen-binding molecule of [3], wherein the immune-related molecule is a molecule present on the cell membrane of an immune cell.
[5] The second antigen-binding molecule of [4], wherein the immune cell is at least one selected from the group consisting of a granulocyte, a macrophage, a dendritic cell, a T cell, and a B cell.
[6] The second antigen-binding molecule of any one of [3] to [5], wherein the immune-related molecule is CD3.
[7] The second antigen-binding molecule of [6], wherein the first antigen-binding molecule comprises:

a CD3-binding polypeptide consisting of any combination of heavy chain variable region and light chain variable region amino acid sequences selected from SEQ ID NO: 1 and SEQ ID NO: 122, SEQ ID NO: 114 and SEQ ID NO:115, SEQ ID NO: 116 and SEQ ID NO: 117, SEQ ID NO: 118 and SEQ ID NO: 119, and SEQ ID NO:

120 and SEQ ID NO: 121, respectively; or

a first modified polypeptide produced by modifying the CD3-binding polypeptide, wherein the CD3-binding activity of the first modified polypeptide is lower than that of the CD3-binding polypeptide.

[8] The second antigen-binding molecule of [3], wherein the cellular metabolite is adenosine or a derivative thereof.

[9] The second antigen-binding molecule of [8], wherein the first antigen-binding molecule comprises:

an adenosine-binding polypeptide consisting of any combination of heavy chain variable region and light chain variable region amino acid sequences selected from SEQ ID NO: 106 and SEQ ID NO: 107, SEQ ID NO: 108 and SEQ ID NO:109, SEQ ID NO: 110 and SEQ ID NO: 111, and SEQ ID NO: 112 and SEQ ID NO: 113, respectively; or

a second modified polypeptide produced by modifying the adenosine-binding polypeptide, wherein the adenosine-binding activity of the second modified polypeptide is lower or higher than that of the adenosine-binding polypeptide.

[10] The second antigen-binding molecule of any one of [1] to [9], wherein the first antigen-binding molecule has multiple antigen specificity and further binds to at least a second antigen.

[11] The second antigen-binding molecule of [10], wherein the second antigen is a cancer antigen or an immune-related molecule.

[12] The second antigen-binding molecule of any one of [1] to [11], which has multiple antigen specificity and further binds to at least a third antigen.

[13] The second antigen-binding molecule of [12], wherein the third antigen is a cancer antigen or an immune-related molecule.

[14] The second antigen-binding molecule of any one of [1] to [13], wherein the first antigen-binding molecule has multiple antigen specificity and further binds to at least a second antigen, wherein the second antigen-binding molecule has multiple antigen specificity and further binds to at least a third antigen, and wherein the combination of the first antigen, the second antigen, and the third antigen is any one of the combinations (1) to (5) below:

(1) a combination in which the first antigen is an immune-related molecule, the second antigen is a first cancer antigen, and the third antigen is a second cancer antigen;

(2) a combination in which the first antigen is a cellular metabolite of a target cell, the second antigen is a cancer antigen, and the third antigen is an immune-related molecule;

(3) a combination in which the first antigen is a cellular metabolite of a target cell, the second antigen is an immune-related molecule, and the third antigen is a cancer antigen;

(4) a combination in which the first antigen is a first immune-related molecule, the second antigen is a cancer antigen, and the third antigen is a second immune-related molecule; and

(5) a combination in which the first antigen is a first immune-related molecule, the second antigen is a second immune-related molecule, and the third antigen is a cancer antigen.

[15] A combination of the first antigen-binding molecule and the second antigen-binding molecule of [1].

[16] A first antigen-binding molecule, which binds to a first antigen, wherein the binding activity of the first antigen-binding molecule to the first antigen is enhanced by a second antigen-binding molecule which binds to an antigen/antigen-binding molecule complex comprising the first antigen and the first antigen-binding molecule.

[17] The first antigen-binding molecule of [16], wherein the binding activity of the second antigen-binding molecule to the first antigen is higher in the presence of the first antigen-binding molecule than in the absence of the first antigen-binding molecule.

[18] The first antigen-binding molecule of [16] or [17], wherein the first antigen is an immune-related molecule or a cellular metabolite.

[19] The first antigen-binding molecule of [18], wherein the immune-related molecule is a molecule present on the cell membrane of an immune cell.

[20] The first antigen-binding molecule of [19], wherein the immune cell is at least one selected from the group consisting of a granulocyte, a macrophage, a dendritic cell, a T cell, and a B cell.

[21] The first antigen-binding molecule of any one of [19] to [20], wherein the immune-related molecule is CD3.

[22] The first antigen-binding molecule of [21], wherein the first antigen-binding molecule comprises:

a CD3-binding polypeptide consisting of any combination of heavy chain variable region and light chain variable region amino acid sequences selected from SEQ ID NO: 1 and SEQ ID NO: 122, SEQ ID NO: 114 and SEQ ID NO:115, SEQ ID NO: 116 and SEQ ID NO: 117, SEQ ID NO: 118 and SEQ ID NO: 119, and SEQ ID NO:

120 and SEQ ID NO: 121, respectively; or
a first modified polypeptide produced by modifying the CD3-binding polypeptide, wherein the CD3-binding activity of the first modified polypeptide is lower than that of the CD3-binding polypeptide.

[23] The first antigen-binding molecule of [18], wherein the cellular metabolite is adenosine or a derivative thereof.
[24] The first antigen-binding molecule of [23], wherein the first antigen-binding molecule comprises:

an adenosine-binding polypeptide consisting of any combination of heavy chain variable region and light chain variable region amino acid sequences selected from SEQ ID NO: 106 and SEQ ID NO: 107, SEQ ID NO: 108 and SEQ ID NO:109, SEQ ID NO: 110 and SEQ ID NO: 111, and SEQ ID NO: 112 and SEQ ID NO: 113, respectively; or
a second modified polypeptide produced by modifying the adenosine-binding polypeptide, wherein the adenosine-binding activity of the second modified polypeptide is lower or higher than that of the adenosine-binding polypeptide.

[25] The first antigen-binding molecule of any one of [16] to [24], which has multiple antigen specificity and further binds to at least a second antigen.
[26] The first antigen-binding molecule of [25], wherein the second antigen is a cancer antigen or an immune-related molecule.
[27] The first antigen-binding molecule of any one of [16] to [26], wherein the second antigen-binding molecule has multiple antigen specificity and further binds to at least a third antigen.
[28] The first antigen-binding molecule of [27], wherein the third antigen is a cancer antigen or an immune-related molecule.
[29] The first antigen-binding molecule of any one of [16] to [28], wherein the first antigen-binding molecule has multiple antigen specificity and further binds to at least a second antigen, wherein the second antigen-binding molecule has multiple antigen specificity and further binds to at least a third antigen, and wherein the combination of the first antigen, the second antigen, and the third antigen is any one of the combinations (1) to (5) below:

(1) a combination in which the first antigen is an immune-related molecule, the second antigen is a first cancer antigen, and the third antigen is a second cancer antigen;
(2) a combination in which the first antigen is a cellular metabolite of a target cell, the second antigen is a cancer antigen, and the third antigen is an immune-related molecule;
(3) a combination in which the first antigen is a cellular metabolite of a target cell, the second antigen is an immune-related molecule, and the third antigen is a cancer antigen;
(4) a combination in which the first antigen is a first immune-related molecule, the second antigen is a cancer antigen, and the third antigen is a second immune-related molecule; and
(5) a combination in which the first antigen is a first immune-related molecule, the second antigen is a second immune-related molecule, and the third antigen is a cancer antigen.

[30] A combination of the first antigen-binding molecule and the second antigen-binding molecule of [16].
[31] The combination of [15] or [30], which is a pharmaceutical composition.
[32] The combination of [31], wherein the first antigen-binding molecule and the second antigen-binding molecule are administered simultaneously or separately.
[33] A screening method comprising identifying one compound, or antibody or fragment thereof, arbitrarily selected from a library of compounds or antibodies or fragments thereof, as a second antigen-binding molecule when the binding activity of a first antigen-binding molecule to a first antigen assayed using at least one selected from SPR, BLI, and ELISA is detected in the presence of the compound or the antibody or fragment thereof but cannot be detected in the absence of the compound or the antibody or fragment thereof.
[34] A screening method comprising identifying one compound, or antibody or fragment thereof, arbitrarily selected from a library of compounds or antibodies or fragments thereof, as a second antigen-binding molecule when the binding activity of a first antigen-binding molecule to a first antigen assayed using at least one selected from SPR, BLI, and ELISA is higher in the presence of the compound or the antibody or fragment thereof than in the absence of the compound or the antibody or fragment thereof.
[35] A screening method comprising the steps of:

(a) immunizing a mammal with an antigen/antigen-binding molecule complex comprising a first antigen and a first antigen-binding molecule, and obtaining a first group of antibody-producing cells which produce monoclonal antibodies that bind to the complex;

(b) selecting from the first group, a second group which produces monoclonal antibodies whose binding activity to either or both of the first antigen not in the form of the complex and the first antigen-binding molecule not in the form of the complex cannot be detected in at least one assay selected from SPR, BLI, and ELISA; and
(c) selecting from the second group, a third group which produces monoclonal antibodies that enhance the binding activity of the first antigen-binding molecule to the first antigen in at least one assay selected from SPR, BLI, and ELISA, and identifying the third group as antibody-producing cells that produce a second antigen-binding molecule.

[36] A screening method comprising the steps of:

(a) immunizing a mammal with an antigen/antigen-binding molecule complex comprising a first antigen and a first antigen-binding molecule, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding molecule not in the form of the complex is lower than their binding to the complex in at least one assay selected from SPR, BLI, and ELISA; and
(c) selecting from the second group, a third group which produces monoclonal antibodies that enhance the binding activity of the first antigen-binding molecule to the first antigen in at least one assay selected from SPR, BLI, and ELISA, and identifying the third group as antibody-producing cells that produce a second antigen-binding molecule.

[37] A screening method comprising the steps of:

(a) immunizing a mammal with an antigen/antigen-binding region complex comprising a first antigen and a first antigen-binding region, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding region not in the form of the complex cannot be detected in at least one assay selected from SPR, BLI, and ELISA; and
(c) selecting from the second group, a third group which produces monoclonal antibodies that enhance the binding activity of the first antigen-binding region to the first antigen in at least one assay selected from SPR, BLI, and ELISA, and identifying the third group as antibody-producing cells that produce a second antigen-binding molecule.

[38] A screening method comprising the steps of:

(a) immunizing a mammal with an antigen/antigen-binding region complex comprising a first antigen and a first antigen-binding region, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding region not in the form of the complex is lower than their binding to the complex in at least one assay selected from SPR, BLI, and ELISA; and
(c) selecting from the second group, a third group which produces monoclonal antibodies that enhance the binding activity of the first antigen-binding region to the first antigen in at least one assay selected from SPR, BLI, and ELISA, and identifying the third group as antibody-producing cells that produce a second antigen-binding molecule.

[39] A method for producing a second antigen-binding molecule, comprising the steps of:

(d) culturing antibody-producing cells obtained from a screening method comprising the following steps (a) to (c):

(a) immunizing a mammal with an antigen/antigen-binding molecule complex comprising a first antigen and a first antigen-binding molecule, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding molecule not in the form of the complex cannot be detected in at least one assay selected from SPR, BLI, and ELISA; and

(c) selecting from the second group, a third group which produces monoclonal antibodies that enhance the binding activity of the first antigen-binding molecule to the first antigen in at least one assay selected from SPR, BLI, and ELISA, and identifying the third group as antibody-producing cells that produce a second antigen-binding molecule;

(e) obtaining a culture supernatant or a cell homogenate from the antibody-producing-cell culture; and
(f) purifying the second antigen-binding molecule from the culture supernatant or the cell homogenate.

[40] A method for producing a second antigen-binding molecule, comprising the steps of:

(d) culturing antibody-producing cells obtained from a screening method comprising the steps (a) to (c) below:

(a) immunizing a mammal with an antigen/antigen-binding molecule complex comprising a first antigen and a first antigen-binding molecule, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding molecule not in the form of the complex is lower than their binding to the complex in at least one assay selected from SPR, BLI, and ELISA; and
(c) selecting from the second group, a third group which produces monoclonal antibodies that enhance the binding activity of the first antigen-binding molecule to the first antigen in at least one assay selected from SPR, BLI, and ELISA, and identifying the third group as antibody-producing cells that produce a second antigen-binding molecule;

(e) obtaining a culture supernatant or a cell homogenate from the antibody-producing-cell culture; and
(f) purifying the second antigen-binding molecule from the culture supernatant or the cell homogenate.

[41] A method for producing a second antigen-binding molecule, comprising the steps of:

(d) culturing antibody-producing cells obtained from a screening method comprising the steps (a) to (c) below:

(a) immunizing a mammal with an antigen/antigen-binding region complex comprising a first antigen and a first antigen-binding region, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding region not in the form of the complex cannot be detected in at least one assay selected from SPR, BLI, and ELISA; and
(c) selecting from the second group, a third group which produces monoclonal antibodies that enhance the binding activity of the first antigen-binding region to the first antigen in at least one assay selected from SPR, BLI, and ELISA, and identifying the third group as antibody-producing cells that produce a second antigen-binding molecule;

(e) obtaining a culture supernatant or a cell homogenate from the antibody-producing-cell culture; and
(f) purifying the second antigen-binding molecule from the culture supernatant or the cell homogenate.

[42] A method for producing a second antigen-binding molecule, comprising the steps of:

(d) culturing antibody-producing cells obtained from a screening method comprising the steps (a) to (c) below:

(a) immunizing a mammal with an antigen/antigen-binding region complex comprising a first antigen and a first antigen-binding region, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding region not in the form of the complex is lower than their binding to the complex in at least one assay selected from SPR, BLI, and ELISA; and

(c) selecting from the second group, a third group which produces monoclonal antibodies that enhance the binding activity of the first antigen-binding region to the first antigen in at least one assay selected from SPR, BLI, and ELISA, and identifying the third group as antibody-producing cells that produce a second antigen-binding molecule;
(e) obtaining a culture supernatant or a cell homogenate from the antibody-producing-cell culture; and
(f) purifying the second antigen-binding molecule from the culture supernatant or the cell homogenate.

[43] A method for producing a phage display library of antigen-binding molecules, comprising:

a first step of modifying an amino acid in a first antigen-binding molecule that binds to a first antigen to obtain a variant of the first antigen-binding molecule, whose binding to the first antigen is lowered or is below the detection limit in at least one assay selected from SPR, BLI, and ELISA;
a second step of obtaining a first phage display library of antigen-binding molecules from an existing phage display library of antigen-binding molecules by removing phages presenting antigen-binding molecules that bind to either or both of the first antigen and the variant; and
a third step of obtaining a second phage display library of antigen-binding molecules from the first phage display library of antigen-binding molecules by enrichment for phages presenting antigen-binding molecules that bind to an antigen/antigen-binding molecule complex comprising the first antigen and the first antigen-binding molecule.

[44] The production method of [43], wherein the second and third steps are repeated using the second phage display library of antigen-binding molecules as the existing phage display library of antigen-binding molecules.
[45] A method for producing a phage display library of antigen-binding molecules, comprising:

a first step of obtaining a first phage display library of antigen-binding molecules from an existing phage display library of antigen-binding molecules by removing phages presenting antigen-binding molecules that (i) bind to a first antigen-binding molecule that may bind to a first antigen but is not bound to the first antigen and (ii) bind to the first antigen not bound to the first antigen-binding molecule; and
a second step of obtaining a second phage display library of antigen-binding molecules from the first phage display library of antigen-binding molecules by enrichment for phages presenting antigen-binding molecules that bind to antigen/antigen-binding molecule complex comprising the first antigen and the first antigen-binding molecule.

[46] The production method of [45], wherein the first and second steps are repeated using the second phage display library of antigen-binding molecules as the existing phage display library of antigen-binding molecules.

[Effects of the Invention]

According to the present invention, binding of a second antigen-binding molecule to an antigen/antigen-binding molecule complex comprising a first antigen and a first antigen-binding molecule can enhance the binding activity of the first antigen-binding molecule to the first antigen.

[Brief Description of Drawings]

Fig. 1 schematically illustrates the binding mechanism of one embodiment of the first antigen-binding molecule and one embodiment of the second antigen-binding molecule when both molecules are used in combination.
Fig. 2 schematically illustrates the mechanisms of action when one embodiment of the first antigen-binding molecule and one embodiment of the second antigen-binding molecule crosslink a target cell and an effector cell.
Fig. 3 is a diagram showing the CD3 signal-inducing abilities of a group of clamping antibody candidates prepared in Example 3, which abilities were observed by a functional assay.
Fig. 4 is a graph showing the binding activities of the clamping antibodies prepared in Example 3.
Fig. 5 is a set of graphs showing stabilization of complexes of CD3 and an anti-CD3 antibody by clamping antibodies.
Fig. 6 is a set of graphs showing TDCC activities using the same antigen.
Fig. 7 is a set of graphs showing TDCC activities against EREG/GPC3 double-positive cells.

Fig. 8 is a diagram showing the binding of adenosine, anti-adenosine antibody, and clamping antibody.
Fig. 9 is a graph showing the affinities of adenosine-clamping antibodies.
Fig. 10 is a diagram showing adenosine concentration-dependent binding between an anti-adenosine antibody and a clamping antibody.
Fig. 11 is a diagram showing adenosine concentration-dependent cytotoxic activities of a bispecific antibody using an adenosine-clamping antibody.
Fig. 12 is a diagram showing the crystal structure of an epitope peptide-fused anti-CD3 antibody Fab and a clamping antibody.
Fig. 13 is a set of graphs showing the TDCC activities against GPC3/CLDN6 double-positive cells.
Fig. 14 is a set of graphs showing the TDCC activities against GPC3/Her2 double-positive cells.
Fig. 15 is a diagram showing effector cell-specific activation.
Fig. 16 is a graph showing the TDCC activities specific to CD8-positive T cells resulting from administration of anti-cancer antigen/attenuated CD3 antibody and anti-CD8 clamping antibody.
Fig. 17 is a graph showing the antitumor effects resulting from administration of anti-cancer antigen antibody / attenuated CD3 antibody and anti-cancer antigen antibody / clamping antibody.

[Description of Embodiments]

A. Definitions

Herein, the term "polypeptide" encompasses all peptides with a plurality of amino acids linked by peptide bonds. Herein, polypeptides are sometimes referred to as "peptides" or "proteins."

Herein, the term "antigen-binding region" means a compound having an activity of specifically binding to an antigen. The antigen-binding region may be peptidic or non-peptidic.

Herein, "CH1" means a single polypeptide chain of CH1 of an antibody. Specifically, CH1 is a region represented by amino acid residues at positions 118 to 215 of a heavy chain in the EU numbering system, and herein encompasses the wild-type and also variants produced by introducing amino acid residue substitutions, additions, or deletions into the wild-type.

Herein, "CH2" means a single polypeptide chain of CH2 of an antibody. Specifically, CH2 is a region represented by amino acid residues at positions 231 to 340 of a heavy chain in the EU numbering system, and herein encompasses the wild-type and also variants produced by introducing amino acid residue substitutions, additions, or deletions into the wild-type.

Herein, "CH3" means a single polypeptide chain of CH3 of an antibody. Specifically, CH3 is a region represented by amino acid residues from position 341 to the C-terminus of a heavy chain in the EU numbering system, and herein encompasses the wild-type and also variants produced by introducing amino acid residue substitutions, additions, or deletions into the wild-type.

Herein, "CL" means a single polypeptide chain of CL of an antibody. Specifically, CL is a region represented by amino acid residues from position 108 to the C-terminus of a light chain in the EU numbering system, and herein encompasses the wild-type and variants produced by introducing amino acid residue substitutions, additions or deletions into the wild-type.

Herein, "antibody-half molecule" means a single molecule when the binding between heavy chains in an antibody is dissociated. Examples of an antibody-half molecule in the case where the antibody is IgG include a complex composed of one heavy chain and one light chain. Antibody-half molecules include molecules consisting of one heavy chain which are produced by dissociating the inter-heavy chain bonds of so-called heavy chain antibodies (also called VHHs (VH originating from heavy-chain antibody)), which are antibodies consisting of two heavy chains found in camelid antibodies and such.

In one embodiment, the antibody-half molecules include those derived from chimeric antibodies or humanized antibodies.

In one embodiment, the antibody-half molecules include those derived from various isotypes such as IgG, IgM, IgA, IgD, and IgE. The antibody-half molecules are preferably those derived from IgG. There are IgG1, IgG2, IgG3, and IgG4 in IgG. The antibody-half molecules may be derived from any of these subtypes. The antibody-half molecules may be molecules produced by dissociating the inter-heavy chain bonds of naturally-occurring antibodies or may be genetic recombinants produced by introducing amino acid residue substitutions, additions or deletions into the natural-occurring antibodies.

A "hinge region" as used herein is a region located between CH1 and CH2 in an antibody. Specifically, the hinge region is a region represented by amino acid residues at positions 216 to 230 in the EU numbering system, and herein encompasses the wild-type and also variants produced by introducing amino acid residue substitutions, additions, or deletions into the wild-type. Herein, the "hinge region portion in an antibody-half molecule" means a hinge region portion in one heavy chain, and it means a portion consisting of a single chain polypeptide.

Herein, a "constant region" is a region including CH1, CH2, CH3, CL, and a hinge region in an antibody. Herein, a "constant region portion in an antibody-half molecule" means a constant region portion in an antibody-half molecule.

The term "Fc region" herein is used to define a C-terminal region of an immunoglobulin heavy chain that contains at least a portion of the constant region. The term includes native sequence Fc regions and variant Fc regions. In one embodiment, a human IgG heavy chain Fc region extends from Cys226, or from Pro230, to the carboxyl-terminus of the heavy chain. However, the C-terminal lysine (Lys447) or glycine-lysine (residues 446-447) of the Fc region may or may not be present. Unless otherwise specified herein, numbering of amino acid residues in the Fc region or constant region is according to the EU numbering system, also called the EU index, as described in Kabat et al., Sequences of Proteins of Immunological Interest, 5th Ed. Public Health Service, National Institutes of Health, Bethesda, MD, 1991.

Herein, "effector functions" refer to those biological activities attributable to the Fc region of an antibody, which vary with the antibody isotype, and activities of controlling immune cell response by a modified antibody. Examples of antibody effector functions include: C1q binding and complement dependent cytotoxicity (CDC); Fc receptor binding; antibody-dependent cell-mediated cytotoxicity (ADCC); T-cell dependent cytotoxicity (TDCC); phagocytosis; down regulation of cell surface receptors (e.g. B cell receptor); and B cell activation.

The term "Fc receptor" or "FcR" refers to a receptor that binds to the Fc region of an antibody. In some embodiments, an FcR is a native human FcR. In some embodiments, an FcR is one which binds an IgG antibody (a gamma receptor) and includes receptors of the Fc gamma RI, Fc gamma RII, and Fc gamma RIII subclasses, including allelic variants and alternatively spliced forms of those receptors. Fc gamma RII receptors include Fc gamma RIIA (an "activating receptor") and Fc gamma RIIB (an "inhibiting receptor"), which have similar amino acid sequences that differ primarily in the cytoplasmic domains thereof. Activating receptor Fc gamma RIIA contains an immunoreceptor tyrosine-based activation motif (ITAM) in its cytoplasmic domain. Inhibiting receptor Fc gamma RIIB contains an immunoreceptor tyrosine-based inhibition motif (ITIM) in its cytoplasmic domain. (see, *e.g.,* Daeron, Annu. Rev. Immunol. 15:203-234 (1997)). FcRs are reviewed, for example, in Ravetch and Kinet, Annu. Rev. Immunol 9:457-92 (1991); Capel et al., Immunomethods 4:25-34 (1994); and de Haas et al., J. Lab. Clin. Med. 126:330-41 (1995). Other FcRs, including those to be identified in the future, are encompassed by the term "FcR" herein.

The term "covalent bond" herein includes all those generally known. "Covalent bonds" includes, for example, disulfide bonds and carbon-carbon bonds.

The term "cytotoxic agent" as used herein refers to a substance that inhibits or prevents a cellular function and/or causes cell death or destruction. Cytotoxic agents include, but are not limited to, radioactive isotopes (e.g., $^{211}At$, $^{131}I$, $^{125}I$, $^{90}Y$, $^{186}Re$, $^{188}Re$, $^{153}Sm$, $^{212}Bi$, $^{32}P$, $^{212}Pb$ and radioactive isotopes of Lu); chemotherapeutic agents or drugs (e.g., methotrexate, adriamycin, vinca alkaloids (vincristine, vinblastine, etoposide), doxorubicin, melphalan, mitomycin C, chlorambucil, daunorubicin or other intercalating agents); growth inhibitory agents; enzymes and fragments thereof such as nucleolytic enzymes; antibiotics; toxins such as small molecule toxins or enzymatically active toxins of bacterial, fungal, plant or animal origin, including fragments and/or variants thereof; and the various antitumor or anticancer agents disclosed below.

The term "binding activity" as used herein is used to refer to the strength of bonds formed between molecules. The types of bonds formed between molecules do not include covalent bonds, but include intermolecular bonds such as hydrogen bonds, electrostatic forces, van der Waals forces, and hydrophobic bonds. The sum of these bonds determines the binding activity between molecules. Herein, the binding activity is expressed in particular by the dissociation constant KD. KD can be determined using data from known assays that examine binding between molecules. Examples of the assays include surface plasmon resonance (SPR), biolayer interference (BLI), enzyme-linked immunosorbent assay (ELISA), and fluorescence-activated cell sorter (FACS). Of these, SPR is preferable. For example, Biacore (registered trademark) T200 (GE Healthcare) is used for measuring the binding activity by SPR.

KD when measured by SPR using Biacore (registered trademark) T200 can range from approximately $1 \times 10^{-12}$ to approximately $1 \times 10^{-4}$. The larger the KD is within this range ($1 \times 10^{-12}$ to $1 \times 10^{-4}$), the lower the binding activity, and the smaller the KD, the higher the binding activity. In the binding activity of an antigen-binding molecule to an antigen, when KD is $1 \times 10^{-6}$ or more, the antigen-binding molecule often cannot easily exhibit a physiological function. For example, when the antigen-binding molecule is an antibody, it is often difficult to exert its effector function. Therefore, herein, a case where KD is $1 \times 10^{-6}$ or more as measured by SPR is defined as "low binding activity", and a case where KD is lower than $1 \times 10^{-6}$ is defined as "high binding activity".

The temperature conditions for intermolecular binding assays are usually 25°C to 37°C. The temperature condition in the case of SPR is preferably 25°C or 37°C, and more preferably 37°C. The temperature condition for BLI is preferably 30°C. The temperature condition in the case of ELISA is preferably 25°C. As the running buffer used for the binding assay, a commercially available buffer may be used, or it may be prepared at the time of use. Examples of the commercially available buffers include HBS-EP+ (GE Healthcare) (0.01 M HEPES, 0.15 M NaCl, 3 mM EDTA, 0.05% (v/v) polyoxyethylene (20) sorbitan monolaurate, pH 7.4). Example of the buffers prepared at the time of use include ACES buffer (20 mM ACES (Nacalai tesque), 150 mM NaCl, 0.05% (w/v) polyoxyethylene (20) sorbitan monolaurate (Junsei Chemical), pH 7.4). The test compound is dissolved in the desired buffer. The main constituent that may affect intermolecular binding during the assay is NaCl. The concentration of NaCl differs depending on the purpose of the experiment to be performed, and the concentration of NaCl in buffers used in conventional experiments performed without examining salt concentration conditions is 150 mM. That is, the concentration of NaCl in conventionally used running buffers is preferably 150 mM. pH can also affect intermolecular binding during the assay, and buffers used in conventional experiments performed without examining pH conditions have pH 7.4. That is, pH 7.4 is preferred for conventionally used running buffers.

B. First antigen-binding molecule

The first antigen-binding molecule of the present invention binds to a first antigen. That is, the first antigen-binding molecule includes a first antigen-binding region that binds to the first antigen. The first antigen-binding molecule binds to the first antigen to form an antigen/antigen-binding molecule complex. In certain embodiments, the first antigen-binding molecule is an antigen-binding molecule that is not expressed *in vivo.* The first antigen-binding molecule is preferably an antigen-binding molecule that is not expressed *in vivo.* "An antigen-binding molecule is not expressed *in vivo"* means that "an antigen-binding molecule is not a protein or fragment thereof that is ordinarily expressed in a living body that has not undergone any artificial treatment such as medication or immunization".

In one embodiment, the binding activity of the first antigen-binding molecule or the first antigen-binding region to the first antigen is enhanced by the undermentioned second antigen-binding molecule (herein, sometimes called a "clamping molecule") that binds to the antigen/antigen-binding molecule complex. The first antigen-binding molecule is not particularly limited as long as the binding activity to the first antigen is enhanced by the second antigen-binding molecule, and it may be a complete antibody consisting of two light chain molecules and two heavy chain molecules, such as a native antibody, or may be an antibody fragment such as an antibody half-molecule, diabody (Db), scFv, single chain antibody, $sc(Fv)_2$, or $sc(Fab')_2$.

In another embodiment, when the first antigen is a receptor described below, the first antigen-binding molecule or the first antigen-binding region may be a ligand for the receptor. For example, if the first antigen is a T cell receptor complex, co-stimulatory molecule, or coinhibitory molecule, the first antigen-binding molecule or first antigen-binding region may be their ligand. Specifically, when the first antigen is PD-1, the first antigen-binding molecule or the first antigen-binding region may be PD-L1 or PD-L2.

Whether the binding activity of the first antigen-binding molecule or the first antigen-binding region to the first antigen is enhanced by the second antigen-binding molecule that binds to the antigen/antigen-binding molecule complex is determined, for example, from the value of KD $(clamping^-)$ / KD $(clamping^+)$, i.e. KD $(clamping^-)$ divided by KD $(clamping^+)$, wherein KD $(clamping^-)$ is the dissociation constant of the first antigen-binding molecule or the first antigen-binding region for the first antigen in the absence of the second antigen-binding molecule $(clamping^-)$, and KD $(clamping^+)$ is the dissociation constant of the first antigen-binding molecule or the first antigen-binding region for the first antigen in the presence of the second antigen-binding molecule $(clamping^+)$, as determined by SPR. The above-mentioned phrase "the binding activity of the first antigen-binding molecule or the first antigen-binding region to the first antigen is enhanced by the second antigen-binding molecule described below that binds to the antigen/antigen-binding molecule complex" means that KD $(clamping^-)$ / KD $(clamping^+)$ is higher than 1.

A higher KD $(clamping^-)$ / KD $(clamping^+)$ means that the increase rate of the binding activity of the first antigen-binding molecule or the first antigen-binding region to the first antigen in the presence of the second antigen-binding molecule relative to that in the absence of the second antigen-binding molecule is higher. In other words, this means that the presence of the second antigen-binding molecule switches on/off more distinctly the binding of the first antigen-binding molecule or the first antigen-binding region to the first antigen.

In one aspect, the binding activity of the first antigen-binding molecule to the first antigen may be high binding activity or low binding activity as measured by SPR, and it may be as low as undetectable by SPR.

When the first antigen-binding molecule is a bispecific antibody and the first antigen is an immune-related molecule described later, from the viewpoint of reducing side effects, preferably, the binding activity of the first antigen-binding molecule to the first antigen is low binding activity as measured by SPR or as low as undetectable by SPR. Here, the expression "as low as undetectable by SPR" means that the binding activity of the first antigen-binding molecule to the first antigen cannot be detected by SPR, but the first antigen-binding molecule specifically binds to the first antigen even slightly. When the binding activity of the first antigen-binding molecule to the first antigen is as low as undetectable by SPR, the above-mentioned KD $(clamping^-)$ / KD $(clamping^+)$ is not used.

In one embodiment, the binding activity of the second antigen-binding molecule to the first antigen in the presence of the first antigen-binding molecule is higher than that in the absence of the first antigen-binding molecule. It is presumed that this is due to any one of the following mechanisms: the mechanism in which the binding activity of the second antigen-binding molecule to the antigen/antigen-binding molecule complex is higher than to the free first antigen; the mechanism in which binding of the second antigen-binding molecule to the free first antigen-binding molecule enhances the binding activity of the second antigen-binding molecule to the free first antigen; the mechanism in which the complex composed of the first antigen and the second antigen-binding molecule is stabilized by binding to the free first antigen-binding molecule; or a combination thereof.

In this embodiment, as an indicator of the binding activity of the second antigen-binding molecule to the first antigen in the presence of the first antigen-binding molecule compared to that in the absence of the first antigen-binding molecule, for example, KD (first antigen-binding molecule -) / KD (first antigen-binding molecule +), i.e. KD (first antigen-binding molecule -) divided by KD (first antigen-binding molecule +) is used, wherein KD (first antigen-binding molecule -) is the dissociation constant of the second antigen-binding molecule for the free first antigen in the absence of the first antigen-binding molecule, and KD (first antigen-binding molecule +) is the dissociation constant of the second antigen-binding molecule for the first antigen in the presence of the first antigen-binding molecule, as determined by SPR. The above-mentioned expression "binding activity of the second antigen-binding molecule to the first antigen in the presence of the first antigen-binding molecule is higher than that in the absence of the first antigen-binding molecule" means that the KD (first antigen-binding molecule -) / KD (first antigen-binding molecule +) is higher than 1.

In one aspect, the binding activity of the second antigen-binding molecule to the free first antigen may be high binding activity or low binding activity as measured by SPR, and it may be as low as undetectable by SPR.

When the second antigen-binding molecule is a bispecific antibody and the first antigen is an immune-related molecule described below, from the viewpoint of reducing side effects, preferably, the binding activity of the second antigen-binding molecule to the free first antigen is low binding activity as measured by SPR or as low as undetectable by SPR. Here, the expression "as low as undetectable by SPR" means that the binding activity of the second antigen-binding molecule to the free first antigen cannot be detected by SPR, but the second antigen-binding molecule specifically binds to the free first antigen even slightly. When the binding activity of the second antigen-binding molecule to the free first antigen is as low as undetectable by SPR, the above-mentioned KD (free) / KD (complex) is not used.

In one embodiment, as the binding activity of the second antigen-binding molecule to the antigen/antigen-binding molecule complex, KD of the second antigen-binding molecule for the antigen/antigen-binding molecule complex as determined by SPR is used. The KD is usually indicated in the range of approximately $1 \times 10^{-12}$ to approximately $1 \times 10^{-4}$. The lower the KD, the stronger the binding of the second antigen-binding molecule to the antigen/antigen-binding molecule complex.

In one embodiment, in measuring the binding activity of the second antigen-binding molecule to the first antigen in the presence of the first antigen-binding molecule, and in measuring the binding activity of the second antigen-binding molecule to the antigen/antigen-binding molecule complex, the first antigen fused with the first antigen-binding molecule may be used.

In one embodiment, when comparing the binding activity of the second antigen-binding molecule to the antigen/antigen-binding molecule complex with the binding activity of the second antigen-binding molecule to the first antigen-binding molecule, the binding activity of the second antigen-binding molecule to the first antigen-binding molecule measured in the presence of the first antigen can be compared with the binding activity of the second antigen-binding molecule to the first antigen-binding molecule measured in the absence of the first antigen.

a. First antigen

The first antigen is not particularly limited, and includes any antigens. Specific antigen types include those described in WO2013/180200. The first antigen is preferably, but is not limited to, an immune-related molecule or cellular metabolite.

In one embodiment, the first antigen is an extracellular protein. The extracellular proteins include cell membrane proteins. Preferably, the extracellular protein is a cell membrane protein.

In another embodiment, the first antigen is a native protein. This native protein is not a protein expressed in cells by genetic engineering. The first antigen is preferably a native cell membrane protein.

The immune-related molecule herein includes any molecule as long as it is a molecule produced by immune cells. The immune-related molecule may be, for example, a molecule present on cell membrane or a molecule released extracellularly.

Specific examples of molecules present on the cell membrane of immune cells include T cell-activating factors such as T cell receptor complexes, co-stimulatory molecules, and coinhibitory molecules. Of these, T cell receptor complexes and co-stimulatory molecules are preferred. More preferably, the molecule present in the cell membrane of the immune cell is a native protein rather than a protein expressed by genetic engineering.

Examples of co-stimulatory molecules include CD2, CD27, CD28, CD40, CD137 (4-1BB), CD40, OX40 (CD134), ICOS (inducible co-stimulator), DR3, GITR, CD30, TIM1, SLAM, and CD226. The T cell receptor complex includes its constituent, for example, CD3. CD3 has subtypes, CD3γ, CD3δ, CD3ε, and CD3ζ. Among these, CD3ε is preferable.

Examples of co-inhibitory molecules include CTLA4, PD1, TIM3, TIGIT, CD160, LAG3, LAIR1, B7-1, and B7-H1.

Examples of the molecules released extracellularly include various cytokines.

Examples of the above-mentioned immune cells include granulocytes, macrophages, dendritic cells, T cells, and B cells. The immune cell is preferably at least one selected from the group consisting of granulocytes, macrophages, dendritic cells, T cells, and B cells, and is more preferably T cells. Examples of T cell types include CD4-positive, CD8-positive, Th1, Th2, and Th12. Among these, CD8-positive is preferable.
The cellular metabolite herein is a cellular metabolite released extracellularly. The cellular metabolite is not particularly limited, and includes any metabolites. The cellular metabolite is preferably a compound that is not administered to a living body but is generated internally from any tissue in the living body. Specific types of cell metabolites include cancer tissue-specific metabolites and inflammatory tissue-specific metabolites described in WO2013/180200.
Examples of cancer tissue-specific metabolites include primary metabolites of the glycolytic pathway or the Krebs cycle, such as lactic acid, succinic acid and citric acid, amino acids such as alanine, glutamic acid, and aspartic acid, and amino acid metabolites such as kynurenine, arachidonic acid metabolites such as prostaglandin E2, nucleosides having a purine ring structure such as adenosine, adenosine triphosphate (ATP), adenosine diphosphate (ADP), and adenosine monophosphate (AMP), uric acid, and 1-methylnicotinamide. Among these, a nucleoside carrying a purine ring structure is preferable, and adenosine is more preferable.
Examples of inflammatory tissue-specific metabolites include arachidonic acid metabolites such as prostaglandin E2, nucleosides carrying a purine ring structure such as adenosine, adenosine triphosphate (ATP), adenosine diphosphate (ADP), and adenosine monophosphate (AMP), and uric acid. Among these, a nucleoside carrying a purine ring structure is preferable, and adenosine is more preferable.

b. Second antigen

The first antigen-binding molecule may bind to a single antigen or may bind to a plurality of antigens and have a so-called multiple antigen specificity.
When the first antigen-binding molecule has multiple antigen specificity, the first antigen-binding molecule binds to at least the second antigen. That is, the first antigen-binding molecule comprises a second antigen-binding region that binds to the second antigen. Examples of the second antigen-binding region include antibody fragments. The antibody fragment may be any fragment as long as it can bind to the second antigen. Examples of the antibody fragment include Fv and Fab.
The second antigen is not particularly limited, and includes any antigens. Specific antigen types include those described in WO2013/180200. Preferably, the second antigen is a cancer antigen or an immune-related molecule. More preferably, the second antigen is a cancer antigen.
Specific examples of cancer antigens include cancer-specific antigens exemplified in WO2015/156268. The immune-related molecule and its examples are the same as the immune-related molecule in the first antigen described above. When the first antigen is an immune-related molecule, preferably the second antigen is an antigen other than an immune-related molecule, and is more preferably a cancer antigen.
In one embodiment, the second antigen is an extracellular protein. The extracellular proteins include cell membrane proteins. Preferably, the extracellular protein is a cell membrane protein.

c. First other component

In one embodiment, the first antigen-binding molecule may or may not comprise a component other than the antigen-binding region (first other component). The first other component is, for example, an antibody fragment, a linker, and a cytotoxic agent.
From the viewpoint that various functions can be added to the first antigen-binding molecule, preferably, the first antigen-binding molecule comprises the first other component. In order to improve the stability in plasma, production efficiency, and such of the first antigen-binding molecule, the first other component is preferably an antibody fragment. Antibody fragments include antibody Fc regions and antibody constant regions.
When the first antigen-binding molecule comprises an antibody Fc region, the Fc region may be a native Fc region having the same amino acid sequence as the Fc region of a native antibody, or may be a modified Fc region produced by modifying a native Fc region. In this case, the Fc region of the antibody is preferably derived from the Fc region of IgG The IgG is preferably human-derived.
When the first antigen-binding molecule comprises an antibody constant region, the constant region may be a native constant region having the same amino acid sequence as the constant region of a native antibody, or may be a modified constant region produced by modifying the native constant region. In this case, the constant region of the antibody is preferably derived from an IgG constant region. The IgG is preferably human-derived.
In one embodiment, when the first antigen-binding molecule comprises a modified Fc region or a modified constant region as the first other component, from the viewpoint of suppressing an undesired immune response such as a cytokine storm, the modified Fc region or the modified constant region is a modified Fc region or a modified constant region that has suppressed or no binding to FcγR.

Examples of the modified Fc regions or modified constant regions which have suppressed or no binding to FcγR include the modified Fc region, modified constant region, or such described in WO2012/073985.

In one embodiment, when the first antigen-binding molecule has dual antigen specificity and when a heterodimer of a polypeptide comprising a first antigen-binding region and a polypeptide comprising a second antigen-binding region is formed, from the viewpoint of production efficiency, the first antigen-binding molecule preferably comprises a modified Fc region or a modified constant region.

In this case, for example, the polypeptide comprising the first antigen-binding region and the polypeptide comprising the second antigen-binding region each have at least a first CH3 and a second CH3. Specific examples of the modified Fc regions or the modified constant regions in this case include the modifications (i) to (iii) below.

(i) a modification where either one of the first CH3 and the second CH3 has a positively-charged region and the other has a negatively-charged region, and when the heterodimer is formed, the positively-charged region interacts with the negatively-charged region;
(ii) a modification where either one of the first CH3 and the second CH3 has a convex portion and the other has a concave portion, and when the heterodimer is formed, the convex portion fits into and interacts with the concave portion; and
(iii) a modification where the first CH3 and the second CH3 are modified IgG CH3, a part of which is replaced with a part of IgA CH3, and when the heterodimer is formed, the replaced part of IgA CH3 in the first CH3 interacts with the replaced part of IgA CH3 in the second CH3.

Examples of the modifications of (i) above are described in WO 2006/106905, WO 2009/089004, WO 2010/129304, and WO 2014/084607.

Specific examples include: modifying at least one combination from among the combinations of positions 356 and 439, positions 357 and 370, and position 399 and 409 according to the EU numbering system in the amino acid sequence of one heavy chain constant region, to amino acids having the same charge; and modifying at least one combination from among the combinations of positions 356 and 439, positions 357 and 370, and positions 399 and 409 according to the EU numbering system in the other heavy chain constant region, to amino acids having a charge opposite to that of the one heavy chain constant region. More specifically, for example, either one of the heavy chain constant regions is introduced with a mutation that substitutes Glu at position 356 in the EU numbering system with Lys, and the other heavy chain constant region is introduced with a mutation that substitutes Lys at position 439 in the EU numbering system with Glu.

Examples of the modification of (ii) above are described in WO 96/027011 and Margaret Merchant et al., Nature Biotechnology 1998, 16, 677-681. Specific examples include: the combination of introducing T366Y to one CH3 and Y407A to the other CH3; or the combination of introducing T366W to one CH3 and Y407A to the other CH3, or the combination of introducing F405A to one CH3 and T394W to the other CH3, or the combination of introducing Y407T to one CH3 and T366Y to the other CH3, or the combination of introducing T366Y/F405A to one CH3 and T394W/Y407T to the other CH3, or the combination of introducing T366W/F405W to one CH3 and T394S/Y407A to the other CH3, or the combination of introducing F405W/Y407A to one CH3 and T366W/T394S to the other CH3, or the combination of introducing F405W to one CH3 and T394S to the other CH3, or the combination of introducing T366W to one CH3 and T366S/L368A/Y407V to the other CH3. The modification of (ii) can be combined with the modification of (i). Examples of such combinations include those described in WO 2012/058768.

The modification of (iii) above is a technique of using strand-exchange engineered domain CH3s, in which a part of one heavy chain CH3 of an antibody is modified to a sequence derived from IgA corresponding to that part, and the complementary part of the other heavy chain CH3 is introduced with an IgA-derived sequence corresponding to that part, to efficiently induce the interaction of polypeptides having different sequences by complementary interaction of the CH3s (Protein Engineering Design & Selection, 23; 195-202, 2010). This known technique can also be used to efficiently produce a first antigen-binding molecule having multiple antigen specificity. Examples of the modification of (iii) include the modification technique described in WO 2007/110205.

In addition to the modifications (i) to (iii) above, modifications in CH3 described in WO96/027011 may be used for the modified Fc region or the modified constant region. Furthermore, the modification in the hinge region portion described in WO2011/143545 and the FAE technique described in WO2014/104165 may be used for the modified constant region.

d. Examples of the first antigen-binding molecules

Examples of the first antigen-binding molecules or the first antigen-binding regions when the first antigen is CD3 are shown below. In this case, as long as the first antigen-binding molecule is a molecule that binds to CD3, it may be a newly prepared molecule or a known molecule such as those described in WO2016/020444, WO2008/119565, or WO2007/042261.

As a specific example of the present examples, the first antigen-binding molecule or the first antigen-binding region comprises a CD3-binding polypeptide consisting of any combination of heavy chain variable region and light chain variable region amino acid sequences selected from SEQ ID NO: 1 and SEQ ID NO: 122, SEQ ID NO: 114 and SEQ ID NO:115, SEQ ID NO: 116 and SEQ ID NO: 117, SEQ ID NO: 118 and SEQ ID NO: 119, and SEQ ID NO: 120 and SEQ ID NO: 121, respectively; or a first modified polypeptide produced by modifying the CD3-binding polypeptide. The CD3-binding activity of the first modified polypeptide is lower than that of the CD3-binding polypeptide.

In this specific example, the first antigen-binding molecule or the first antigen-binding region preferably comprises a first modified polypeptide formed by modifying a CD3-binding polypeptide consisting of any combination selected from SEQ ID NO: 1 and SEQ ID NO: 122, SEQ ID NO: 114 and SEQ ID NO: 115, SEQ ID NO: 116 and SEQ ID NO: 117, SEQ ID NO: 118 and SEQ ID NO: 119, and SEQ ID NO: 120 and SEQ ID NO: 121. Such modification includes any modification as long as the CD3-binding activity of the first modified polypeptide is lower than that of the CD3-binding polypeptide. The amino acid sequence homology between the first modified polypeptide and the original CD3-binding polypeptide for modification is preferably 80% or more, and more preferably 90% or more.

In this specific example, as an index of comparison between the CD3-binding activity of the first modified polypeptide and the CD3-binding activity of the pre-modified CD3-binding polypeptide, for example, KD (before modification) / KD (after modification), i.e. KD (before modification) divided by KD (after modification) is used, wherein KD (before modification) is the dissociation constant of the pre-modified CD3-binding polypeptide for CD3, and KD (after modification) is that of the first modified polypeptide for CD3, as determined by SPR. The above-mentioned expression "CD3-binding activity of the first modified polypeptide is lower than that of the CD3-binding polypeptide" means that KD (before modification) / KD (after modification) is higher than 1.

The CD3-binding activity of the first modified polypeptide may be high binding activity or low binding activity as measured by SPR, or it may be as low as undetectable by SPR. Preferably, the CD3-binding activity of the first modified polypeptide is low binding activity as measured by SPR or so low that its detection by SPR is impossible.

When the CD3-binding activity of the first modified polypeptide is as low as undetectable by SPR, the above-mentioned KD (before modification) / KD (after modification) is not used, and the KD value of the first modified polypeptide for CD3 in the presence of the second antigen-binding molecule described later is used. The CD3-binding activity of the first modified polypeptide in the presence of the second antigen-binding molecule, for example, when the first antigen-binding molecule is an antibody, may be within a range where that antibody can exert effector functions. Preferably, the CD3-binding activity of the first modified polypeptide in the presence of the second antigen-binding molecule is high binding activity.

In this specific example, the first antigen-binding molecule is not likely to bind to CD3 in the absence of the second antigen-binding molecule, but is more likely to bind to CD3 in the presence of the second antigen-binding molecule. This is useful for the on/off mechanism of the binding of the first antigen-binding molecule to CD3 mediated by the second antigen-binding molecule. For example, when a first antigen-binding molecule and a second antigen-binding molecule, both having multiple antigen specificities are used in combination as a medicine, the T cell-mediated cytotoxic activity is induced more specifically to the target cell by this mechanism. Therefore, side effects will be further reduced.

The subtype of CD3 used for SPR when determining the KD values for CD3 described above may be any one of CD3γ, CD38, CD3ε and CD3ζ, or a combination thereof. Among these, CD3ε is preferable as the subtype of CD3. All subtypes of CD3 are preferably human-derived.

The epitope of CD3ε to which the first antigen-binding molecule binds is not particularly limited, and preferably, the epitope of CD3ε comprises at least the amino acid sequence from the N-terminal to position 27 of CD3ε, and more preferably, the epitope of CD3ε comprises at least the amino acid sequence from the N-terminus to position 8 of CD3ε, and most preferably, the epitope of CD3ε comprises at least the amino acid sequence from the N-terminus to position 5 of CD3ε.

Examples of the first antigen-binding molecules or the first antigen-binding regions when the first antigen is adenosine are shown below. As a specific example of the present examples, the first antigen-binding molecule or the first antigen-binding region comprises an adenosine-binding polypeptide consisting of any combination of heavy chain variable region and light chain variable region amino acid sequences selected from SEQ ID NO: 106 and SEQ ID NO: 107, SEQ ID NO: 108 and SEQ ID NO:109, SEQ ID NO: 110 and SEQ ID NO: 111, and SEQ ID NO: 112 and SEQ ID NO: 113, respectively; or a second modified polypeptide produced by modifying the adenosine-binding polypeptide. In this specific example, the adenosine-binding activity of the second modified polypeptide may be higher or lower than that of the adenosine-binding polypeptide. Such modifications include any modifications. The amino acid sequence homology between the second modified polypeptide and the original adenosine-binding polypeptide for modification is preferably 80% or more, and more preferably 90% or more. In this specific example, when the heavy chain variable region or the light chain variable region is derived from a non-human animal, the second modified polypeptide includes a humanized polypeptide.

In this specific example, as an index of comparison between the adenosine-binding activity of the second modified polypeptide and the adenosine-binding activity of the pre-modified adenosine-binding polypeptide, for example, KD (before modification) / KD (after modification), i.e. KD (before modification) divided by KD (after modification) is used, wherein KD (before modification) is the dissociation constant of the pre-modified adenosine-binding polypeptide for adenosine, and KD (after modification) is that of the second modified polypeptide for adenosine, as determined by SPR. The above-mentioned expression "adenosine-binding activity of the second modified polypeptide is lower than that of the adenosine-binding polypeptide" means that KD (before modification) / KD (after modification) is higher than 1. Conversely, the expression "adenosine-binding activity of the second modified polypeptide is higher than that of the adenosine-binding polypeptide" means that KD (before modification) / KD (after modification) is lower than 1.

The adenosine-binding activity of the second modified polypeptide may be high binding activity or low binding activity as measured by SPR, or it may be as low as undetectable by SPR.

When the adenosine-binding activity of the second modified polypeptide is as low as undetectable by SPR, the above-mentioned KD (before modification) / KD (after modification) is not used, and the KD value of the second modified polypeptide for adenosine in the presence of the second antigen-binding molecule described below is used. The adenosine-binding activity of the second modified polypeptide in the presence of the second antigen-binding molecule, for example, when the first antigen-binding molecule is an antibody, may be within a range where that antibody can exert effector functions. Preferably, the binding activity of the second modified polypeptide to adenosine in the presence of the second antigen-binding molecule is high binding activity.

In the above-mentioned specific examples, an example in which the first antigen is CD3 or adenosine has been shown. Needless to say, the antibody modification technique of the present invention described below that enhances the binding activity of the first antigen-binding molecule to the first antigen by using the second antigen-binding molecule, can also be applied to first antigens other than CD3 and adenosine.

e. Third antigen

The second antigen-binding molecule used in combination with the first antigen-binding molecule may bind to a single antigen, or it may bind to a plurality of antigens and have a so-called multiple antigen specificity. A third antigen-binding region and a third antigen are the same as those in the second antigen-binding molecule described below.

C. Second antigen-binding molecule

The second antigen-binding molecule of the present invention binds to an antigen/antigen-binding molecule complex. That is, the second antigen-binding molecule comprises a complex-binding region that binds to the complex. The complex comprises a first antigen and a first antigen-binding molecule that binds to the first antigen.

The second antigen-binding molecule is not particularly limited as long as it comprises a complex-binding region and enhances the binding activity of the first antigen-binding molecule to the first antigen, and it may be a complete antibody consisting of two light chain molecules and two heavy chain molecules, such as a native antibody, or may be an antibody fragment such as an antibody half-molecule, diabody (Db), scFv, single chain antibody, $sc(Fv)_2$, or $sc(Fab')_2$.

The mechanism by which the second antigen-binding molecule binds to the complex is not particularly limited as long as it binds to the complex. Preferably, the second antigen-binding molecule binds to both the first antigen and the first antigen-binding molecule when binding to the complex. That is, the epitopes in the complex for the second antigen-binding molecule are included in both the first antigen-binding molecule and the first antigen.

When the first antigen-binding molecule is an antibody comprising a heavy chain variable region and a light chain variable region, the epitopes for the second antigen-binding molecule are included in the first antigen and in either or both of the heavy chain variable region and the light chain variable region in the first antigen-binding molecule. In this case, the epitopes to which the second antigen-binding molecule binds are preferably included in the first antigen and in the heavy chain variable region in the first antigen-binding molecule.

The second antigen-binding molecule binds to the first antigen and the first antigen-binding molecule before and after the complex is formed, thereby increasing the binding activity of the first antigen-binding molecule to the first antigen. That is, the second antigen-binding molecule stabilizes the complex.

In one embodiment, the second antigen-binding molecule has a higher binding activity to the first antigen in the presence of the first antigen-binding molecule than in the absence of the first antigen-binding molecule. It is presumed that this is due to either or both of the mechanisms: the mechanism in which the second antigen-binding molecule has a higher binding activity to the complex than to the first antigen that is free from the first antigen-binding molecule; and the mechanism in which binding to the first antigen-binding molecule that is free from the first antigen enhances the binding activity of the second antigen-binding molecule to the first antigen that is free from the first antigen-binding molecule.

In this embodiment, an index of comparison between the binding activity of the second antigen-binding molecule to the first antigen that is free from the first antigen-binding molecule and the binding activity of the second antigen-binding molecule to the first antigen in the presence of the first antigen-binding molecule in this embodiment is the same as the index for the above-mentioned first antigen-binding molecule.

In this embodiment, binding of the second antigen-binding molecule to the first antigen is enhanced by the presence of the first antigen-binding molecule. This means that the specificity of the second antigen-binding molecule for the first antigen becomes higher due to the presence of the first antigen-binding molecule. By utilizing this characteristic, side effects are further reduced, particularly when the first antigen-binding molecule and the second antigen-binding molecule have dual antigen specificity, and both molecules are used in combination as a medicine.

a. Examples of the first antigen-binding molecules

Examples of the second antigen-binding molecules or the second antigen-binding regions when the first antigen is CD3 are shown below. A specific example of the present examples is a second antigen-binding molecule or a second antigen-binding region comprising a polypeptide consisting of any combination of heavy chain variable region and light chain variable region amino acid sequences selected from SEQ ID NO: 45 and SEQ ID NO: 46, SEQ ID NO: 47 and SEQ ID NO: 48, SEQ ID NO: 49 and SEQ ID NO: 50, and SEQ ID NO: 51 and SEQ ID NO: 52, respectively.

Example of the second antigen-binding molecules or the second antigen-binding regions when the first antigen is adenosine are shown below. A specific example of the present examples is a second antigen-binding molecule or a second antigen-binding region comprising a polypeptide consisting of a combination of the following heavy-chain variable-region and the light-chain variable-region amino acid sequences.

The polypeptide consists of any combination of heavy chain variable region and light chain variable region amino acid sequences selected from SEQ ID NO: 160 and SEQ ID NO: 161, SEQ ID NO: 162 and SEQ ID NO: 163, and SEQ ID NO: 164 and SEQ ID NO: 165, respectively.

b. Third antigen

The second antigen-binding molecule may be a molecule that binds to a single antigen or a molecule that binds to a plurality of antigens and has a so-called multiple antigen specificity.

When the second antigen-binding molecule has multiple antigen specificity, the second antigen-binding molecule binds to at least a third antigen. That is, the second antigen-binding molecule includes a third antigen-binding region that binds to a third antigen. Examples of the third antigen-binding regions include antibody fragments. The antibody fragment may be any fragment as long as it can bind to the third antigen. Examples of the antibody fragment include Fv and Fab.

The third antigen is not particularly limited, and includes any antigens. Specific antigen types include antigens described in WO2013/180200. Preferably, the third antigen is a cancer antigen or an immune-related molecule. More preferably, the third antigen is a cancer antigen. When the third antigen is an immune-related molecule, CD8 is exemplified as the immune-related molecule.

Specific examples of cancer antigens are the same as the cancer antigens in the second antigen described above. However, when both the second antigen and the third antigen are cancer antigens, preferably, the second antigen and the third antigen are different types of cancer antigens. More preferably, the second and third antigens are different types of cancer antigens expressed in the same cancer cell or cancer tissue.

The immune-related molecule and its examples are the same as the immune-related molecule in the first antigen described above. However, when both the first antigen and the third antigen are immune-related molecules, preferably, the first antigen and the third antigen are different types of immune-related molecules. The term "different types" as used herein includes the case where they are different regions on the surface of the primary structure or higher-order structure of a single protein.

In one embodiment, the third antigen is an extracellular protein. The extracellular proteins include cell membrane proteins. Preferably, the extracellular protein is a cell membrane protein.

c. Second other component

In one embodiment, the second antigen-binding molecule may or may not comprise a component other than the antigen-binding region (second other component). The second other component is, for example, an antibody fragment, a linker, and a labeling compound.

From the viewpoint that various functions can be added to the second antigen-binding molecule, preferably, the second antigen-binding molecule comprises the second other component. In order to improve the stability in plasma, production efficiency, and such of the first antigen-binding molecule in plasma, the second other component is preferably an antibody fragment. Antibody fragments include antibody Fc regions and antibody constant regions.

When the second antigen-binding molecule comprises an antibody Fc region, the Fc region may be a native Fc region having the same amino acid sequence as the Fc region of a native antibody, or may be a modified Fc region produced by modifying a native Fc region. In this case, the Fc region of the antibody is preferably derived from the Fc region of IgG The IgG is preferably human-derived.

When the second antigen-binding molecule comprises an antibody constant region, the constant region may be a native constant region having the same amino acid sequence as the constant region of a native antibody, and a modified constant region formed by modifying the native constant region. In this case, the constant region of the antibody is preferably derived from an IgG constant region. The IgG is preferably human-derived.

In one embodiment, when the second antigen-binding molecule has dual antigen specificity and when a heterodimer of a polypeptide comprising a complex-binding region and a polypeptide comprising a third antigen-binding region is formed, from the viewpoint of production efficiency, preferably, the Fc region or the constant region is a modified Fc region or a modified constant region, respectively. As a specific example of the modified Fc region or modified constant region in this case, at least one modification of (i) to (iii) in the first other component described above, or modification in the hinge region portion may be applied. In this case, the modification in the first other component and the modification in the second other component may be combined so that a heterodimer is more likely to be formed.

d. First antigen

The first antigen of the first antigen-binding molecule used in combination with the second antigen-binding molecule is the same as that in the first antigen-binding molecule described above.

e. Second antigen

The first antigen-binding molecule used in combination with the second antigen-binding molecule may bind to a single antigen, or may bind to a plurality of antigens and have a so-called multiple antigen specificity. The second antigen-binding region and the second antigen are the same as those in the first antigen-binding molecule described above.

D. Suitable combination of the first antigen, the second antigen, and the third antigen

When the first antigen-binding molecule and the second antigen-binding molecule both have multiple antigen specificity, the combination of the types of the first antigen, the second antigen, and the third antigen is preferably any one of the combinations (1) to (5) below:

(1) a combination in which the first antigen is an immune-related molecule, the second antigen is a first cancer antigen, and the third antigen is a second cancer antigen;
(2) a combination in which the first antigen is a cellular metabolite of a target cell, the second antigen is a cancer antigen, and the third antigen is an immune-related molecule;
(3) a combination in which the first antigen is a cellular metabolite of a target cell, the second antigen is an immune-related molecule, and the third antigen is a cancer antigen;
(4) a combination in which the first antigen is a first immune-related molecule, the second antigen is a cancer antigen, and the third antigen is a second immune-related molecule; and
(5) a combination in which the first antigen is a first immune-related molecule, the second antigen is a second immune-related molecule, and the third antigen is a cancer antigen.

In any of (1) to (5) above, side effects are expected to be reduced.

Particularly in (1) above, from the viewpoint of further reducing the side effects, the second cancer antigen is preferably different in type from the first antigen. In this case, more preferably, the first antigen is specifically expressed in the same cancer tissue or inflammatory tissue as the second antigen.

In (4) above, from the viewpoint of further reducing side effects, the second immune-related molecule is preferably different in type from the first immune-related molecule. In this case, more preferably the second immune-related molecule is CD8. Even more preferably, the first immune-related molecule is CD3.

E. Production methods

a. First antigen-binding molecule

The compound used as the first antigen-binding molecule may be any compound as long as it is a molecule comprising a first antigen-binding region that binds to the first antigen. The first antigen-binding molecule may be a low molecular weight compound, a high molecular weight compound, or a fusion molecule thereof.

Examples of the first antigen-binding regions include antibody variable regions. The cDNA encoding the antigen-binding region can be obtained by a general antibody production procedure such as immunization with purified antigen or DNA immunization, collection of immune cells from the immunized animal, formation of hybridomas, and cloning of cDNA encoding the variable region from the hybridoma, as described in WO2013/180200. The variable region may be humanized. The variable region expressed by a known protein expression system using the cloned cDNA is directly used as the first antigen-binding molecule.

When the first antigen-binding molecule further comprises a second antigen-binding region or a first other component, the first antigen-binding molecule may be expressed as a fusion protein with the first antigen-binding region, or may be expressed as a complex protein formed by intermolecular forces or covalent bonds. For example, when the first antigen-binding molecule is a bispecific antibody, the first antigen-binding molecule is expressed as a complex protein in which a first antigen-binding region and a second antigen-binding region are the respective variable regions for the bispecific antibodies, and in which the first other component comprises an Fc region. In this case, the second antigen-binding region is produced in the same manner as the first antigen-binding region described above. Regarding the Fc region, for example, those described for the bispecific antibodies and methods for producing them of WO2013/180200 are used.

In the technique for stabilizing the antigen/antigen-binding molecule complex formed of the first antigen and the first antigen-binding molecule by the second antigen-binding molecule in the present invention, for example, the binding activity of the first antigen-binding molecule to the first antigen may be high binding activity or low binding activity as measured by SPR, or may be as low as undetectable by SPR.

A first antigen-binding molecule that has a low binding activity to the first antigen as measured by SPR or has binding activity as low as undetectable by SPR can be prepared by attenuating the antigen binding activity of a molecule having high binding activity to the first antigen as measured by SPR by an antibody modification technique such as alanine scanning (Biochemistry Vol.32, No.27, 1993, 6828-6835). The first antigen-binding molecule having such a low binding ability that the KD value cannot be calculated from the kinetic analysis by SPR is prepared by the above-mentioned general antibody production procedure. For example, the first antigen-binding molecule having binding activity as low as undetectable by SPR is prepared by a screening method in which a group of candidate polypeptides are first obtained whose binding activity to the first antigen has been detected by a different mode of intermolecular interaction measurement such as ELISA; and then a polypeptide that cannot be detected by SPR is identified from the group of polypeptide candidates.

b. Second antigen-binding molecule

The second antigen-binding molecule is identified by screening for molecules that enhance the binding activity of the first antigen-binding molecule to the first antigen. The second antigen-binding molecule may be identified, for example, from a library of compounds or antibodies or fragments thereof by a known method for measuring binding activity. Specific examples are (Method I) and (Method II) below.

(Method I)

A screening method comprising identifying one compound, or antibody or fragment thereof, arbitrarily selected from a library of compounds or antibodies or fragments thereof, as a second antigen-binding molecule when the binding activity of a first antigen-binding molecule to a first antigen assayed using at least one selected from SPR, BLI, and ELISA is detected in the presence of the compound, or the antibody or fragment thereof but cannot be detected in the absence of the compound or the antibody or fragment thereof.

(Method II)

A screening method comprising identifying one compound, or antibody or fragment thereof, arbitrarily selected from a library of compounds or antibodies or fragments thereof, as a second antigen-binding molecule when the binding activity of a first antigen-binding molecule to a first antigen assayed using at least one selected from SPR, BLI, and ELISA is higher in the presence of the compound or the antibody or fragment thereof than in the absence of the compound or the antibody or fragment thereof.

A second antigen-binding molecule whose binding activity to the free first antigen is undetectable in at least one assay selected from SPR, BLI, and ELISA, or a second antigen binding molecule which has higher binding activity to the first antigen in the presence of the first antigen-binding molecule than in the absence of the first antigen-binding molecule in at least one assay selected from SPR, BLI, and ELISA can be obtained by the screening methods of (Method III) to (Method VI) below.

(Method III)

A screening method comprising the steps of:

(a) immunizing a mammal with an antigen/antigen-binding molecule complex comprising a first antigen and a first antigen-binding molecule, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding molecule not in the form of the complex cannot be detected in at least one assay selected from SPR, BLI, and ELISA; and
(c) selecting from the second group, a third group which produces monoclonal antibodies that enhance the binding activity of the first antigen-binding molecule to the first antigen in at least one assay selected from SPR, BLI, and ELISA, and identifying the third group as antibody-producing cells that produce a second antigen-binding molecule.

(Method IV)

A screening method comprising the steps of:

(a) immunizing a mammal with an antigen/antigen-binding molecule complex comprising a first antigen and a first antigen-binding molecule, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding molecule not in the form of the complex is lower than their binding to the complex in at least one assay selected from SPR, BLI, and ELISA; and
(c) selecting from the second group, a third group which produces monoclonal antibodies that enhance the binding activity of the first antigen-binding molecule to the first antigen in at least one assay selected from SPR, BLI, and ELISA, and identifying the third group as antibody-producing cells that produce a second antigen-binding molecule.

In t Methods III and IV described above, the antigen/antigen-binding molecule complex is used in the immunization in step (a); however, the antigen/antigen-binding region complex obtained by changing the antigen-binding molecule to a polypeptide not containing the portion other than the antigen-binding region can also be used in the immunization.

(Method V)

A screening method comprising the steps of:

(a) immunizing a mammal with an antigen/antigen-binding region complex comprising a first antigen and a first antigen-binding region, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding region not in the form of the complex cannot be detected in at least one assay selected from SPR, BLI, and ELISA; and
(c) selecting from the second group, a third group which produces monoclonal antibodies that enhance the binding activity of the first antigen-binding region to the first antigen in at least one assay selected from SPR, BLI, and ELISA, and identifying the third group as antibody-producing cells that produce a second antigen-binding molecule.

(Method VI)

A screening method comprising the steps of:

(a) immunizing a mammal with an antigen/antigen-binding region complex comprising a first antigen and a first antigen-binding region, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding region not in the form of the complex is lower than their binding to the complex in at least one assay selected from SPR, BLI, and ELISA; and
(c) selecting from the second group, a third group which produces monoclonal antibodies that enhance the binding activity of the first antigen-binding region to the first antigen in at least one assay selected from SPR, BLI, and ELISA, and identifying the third group as antibody-producing cells that produce a second antigen-binding molecule.

In Method I to Method VI described above, instead of the "at least one assay selected from SPR, BLI, and ELISA", an assay capable of indirectly showing binding activity, for example, a pharmacological assay using cells may be used.

Among the (Method I) to (Method VI), from the viewpoint of reducing side effects when using a bispecific antibody prepared from the first antigen-binding molecule and the second antigen-binding molecule as a medicine, (Method III) to (Method VI) are preferred. From the viewpoint of screening efficiency, (Method V) and (Method VI) are more preferable, and from the viewpoint of further reducing side effects, (Method III) and (Method V) are more preferable, and from both viewpoints, (Method V) is most preferable.

Examples of methods for producing the second antigen-binding molecule include a method of culturing antibody-producing cells that produce the second antigen-binding molecule screened in the above-mentioned (Method III) to (Method VI), and purifying the second antigen-binding molecule from the culture supernatant or the cell homogenate. That is, the second antigen-binding molecule is produced by (Method III') to (Method VI') below.

(Method III')

A method for producing a second antigen-binding molecule, comprising the steps of:

(d) culturing antibody-producing cells obtained from a screening method comprising the following steps (a) to (c):

(a) immunizing a mammal with an antigen/antigen-binding molecule complex comprising a first antigen and a first antigen-binding molecule, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding molecule not in the form of the complex cannot be detected in at least one assay selected from SPR, BLI, and ELISA; and
(c) selecting from the second group, a third group which produces monoclonal antibodies that enhance the binding activity of the first antigen-binding molecule to the first antigen in at least one assay selected from SPR, BLI, and ELISA, and identifying the third group as antibody-producing cells that produce a second antigen-binding molecule;

(e) obtaining a culture supernatant or a cell homogenate from the antibody-producing-cell culture; and
(f) purifying the second antigen-binding molecule from the culture supernatant or the cell homogenate.

(Method IV')

A method for producing a second antigen-binding molecule, comprising the steps of:

(d) culturing antibody-producing cells obtained from a screening method comprising the steps (a) to (c) below:

(a) immunizing a mammal with an antigen/antigen-binding molecule complex comprising a first antigen and a first antigen-binding molecule, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding molecule not in the form of the complex is lower than their binding to the complex in at least one assay selected from SPR, BLI, and ELISA; and
(c) selecting from the second group, a third group which produces monoclonal antibodies that enhance the binding activity of the first antigen-binding molecule to the first antigen in at least one assay selected from SPR, BLI, and ELISA, and identifying the third group as antibody-producing cells that produce a second antigen-binding molecule;

(e) obtaining a culture supernatant or a cell homogenate from the antibody-producing-cell culture; and
(f) purifying the second antigen-binding molecule from the culture supernatant or the cell homogenate.

(Method V')

A method for producing a second antigen-binding molecule, comprising the steps of:

(d) culturing antibody-producing cells obtained from a screening method comprising the steps (a) to (c) below:

(a) immunizing a mammal with an antigen/antigen-binding region complex comprising a first antigen and a first antigen-binding region, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding region not in the form of the complex cannot be detected in at least one assay selected from SPR, BLI, and ELISA; and
(c) selecting from the second group, a third group which produces monoclonal antibodies that enhance the binding activity of the first antigen-binding region to the first antigen in at least one assay selected from SPR, BLI, and ELISA, and identifying the third group as antibody-producing cells that produce a second antigen-binding molecule;

(e) obtaining a culture supernatant or a cell homogenate from the antibody-producing-cell culture; and
(f) purifying the second antigen-binding molecule from the culture supernatant or the cell homogenate.

(Method VI')

A method for producing a second antigen-binding molecule, comprising the steps of:

(d) culturing antibody-producing cells obtained from a screening method comprising the steps (a) to (c) below:

(a) immunizing a mammal with an antigen/antigen-binding region complex comprising a first antigen and a first antigen-binding region, and obtaining a first group of antibody-producing cells which produce monoclonal antibodies that bind to the complex;
(b) selecting from the first group, a second group which produces monoclonal antibodies whose binding activity to either or both of the first antigen not in the form of the complex and the first antigen-binding region not in the form of the complex is lower than their binding to the complex in at least one assay selected from SPR, BLI, and ELISA; and
(c) selecting from the second group, a third group which produces monoclonal antibodies that enhance the binding activity of the first antigen-binding region to the first antigen in at least one assay selected from SPR, BLI, and ELISA, and identifying the third group as antibody-producing cells that produce a second antigen-binding molecule;

(e) obtaining a culture supernatant or a cell homogenate from the antibody-producing-cell culture; and
(f) purifying the second antigen-binding molecule from the culture supernatant or the cell homogenate.

The types of cells from which the antibody-producing cells in (Method III) to (Method VI) and (Method III') to (Method VI') described above are derived include any types of cells as long as they are known types of cells producing an antibody. Examples of the types of cells from which antibody-producing cells are derived include B cells and hybridomas.
In another embodiment, the library used in Method I and Method II described above can be produced as an antigen-binding-molecule library for phage display by Method VII or Method VIII below.

(Method VII)

A method for producing a phage display library of antigen-binding molecules, comprising:

a first step of modifying an amino acid in a first antigen-binding molecule that binds to a first antigen to obtain a variant of the first antigen-binding molecule, whose binding to the first antigen is lowered or is below the detection limit in at least one assay selected from SPR, BLI, and ELISA;
a second step of obtaining a first phage display library of antigen-binding molecules from an existing phage display library of antigen-binding molecules by removing phages presenting antigen-binding molecules that bind to either or both of the first antigen and the variant; and
a third step of obtaining a second phage display library of antigen-binding molecules from the first phage display library of antigen-binding molecules by enrichment for phages presenting antigen-binding molecules that bind to an antigen/antigen-binding molecule complex comprising the first antigen and the first antigen-binding molecule.

Another embodiment of Method VII described above can provide a method for producing a phage display library of antigen-binding molecules, wherein the second and third steps are repeated using the second phage display library of antigen-binding molecules as the existing phage display library of antigen-binding molecules. By repeating the second step and the third step, a phage display library of antigen-binding molecules containing phages displaying the second antigen-binding molecules described above at a high density can be produced.

(Method VIII)

A method for producing a phage display library of antigen-binding molecules, comprising:

a first step of obtaining a first phage display library of antigen-binding molecules from an existing phage display library of antigen-binding molecules by removing phages presenting antigen-binding molecules that (i) bind to a first antigen-binding molecule that may bind to a first antigen but is not bound to the first antigen and (ii) bind to the first antigen not bound to the first antigen-binding molecule; and
a second step of obtaining a second phage display library of antigen-binding molecules from the first phage display library of antigen-binding molecules by enrichment for phages presenting antigen-binding molecules that bind to antigen/antigen-binding molecule complex comprising the first antigen and the first antigen-binding molecule.

Another embodiment of Method VIII described above can provide a method for producing a phage display library of antigen-binding molecules, wherein the first and second steps are repeated using the second phage display library of antigen-binding molecules as the existing phage display library of antigen-binding molecules. By repeating the first step and the second step, a phage display library of antigen-binding molecules containing phages displaying the second antigen-binding molecules described above at a high density can be produced.

F. Combinations

The combination of the present invention is a combination of the above-described first antigen-binding molecule and the above-mentioned second antigen-binding molecule. When the combination of the first antigen, the second antigen, and the third antigen is any one of the combinations (1) to (5) in "D. Suitable combination of the first antigen, the second antigen, and the third antigen" described above, the combination is preferably a TDCC activity inducer or an ADCC activity inducer, and more preferably a TDCC activity inducer.

a. Pharmaceutical compositions

The combination is preferably a pharmaceutical composition. When the combination is a pharmaceutical composition, the first antigen-binding molecule and the second antigen-binding molecule may be administered simultaneously or separately. Preferably, the first antigen binding molecule and the second antigen binding molecule are administered separately.
The first antigen-binding molecule and the second antigen-binding molecule may be intended to be administered simultaneously and may be formulated in the same formulation, or may be intended to be administered separately and may be formulated separately.
When the first antigen-binding molecule and the second antigen-binding molecule are formulated separately, they may be combined to prepare a kit; or the package insert of one formulation may indicate the one formulation may be used in combination with the other formulation. As an example of the former, the first antigen-binding molecule and the second antigen-binding molecule are filled in separate ampules, and both ampoules are packed in one box to prepare a kit.

b. Other components

The pharmaceutical compositions may contain other components besides the first antigen-binding molecule and the second antigen-binding molecule.
Examples of the other components include pharmaceutically acceptable carriers.
The pharmaceutical compositions can be formulated by methods known to those skilled in the art. For example, such pharmaceutical compositions can be used parenterally, as injections which are sterile solutions or suspensions including an antibody along with water or another pharmaceutically acceptable liquid. For example, such compositions may be formulated as unit doses that meet the requirements for the preparation of pharmaceuticals by appropriately combining the antibody with pharmaceutically acceptable carriers or media, specifically with sterile water, physiological saline, a vegetable oil, emulsifier, suspension, detergent, stabilizer, flavoring agent, excipient, vehicle, preservative, binder, or such. In such preparations, the amount of active ingredient is adjusted such that the dose falls within an appropriately pre-determined range.
Sterile compositions for injection can be formulated using vehicles such as distilled water for injection, according to standard protocols for formulation.
Aqueous solutions for injection include, for example, physiological saline and isotonic solutions containing dextrose or other adjuvants (for example, D-sorbitol, D-mannose, D-mannitol, and sodium chloride). Appropriate solubilizers, for example, alcohols (ethanol and such), polyalcohols (propylene glycol, polyethylene glycol, and such), nonionic detergents (polysorbate 80™, HCO-50, and such), may be used in combination.
Oils include sesame and soybean oils. Benzyl benzoate and/or benzyl alcohol can be used in combination as solubilizers. Buffers (for example, phosphate buffer and sodium acetate buffer), soothing agents (for example, procaine hydrochloride), stabilizers (for example, benzyl alcohol and phenol), and/or antioxidants can also be combined. Prepared injectables are generally filled into appropriate ampules.

c. Dosage form

The pharmaceutical compositions are preferably administered parenterally. For example, the compositions may be injections, transnasal compositions, transpulmonary compositions or transdermal compositions. For example, such compositions can be administered systemically or locally by intravenous injection, intramuscular injection, intraperitoneal injection, subcutaneous injection, or such.

d. Target disease

The target disease of the pharmaceutical composition is not particularly limited. Preferably, the target disease is a disease in which when the first antigen is a cellular metabolite, the cellular metabolite is expressed at a higher level than in normal tissues, and a disease in which when the first antigen is an immune metabolite, the second and third antigens are expressed at higher levels than in normal tissues. That is, the disease is a disease in which it is desirable that the first antigen-binding molecule and the second antigen-binding molecule jointly direct effector cells, particularly T cells, to the target cell to exert effector functions, particularly TDCC activity.
Specific examples of target diseases include cell proliferative diseases, immune enhancing diseases, and infectious diseases. Examples of the cell proliferative diseases include a tumor. Examples of the immune-enhancing diseases include autoimmune diseases. Examples of the infectious diseases include bacterial infections and viral infections.

e. Other uses

The combinations of the present invention can also be used for uses other than pharmaceutical compositions.
In one embodiment, when the first antigen is a low molecular weight compound such as a cellular metabolite, the combination of the present invention is useful for a simpler assay for detecting the low molecular weight compounds. For detection of low molecular weight compounds, SPR and HPLC are usually used, and sandwich methods used in ELISA and the like are often difficult to be applied because the antigen is too small. However, this combination may provide a simpler detection system for a low molecular weight compound because the first antigen-binding molecule and the second antigen-binding molecule can simultaneously bind to the low molecular weight compound from both sides.
In other embodiments, the combinations of the invention are useful for *in vivo* imaging. As described above, the conventional efforts to allow antibody drugs to exert the effector functions specifically on target tissue is still in progress, and more efforts are desired. Similarly, when target tissue-specific *in vivo* imaging is performed using an antibody, noise due to binding of the antibody to tissue other than the target tissue may occur. On the other hand, if this combination is used, this noise can be further reduced. For example, when the first antigen is a cell metabolite and the second antigen is a target cell-specific antigen, using the second antigen-binding molecule comprising a complex-binding region and a labeling compound(for example, a radioisotope and a fluorescent dye) as the second other component, the combination can be applied to *in vivo* imaging. In this case, noise can be reduced and detection sensitivity can be improved in imaging of target tissue in which cell metabolites are present.

G Treatment methods

When the first antigen-binding molecule and the second antigen-binding molecule described above are used in combination as a medicine, the first antigen-binding molecule and the second antigen-binding molecule may be administered simultaneously or separately. How to administer them may be determined based on the pharmacokinetics and action mechanism of the first antigen-binding molecule and the second antigen-binding molecule.

The dose and administration method vary depending on the patient's body weight, age, symptoms, and such, but those skilled in the art can set an appropriate dose and administration method by considering these conditions.

[Examples]

In the following examples, antibodies prepared as one embodiment of the second antigen-binding molecule are referred to as "clamping antibodies" because of their function. Bispecific antibodies are also abbreviated as "BiAbs".

The present Examples show one embodiment of the present invention. Antigens in the present invention are not necessarily limited to those used in the present Examples.

[Example 1]

The concept of an antibody that recognizes and binds to an antibody bound to an antigen.

To exert medicinal effects while avoiding side effects, there is a need for techniques to discover drugs that do not act systemically in normal tissues or blood, but act only at lesion sites such as cancer and at inflamed sites. For example, EGFR-BiTE (Non-Patent Literature: BiTE: Baeuerle PA et. al. Curr. Opin. Mol. Ther. 2009. 11, 22-30) exerts antitumor effects by recruiting and activating T cells *via* CD3. Therefore, if the EGFR-BiTE can be provided with the property of binding to CD3 expressed in T cells in the vicinity of cancer cells but not binding to CD3 expressed in T cells not in the vicinity of cancer cells, modified EGFR-BiTE provided with such a property can activate T cells only in cancer, and thereby exert strong antitumor effects while avoiding side effects.

In addition to antibody drugs against cancer, if antibody molecules bind to cytokines only in the synovial fluid of the joints that are inflamed in rheumatoid arthritis and inhibit their actions, and do not cause systemic inhibition, the antibody molecules were considered to be able to exert high therapeutic effects on inflammatory diseases and autoimmune diseases such as rheumatoid arthritis while avoiding increase in risk of infection due to systemic cytokine neutralization.

Thus, antibodies that crosslink antigen double-positive target cells and effector cells or activate T cells only in cancer or at inflamed sites can exert their drug efficacy while avoiding side effects. However, no ideal antibody having such characteristics has been reported so far. Accordingly, the inventors considered that combination of: a clamping antibody, an antibody as shown in Fig. 1 that provides crosslinking *via* a small molecule present at high concentrations in target disease sites such as cancer or *via* CD3 expressed on T cells in the vicinity of cancer cells; and an antibody that recognizes an antigen on a target disease cell would enable crosslinking cells at target tissue in which small molecules are present at high concentration, and inducing CD3 signals, as shown in Fig. 2.

Therefore, an effort was made to obtain a clamping antibody against an antibody that binds to adenosine which is known as a small molecule considered to be present at high concentration in cancer tissues, and a clamping antibody against an anti-CD3 antibody capable of inducing CD3 signals.

[Example 2]

Acquirement of attenuated CD3 antibody

The anti-CD3 antibody CE115HA000 is an antibody prepared by humanizing a rat-derived antibody that recognizes CD3ε. Variants with different TDCC inducibilities have been obtained by introducing mutations into the antigen-determining regions (CDRs). Of these, CE115HA146 with attenuated TDCC activity and CE115HA056 with TDCC activity not found were used. The amino acid sequences of the heavy chain variable regions are shown in SEQ ID NOs: 1-3.

[Example 3]

Acquirement of a clamping antibody against an anti-CD3 antibody

(1) Antigen preparation

A gene encoding an antibody (CE115HAPG13-rabCH1hG1m, SEQ ID NO: 6) designed to have eight amino acid residues of CD3ε (QDGNEEMQ SEQ ID NO: 5), which is the epitope, linked *via* a GS linker (GGGSGGGS, SEQ ID NO: 4) to the N-terminal side of the CE115HA146 heavy chain was prepared and inserted into an expression vector for mammalian cultured cells. The gene encoding the corresponding light chain (GLS3000-rabk, SEQ ID NO: 7) was similarly inserted into an expression vector for mammalian cultured cells. These expression vectors were introduced into FreeStyle 293F cells (Thermo Fisher Scinetific) using the transfection reagent 293fectin Tranfection Reagent (Thermo Fisher Scientific) according to the instructions provided by the manufacturer, the cells were cultured for five days, and the culture medium was harvested. The antibody was purified from the collected culture medium by affinity purification using rProtein A Sepharose Fast Flow resin (GE Healthcare). A 1500 units of the protease FabRICATOR (Genovis) was added to 10 mg of the purified antibody, and cleavage was carried out at 37°C for 15 hours. Thereafter, the flow-through fraction of Eshmuno A resin (Merck Millipore) was applied to a gel filtration column to prepare a F(ab')2 fragment fused to the CD3ε epitope. The concentration of the purified protein was calculated by measuring the absorbance at 280 nm using a spectrophotometer and using the extinction coefficient calculated from the obtained value by the PACE method (Protein Science 1995; 4: 2411-2423).

(2) Immunization to rabbits, selection of antibody-producing cells, and isolation of antibody genes

Rabbit immunization was performed by subcutaneously injecting an emulsion prepared by mixing a CD3ε epitope-fused F(ab')2 solution and TiterMax Gold (TiterMax USA). After four times of immunization, blood and spleen were collected from rabbits found to produce the antibody. In order to concentrate B cells presenting antigen-specific B cell receptors on the surface, peripheral blood mononuclear cells and splenocytes were prepared, they were reacted with CD3ε epitope-fused CE115HA146 (CE115HAPG13rabCH1hG1m/GLS3000-rabk, SEQ ID NOs: 6 and 7) and Alexa647 (Invitrogen)-labeled CE115HA146 (CE115HA146-rabCH1hG1m/GLS3000-rabk, SEQ ID NOs: 8 and 7), and the bound antibodies were stained using a DyLite488-labeled anti-human IgG antibody, Goat anti-Human IgG Fc Cross-Absorbed DyLight488 conjugate (Thermo-Fisher-Scientific). Cells stained with DyLite488 alone were separated using a cell sorter (FACS Aria III, BD), seeded in a 96 well plate at 1 cell/well, and cultured in the presence of EL4 cells at 25000 cells/well in a BT medium for ten days. EL4 cells whose cell growth was suppressed by pretreatment with mytomycin C (Sigma-Aldrich) were used. BT medium was prepared by adding Fetal Bovine Serum, ultra-low IgG (Life technologies); and 1/20 volume rabbit T cell culture medium to RPMI 1640 with L-Gln (nacalai tesque). Rabbit T cell culture medium was prepared by culturing rabbit T cells in RPMI-1640 medium supplemented with Phytohemagglutinin-M (Roche), phorbol-12-myristate 13-acetate (Sigma-Aldrich), and 2% FBS.

As primary screening, the binding property between the antibody secreted into the B cell culture medium and the CD3ε epitope-fused CE115HA146 was evaluated by ELISA. An anti-human F(ab')2 antibody, Affipure F(ab')2 Fragment Donkey Anti-Human (Jackson Immuno Research) was immobilized onto a 384-well plate, and CD3ε epitope-fused CE115HA146 (CE115HAPG13-rabCH1hG1m/GLS3000-rabk, SEQ ID NOs.: 6 and 7) or CE115HA146 not fused with the CD3ε epitope (CE115HA146-rabCH1hG1m/GLS3000-rabk, SEQ ID NOs: 8 and 7) was bound to the plate. After adding the B cell culture supernatant, peroxidase-labeled anti-rabbit Fc antibody (Biolegend) was reacted, and the rabbit antibody bound to the antigen was detected using the ABTS Microwell Peroxidase Substrate (1-Component System) (KPL) by measuring the absorbance at 405 nm on a SpectraMax 340PC384 plate reader (Molecular device). Secondary screening was performed on clones found to specifically bind to the CD3ε epitope-fused CE115HA146. As secondary screening, the properties of binding to the CD3ε epitope-fused control antibody (hGC33VHGP01-rabCH1hG1m/hGC33VL-rabk, SEQ ID NOs: 9 and 10) was similarly evaluated by ELISA, and thereby, antibodies that recognize only the peptide sequence without depending on the backbone antibody sequence were excluded. Screening of 10560 clones was carried out, 352 clones were selected, and RNAs were extracted and purified from the selected B cells using ZR-96 Quick RNA Kit (Zymo research). The primer sets (SEQ ID NOs: 11 and 12, and SEQ ID NOs: 13 and 14) corresponding to DNAs encoding the heavy chain variable region and the light chain variable region, respectively of the antibody gene, and PrimeScript II High Fidelity One Step RT-PCR Kit (TAKARABIO) was used to perform RT-PCR, and the respective PCR products were obtained. The obtained PCR products of the heavy chain variable region and the light chain variable region were cloned into plasmid DNA encoding human heavy chain constant region and plasmid DNA encoding human light chain constant region, respectively, using In-fusion HD Cloning Kit (Takara Bio). The nucleotide sequences of the heavy chain constant region and the light chain constant region are shown in SEQ ID NOs: 15 and 16, respectively. As described above, vectors expressing a polypeptide in which a heavy chain variable region and a human heavy chain constant region are fused and a polypeptide in which a light chain variable region and a human light chain constant region are fused were produced.

(3) Preparation of bispecific antibodies (BiAbs)

The antibody genes selected by B cell cloning and screening by ELISA were introduced into FreeStyle 293F cells to express the antibodies. 5 mL of medium per well was added to a 6-well cell culture plate and transfection was carried out according to the manufacturer's instructions. After culturing for four days, cell supernatants were prepared, and purified antibodies were obtained by a method known to those skilled in the art by batch purification using rProtein A Sepharose Fast Flow (GE Healthcare). Antibody concentration was calculated by the same method as the protein concentration calculation in (1) described above. There were 212 clones with sufficient expression.

BiAbs were prepared by FAE technology. First, 20 μg each of the two types of antibodies were mixed, 10 μL of 2-Mercaptoethylamine-HCl (2-MEA, Sigma-Aldrich) prepared at 250 mM using Tris-Buffered Saline (TBS, Takara Bio) was added, and the total volume was increased to 100 μL using TBS buffer. This reaction solution was incubated at 37°C for 90 minutes, and then 2-MEA was removed and replaced with D-PBS (-) (Wako Pure Chemicals) using Zeba 96-well Spin Plates (Thermo Fisher Scientific). As an example of the first antigen-binding molecule, BiAb1 was prepared using anti-human glypican 3 (GPC3) antibody (GCH065-F760mnN17/L0011-k0, SEQ ID NOs: 17 and 18) and CE115HA146 (CE115HA146-F760mnP17/GLS3000-k0, SEQ ID NOs: 19 and 20), and BiAb2 was prepared using an anti-GPC3 antibody (GCH065-F760mnN17/L0011-k0, SEQ ID NOs: 17 and 18) and the antibody derived from rabbit B cell cloning obtained in (2) mentioned above.

(4) CD3 signal reporter assay using Jurkat-luc cells (Jurkat-Luc assay)

SK-pca60 cell line, which is SK-HEP-1 cells (ATCC HTB-52) forced to express human GPC3, was used as target cells, and NFAT-RE-luc2-Jurkat cells (Jurkat-luc cells, Promega) that express Luciferase in response to a CD3 signal were used as effector cells. To 25 μL of Assay buffer (RPMI-1640 (Nacalai tesque) containing 10% FBS (HyClone), 1x MEM Non-Essential Amino Acids Solution (Gibco), and 1 mM Sodium Pyruvate) in white 96-well plates (Corning), 0.09 μg/mL of BiAb1 and 0.3 μg/mL of BiAb2 were added. For signal correction and control between plates, each plate was prepared to have wells with no antibody addition, addition of BiAb1 only, and addition of BiAb2 only, respectively, and the amount of added solution was adjusted to 25 μL by using the Assay buffer when deficient. After adding the antibody solution, the target cells were seeded at 25 μL/well (1 x $10^4$ cells/well), Jurkat-Luc cell suspension was seeded at 25 μL/well (1 x $10^4$ cells/well), and the cells were cultured under conditions of 37°C and 5% $CO_2$ for 6 hours. After allowing the 96-well plates to stand at normal temperature for 15 minutes, 75 μL of Bio-Glo Luciferase assay reagent (Promega) was added, stirred, and then reacted for ten minutes, and luminescence was measured using a plate reader EnVision (Perkin-Elmer).

The average relative luminescence intensity value (RLU) used as an index of TDCC activity was corrected by the following Formula 1.

(Formula 1)

$$\text{Normalized RLU} = RLU \times \frac{B}{A}$$

In the above Formula 1, "A" represents a value obtained by averaging the average RLU values of wells to which only BiAb1 was added in multiple different plates, and "B" represents the average RLU value for wells to which only BiAb1 was added in each plate. The term obtained by dividing B by A was used as a correction term between plates for activation of the CD3 signal by each antibody. Results of the Jurkat-Luc assay are shown in Fig. 3. In the activation of the CD3 signal by BiAb1, six clones out of the 212 clones had an enhancement of more than twice the standard deviation by addition of BiAb2, and as a result of sequence analysis, four clones which are CLA0022, CLA0028, CLA0311, CLA0344, excluding overlapping sequences, were obtained as the second antigen-binding molecules. The antigen-determining site amino acid sequences (SEQ ID NOs: 21 to 44) and variable region amino acid sequences (SEQ ID NOs: 45 to 52) of the obtained antibodies are shown. In this assay, clones that attenuate the CD3 signal were also obtained.

[Example 4]

Evaluation of binding property between anti-CD3 antibody/CD3ε peptide complex and clamping antibody by surface plasmon resonance (SPR)

(1) Preparation of analytes and ligands

The analytes used were Fab fragments prepared from the four clones which are the CLA0022, CLA0028, CLA0311 and CLA0334 antibodies obtained in Example 3. Specifically, expression vectors prepared by inserting genes encoding the sequence of each of the antibodies: CLA0022VH-F760mnP17/CLA0022VL-k0C (SEQ ID NOs: 53 and 54); CLA0028VH-F760mnP17/CLA0028VL-k0C (SEQ ID NOs: 55 and 56); CLA0311VH-F760mnP17/CLA0311VL-k0C (SEQ ID NOs: 57 and 58); or CLA0334VH-F760mnP17/CLA0334VL-k0C (SEQ ID NOs: 59 and 60) were introduced into Expi293F using ExpiFectamine293 (Thermo Fisher Fisher Scientific), the culture supernatant on the fifth day of culturing was collected, and the antibodies were prepared by a method known to those skilled in the art using HiTrap MabSelect SuRe. Fab fragments were prepared from the purified antibodies using Pierce Fab Preparation Kit (Thermo Fisher Scientific) according to the manufacturer's instructions. The concentrations of the obtained Fab fragments were calculated by a method similar to that for protein concentration calculation in Example 3 (1).

Ligands were prepared as follows: mammalian expression vectors prepared by inserting genes encoding CD3ε epitope-fused CE115HA146 (CE115HAGP13-rabIgG/GLS3000-rabk, SEQ ID NOs: 61 and 7), CD3ε epitope-fused CE115HA056 (CE115HAGP12-rabIgG/GLS3000-rabk, SEQ ID NOs: 62 and 7), CD3ε epitope-fused GPC3 antibody (hGC33VHGP01-rabCH1hG1m/hGC33VL-rabk, SEQ ID NOs: 9 and 10), CD3 antibodies CE115HA000 (CE115HA000-F760mnP17/GLS3000-k0, SEQ ID NOs: 63 and 20), CE115HA056 (CE115HA056-F760mnP17/GLS3000-k0, SEQ ID NOs: 64 and 20), and CE115HA146 (CE115HA146-F760mnP17/GLS3000-k0, SEQ ID NOs: 19 and 20), and negative control IC17 (IC17Hdk-F760mnP17/IC17L-k0, SEQ ID NOs: 65 and 66) were introduced into Expi293F, and the ligands were prepared by a method similar to that for antibodies for Fab preparation.

(2) Evaluation of binding properties of clamping antibodies by SPR

SuRe protein A (GE Healthcare) prepared at 25 μg/mL using Acetate4.5 (GE Healthcare) was immobilized on sensor chip CM4 at approximately 1000 RU per flow cell using an amine coupling kit (GE Healthcare).

First, to evaluate the binding specificity of the clamping antibodies, a ligand was reacted at 37°C for 60 seconds at a flow rate of 10 μL/min to capture 1000 RU, and an analyte prepared at 100 nM was allowed to act for 180 seconds at a flow rate of 30 μL/min, and the amount of binding was measured. By subtracting the value of the flow cell that did not capture the ligand, the amount of binding per 1 RU of ligand was calculated. As shown in Fig. 4, the clamping antibody bound to CD3ε epitope-fused CE115HA146 (CE115HAPG13) and CD3ε epitope-fused CE115HA056 (CE115HAPG12), but hardly showed any binding to CE115HA000, CE115HA056, and CE115HA146 CD3 antibodies alone, and to CD3ε epitope-fused GPC3 antibody, and negative control IC17.

Next, for the purpose of measuring affinity, the ligand was reacted at 37°C at a flow rate of 10 μL/min for 60 seconds to capture 75 RU, and the 0, 25, 50, 100, 200, and 400 nM clamping antibody-derived Fab fragments used as an analyte were allowed to act for 180 seconds at a flow rate of 30 μL/min, and then dissociation was observed for 300 seconds. The sensor chip was regenerated by passing Glycine 1.5 and 25 mM NaOH, each at a flow rate of 30 μL/min for 15 seconds. The dissociation constant KD (M) was calculated based on the association rate constant ka (1/Ms) and the dissociation rate constant kd (1/s), which are kinetic parameters calculated from the sensorgram obtained by the measurement. Biacore T200 Evaluation Software (GE Healthcare) was used for calculation of each parameter. The obtained KD values are shown in Table 1.

[Table 1]

| Affinity to CD3ε epitope-fused CD3 antibody | | |
|---|---|---|
| Clamping antibody | CE115HAPG12 | CE115HAPG13 |
| CLA0022 | 1.4 x $10^{-8}$ M | 1.7 x $10^{-8}$ M |
| CLA0028 | 7.4 x $10^{-8}$ M | 7.8 x $10^{-8}$ M |
| CLA0311 | N.D*. | N. D. |
| CLA0334 | 1.1 x $10^{-7}$ M | 1.5 x $10^{-7}$ M |
| N. D. not determined | | |

Formation of a complex between the CD3ε epitope-fused CD3 antibody and the clamping antibody was also confirmed by crystal structure analysis.

[Example 5]

Evaluation of binding property to CD3εδ using Bio-layer Interferometry (BLI)

(1) Preparation of biotinylated human CD3εδ heterodimer

Biotinylated human CD3εδ heterodimer (hereinafter CD3εδ) was prepared by a method known to those skilled in the art. Specifically, a gene fragment encoding an antibody constant region, a gene fragment encoding a sequence (ENLYFQG, SEQ ID NO: 67) cleaved by TEV protease, and a gene fragment encoding Avi tag (GLNDIFEAQKIEWHE, SEQ ID NO: 68) to which biotin is added by biotin ligase were linked downstream of a gene fragment encoding the extracellular region of human CD3ε, *via* a gene fragment encoding a linker composed of glycine and serine. A gene fragment encoding a protein in which the extracellular region of the human CD3ε, the antibody constant region, the TEV protease cleavage sequence, and Avi tag are linked (Fc-fused human CD3ε, SEQ ID NO: 69) was inserted into an animal cell expression vector. Next, the gene fragment encoding the antibody constant region and a gene fragment encoding Flag tag (DYKDDDDK, SEQ ID NO: 70) were linked downstream of a gene fragment encoding the extracellular region of human CD3δ. A gene fragment encoding a protein in which the extracellular region of human CD3δ, the antibody constant region, and Flag tag are linked (Fc-fused human CD3δ, SEQ ID NO: 71) was inserted into an animal cell expression vector. The two constructed plasmid vectors were introduced into FreeStyle 293F cells (Invitrogen) using ExpiFectamine-293 (Thermo Fisher Scientific). At the time of transfection, a biotin ligase (BirA, SEQ ID NO: 72) -expressing gene and biotin were added for the purpose of biotinylating the Avi tag of CD3ε. The transfected cells were cultured at 37°C under 8% $CO_2$ to secrete the protein of interest into the culture supernatant. The cell culture medium was filtered through a 0.22-μm bottle top filter to obtain a culture supernatant.

The culture supernatant was added to a column prepared using Eshmuno A resin (Merck Millipore) to allow the protein of interest to bind to the column. Then, elution was carried out using 20 mM sodium citrate pH 2.7 solution. After neutralizing the eluted fraction, this was added to and adsorbed onto the Anti-FLAG M2 column prepared using Anti-FLAG M2 agarose resin (Sigma-Aldrich), and the protein of interest was eluted using the FLAG peptide dissolved in D-PBS (-). From this eluted solution, aggregates and FLAG peptide were removed by gel filtration chromatography using Superdex 26/600 (GE healthcare) to obtain purified CD3εδ. The concentration of the obtained purified protein was calculated by a method similar to that for protein concentration calculation in Example 3 (1).

(2) Preparation of one-arm antibodies

In order to bind the antigen CD3εδ and the antibody in a 1:1 ratio, one-arm antibodies CE115HA000 one arm (CE115HA000-pE22Hh/GLS3000-k0//Kn010, SEQ ID NOs: 73, 20, and 74), CE115HA056 one arm (CE115HA056-pE22Hh/GLS3000-k0//Kn010, SEQ ID NOs: 75, 20, and 74), and CE115HA146 one arm (CE115HA146-pE22Hh/GLS3000-k0//Kn010, SEQ ID NOs: 76, 20, and 74)), and negative control anti-KLH antibody IC17 one arm (IC17Hdk-pE22Hh/IC17L-k0//Kn010, SEQ ID NOs: 77, 66, and 74) were prepared. The respective antibody heavy chain-encoding gene, light chain-encoding gene, and a gene encoding an antibody fragment having no variable region were introduced into Expi293F using ExpiFectamine293 (Thermo Fisher Scientific), and purified antibodies were prepared by a method similar to that in Example 4 (1).

(3) Evaluation of ternary complex formation by Octet

Using an Octet RED 384 (ForteBio), a streptavidin sensor (ForteBio) was reacted with CD3εδ prepared at 0.2 μM in ACES at 37°C for 300 seconds. Next, CE115HA000 one arm, CE115HA056 one arm, and CE115HA146 one arm at 0, 111, 333, and 1000 nM, and negative control IC17one arm were reacted for 120 seconds, and then dissociation was monitored for 120 seconds in HBS-EP(+) buffer containing 1 μM of clamping antibody Fab fragment (see Example 4 (1)),. Response values were extracted every three seconds, and graphs were depicted using Microsoft Excel 2013 (Microsoft). As shown in Fig. 5, when running buffer alone or negative control IC17 Fab was added, CD3 antibody rapidly dissociated, whereas when Fabs prepared from clamping antibodies CLA0022, CLA0028, CLA0311, and CLA0334 were added, further increase in response or delay in dissociation was observed in the binding response of anti-CD3 antibody, and therefore, the addition of a clamping antibody was considered to stabilize the CD3εδ/anti-CD3 antibody complex.

[Example 6]

Evaluation of TDCC activity using human peripheral blood monocytes (PBMC)

(1) Preparation of human PBMC solution

Using a syringe loaded in advance with 200 μL of a 1000 units/mL heparin solution (Novo-Heparin injection 5,000 units, Novo Nordisk), 50 mL of peripheral blood was collected from healthy volunteers at Chugai Pharmaceutical Co., Ltd. The peripheral blood diluted two-fold with PBS (-) was divided into 4 equal parts, then added to a Leucosep lymphocyte separation tube (Greiner bio-one) prefilled with 15 ml of Ficoll-Paque PLUS and centrifuged. The separation tube into which the peripheral blood was dispensed was centrifuged at a speed of 1,000 x g for ten minutes at room temperature, and then the mononuclear cell fraction layers were collected. The cells in each of the layers were washed once using 10 mL of RPMI-1640 Medium containing 10% FBS (hereinafter referred to as 10% FBS/RPMI), then depending on the target cell, 10% FBS/RPMI, Dulbecco's Modified Eagle's Medium (hereinafter, 10% FBS/D-MEM), and Eagle's Minimal Essential Medium (hereinafter, 10% FBS/E-MEM) were used to suspend the cells to 5 x $10^5$ cells/mL or 1 x $10^6$ cells/mL, and then this was subjected to subsequent experiments as a human PBMC solution.

(2) Preparation of target cells

SK-pca60 (GPC3-positive cells), NCI-H446 (ATCC HTB-171, GPC3-positive cells), SKE-4B2 (human EREG-positive cells), which is SK-HEP-1 cells forced to express human EREG, and hEREG/SK-pca60 (human EREG, GPC3-positive cells), which is SK-HEP-1 cells forced to express human EREG and human GPC3, were detached from dishes using Cell dissociation buffer, SK-pca60 was suspended in 10% FBS/D-MEM at cell density of $6 \times 10^4$ cells/mL, NCI-H446 was suspended in 10% FBS/RPMI at cell density of $2 \times 10^5$ cells/mL, and SKE-4B2 was suspended in 10% FBS/E-MEM at cell density of $1 \times 10^5$ cells/mL, and hEREG/SK-pca60 was suspended in 10% FBS/E-MEM at cell density of $1 \times 10^5$ cells/mL. The cell suspension solution was used as target cells for subsequent experiments.

(3) Preparation of BiAbs

Each of the antibodies, GCH065-F760mnN17/L0011-k0 (SEQ ID NOs: 17 and 18), EGLVH-F760mnN17/EGLVL-KT0 (SEQ ID NOs: 78 and 79), CE115HA000-F760mnP17/GLS3000-k0 (SEQ ID NOs: 63 and 20), CE115HA056-F760mnP17/GLS3000-k0 (SEQ ID NOs: 64 and 20), CLA0028VH-F760-mnP17/CLA0028VL-k0C (SEQ ID NOs: 55 and 56), and IC17Hdk-F760mnN17/IC17L-k0 (SEQ ID NOs: 65 and 66), were prepared as follows: expression vectors prepared by inserting the respective heavy chain and light chain genes were introduced into Expi293F cells (Thermo Fisher Scientific) using ExpiFectamine293 (Thermo Fisher Scientific) and purified antibodies were prepared by a method similar to that in Example 4(1).

The obtained antibodies were mixed in equal amounts (200 μg or 500 μg) in the combinations set forth below, and one-tenth that amount of 2-MEA (Sigma-Aldrich) prepared at 250 mM in TBS (Takara Bio) was added, and the procedure was performed on a 500-μL or 1000-μL scale. This reaction solution was incubated at 37°C for 90 minutes, then PD-Minitrap G-25 or PD-Miditrap G25 (GE Healthcare) was used to remove 2-MEA and replace it with D-PBS(-) (Wako Pure Chemicals). The prepared BiAbs are shown below.

[Table 2]

| Prepared BiAbs | | |
|---|---|---|
| BiAb | Antibodies used | SEQ ID NOs |
| GCH065/CE115HA000 | GCH065-F760mnN17/L0011-k0 + CE115HA000-F760mnP17/GLS3000-k0 | 17, 18, 63, 20 |
| EGL/CE115HA000 | EGLVH-F760mnN17/EGLVL-KT0 + CH115HA000-F760mnP17/GLS3000-k0 | 78, 79, 63, 20 |
| GCH065/CE115HA056 | GCH065-F760mnN17/L0011-k0 + CE115HA056VL-F760mnP17/GLS3000-k0 | 17, 18, 64, 20 |
| GCH065/CLA0028 | GCH065-F760mnN17/L0011-k0 + CLA0028VH-F760mnP17/CLA0028VL-k0C | 17, 18, 55, 56 |

(continued)

| Prepared BiAbs | | |
|---|---|---|
| BiAb | Antibodies used | SEQ ID NOs |
| EGL/CLA0028 | EGLVH-F760mnN17/EGLVL-KT0 + CLA0028VH-F760mnP17/CLA0028VL-k0C | 78, 79, 55, 56 |
| IC17/CLA0028 | IC17Hdk-F760mnN17/IC17L-k0 + CLA0028VH-F760mnP17/CLA0028VL-k0C | 65, 66, 55, 56 |

(4) Cytotoxicity assay (TDCC assay)

TDCC activity was evaluated by measuring the level of the electrical resistance generated accompanying adhesion of cells to the electrode, using xCELLigence (ACEA Biosciences). First, the medium used for target cell preparation was added at 50 μL/well to RTCA Resistor plate 96 to correct the background value.

Next, target cell suspension solutions prepared as in Example 6(2) were used at 50 μL/well for seeding (SK-pca60: 3 x $10^3$ cells/well, NCI-H446: 1 x $10^4$ cells/well, SKE-4B2: 5 x $10^3$ cells/well, hEREG/SK-pca60: 5 x $10^3$ cells/well), the plate was placed into xCELLigence, and cultured overnight under conditions of 37°C and 5% $CO_2$. When the target cell was SK-pca60, on the day after seeding the cells, 25 μL/well of BiAb2 prepared at each of the concentrations (0, 0.008, 0.08, 0.8, 8, and 80 μg/mL) and 25 μL/well of BiAb1 prepared at each of the concentrations (0, 0.0008, 0.008, 0.08, 0.8, and 8 μg/mL) were added. Thereafter, human PBMC suspension solution containing cells at ten times the number of target cells was added at 50 μL/well, the plate was placed into xCELLigence, and then cultured under conditions of 37°C and 5% $CO_2$ for 36 hours, during which the electrical resistance value (Cell index) was measured at 10 minute intervals over time. On the other hand, when NCI-H446, SKE-4B2, and hEREG / SK-pca60 were used as target cells, on the day after seeding the cells, 25 μL/well of BiAb2 prepared at each of the concentrations (0, 0.008, 0.08, 0.8, and 8 μg/mL) and 25 μL/well of BiAb1 prepared at each of the concentrations (0, 0.008, 0.08, 0.8, and 8 μg/mL) were added. Thereafter, human PBMC suspension solution containing cells at five times the number of target cells was added at 50 μL/well, the plate was placed into xCELLigence, and then cultured under conditions of 37°C and 5% $CO_2$ for 120 hours, during which the electrical resistance value (Cell index) was measured at 10 minute intervals over time.

The cell growth inhibition rate (CGI) was calculated by the following Formula 2 as an index of TDCC activity.

(Formula 2)

$$\mathrm{CGI\ [\%\ no\ Ab\ control]} = \left(\frac{X - Y}{X - 1} \times 100\right) \times \frac{B}{A}$$

In the above Formula 2, all of the Cell Indices used were calculated using the Delta Cell Index where the first resistance value measurement point after antibody addition is taken to be 1. "X" represents the average value of Delta Cell Indices at the final measurement point of the antibody-free wells, "Y" represents the average value of Delta Cell Indices at the final measurement point of the antibody-added wells, and "A" is an averaged value of the average Delta Cell Index values at the final measurement point when only 0.1 μg/mL of the positive control BiAb (GCH065/CE115HA000 for SK-pca60, NCI-H446, and hEREG/SK-pca60; and EGL/CE115HA000 for SKE-4B2) is added in multiple different plates of the same target cell, and "B" is the average Delta Cell Index value of the final measurement point when only 0.1 μg/mL of the positive control BiAb (GCH065/CE115 for SK-pca60, NCI-H446, and hEREG/SK-pca60; and EGL/CE115 for SKE-4B2) is added in each plate of the same target cell. The term obtained by dividing B by A was used as a correction term between plates for TDCC activity against the same target cell.

First, GCH065/CE115HA056 was used as BiAb1 and GCH065/CL0028 was used as BiAb2, and antigen-binding-dependent TDCC activities were evaluated. SK-pca60 cells that constantly express GPC3 were used as target cells, and GCH065/CE115HA000 that exerts TDCC activity with BiAb alone was used as a positive control. As shown on the left side of Fig. 6, GCH065/CE115HA000 showed remarkable TDCC activity from an added amount of 0.001 μg/mL, and reached a plateau at 0.01 μg/mL or more. On the other hand, GCH065/CE115HA056 of BiAb1 alone did not show TDCC activity, but TDCC activity was found to be shown by addition of clamping antibody BiAb2 which binds to GPC3. BiAb 1 concentration-dependent TDCC activity was observed in the presence of BiAb2 at 0.01 μg/mL. BiAb1 was found to show almost the same TDCC activity as that of the positive control GCH065/CE115HA000 upon addition of BiAb2 at concentration of 0.1 μg/mL to 10 μg/mL. On the other hand, as shown on the right side of Fig. 6, when the clamping antibody IC17/CLA0028 having no antigen binding ability was added as BiAb2, no remarkable TDCC activity was observed.

Next, double-positive cell-specific TDCC activity was evaluated using GPC3 and EREG GCH065/CE115HA000 and EGL/CE115HA000 were used as positive controls. As shown in Fig. 7, GCH065/CE115HA000 and EGL/CE115HA000 showed TDCC activity against the respective antigen single-positive cells NCI-H446 (GPC3) and SKE-4B2 (EREG), and both antibodies showed TDCC activity against the double-positive cell hEREG/SK-pca60 (EREG/GPC3). When GCH065/CE115HA056 of BiAb1 alone was allowed to act, TDCC activity was not shown against any cell line, but in the presence of BiAb2, EGL/CLA0028 at 1 μg/mL, TDCC activity was shown solely for hEREG/SK-pca60 which is an antigen double-positive cell. In contrast, GCH065/CE115HA056 did not show TDCC activity for all cell lines in the presence of 1 μg/mL of IC17/CLA0028, which is BiAb2 having no antigen-binding ability.

Based on the above results, the present inventors succeeded in producing antibodies that specifically exhibit TDCC activity on antigen double-positive cells.

[Example 7]

Acquirement of clamping antibodies that recognize complexes of adenosine or an adenosine derivative with an adenosine-binding antibody from an antibody library

Clamping antibodies that bind to complexes of adenosine or an adenosine derivative with an adenosine-binding antibody were obtained from the naive human antibody phage display library and synthetic human antibody phage display library described in WO2015/156268 by a phage display method. By referring to the heavy chain variable region and the light chain variable region obtained in WO2015/083764, SMB0002hH-G1m3/SMB0002hL-k0a (SEQ ID NOs: 80 and 81) was used as the adenosine-binding antibody. That is, phages showing binding activity to the adenosine-binding antibody SMB0002hH-G1m3/SMB0002hL-k0a captured on the magnetic beads in the presence of adenosine or an adenosine derivative, but showing no binding activity to the variants with attenuated adenosine-binding activity which were prepared by performing single-amino acid-substitution on SMB0002hH-G1m3/SMB0002hL-k0a, SMBh068-G1m3/SMB0002hL-k0a (SEQ ID NOs: 82 and 81), SMBh508-G1m3/SMB0002hL-k0a (SEQ ID NOs: 83 and 81), SMBh606-G1m3/SMB0002hL-k0a (SEQ ID NOs: 84 and 81), SMB0002hH-G1m3/SMB1234-k0a (SEQ ID NOs: 80 and 85), and SMB0002hH-G1m3/SMB1255-k0a (SEQ ID NOs: 80 and 86), were collected. In this acquiring method, biotinylated adenosine-binding antibody and its varitant, which were biotiniylated with EZ-link Sulfo-NHS-SS-Biotin (Thermo Fisher Scientific) by a method known to those skilled in the art, were used as panning antigens.

*Escherichia coli* carrying a phage display phagemid vector of a naive human antibody library or a synthetic human antibody library constructed by a method known to those skilled in the art was infected with the helper phage M13KO7TC described in WO2015/046554, and after culturing overnight at 30°C, the phages were collected from the culture supernatant. Antibody-displaying phage library solution was prepared by adding 1/5 volume of 2.5 M NaCl/10% PEG to phage produced *Escherichia coli* culture medium to precipitate the phage population followed by diluting with TBS. Next, BSA was added to the phage library solution at a final concentration of 4%. Panning with antigen immobilized on magnetic beads was performed. Sera-Mag SpeedBeads NeutrAvidin-coated (Thermo Fisher Scientific), FG beads NeutrAvidin (Tamagawa Seiki), or Dynabeads MyOne Streptavidin T1 (Thermo Fisher Scientific) was used as the magnetic beads.

In the first round of panning, in order to remove the phages that bind to the adenosine-binding antibody in the absence of adenosine, negative selection was carried out using five variants of the adenosine-binding antibody which have attenuated adenosine binding (SMBh068-G1m3/SMB0002hL-k0a, SMBh508-G1m3/SMB0002hL-k0a, SMBh606-G1m3/SMB0002hL-k0a, SMB0002hH-G1m3/SMBl234-k0a, and SMB0002hH-G1m3/SMBl255-k0a). Specifically, a solution prepared by mixing equimolar amounts of five types of SMB0002hH-G1m3/SMB0002hL-k0a variants biotinylated by the above-described method was added to Sera-Mag SpeedBeads NeutrAvidin-coated blocked with BSAto add a total of 2000 pmol of variants to the beads, and this was subjected to reaction at room temperature for 15 minutes. To the beads washed three times with TBS, 0.5 mL of phage library solution blocked with BSA was added and allowed to bind at room temperature for one hour. Phages that did not bind to antigen and beads, were recovered by separating the beads using magnetic stand.

Subsequently, antibodies that bind to SMB0002hH-G1m3/SMB0002hL-k0a in the presence of adenosine were selected. The phage library recovered by the method described above, was contacted with the antigen and adenosine at room temperature for 15 minutes by adding 700 pmol of biotinylated SMB0002hH-G1m3/SMB0002hL-k0a and adenosine at a final concentration of 500 μM. Thereafter, contact was carried out for 45 minutes at 4°C. Next, magnetic beads, FG beads NeutrAvidin or Dynabeads MyOne Streptavidin T1, blocked with BSA were added to the mixed solution of the labeled antigen and adenosine with phage library, and binding of the complex of the antigen and adenosine with the phage to the magnetic beads was carried out at 4°C for 30 minutes. The beads were washed once with 1 mL of ice-cooled adenosine/TBST (500 μM adenosine, 0.1% Tween 20, TBS buffer) and once with ice-cooled adenosine/TBS

(500 μM adenosine, TBS buffer). Thereafter, a DTT solution was added at a final concentration of 25 mM, and after stirring the mixture at room temperature for ten minutes, phages were recovered from the beads separated using a magnetic stand. Furthermore, a trypsin solution was added to the mixture to a final concentration of 1 mg/mL. The mixed solution was stirred at room temperature for 15 minutes, and then phages were recovered from the beads separated using a magnetic stand. The recovered phages were added to 20 mL of *E. coli* strain ER2738 in the logarithmic growth phase (OD600 of 0.4 to 0.7). *E. coli* was infected with the phage by incubating the *E. coli* with gentle stirring at 37°C for one hour. Infected *E. coli* was seeded onto a 225 mm x 225 mm-plate medium. Next, the seeded *E. coli* culture medium was infected with M13KO7TC, and upon culturing overnight at 30°C, phages were collected from the culture supernatant to prepare an antibody-displaying phage library solution.

Using the prepared antibody-displaying phage library solution, the second and subsequent pannings were performed by a similar method, and repeated up to the fifth panning. It was noted that, in negative selection, 800 pmol of the antigen was used in the second panning, and 400 pmol of the antigen was used in the third and subsequent pannings. As for SMB0002hH-G1m3/SMB0002hL-k0a used as an antigen after negative selection, 300 pmol of the antigen was used in the second panning, and 150 pmol of the antigen was used in the third and subsequent pannings. In the bead washing operation after binding the complex of adenosine and phage with SMB0002hH-G1m3/SMB0002hL-k0a to the magnetic beads, in the second panning, washing with adenosine/TBST twice and then washing with adenosine/TBS once were performed. In the third and subsequent pannings, washing with adenosine/TBST three times and then washing with adenosine/TBS twice were performed. After panning, the recovered phages were used to infect *E. coli,* and the *E. coli* was seeded onto a plate medium to obtain a single colony of *E. coli* infected with the phage.

The same panning operation was performed by adding an adenosine derivative.

[Example 8]

Evaluation of binding activity to a complex of adenosine with an adenosine-binding antibody by phage ELISA

From a single colony of *E. coli* obtained in Example 7, phage-containing culture supernatant was recovered by following a standard method (Methods Mol. Biol. (2002) 178, 133-145). The nucleotide sequence of the antibody gene was determined from a single colony of *E. coli* by a method known to those skilled in the art. The collected culture supernatant was ultrafiltered using NucleoFast 96 (MACHEREY-NAGEL). The flow-through was removed by centrifuging NucleoFast 96 in which 200 μL of each culture supernatant was applied to each well (centrifugation at 6000 x g and 4°C for 40 minutes). The NucleoFast 96 with 200 μL of $H_2O$ added to each well was washed by centrifugation again (centrifugation at 6000 x g and 4°C for 20 minutes). Finally, 200 μL of TBS was added, the phage contained in the supernatant of each well of the NucleoFast 96 that was allowed to stand at room temperature for five minutes was recovered as a purified phage. Purified phage with TBS or adenosine/TBS added was subjected to ELISA by the following procedure. A 10 μL TBS solution containing the above described biotinylated SMB0002hH-G1m3/SMB0002hL-k0a or five variants thereof at 25 pmol/mL was used for at least one hour to coat 384-well streptavidin-coated microplates (Greiner Bio-One). Each well of the plate was washed with TBST to remove the biotinylated antigen not bound to the plate, and then the well was blocked with 80 μL of 2% skim milk-TBS for 1 hour or longer. After removing 2% skim milk/TBS, the plate with purified phage added to each well was allowed to stand at room temperature for 1 hour, making the phage displaying antibody bound to the biotinylated antigen present in each well in the presence or absence of adenosine at a final concentration of 500 μM. A plate to which an HRP-conjugated anti-M13 antibody (GE Healthcare) diluted with adenosine/TBST or TBST was added to each well washed with adenosine/TBST or TBST, was incubated for 1 hour. After washing with adenosine/TBST or TBST, the color reaction of the solution in each well to which TMB single solution (ZYMED) was added was stopped by addition of sulfuric acid, and then the color was measured from the absorbance at 450 nm. Moreover, the same screening was performed using an adenosine derivative. As a result, a plurality of antibody-displaying phages that bound to SMB0002hH-G1m3/SMB0002hL-k0a in the presence of adenosine or the adenosine derivative, and did not bind in the absence of adenosine or the adenosine derivative were confirmed. In addition, among them, there were a plurality of phages that did not bind in the presence of adenosine or an adenosine derivative to the plate onto which the mixed solution of the five types of SMB0002hH-G1m3/SMB0002hL-k0a variants with attenuated adenosine binding ability was immobilized. From these results, it was shown that antibodies showing binding activity to an adenosine-binding antibody only in the presence of adenosine or an adenosine derivative can be obtained from an antibody-displaying phage library. Of the 768 clones evaluated by phage ELISA, 40 different antibodies showing such binding ability, which exclude overlapping sequences, were obtained as candidates for a clamping antibody that recognizes a complex of adenosine or adenosine derivative with an adenosine-binding antibody.

[Example 9]

Preparation of biotinylated SMB0002Fab

A gene fragment encoding SMB0002hL-k0aTEVBAP (SEQ ID NO: 87) prepared by linking a TEV protease cleavage sequence and an AviTag sequence are linked to the C-terminus of the light chain of SMB0002 *via* a linker was introduced into an animal expression vector. Animal expression vectors SMB0002hH-G1m3 and SMB0002hL-k0aTEVBAP were introduced into Expi293 cells (Life Technologies) using 293Fectin (Life Technologies). At this time, a gene expressing EBNA1 and a gene expressing biotin ligase (BirA) were co-introduced, and further, biotin was added for the purpose of biotinylating the C-terminus of the light chain of SMB0002hH-G1m3/SMB0002hL-k0aTEVBAP (SEQ ID NO: 80, SEQ ID NO: 87). Cells into which the antibody expression vector had been introduced were cultured at 37°C under 8% $CO_2$, and SMB0002hH-G1m3/SMB0002hL-k0aTEVBAP in which the C-terminus of the light chain was biotinylated was secreted into the culture supernatant. The cell culture solution was centrifuged, and the supernatant was filtered through SARTPORE 2 300 (Sartorius) to obtain the culture supernatant. Addition of the culture supernatant to a 5-mL size Protein A carrier column HiTrap MabSelect Sure pcc (GE Healthcare) equilibrated with D-PBS(-), and addition of four column volumes of 50 mM acetate buffer at 5 mL/min led to elution of the antibody, and addition of 1.5 M Tris-HCl at pH 7.4 for neutralization yielded a purified fraction of the antibody.

The purified fraction of the antibody was concentrated by exchanging the buffer solution with 100 mM Tris-HCl at pH 8.0 with a Jumbosep 30K disc (Pall), and then diluted to 2 mg/mL with 100 mM Tris-HCl at pH 8.0. Lys-C (Roche) at a mass ratio of 1/2000 was added to the diluted full-length antibody, and this was allowed to stand at 35°C for 2.5 hours. Thereafter, the reaction was stopped by adding 1/10 volume equivalent of a solution prepared by dissolving 2 tablets of cOmplet EDTA-free Protease Inhibitor Cocktail (Roche) in 10 mL of MQ.

Next, this sample was added to a 5-mL HiTrap Mabselect Sure connected in tandem to a 5-mL HiTrap Mabselect Sure pcc equilibrated with D-PBS(-), the flow-through was collected. 1 M Arginine-HCl at 1/6 liquid volume equivalent was added to the collected sample, and this was concentrated using Jambosep 10 K.
This was separated and purified using a gel filtration column Superdex 75 pg 26/60 (GE Healthcare) equilibrated with D-PBS(-). This was concentrated using an Amicon-Ultra 15 10 K (Merck Millipore), and D-PBS(-) containing 8M Urea was added at 1.6-times that volume. Then, using Slide-A-Lyzer G2 Dialysis Cassettes 20K (Thermo Fisher Scientific), stepwise dialysis was performed in sufficient amount of D-PBS (-) containing 6 M Urea, 4 M Urea, and 2 M Urea as the external dialysis solution, and subsequently, dialysis was performed twice with D-PBS(-), and the prepared Fab fragment was refolded. Then, the Fab solution after refolding was concentrated using Amicon-Ultra4 10 K (Merck Millipore), filtered through Millex GV filter unit 0.22 um (Merck Millipore), and a purified Fab fragment of SMB0002hH-G1m3/SMB0002hL-k0aTEVBAP in which the C-terminus of the light chain was biotinylated was obtained. This is designated as biotinylated SMB0002Fab.

[Example 10]

Evaluation of binding of an adenosine-clamping antibody to a complex of adenosine with an adenosine-binding antibody using the BLI method for the obtained antibody

The heavy-chain and light-chain variable region sequences of adenosine-clamping antibodies obtained in Example 8 were inserted into animal expression plasmids having an antibody heavy chain constant region, a light chain kappa constant region sequence, or a light chain lambda constant region sequence, respectively, to prepare antibody expression vectors. The nucleotide sequences of the obtained expression vectors were determined by a method known to those skilled in the art.

The antibody expression vectors were transiently introduced into FreeStyle293F cells (Thermo Fisher Scientific) or Expi293 cells (Thermo Fisher Scientific) to express the antibody. From the obtained culture supernatant, the antibody was purified using rProtein A Sepharose (registered trademark) Fast Flow (GE Healthcare) or Bravo AssayMAP (Agilent) and Protein A (PA-W) Cartrige (Agilent) by a method known to those skilled in the art. The purified antibody concentration was calculated by measuring the absorbance at 280 nm using a spectrophotometer, and calculating the antibody concentration from the obtained value using the extinction coefficient calculated by the PACE method (Protein Science 1995; 4: 2411- 2423).

Evaluation of the binding of each prepared and purified antibody to a complex of adenosine with an adenosine-binding antibody was performed using OctetHTX (ForteBio). Specifically, biotinylated SMB0002Fab prepared by the method described in Example 9, which was prepared with TBS or adenosine/TBS or SMB0002hH-G1m3/SMB0002hL-k0a biotinylated with EZ-Link Sulfo-NHS-SS-Biotin was bound to Dip and Read™ Streptavidin (SA) Biosensors (ForteBio). Subsequently, each purified antibody prepared at 10 μg/mL using TBS or adenosine/TBS was allowed to act, and the binding at 30°C was evaluated. Fig. 8 shows sensorgrams representing the amount of binding over time measured with OctetHTX. SC001 (heavy chain / light chain (SEQ ID NOs: 88 and 89)), SC002 (heavy chain / light chain (SEQ ID NOs: 90 and 91)), SC003 (heavy chain / light chain (SEQ ID NOs: 123 and 124)) SC014 (heavy chain / light chain (SEQ ID NOs: 92 and 93)), SC016 (heavy chain / light chain (SEQ ID NOs: 94 and 95)), SC019 (heavy chain / light chain (SEQ ID NOs: 96 and 97))), SC032 (heavy chain / light chain (SEQ ID NOs: 125 and 126)), SC034 (heavy chain / light chain (SEQ ID NOs: 127 and 128)), SC044 (heavy chain / light chain (SEQ ID NOs: 129 and 130))), SC045 (heavy chain / light chain (SEQ ID NOs: 131 and 132)), and SC048 (heavy chain / light chain (SEQ ID NOs: 133 and 134)) showed higher binding signals for biotinylated SMB0002Fab and biotinylated SMB0002hH-G1m3/SMB0002hL-k0 in the presence of adenosine, than in the absence of adenosine. On the other hand, SC009 (heavy chain / light chain (SEQ ID NOs: 98 and 99)) showed similar binding signals to biotinylated SMB0002Fab and to biotinylated SMB0002hH-G1m3/SMB0002hL-k0 in the presence and absence of adenosine.

[Example 11]

Evaluation of binding of an adenosine-clamping antibody to a complex of adenosine with an adenosine-binding antibody using the SPR method

Regarding the clamping antibodies that bind to the complex of adenosine with adenosine-binding antibody obtained in Example 10, their affinity for the adenosine-binding antibody in the presence of adenosine was analyzed using Biacore T200 (GE Healthcare). The antibody of interest was captured on a Sensor chip CM4 (GE Healthcare) onto which an appropriate amount of protein G (Invitrogen) was immobilized by the amine coupling method. Two types of buffers were used as running buffers: 20 mM ACES, 150 mM NaCl, 0.05% (w/v) Tween 20; or 20 mM ACES, 150 mM NaCl, 0.05% (w/v) Tween 20, 500 μM adenosine. The biotinylated SMB0002Fab prepared in Example 9 was prepared in the respective running buffers at final concentrations of 250 nM, 62.5 nM, and 15.6 nM, and the binding between each antibody and SMB0002Fab was measured under conditions of binding time of three minutes and dissociation time of five minutes for each ligand concentration at a flow rate of 30 μL/min using the single cycle kinetic function of Biacore T200 Control Software (GE Healthcare). Thereafter, the sensor chip was regenerated by injecting 10 mM Glycine-HCl (pH 2.5) and 10 mM NaOH, each at a flow rate of 30 μL/min for ten seconds. All measurements were performed at 25°C. Fig. 9 shows the affinity of each antibody for SMB0002Fab in the presence and absence of 500 μM adenosine (ADO) measured by the above-mentioned method. SC001, SC002, SC003, SC014, SC016, SC019, SC032, SC044, SC045, and SC048 were confirmed to have a smaller KD value for binding to SMB0002Fab, which is an adenosine-binding antibody, in the presence of adenosine than in the absence of adenosine, and to bind to the adenosine-binding antibody more strongly in the presence of adenosine. Since the binding activity of SC001 and SC019 to the adenosine-binding antibody in the absence of adenosine was low, the KD value could not be determined.

[Example 12]

Evaluation of the ability of an adenosine-clamping antibody to enhance the binding activity between an adenosine-binding antibody and adenosine, by using the SPR method

Regarding the clamping antibodies obtained in Example 10 that bind to a complex of adenosine with an adenosine-binding antibody, adenosine concentration-dependent binding to an adenosine-binding antibody was evaluated using Biacore T200 (GE Healthcare). The antibody of interest was captured on a Sensor chip CM4 (GE Healthcare) onto which an appropriate amount of protein G (Invitrogen) was immobilized by the amine coupling method. As the running buffer, 20 mM ACES, 150 mM NaCl, 0.05% (w/v) Tween 20 was used. 500 nM biotinylated SMB0002Fab prepared in Example 9 was prepared using running buffers containing adenosine at final concentrations of 100 μM, 20 μM, 4 μM, 800 nM, 160 nM, 32 nM, 6.4 nM, and 1.28 nM, and the binding between each antibody and SMB0002Fab when injected under conditions of binding time of three minutes and dissociation time of five minutes at a flow rate of 30 μL/min was measured. Thereafter, the sensor chip was regenerated by injecting 10 mM Glycine-HCl (pH 2.5) and 10 mM NaOH at a flow rate of 30 μL/min for 10 seconds each. All measurements were performed at 25°C. Fig. 10 shows sensorgrams obtained by measuring the binding between each antibody and 500 nM SMB0002Fab in the presence of each concentration of adenosine, by the above method. Moreover, Table 3 shows the results of calculating from the above-mentioned results the KD value of the binding affinity of each antibody to adenosine in the presence of 500 nM SMB0002Fab by performing steady state analysis using Biacore T200 Evaluation Software.

[Table 3]

| Ligand | SMB0002 Fab conc. (nM) | KD (M) |
|---|---|---|
| SC001 | 0 | N.D |

(continued)

| Ligand | SMB0002 Fab conc. (nM) | KD (M) |
|---|---|---|
| SC002 | 0 | N.D |
| SC003 | 0 | N.D |
| SC009 | 0 | N.D |
| SC014 | 0 | N.D |
| SC016 | 0 | N.D |
| SC019 | 0 | N.D |
| SC032 | 0 | N.D |
| SC044 | 0 | N.D |
| SC045 | 0 | N.D |
| SC048 | 0 | N.D |
| Blank | 0 | N.D |
| SC001 | 500 | 1.63E-07 |
| SC002 | 500 | 1.49E-07 |
| SC003 | 500 | 1.39E-07 |
| SC009 | 500 | N.D |
| SC014 | 500 | 1.68E-07 |
| SC016 | 500 | 1.84E-07 |
| SC019 | 500 | 1.90E-07 |
| SC032 | 500 | 1.99E-07 |
| SC044 | 500 | 2.07E-07 |
| SC045 | 500 | N.D |
| SC048 | 500 | 1.69E-07 |
| Blank | 500 | N.D |

SC001, SC002, SC003, SC014, SC016, SC019, SC032, SC044, SC045, and SC048 were observed to increase the binding response to SMB0002Fab in an adenosine concentration-dependent manner. Results obtained in Examples 10, 11, and 12 showed that antibodies having adenosine-clamping ability can be obtained by the panning method described in Example 7. From the above-mentioned results, it was shown that the obtained clamping antibodies are not limited to the CD3-clamping antibodies obtained in Example 3, and clamping antibodies for adenosine and adenosine-binding antibody can be also obtained.

[Example 13]

Evaluation of adenosine-dependent cytotoxic activity using adenosine-clamping antibodies

Animal cell expression vectors inserted with the adenosine-binding antibody SMB0002hH-F760mnP17/SMB0002hL-k0a (heavy chain / light chain (SEQ ID NOs: 100 and 81)), CD3 agonist antibody CE115HA000-F760mnN17/L0011-k0a (heavy chain / light chain (SEQ ID NOs: 135 and 105)), adenosine-clamping antibody SC003H-F760mnP17/SC003L-SCL3 (heavy chain / light chain (SEQ ID NOs: 136 and 124), GPC3-binding antibody GCH065-F760mnN17/L0011-k0a (heavy chain / light chain (SEQ ID NOs: 104 and 105)), and a negative control antibody IC17HdK-F760mnN17/IC17L-k0a (heavy chain / light chain (SEQ ID NOs: 137 and 138)) or IC17HdK-F760mnP17/IC17L-k0a (heavy chain / light chain (SEQ ID NOs: 139 and 138)) were introduced into Expi293 cells, and by culturing the cells at 37°C under 8% $CO_2$, antibodies were secreted into the culture supernatant. Then, the antibodies were purified using a MonoSpin ProA 96-well plate type (GL Science) by a method known to those skilled in the art. By the method described in Example 3(3), a bispecific antibody of the adenosine-binding antibody and the CD3-binding antibody (SMB0002hH-F760mnP17/SMB0002hL-k0a//CE115HA000-F760mnN17/L0011-k0a), and a bispecific antibody of the adenosine-clamping antibody and the GPC3-binding antibody (SC003H-F760mnP17/SC003L-SCL3//GCH065-F760mnN17/L0011-k0a), and as comparative controls, a bispecific antibody of the adenosine-clamping antibody and a KLH-binding antibody (SC003H-F760mnP17/SC003L-SCL3//IC17HdK-F760mnN17/IC17L-k0a), and a bispecific antibody of the KLH-binding antibody and the GPC3-binding antibody (IC17HdK-F760mnP17/IC17L-k0a//GCH065-F760mnN17/L0011-k0a) were each prepared.

CD3 agonist activity when the two types of prepared bispecific antibodies and adenosine were added simultaneously was evaluated using Jurkat-NFAT reporter cells (NFAT luc2_jurkat cell). Jurkat-NFAT reporter cell is a cell line of human acute T-cell leukemia-derived cells expressing CD3 in which NFAT response element and luciferase (luc2P) are fused and when the signal downstream of CD3 is activated, luciferase is expressed. As a target cell, SK-pca60 cell line established by forcibly expressing human GPC3 in human liver cancer-derived cell line SK-HEP-1 was used. Target cells and reporter cells were added to each well of a white-bottomed 96-well assay plate (Costar) at 1.25E + 04 cells/well and 7.50E + 04 cells/well, respectively, and a mixed solution containing a final concentration of 50 nM bispecific antibody of the adenosine-binding antibody and CD3-binding antibody, a final concentration of 100 nM bispecific antibody of the adenosine-clamping antibody and GPC3-binding antibody, or a bispecific antibody serving as a comparative control was added to the well. Furthermore, adenosine at final concentrations of 1 μM, 10 μM, 100 μM, and 500 μM were added. After incubation at 37°C for six hours in the presence of 5% $CO_2$, luciferase enzyme activity was determined by measuring the amount of luminescence using the Bio-Glo luciferase assay system (Promega) according to the attached protocol. A list of antibodies used is shown in Table 4.

[Table 4]

| Sample No. | Antibody combinations used | SEQ ID NOs | Antibody concentration (nM) |
|---|---|---|---|
| 1 | SMB0002hH-F760mnP17/SMB0002hL-k0a// CE115HA000-F760mnN17/L0011-k0a | 100, 81, 135, 105 | 50 |
| | SC003H-F760mnP17/SC003L-SCL3// GCH065-F760mnN17/ L0011-k0a | 136, 124, 104, 105 | 100 |
| 2 | SMB0002hH-F760mnP17/SMB0002hL-k0a// CE115HA000-F760mnN17/L0011-k0a | 100, 81, 135, 105 | 50 |
| | SC003H-F760mnP17/SC003L-SCL3// IC17HdK-F760mnN1711C17L-k0a | 136, 124, 137, 138 | 100 |
| 3 | SMB0002hH-F760mnP17/SMB0002hL-k0a// CE115HA000-F760mnN17/L0011-k0a | 100, 81, 135, 105 | 50 |
| | IC17HdK-F760mnP17/IC17L-k0a// GCH065-F760mnN17/ L0011-k0a | 139, 138, 104, 105 | 100 |
| 4 | SMB0002hH-F760mnP17/SMB0002hL-k0a// CE115HA000-F760mnN17/L0011-k0a | 100, 81, 135, 105 | 50 |
| | None | - | - |

For detection, 2104 EnVision is used. As a result, when a mixed solution of the bispecific antibody of the adenosine-binding antibody and CD3-binding antibody and the bispecific antibody of the adenosine-clamping antibody and GPC3-binding antibody was added, increase of the luminescence signal of luciferase was observed in an adenosine concentration-dependent manner, and higher signal was shown than when a mixed solution of the bispecific antibody of the adenosine-binding antibody and CD3-binding antibody and the bispecific antibody serving as a comparative control was added (Fig. 11). That is, by adenosine clamping, the bispecific antibody of the adenosine-binding antibody and CD3-binding antibody and the bispecific antibody of the adenosine-clamping antibody and GPC3-binding antibody were able to bring the target cell and reporter cell close together, and activate CD3.

[Example 14]

Evaluation of binding property between the anti-CD3 antibody and CD3εδ in the presence or absence of a clamping antibody by SPR

(1) Preparation of antibodies for immobilization

Expression vectors prepared by inserting genes encoding CE115HA000-BS03a/GLS3000-k0 (SEQ ID NOs: 140 and 20), CE115HA056-BS03a/GLS3000-k0 (SEQ ID NOs: 141 and 20), CE115HA146-BS03a/GLS3000-k0 (SEQ ID NOs: 142/20), IC17Hdk-BS03a/IC17L-k0 (SEQ ID NOs: 143 and 66), and CLA0028VH-BS03b/CLA0028VL-k0C (SEQ ID NOs: 144 and 56) were introduced into Expi293F using ExpiFectamine293 (Thermo Fisher Scientific), the culture supernatant on the fifth day of culturing was collected, and the antibodies were prepared by a method known to those skilled in the art using HiTrap MabSelect SuRe. Preparation of BiAb of the anti-CD3 antibody and clamping antibody CLA0028 was performed by the FAE technique shown in Example 3(3). 500μg each of the anti-CD3 antibody or the negative control IC17 antibody and clamping antibody CLA0028 were mixed, 50 μL of 2-Mercaptoethylamine-HCl (2-MEA, Sigma-Aldrich) prepared at 250 mM in D-PBS(-) (Wako Pure Chemicals) was added, and the total volume was brought to 500 μL using a D-PBS(-) buffer. This reaction solution was incubated at 37°C for 90 minutes, and then PD-minitrap G-25 (GE Healthcare) was used to remove 2-MEA and replace it with D-PBS(-) (Wako Pure Chemicals). The concentrations of the obtained antibodies were calculated by the same method as the protein concentration calculation in Example 3 (1).

(2) Preparation of human CD3εδ heterodimer

Human CD3εδ heterodimer (hereinafter CD3εδ) was prepared by a method known to those skilled in the art. Specifically, a gene fragment encoding a FLAG tag (DYKDDDDK, SEQ ID NO: 70) and a termination codon was linked to a gene encoding the extracellular region (positions 1 to 129) of human CD3ε. A gene fragment encoding His-tag (HHHHHH, SEQ ID NO: 166) and a stop codon was linked to a gene encoding the extracellular region (positions 1 to 106) of human CD3δ. A gene fragment encoding a soluble human CD3ε (SEQ ID NO: 167) with FLAG tag added to the C-terminal side of the extracellular region of human CD3ε, and a gene fragment encoding a soluble human CD3δ (SEQ ID NO: 168) with His tag added to the C-terminus were inserted into animal cell expression vectors. The two types of constructed plasmid vectors were introduced into FreeStyle 293F cells (Invitrogen) using 293fectin (Thermo Fisher Scientific). The transfected cells were cultured at 37°C under 8% $CO_2$ to secrete the protein of interest into the culture supernatant. The cell culture medium was filtered through a 0.22-μm bottle top filter to obtain the culture supernatant.

The culture supernatant was diluted 3-fold with distilled water, adsorbed on Q Sepharose HP (GE Healthcare) equilibrated with 20 mM TrisHCl (pH 7.0), and then eluted with a salt concentration gradient of up to 50% using a buffer of 20 mM TrisHCl, 1M NaCl (pH7.0). Fraction containing the protein of interest was adsorbed onto a HisTrap HP column (GE Healthcare) equilibrated with 20 mM NaPhosphate, 500 mM NaCl, 20 mM Imidazol pH7.5, and elution was performed with a concentration gradient of imidazole up to 50 % using 20 mM NaPhosphate, 500 mM NaCl, 500 mM Imidazol pH7.5. Fraction containing the protein of interest was added to and adsorbed onto an Anti-FLAG M2 column packed with Anti-FLAG M2 agarose resin (Sigma-Aldrich), and the protein of interest was eluted with FLAG peptide dissolved in D-PBS(-). This eluate was subjected to gel filtration chromatography using Superdex 26/600 (GE healthcare) to remove aggregates and FLAG peptide to obtain purified CD3εδ. The concentration of the resulted purified protein was calculated by the same method as the protein concentration calculation in Example 3 (1).

(3) Evaluation of binding properties of clamping antibodies by SPR

Using an amine coupling kit (GE healthcare), SuRe protein A (GE healthcare) prepared at 25 μg/mL in Acetate4.5 (GE healthcare) was immobilized onto Sensor Chip CM4 at approximately 1200 RU per flow cell.

HBS-EP + (GE Healthcare) was used for the running buffer, and the measurement was carried out at 37°C. Each antibody was reacted at a flow rate of 10 μL/min for 60 seconds to capture 1000 RU, and analyte CD3εδ prepared at 0 nM, 4.8 nM, 24 nM, 120 nM, 600 nM, 3000 nM, or 15000 nM was allowed to act at a flow rate of 30 μL/min for 60

seconds to monitor the binding phase, and HBS-EP + was passed at a flow rate of 30 μL/min for 120 seconds to monitor the dissociation phase. The sensor chip was regenerated by passing Glycine1.5 and 25 mM NaOH, each at a flow rate of 30 μL/min for 30 seconds. The dissociation constant KD (M) was calculated based on the association rate constant ka (1/Ms) and the dissociation rate constant kd (1/s), which are kinetic parameters calculated from the sensorgram obtained by the measurement. Biacore T200 Evaluation Software (GE Healthcare) was used for the calculation of each parameter. The obtained KD values are shown in Table 5. The affinity enhancement effect was calculated from the value obtained by dividing the KD value obtained from BiAb with the IC17 arm by the KD value obtained from BiAb with the CLA0028 arm. As a result, enhancement of the KD value was approximately 30-fold for the CE115HA000 arm, approximately 200-fold for CE115HA056, and approximately 70-fold for CE115HA146.

[Table 5]

| Affinity enhancement effect of the clamping arm on CDεδ | | | |
|---|---|---|---|
| | IC17 | CLA0028 | -Fold |
| CE115HA000 | $3.5 \times 10^{-7}$ M | $1.1 \times 10^{-8}$ M | 32 |
| CE115HA056 | $7.4 \times 10^{-6}$ M | $4.0 \times 10^{-8}$ M | 185 |
| CE115HA146 | $1.4 \times 10^{-6}$ M | $2.1 \times 10^{-8}$ M | 67 |

[Example 15]

X-ray crystal structure analysis of a clamping antibody

(1) Antibody preparation

A clamping antibody was prepared as follows: expression vectors prepared by inserting a gene encoding CLA0028VH-F760mnP17/CLA0028VL-k0C (SEQ ID NOs: 55 and 56) were inserted into Expi293F using ExpiFectamine293 (Thermo Fisher Scientific), the culture supernatant was collected on the fifth day of culturing, and the antibody was prepared by a method known to those skilled in the art using HiTrap MabSelect SuRe. A CD3 antibody fused with an epitope peptide of CD3ε was prepared as follows: expression vectors prepared by inserting a gene encoding the CE115HA146 heavy chain (CE115HAPG13-rabCH1hG1m, SEQ ID NO: 6) and a corresponding light chain (GLS3000-rabk, SEQ ID NO: 7) were introduced into FreeStyle 293F cells (Thermo Fisher Scientific) using a transfection reagent 293fectin Tranfection Reagent (Thermo Fisher Scientific) according to the instructions provided by the manufacturer, and from the culture medium after culturing for five day, the antibody was prepared by affinity purification with rProteinA Sepharose Fast Flow resin (GE Healthcare).

(2) Preparation of CLA0028 Fab fragment

A sample of CLA0028VH-F760mnP17/CLA0028VL-k0C was fragmented into Fab and Fc using Lys-C (Roche, 11047825001) under conditions of 35°C x 2 hours. Next, Fab samples were prepared through column purification using HiTrap SP HP 1 ml (GE Healthcare) + HiTrap MabSelect SuRe 1 ml (GE Healthcare) and SEC purification using HiLoad 16/600 Superdex 200 pg (GE Healthcare).

(3) Preparation of CLA0028 Fab/CE115HAPG13 Fab complex

The obtained CLA0028 Fab sample was added to a CE115HAGP13-rabIgG/GLS3000-rabk sample so that the molar ratio of CLA0028 Fab becomes slightly excessive, and by SEC purification using Superdex 200 Increase 10/300 GL (GE Healthcare) using 20 mM HEPES pH 7.3 with 100 mM NaCl as a buffer, a CLA0028 Fab-CE115HAGP13-rabIgG/GLS3000-rabk complex sample was prepared. The obtained sample was fragmented into Fab and Fc using Lys-C (Roche, 11047825001) at room temperature overnight, and then the fragmented sample was passed through HiTrap MabSelect SuRe 1 ml (GE Healthcare) to remove the Fc fragment. Furthermore, CLA0028 Fab - CE115HAPG13 Fab complex sample was prepared by SEC purification with Superdex 200 Increase 10/300 GL (GE Healthcare) using 20 mM HEPES pH7.3 with 100 mM NaCl as a buffer, and by concentrating this through ultrafiltration, a sample of the complex for crystallization was prepared.

(4) Crystallization of a CLA0028 Fab/CE115HAPG13 Fab complex

The obtained sample was crystallized at 21°C under Morpheus (registered trademark) (Molecular Dimensions) F10 reservoir condition, by the sitting-drop vapor diffusion method. A crystal suitable for X-ray structural analysis was obtained.

(5) X-ray diffraction data collection and crystal structure determination from a crystal of the CLA0028 Fab/CE115HAPG13 Fab complex

The obtained crystal was immersed in a reservoir solution of Morpheus (registered trademark) (Molecular Dimensions) F10, and then frozen in liquid nitrogen, and X-ray diffraction data were measured using Swiss Light Source X10SA. During the measurement, the crystal was kept frozen by always placing it under a stream of nitrogen at 100 K. The obtained diffraction image were processed using autoPROC (Acta Cryst. D67: 293-302 (2011)), and diffraction intensity data up to a resolution of 2.5Å was acquired. From the obtained X-ray diffraction intensity data, the initial structure was determined by performing the molecular replacement method by Phaser (J. Appl. Cryst. (2007) 40, 658-674) using the known Fab crystal structure as a search model, and. Thereafter, a model construction and refinement by coot (Acta Cryst. D66: 486-501 (2010)), refmac5 (Acta Cryst. D67: 355-367 (2011)), and phenix.refine (Acta Cryst. D68: 352-367 (2012)) were repeated, and final refined coordinates were obtained. The crystallographic statistics are shown in Table 6.

[Table 6]

| Crystal structure analysis data | |
|---|---|
| **Data measurement** | |
| Measurement wavelength (Å) | 1.00006 |
| Number of measured crystals | 1 |
| Space group | *P*1 |
| Lattice constants | |
| *a b c* (Å) | 76.522 78.332 123.475 |
| $\alpha\ \beta\ \gamma$ (° ) | 107.26 96.11 94.04 |
| Number of complexes in an asymmetric unit | 2 |
| Resolution (Å) | 116.86 - 2.500 (2.59 - 2.50) |
| Number of observed reflections/ number of independent reflections | 158778 / 88255 |
| Redundancy | 1.80 (1.84) |
| Completeness (%) | 94.19 (97.14) |
| Diffraction intensity S/N ratio | 9.3 (2.7) |
| $R_{merge}$ | 0.041 (0.255) |
| **Refinement** | |
| $R_{work}/R_{free}$ | 0.2117 / 0.2562 |
| Number of atoms | 13201 |
| Root mean square deviations from ideal | |
| Bond length (Å) | 0.008 |
| Bond angle (° ) | 1.047 |
| Ramachandran plot | |
| Favored region (%) | 93.89 |
| Allowed region (%) | 5.31 |
| Outlier region (%) | 0.81 |

Values in parentheses are for the highest-resolution shell.

(6) Structure of the CLA0028 Fab/CE115HAPG13 Fab complex

As shown in Fig. 12, it was confirmed that CLA0028 Fab and CE115HAPG13 Fab formed a complex such that the N-terminal 7-residue peptide of CD3e is positioned between them, and that a clamping antibody in agreement with the concept was obtained.

[Example 16]

Evaluation of TDCC activity specific to GPC3 and CLDN6-positive cells using human T cells

(1) Preparation of effector cells

According to a method known to those skilled in the art, T cells were isolated from PBMC (Stemcell) using a T-cell isolation kit (Stemcell), and they were grown on CD3/CD28 beads (Invitrogen) and stored. In subsequent tests, the stored isolated T cells were frozen and thawed, and cultured, and the resulting suspension was used as effector cells.

(2) Preparation of target cells

NCI-H446 (GPC3-positive cells), AGS (CLDN6-positive cells), and GM5.1 (GPC3, CLDN6-positive cells) were subjected to subsequent experiments as target cells.

(3) Preparation of BiAbs

According to the method described above, BiAbs shown in Table 7 below were prepared.

[Table 7]

| Antibody name | BiAb type | Heavy chain of Parent antibody 1 | SEQ ID NO | Light chain of Parent antibody 1 | SEQ ID NO | Heavy chain of Parent antibody 2 | SEQ ID NO | Light chain of Parent antibody 2 | SEQ ID NO |
|---|---|---|---|---|---|---|---|---|---|
| AE3.20/CE115HA000 | BiAb1 | CE115H-BS03bFLAG | 145 | CE115L-SK1 | 146 | AE3.20H-BS03aHis | 147 | AE3.20L-SK1 | 148 |
| GCH065/CE115HA056 | BiAb1 | CE115V95AH-BS03bFLAG | 149 | CE115L-SK1 | 146 | GCH065H-BS03aHis | 150 | TR01L0011-SK1 | 151 |
| GCH065/CE115HA000 | BiAb1 | CE115H-BS03bFLAG | 145 | CE115L-SK1 | 146 | GCH065H-BS03aHis | 150 | TR01L0011-SK1 | 151 |
| IC17/CE115HA056 | BiAb1 | CE115V95AH-BS03bFLAG | 149 | CE115L-SK1 | 146 | cKLHH-BS03aHis | 152 | KLHL-K0 | 153 |
| IC17/CLA0028 | BiAb2 | CLA0028H-BS03bFLAG | 154 | CLA0028L-SK1 | 155 | cKLHH-BS03aHis | 152 | KLHL-K0 | 153 |
| AE3.20/CLA0028 | BiAb2 | CLA0028H-BS03bFLAG | 154 | CLA0028L-SK1 | 155 | AE3.20H-BS03aHis | 147 | AE3.20L-SK1 | 148 |
| GCH065/CLA0028 | BiAb2 | CLA0028H-BS03bFLAG | 154 | CLA0028L-SK1 | 155 | GCH065H-BS03aHis | 150 | TR01L0011-SK1 | 151 |

(4) Cytotoxicity assay (TDCC assay)

TDCC activity was evaluated by measuring the value of electrical resistance generated accompanying cell adhesion to the electrode using xCELLigence (ACEABiosciences). First, the medium used for target cell preparation was added to a RTCA Resistor plate 96, and the background value was corrected.

Next, the target cell suspension solution prepared as in Example 6 was seeded, the plate was placed into xCELLigence, and then the cells were cultured overnight under conditions of 37°C and 5% $CO_2$. On the day after seeding the cells, BiAb1 was added to each well at a final concentration of 0, 0.4, 2, and 10 nM, and BiAb2 was added to each well at a final concentration of 10 nM. Thereafter, an effector cell suspension solution containing cells at five times the number of target cells was added, the plate was placed into xCELLigence, cultured under conditions of 37°C and 5% $CO_2$, during which the electrical resistance value (Cell index) was measured at 10 minute intervals over time.

The cell growth inhibition rate (CGI) was calculated by the following Formula 3 as an index of TDCC activity.

(Formula 3)

$$\mathrm{CGI}\ [\%\ \mathrm{no\ Ab\ control}] = \left(\frac{X-Y}{X-1} \times 100\right)$$

In the above Formula 3, all of the Cell Indices used were calculated using Delta Cell Index where the first resistance value measurement point after antibody addition is taken to be 1. "X" represents the average value of Delta Cell Indices at the final measurement point of the antibody-free wells, and "Y" represents the average value of Delta Cell Indices at the final measurement point of the antibody-added wells.

Fig. 13 is a diagram showing TDCC activity by double antigen-binding (GPC3 and CLDN6).

The TDCC activity specific to double-positive cells was evaluated using GPC3 and CLDN6. GCH065/CE115HA000 (anti-GPC3 TRAB) and AE3.20/CE115HA000 (anti-CLDN6 TRAB) were used as positive controls. As shown in Fig. 13, GCH065/CE115HA000 and AE3.20/CE115HA000 showed TDCC activity against the antigen single-positive cells NCI-H446 (GPC3) and AGS (CLDN6), respectively, and both antibodies showed TDCC activity against the double-positive cells GM5.1 (GPC3/CLDN6).

When GCH065/CE115HA056 of BiAb1 and IC17/CLA0028 of BiAb2 were made to act, when IC17/CE115HA056 of BiAb1 and AE3.20/CLA0028 of BiAb2 were made to act, and when IC17/CE115HA056 of BiAb1 and GCH065/CLA0028 of BiAb2 were allowed to act, none of the pairs indicated TDCC activity on any of the cell lines; however, in the presence of AE3.20/CLA0028 of BiAb2, GCH065/CE115HA056 of BiAb1 showed TDCC activity only on the antigen double-positive cells GM5.1.

Based on the above results, the present inventors succeeded in producing an antibody that exhibits TDCC activity specifically to antigen double-positive cells.

[Example 17]

Evaluation of TDCC activity specific to GPC3- and HER2-positive cells using human T cells

(1) Preparation of effector cell solution

The isolated T cells prepared by the method described above were used as effector cells in subsequent experiments.

(2) Preparation of target cells

NCI-H446 (GPC3-positive cells), NCI-N87 (HER2-positive cells), and GPC3-expressing NCI-N87 (GPC3- and HER2-positive cells) were used as target cells in subsequent experiments.

(3) Preparation of BiAbs

According to the previously described method, BiAbs shown in Table 8 below were prepared.

[Table 8]

| Antibody name | BiAb type | Heavy chain of Parent antibody 1 | SEQ ID NO | Light chain of Parent antibody 1 | SEQ ID NO | Heavy chain of Parent antibody 2 | SEQ ID NO | Light chain of Parent antibody 2 | SEQ ID NO |
|---|---|---|---|---|---|---|---|---|---|
| HER2/CE115HA000 | BiAb1 | CE115H-BS03bFLAG | 145 | CE115L-SK1 | 146 | HER2H-BS03aHis | 156 | HER2L-SK1 | 157 |
| GCH065/CE115HA000 | BiAb1 | CE115H-BS03bFLAG | 145 | CE115L-SK1 | 146 | GCH065H-BS03aHis | 150 | TR01L0011-SK1 | 151 |
| GCH065/CE115HA056 | BiAb1 | CE115V95AH-BS03bFLAG | 149 | CE115L-SK1 | 146 | GCH065H-BS03aHis | 150 | TR01L0011-SK1 | 151 |
| IC17/CE115HA056 | BiAb1 | CE115V95AH-BS03bFLAG | 149 | CE115L-SK1 | 146 | cKLHH-BS03aHis | 152 | KLHL-K0 | 153 |
| HER2/CLA0028 | BiAb2 | CLA0028H-BS03bFLAG | 154 | CLA0028L-SK1 | 155 | HER2H-BS03aHis | 156 | HER2L-SK1 | 157 |
| IC17/CLA0028 | BiAb2 | CLA0028H-BS03bFLAG | 154 | CLA0028L-SK1 | 155 | cKLHH-BS03aHis | 152 | KLHL-K0 | 153 |

(4) Cytotoxic assay (TDCC assay)

Cytotoxic assay using xCELLigence (ACEA Biosciences) described in Example 16 was performed. On the day after seeding the cells, BiAb1 was added to each well at a final concentration of 0, 0.08, 0.4, and 2 nM and BiAb2 was added to each well at a final concentration of 5 nM. Thereafter, an effector cell suspension containing effector cells at five times the number of target cells was added, the plate was placed into xCELLigence, cultured under conditions of 37°C and 5% $CO_2$, during which the electrical resistance value (Cell index) was measured at 10 minute intervals over time.
Fig. 14 is a set of graphs showing TDCC activity by double antigen binding (GPC3 and HER2).
The TDCC activity specific to double-positive cells was evaluated using GPC3 and HER2. GCH065/CE115HA000 (anti-GPC3 TRAB) and HER2/CE115HA000 (anti-Her2 TRAB) were used as positive controls. As shown in Fig. 14, GCH065/CE115HA000 and HER2/CE115HA000 showed TDCC activity against the antigen single-positive cells NCI-H446 (GPC3) and NCI-N87 (HER2), respectively, and both antibodies showed TDCC activity against the double-positive cells GPC3-expressing NCI-N87 (GPC3/HER2).
When GCH065/CE115HA056 of BiAb1 and IC17/CLA0028 of BiAb2 were made to act, and when IC17/CE115HA056 of BiAb1 and HER2/CLA0028 of BiAb2 were made to act, none of the pairs indicated TDCC activity on any of the cell lines; however, in the presence of HER2/CLA0028 of BiAb2, GCH065/CE115HA056 of BiAb1 showed TDCC activity only on the antigen double-positive cells GPC3-expressing NCI-N87.
Based on the above results, the present inventors succeeded in producing an antibody that exhibits TDCC activity specifically to antigen double-positive cells.

[Example 18]

CD8-specific TRABs that use a clamping antibody

A bispecific antibody that exhibits antitumor effects by recruiting and activating T cells *via* CD3 (T cell-redirecting antibody) (abbreviated as "TRAB") is known to induce cytokine release syndrome as side effects while having strong antitumor effects (Non-Patent Literature: Journal for ImmunoTherapy of Cancer 2018. 6, 56).
It has been reported that antitumor activity induced by a TRAB is mainly exerted by CD8-positive T cells, whereas cytokines that cause cytokine release syndrome, such as IL6 induced by a TRAB are mainly released from CD4-positive T cells (Non-patent Document: Immunology. 2017 152 (3): 425-438). If a TRAB that uses only CD8-positive T cells as effector cells can be produced, it can be expected that such TRAB will become an ideal drug that maintains the strong antitumor activity of TRAB while suppressing side effects of cytokine release. Thus, a superior TRAB can be developed by using a technique with which we use only double-positive cells (in this case, CD3/CD8-expressing cells).
The examples below were designed to use CD8, an immune-related molecule instead of a cancer antigen, as a third antigen recognized by a clamping antibody (second antigen-binding molecule). It is expected that only when a CD8-positive T cell bound with this clamping antibody approaches a cancer cell bound with an antibody that recognizes the first antigen (first antigen-binding molecule), the clamping antibody recognizes an antigen/antigen-binding molecule complex formed by CD3 and an antibody that recognizes the first antigen, and induces TDCC activity. In this case, the second antigen is a cancer antigen.
Fig. 15 is a diagram schematically illustrating the mechanism of action when one embodiment of the first antigen-binding molecule and one embodiment of the second antigen-binding molecule crosslink target cells and effector cells.
To confirm this concept, an experiment detailed below was conducted.

(1) Preparation of effector cells

CD8-positive T cells and CD4-positive T cells were isolated from human PBMCs using EasySep Human CD4 + T cell isolation kit (Stemcell) and EasySep Human CD8 + T cell isolation kit (Stemcell), and together with PBMCs, the cells were used as effector cells in subsequent assays.

(2) Preparation of target cells

GPC3-positive cells SKpca60 were used as target cells in subsequent experiments.

(3) Preparation of antibodies

BiAbs shown in Table 9 below were prepared according to the method described above.

[Table 9]

| Antibody name | BiAb type | Heavy chain of Parent antibody 1 | SEQ ID NO | Light chain of Parent antibody 1 | SEQ ID NO | Heavy chain of Parent antibody 2 | SEQ ID NO | Light chain of Parent antibody 2 | SEQ ID NO |
|---|---|---|---|---|---|---|---|---|---|
| GCH065/CE115HA000 | BiAb1 | CE115H-BS03bFLAG | 145 | CE115L-SK1 | 146 | GCH065H-BS03aHis | 150 | TR01L0011-SK1 | 151 |
| GCH065/CE115HA056 | BiAb1 | CE115V95AH-BS03bFLAG | 149 | CE115L-SK1 | 146 | GCH065H-BS03aHis | 150 | TR01L0011-SK1 | 151 |
| IC17/CE115HA056 | BiAb1 | CE115V95AH-BS03bFLAG | 149 | CE115L-SK1 | 146 | cKLHH-BS03aHis | 152 | KLHL-K0 | 153 |
| CD8/CLA0028 | BiAb2 | CLA0028H-BS03bFLAG | 154 | CLA0028L-SK1 | 155 | CD8H-BS03aHis | 158 | CD8L-SK1 | 159 |
| IC17/CLA0028 | BiAb2 | CLA0028H-BS03bFLAG | 154 | CLA0028L-SK1 | 155 | cKLHH-BS03aHis | 152 | KLHL-K0 | 153 |

(4) Cytotoxic assay (TDCC assay)

Cytotoxic assay using xCELLigence (ACEA Biosciences) described in Example 16 was performed.

Fig. 16 shows the TDCC activity specific to CD8-positive T cells by double antigen binding (GPC3 and CD8).

Since SKpca60 used as a target cell is GPC3-positive, GCH065/CE115HA000 (anti-GPC3 TRAB) was used as a positive control. As shown in Fig. 16, the positive control GCH065/CE115HA000 showed TDCC activity, even when any of PBMCs (both CD4-positive T cells and CD8-positive T cells present as a mixture), CD4-positive T cells, and CD8-positive T cells were used as effector cells. On the other hand, GCH065/CE115HA056 + CD8/CLA0028 showed TDCC activity when CD8-positive T cells were used as effector cells, but did not show TDCC activity when CD4-positive T cells were used as effector cells. When PBMCs were used as effector cells, it showed TDCC that was approximately half of that shown when CD8-positive T cells were used as effector cells. It is considered that this may be because the number of CD8-positive T cells contained in PBMCs is less than when CD8-positive T cells are used as effector cells. TDCC activities were not confirmed from IC17/CE115HA056 + CD8/CLA0028 and GCH065/CE115HA056 + IC17/CLA0028 used as negative controls, even when any of the cells were used as effector cells.

Based on the above results, the present inventors succeeded in producing an antibody that exhibits CD8-positive T cell-specific TDCC activity.

[Example 19]

For some of the above-described antibodies, *in vivo* drug efficacy was also evaluated using a cancer-bearing model.

The *in vivo* drug efficacy was evaluated for representative antibodies from among those shown in Table 2, which antibodies were found to have cytotoxic activity in the *in vitro* assay described in Example 6. Human cancer cell line hEREG/SK-pca60 that expresses GPC3 and EREG was transplanted into NOD scid mice. Then, T cells grown by culturing human PBMCs *in vitro* were transferred to the mice with confirmed tumor formation. The mice were treated by administering antibodies (referred to as T cell transferred model).

That is, in the drug efficacy test of the antibody using the hEREG/SK-pca60 T cell-transferred model, the following test was performed. T cells were expanded using PBMCs isolated from blood collected from healthy volunteers and Dynabeads Human T-Activator CD3/CD28 (Thermo Fisher Scientific). 1 x $10^7$ cells of human cancer cell line hEREG/SK-pca60 and Matrigel basement membrane matrix (BD) were mixed and transplanted subcutaneously to the inguinal region of NOD scid mice (CLEA Japan, female, 7W). The day of transplantation was defined as Day 0. Mice were grouped according to tumor size and body weight on the 21st day after transplantation, and then anti-asialo GM1 antibody was administered intraperitoneally at 0.2 mg/mouse. The next day, T cells obtained by the aforementioned expansion were transplanted intraperitoneally at 3 x $10^7$ cells/mouse. Approximately four hours after T cell transplantation, the antibody was administered into the tail vein at 1 mg/kg. Antibody administration was performed twice, that is, on Day 0 and Day 7 (Fig. 17).

## Claims

1. A second antigen-binding molecule, which binds to an antigen/antigen-binding molecule complex comprising a first antigen and a first antigen-binding molecule that binds to the first antigen, and enhances the binding activity of the first antigen-binding molecule to the first antigen.

2. The second antigen-binding molecule of claim 1, which has higher binding activity to the first antigen in the presence of the first antigen-binding molecule than in the absence of the first antigen-binding molecule.

3. The second antigen-binding molecule of claim 1 or claim 2, wherein the first antigen is an immune-related molecule or a cellular metabolite.

4. The second antigen-binding molecule of claim 3, wherein the immune-related molecule is a molecule present on the cell membrane of an immune cell.

5. The second antigen-binding molecule of claim 4, wherein the immune cell is at least one selected from the group consisting of a granulocyte, a macrophage, a dendritic cell, a T cell, and a B cell.

6. The second antigen-binding molecule of any one of claims 3 to 5, wherein the immune-related molecule is CD3.

7. The second antigen-binding molecule of claim 6, wherein the first antigen-binding molecule comprises:

a CD3-binding polypeptide consisting of any combination of heavy chain variable region and light chain variable region amino acid sequences selected from SEQ ID NO: 1 and SEQ ID NO: 122, SEQ ID NO: 114 and SEQ ID NO:115, SEQ ID NO: 116 and SEQ ID NO: 117, SEQ ID NO: 118 and SEQ ID NO: 119, and SEQ ID NO: 120 and SEQ ID NO: 121, respectively; or
a first modified polypeptide produced by modifying the CD3-binding polypeptide, wherein the CD3-binding activity of the first modified polypeptide is lower than that of the CD3-binding polypeptide.

8. The second antigen-binding molecule of claim 3, wherein the cellular metabolite is adenosine or a derivative thereof.

9. The second antigen-binding molecule of claim 8, wherein the first antigen-binding molecule comprises:

an adenosine-binding polypeptide consisting of any combination of heavy chain variable region and light chain variable region amino acid sequences selected from SEQ ID NO: 106 and SEQ ID NO: 107, SEQ ID NO: 108 and SEQ ID NO:109, SEQ ID NO: 110 and SEQ ID NO: 111, and SEQ ID NO: 112 and SEQ ID NO: 113, respectively; or
a second modified polypeptide produced by modifying the adenosine-binding polypeptide, wherein the adenosine-binding activity of the second modified polypeptide is lower or higher than that of the adenosine-binding polypeptide.

10. The second antigen-binding molecule of any one of claims 1 to 9, wherein the first antigen-binding molecule has multiple antigen specificity and further binds to at least a second antigen.

11. The second antigen-binding molecule of claim 10, wherein the second antigen is a cancer antigen or an immune-related molecule.

12. The second antigen-binding molecule of any one of claims 1 to 11, which has multiple antigen specificity and further binds to at least a third antigen.

13. The second antigen-binding molecule of claim 12, wherein the third antigen is a cancer antigen or an immune-related molecule.

14. The second antigen-binding molecule of any one of claims 1 to 13, wherein the first antigen-binding molecule has multiple antigen specificity and further binds to at least a second antigen, wherein the second antigen-binding molecule has multiple antigen specificity and further binds to at least a third antigen, and wherein the combination of the first antigen, the second antigen, and the third antigen is any one of the combinations (1) to (5) below:

(1) a combination in which the first antigen is an immune-related molecule, the second antigen is a first cancer antigen, and the third antigen is a second cancer antigen;
(2) a combination in which the first antigen is a cellular metabolite of a target cell, the second antigen is a cancer antigen, and the third antigen is an immune-related molecule;
(3) a combination in which the first antigen is a cellular metabolite of a target cell, the second antigen is an immune-related molecule, and the third antigen is a cancer antigen;
(4) a combination in which the first antigen is a first immune-related molecule, the second antigen is a cancer antigen, and the third antigen is a second immune-related molecule; and
(5) a combination in which the first antigen is a first immune-related molecule, the second antigen is a second immune-related molecule, and the third antigen is a cancer antigen.

15. A combination of the first antigen-binding molecule and the second antigen-binding molecule of claim 1.

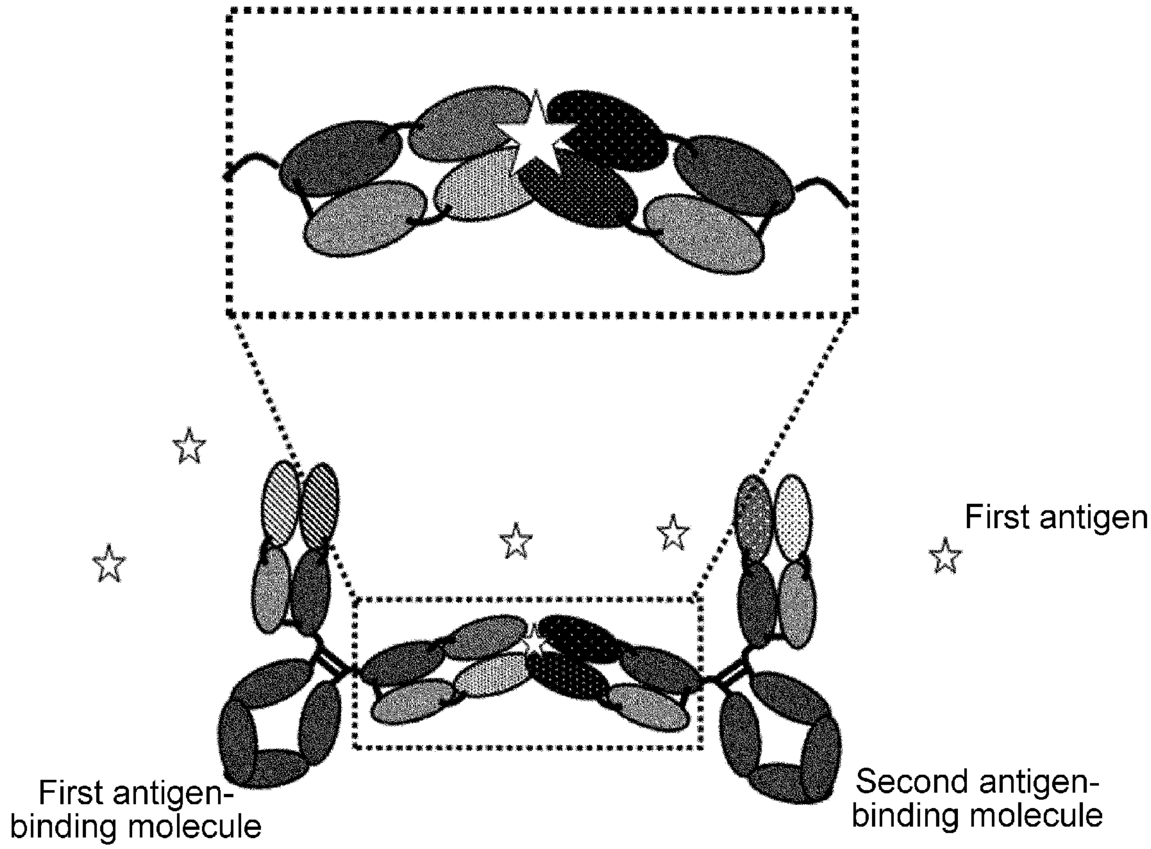
First antigen
First antigen-binding molecule
Second antigen-binding molecule

FIG. 1

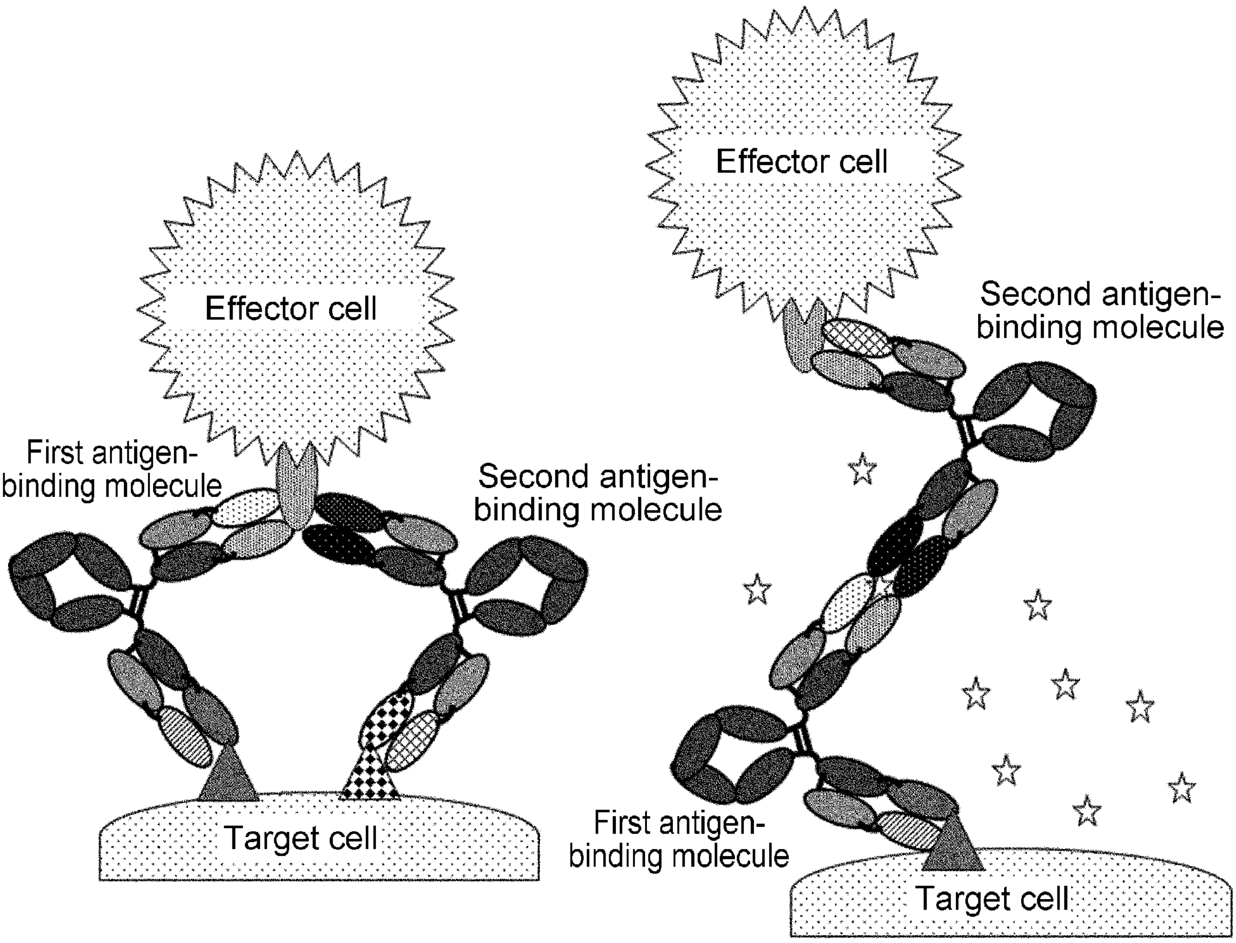
Effector cell
First antigen-binding molecule
Second antigen-binding molecule
Target cell
Effector cell
Second antigen-binding molecule
First antigen-binding molecule
Target cell

FIG. 2

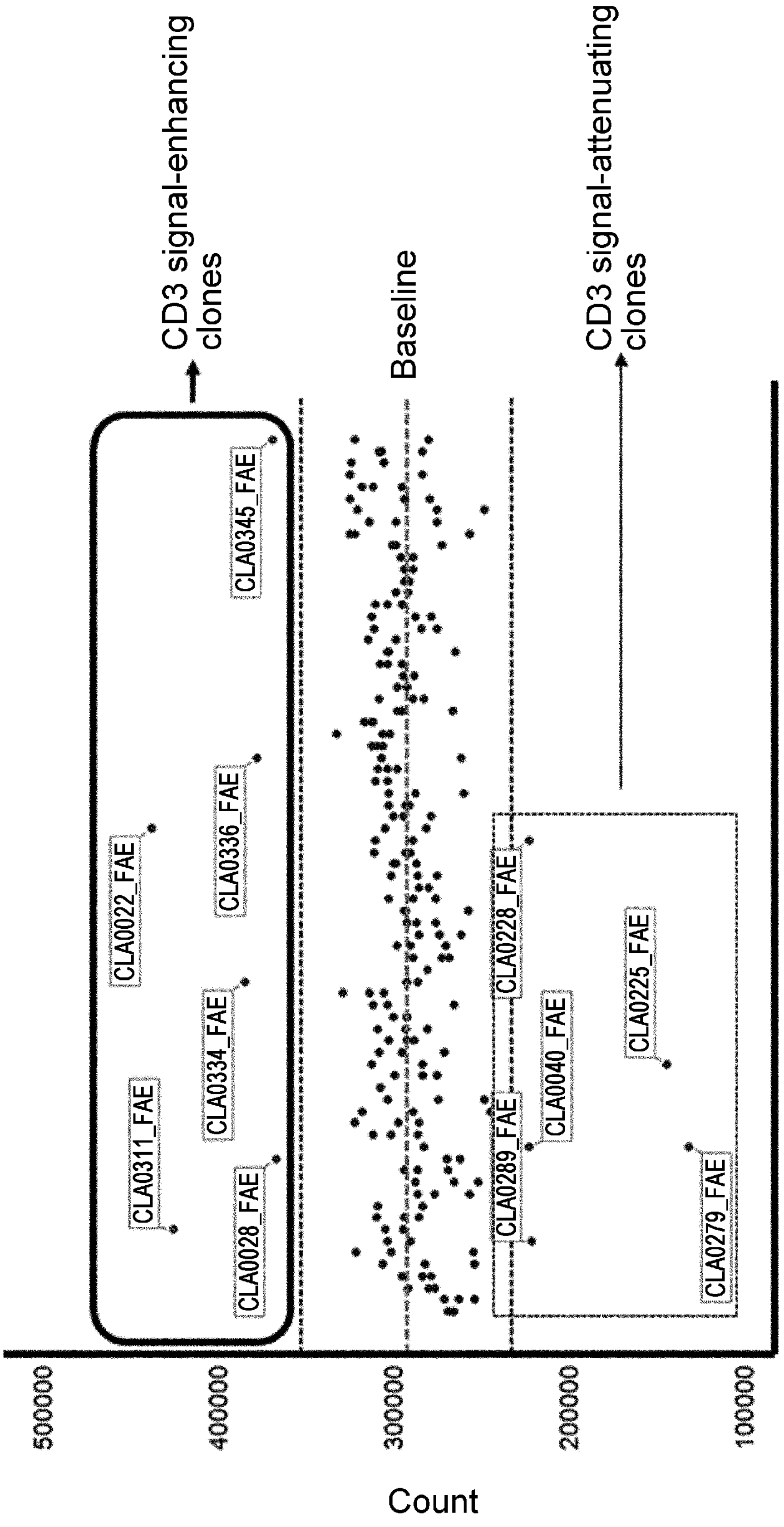
CD3 signal-enhancing clones
Baseline
CD3 signal-attenuating clones
CLA0345_FAE
CLA0022_FAE
CLA0336_FAE
CLA0334_FAE
CLA0311_FAE
CLA0028_FAE
CLA0228_FAE
CLA0225_FAE
CLA0040_FAE
CLA0289_FAE
CLA0279_FAE
500000
400000
300000
200000
100000
Count

FIG. 3

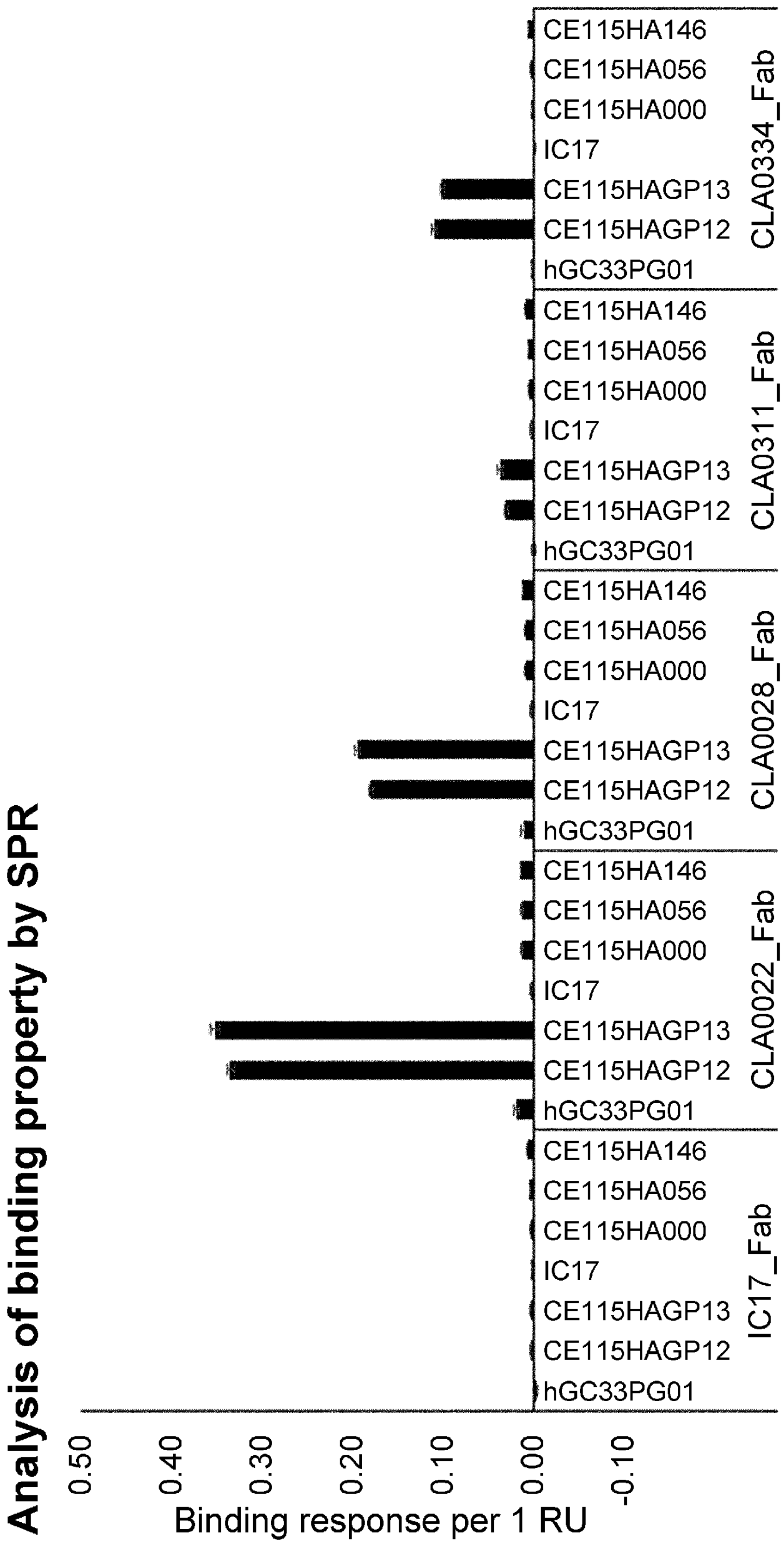
Analysis of binding property by SPR
Binding response per 1 RU
0.50
0.40
0.30
0.20
0.10
0.00
-0.10
CLA0334_Fab
CLA0311_Fab
CLA0028_Fab
CLA0022_Fab
IC17_Fab
CE115HA146
CE115HA056
CE115HA000
IC17
CE115HAGP13
CE115HAGP12
hGC33PG01

FIG. 4

## Stabilization of a CD3/anti-CD3 antibody complex by a clamping antibody

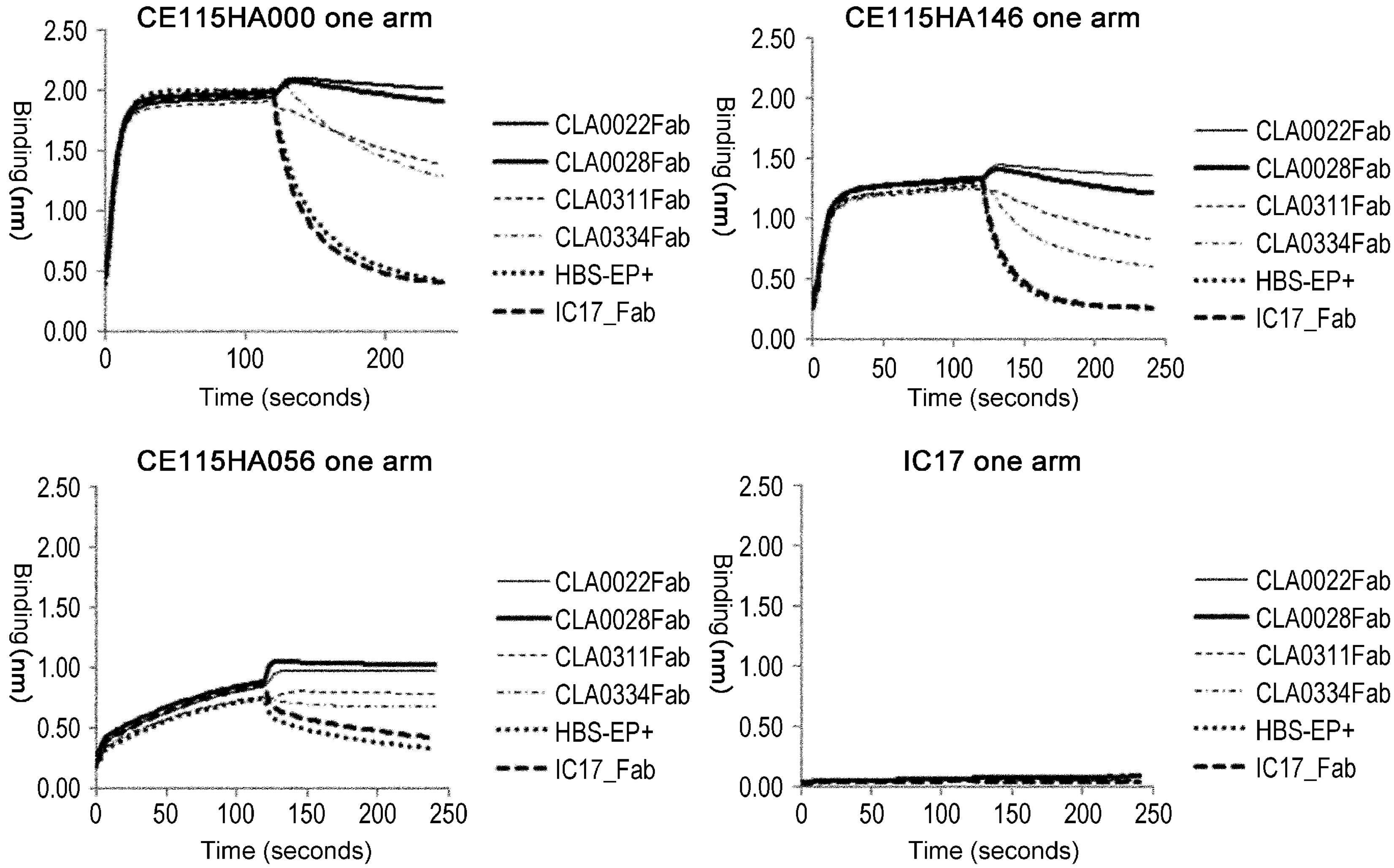


FIG. 5

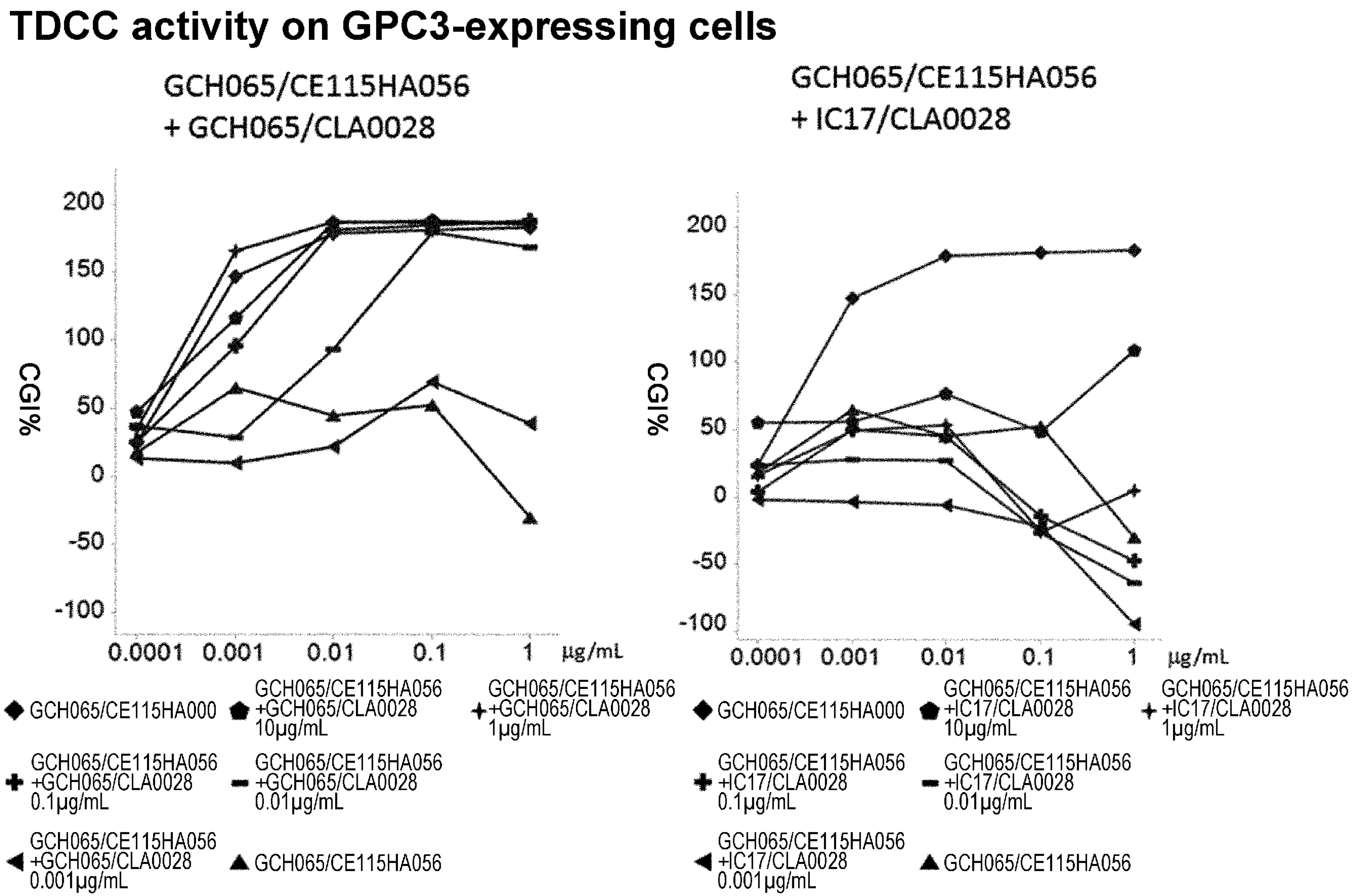
TDCC activity on GPC3-expressing cells
GCH065/CE115HA056
+ GCH065/CLA0028
CGI%
200
150
100
50
0
-50
-100
0.0001
0.001
0.01
0.1
1
µg/mL
GCH065/CE115HA000
GCH065/CE115HA056 +GCH065/CLA0028 10µg/mL
GCH065/CE115HA056 +GCH065/CLA0028 1µg/mL
GCH065/CE115HA056 +GCH065/CLA0028 0.1µg/mL
GCH065/CE115HA056 +GCH065/CLA0028 0.01µg/mL
GCH065/CE115HA056 +GCH065/CLA0028 0.001µg/mL
GCH065/CE115HA056
GCH065/CE115HA056
+ IC17/CLA0028
CGI%
200
150
100
50
0
-50
-100
0.0001
0.001
0.01
0.1
1
µg/mL
GCH065/CE115HA000
GCH065/CE115HA056 +IC17/CLA0028 10µg/mL
GCH065/CE115HA056 +IC17/CLA0028 1µg/mL
GCH065/CE115HA056 +IC17/CLA0028 0.1µg/mL
GCH065/CE115HA056 +IC17/CLA0028 0.01µg/mL
GCH065/CE115HA056 +IC17/CLA0028 0.001µg/mL
GCH065/CE115HA056

FIG. 6

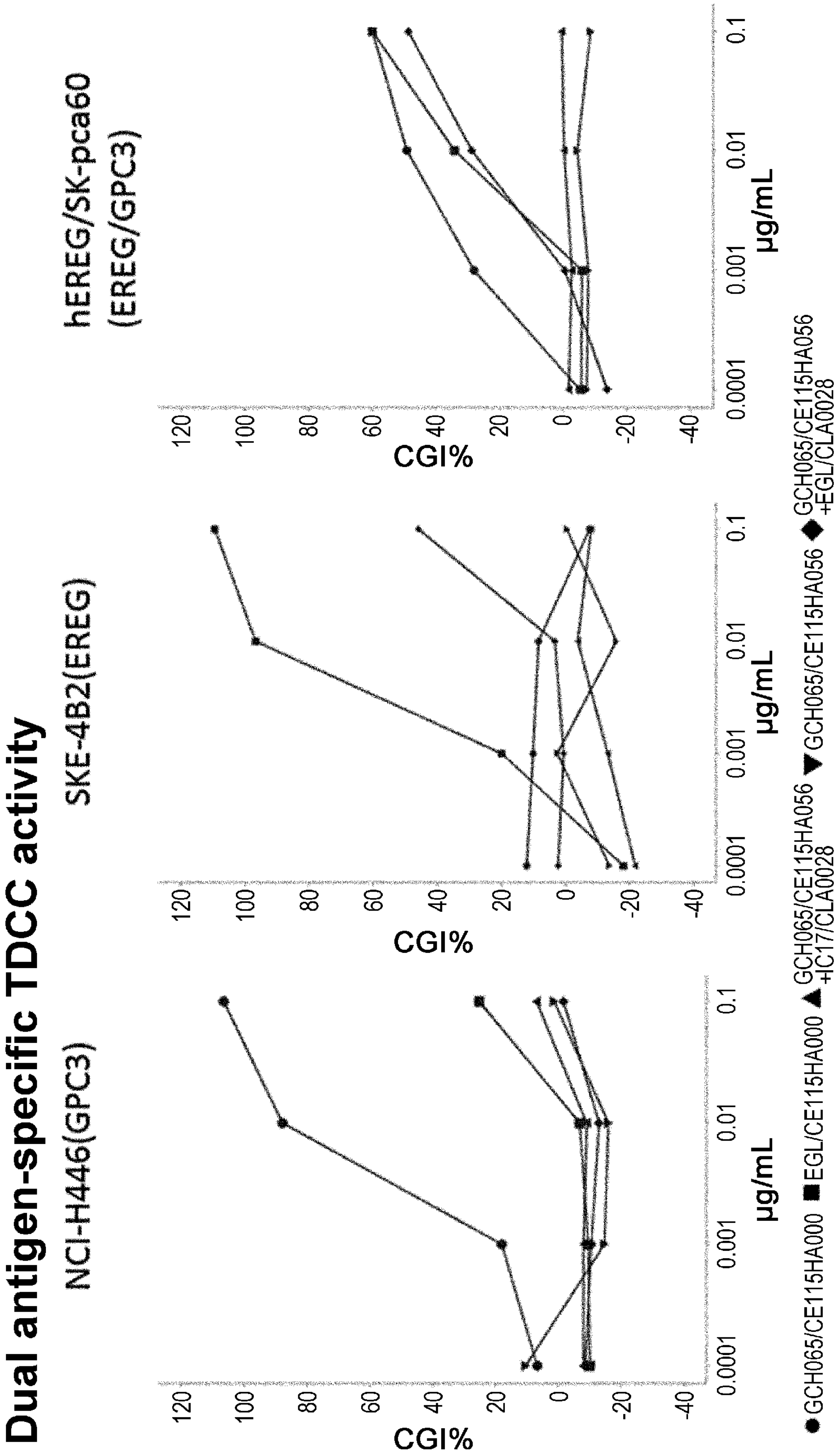
Dual antigen-specific TDCC activity
NCI-H446(GPC3)
SKE-4B2(EREG)
hEREG/SK-pca60
(EREG/GPC3)
CGI%
120
100
80
60
40
20
0
-20
-40
0.0001
0.001
0.01
0.1
µg/mL
● GCH065/CE115HA000
■ EGL/CE115HA000
▲ GCH065/CE115HA056 +IC17/CLA0028
▼ GCH065/CE115HA056
◆ GCH065/CE115HA056 +EGL/CLA0028

FIG. 7

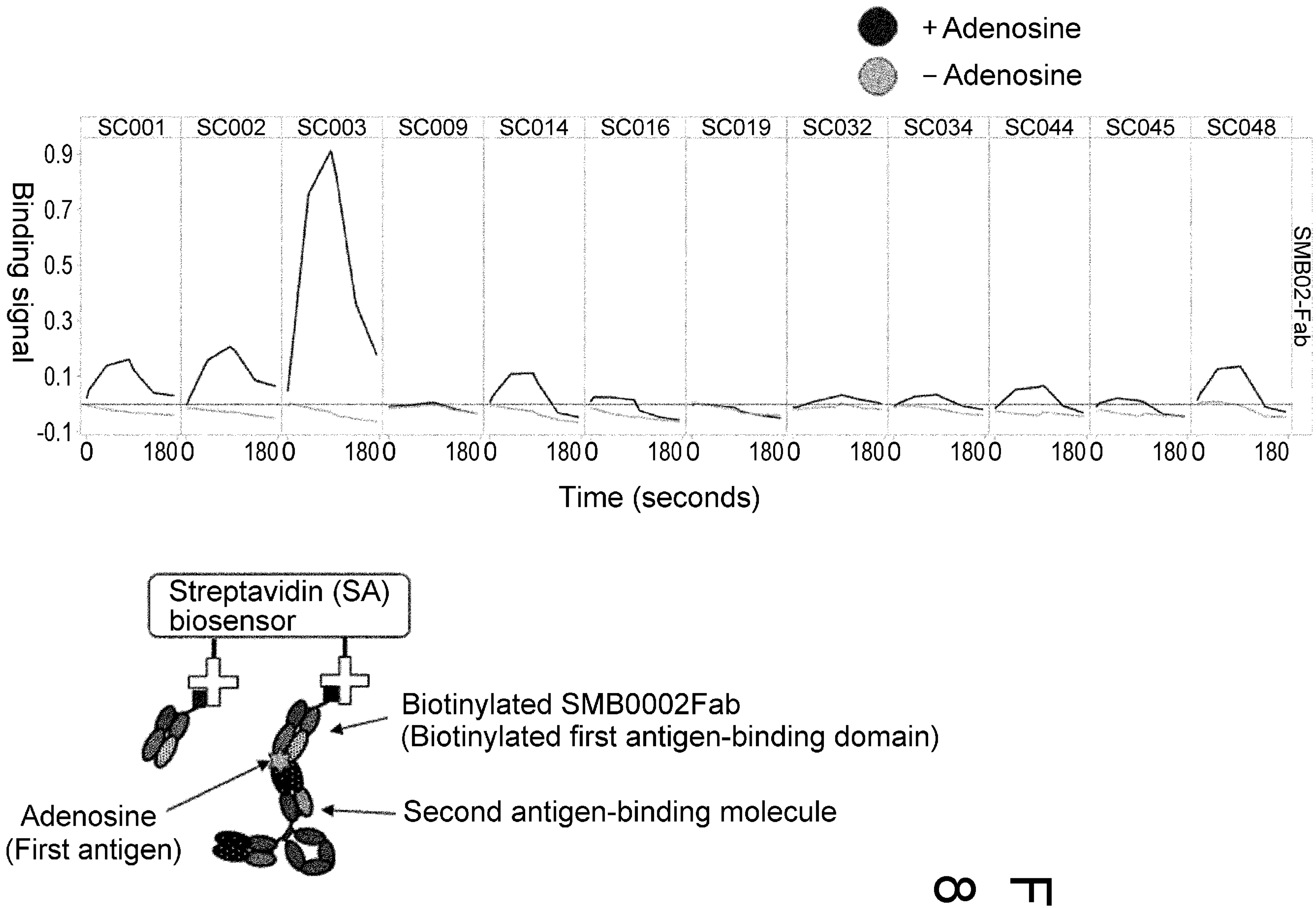
+ Adenosine
− Adenosine
SC001
SC002
SC003
SC009
SC014
SC016
SC019
SC032
SC034
SC044
SC045
SC048
SMB02-Fab
Binding signal
0.9
0.7
0.5
0.3
0.1
-0.1
0
180
Time (seconds)
Streptavidin (SA) biosensor
Biotinylated SMB0002Fab
(Biotinylated first antigen-binding domain)
Second antigen-binding molecule
Adenosine
(First antigen)

FIG. 8

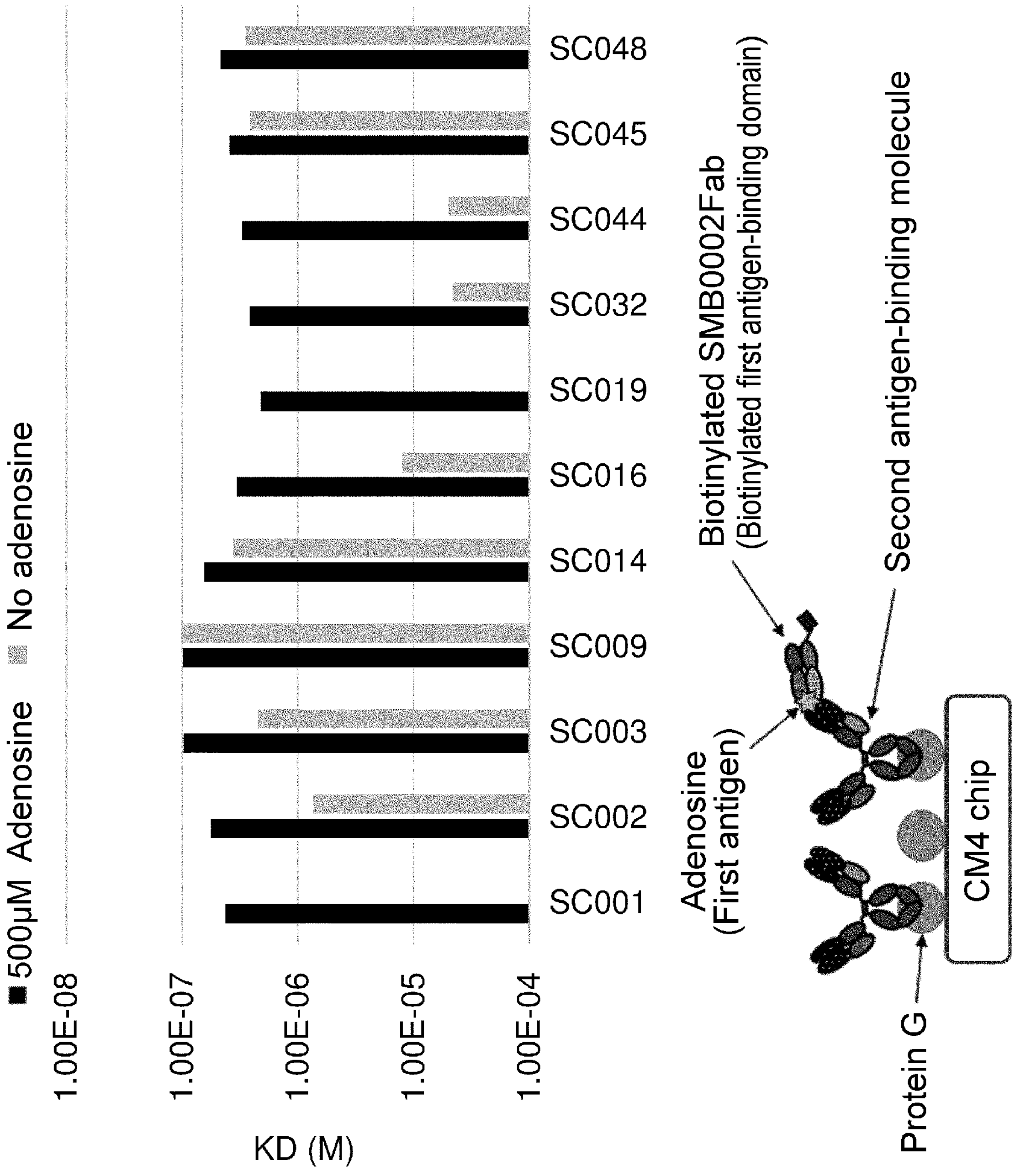
500μM Adenosine
No adenosine
KD (M)
1.00E-08
1.00E-07
1.00E-06
1.00E-05
1.00E-04
SC001
SC002
SC003
SC009
SC014
SC016
SC019
SC032
SC044
SC045
SC048
Biotinylated SMB0002Fab
(Biotinylated first antigen-binding domain)
Second antigen-binding molecule
Adenosine
(First antigen)
Protein G
CM4 chip

FIG. 9

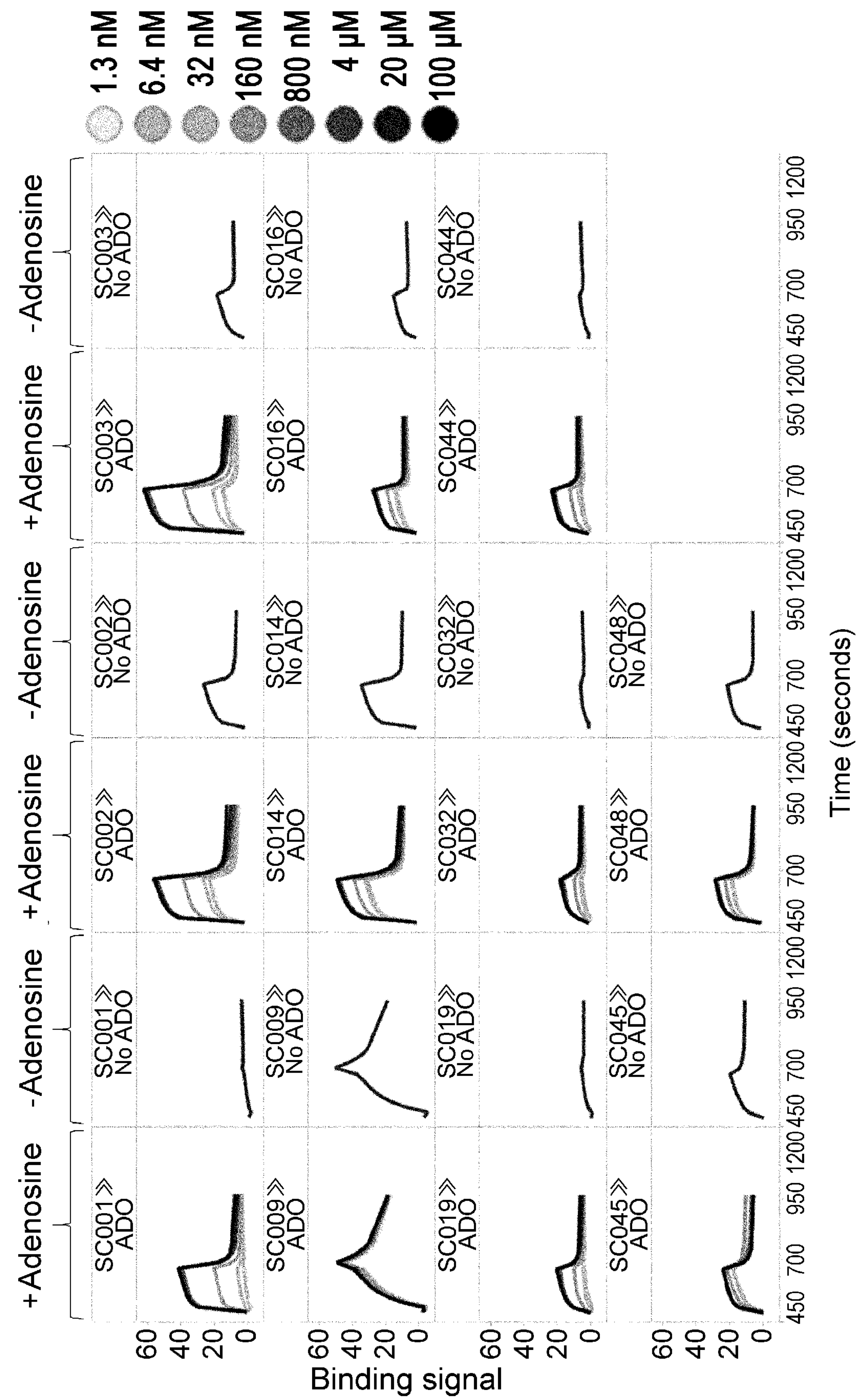

+Adenosine
-Adenosine
+Adenosine
-Adenosine
+Adenosine
-Adenosine
1.3 nM
6.4 nM
32 nM
160 nM
800 nM
4 μM
20 μM
100 μM
SC001≫ ADO
SC001≫ No ADO
SC002≫ ADO
SC002≫ No ADO
SC003≫ ADO
SC003≫ No ADO
SC009≫ ADO
SC009≫ No ADO
SC014≫ ADO
SC014≫ No ADO
SC016≫ ADO
SC016≫ No ADO
SC019≫ ADO
SC019≫ No ADO
SC032≫ ADO
SC032≫ No ADO
SC044≫ ADO
SC044≫ No ADO
SC045≫ ADO
SC045≫ No ADO
SC048≫ ADO
SC048≫ No ADO
Binding signal
Time (seconds)


FIG. 10

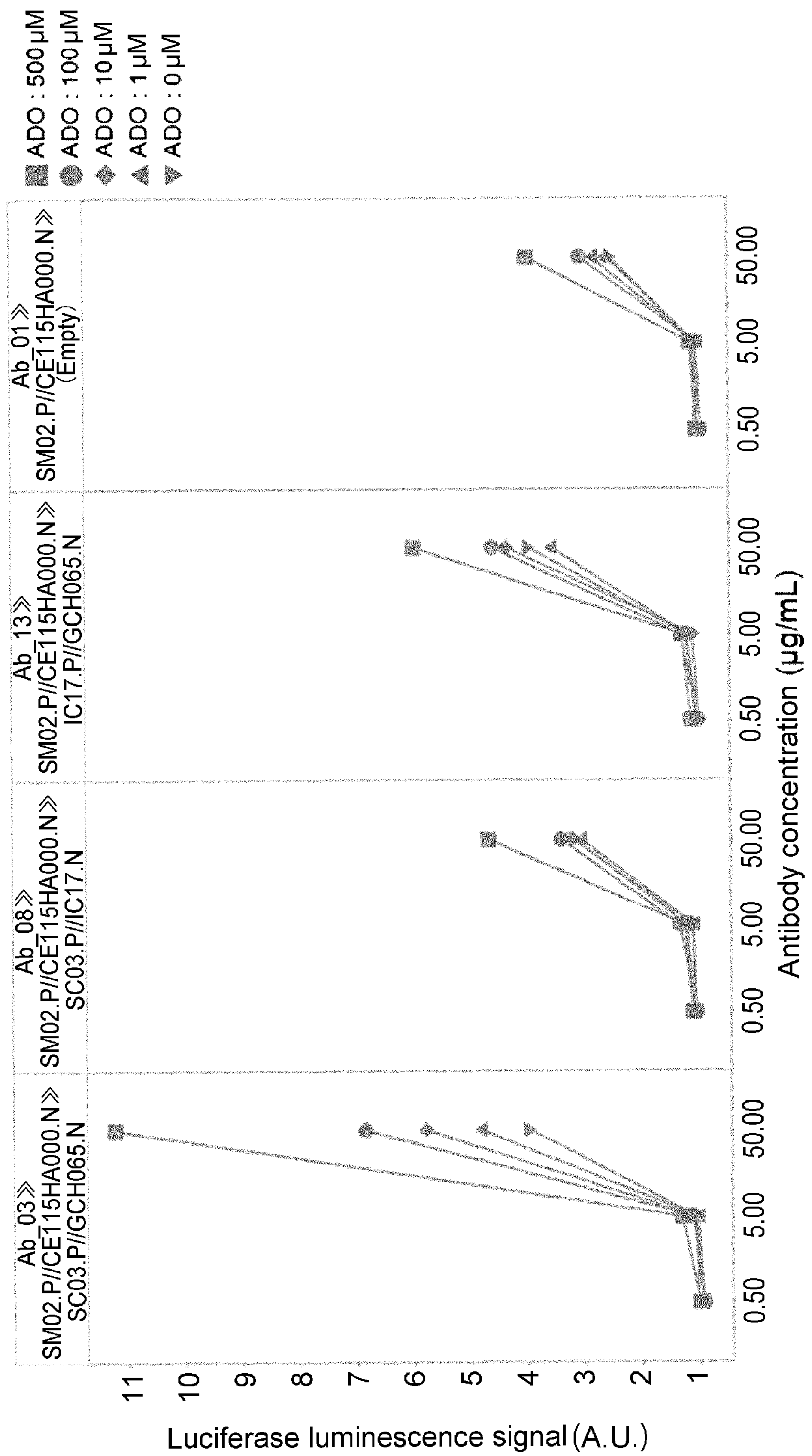
ADO : 500μM
ADO : 100μM
ADO : 10μM
ADO : 1μM
ADO : 0μM
Ab_03》
SM02.P//CE115HA000.N》
SC03.P//GCH065.N
Ab_08》
SM02.P//CE115HA000.N》
SC03.P//IC17.N
Ab_13》
SM02.P//CE115HA000.N》
IC17.P//GCH065.N
Ab_01》
SM02.P//CE115HA000.N》
(Empty)
Luciferase luminescence signal (A.U.)
11
10
9
8
7
6
5
4
3
2
1
0.50
5.00
50.00
Antibody concentration (μg/mL)

FIG. 11

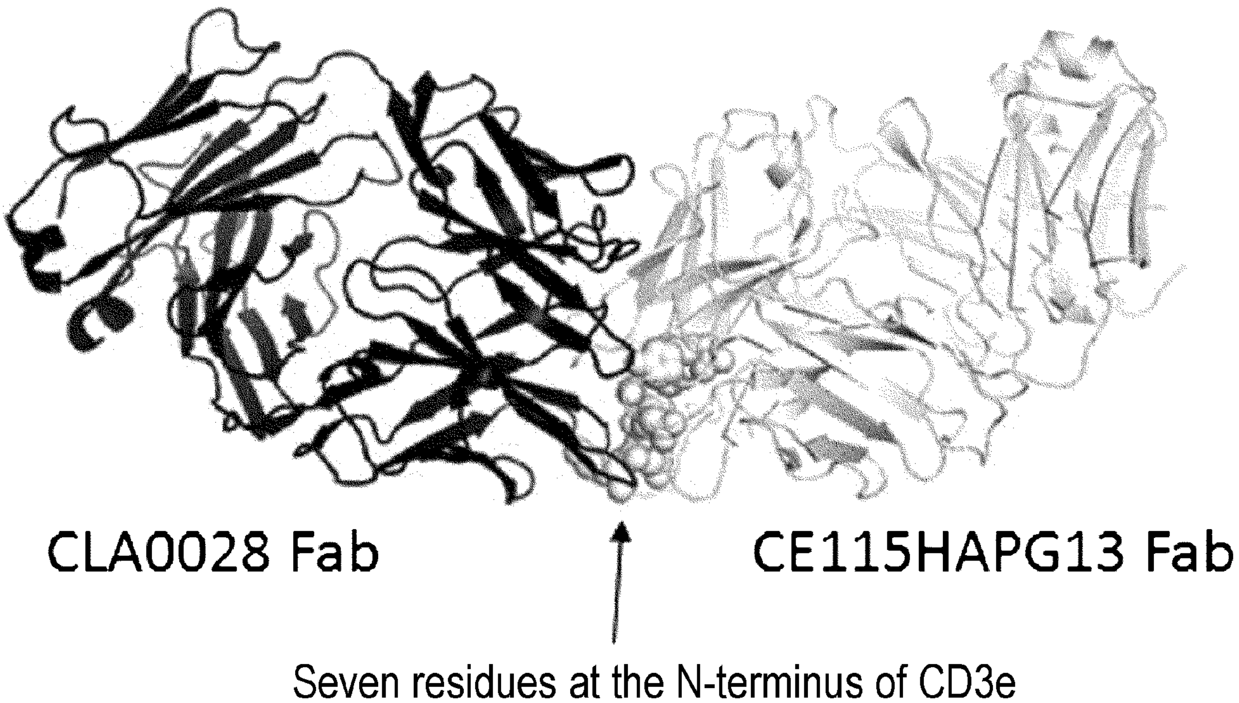
CLA0028 Fab
CE115HAPG13 Fab
Seven residues at the N-terminus of CD3e

FIG. 12

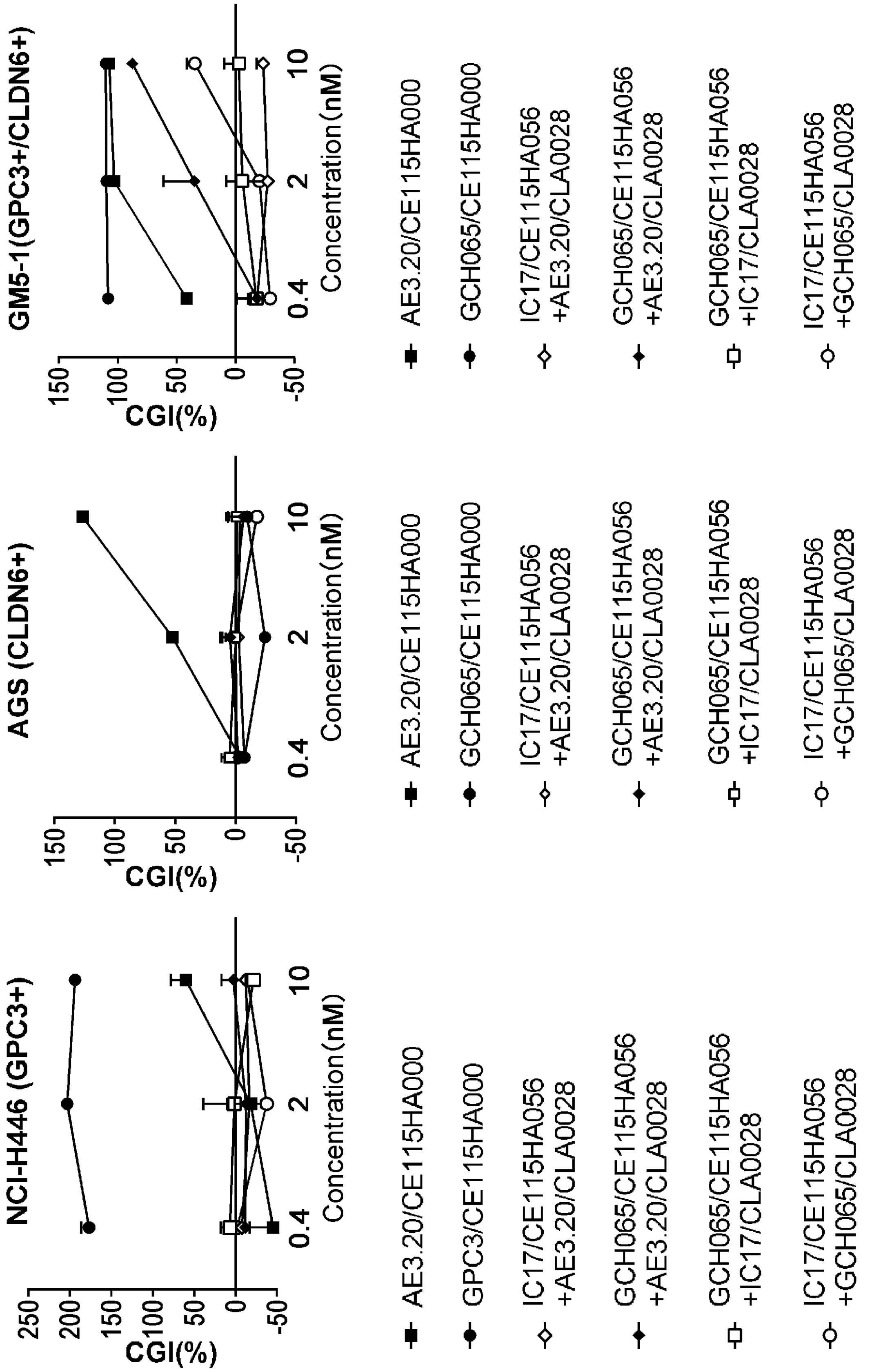
GM5-1(GPC3+/CLDN6+)
AGS (CLDN6+)
NCI-H446 (GPC3+)
CGI(%)
150
100
50
0
-50
250
200
Concentration (nM)
0.4
2
10
AE3.20/CE115HA000
GCH065/CE115HA000
GPC3/CE115HA000
IC17/CE115HA056
+AE3.20/CLA0028
GCH065/CE115HA056
+AE3.20/CLA0028
GCH065/CE115HA056
+IC17/CLA0028
IC17/CE115HA056
+GCH065/CLA0028

FIG. 13

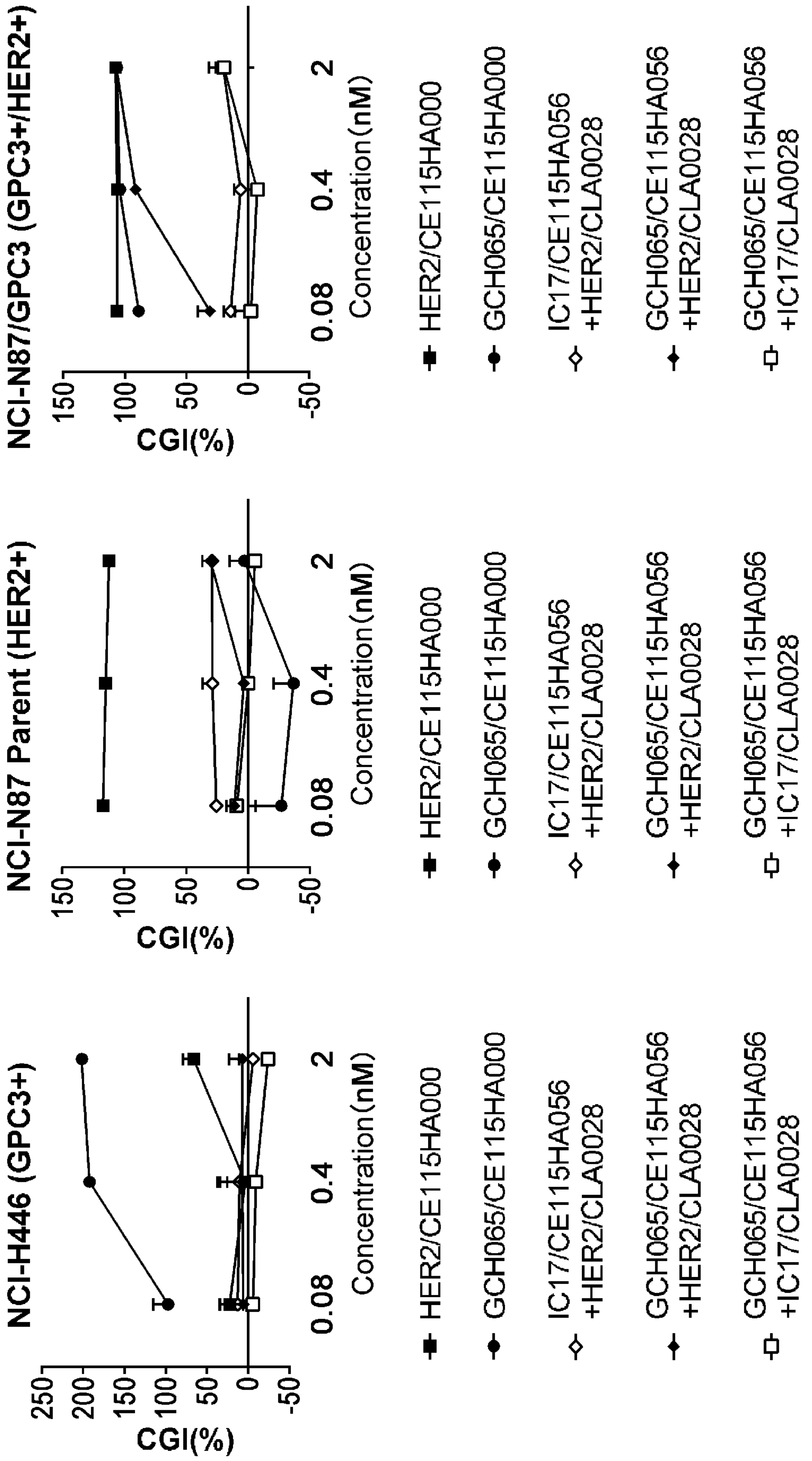
NCI-N87/GPC3 (GPC3+/HER2+)
CGI(%)
150
100
50
0
-50
0.08
0.4
2
Concentration (nM)
HER2/CE115HA000
GCH065/CE115HA000
IC17/CE115HA056
+HER2/CLA0028
GCH065/CE115HA056
+HER2/CLA0028
GCH065/CE115HA056
+IC17/CLA0028
NCI-N87 Parent (HER2+)
CGI(%)
150
100
50
0
-50
0.08
0.4
2
Concentration (nM)
HER2/CE115HA000
GCH065/CE115HA000
IC17/CE115HA056
+HER2/CLA0028
GCH065/CE115HA056
+HER2/CLA0028
GCH065/CE115HA056
+IC17/CLA0028
NCI-H446 (GPC3+)
CGI(%)
250
200
150
100
50
0
-50
0.08
0.4
2
Concentration (nM)
HER2/CE115HA000
GCH065/CE115HA000
IC17/CE115HA056
+HER2/CLA0028
GCH065/CE115HA056
+HER2/CLA0028
GCH065/CE115HA056
+IC17/CLA0028

FIG. 14

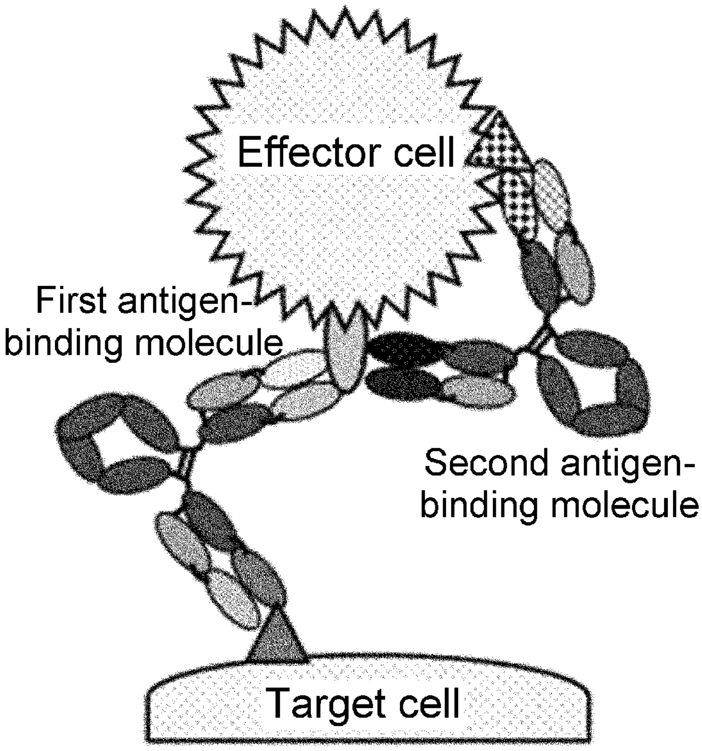
Effector cell
First antigen-binding molecule
Second antigen-binding molecule
Target cell

FIG. 15

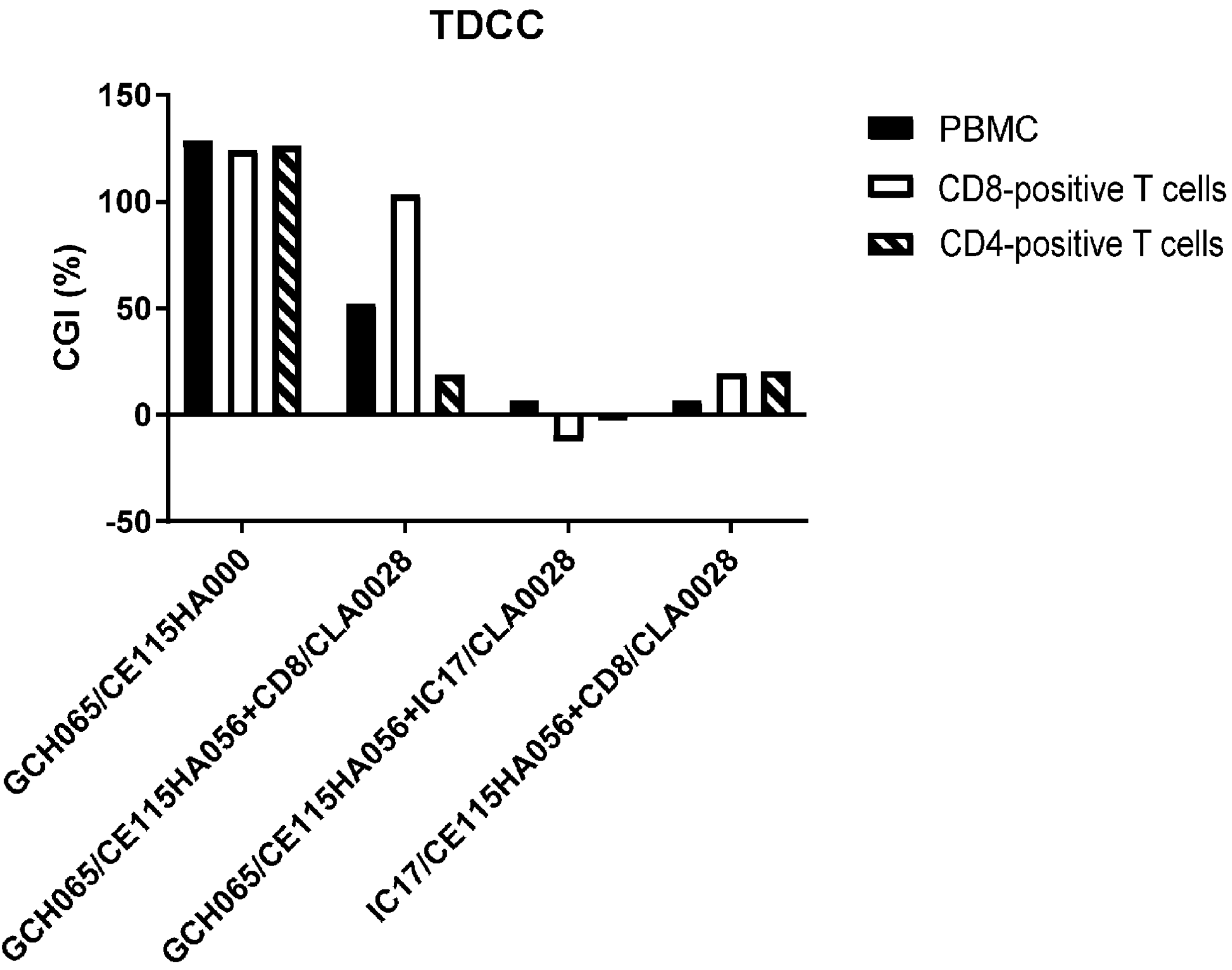
TDCC
150
100
50
0
-50
CGI (%)
PBMC
CD8-positive T cells
CD4-positive T cells
GCH065/CE115HA000
GCH065/CE115HA056+CD8/CLA0028
GCH065/CE115HA056+IC17/CLA0028
IC17/CE115HA056+CD8/CLA0028

FIG. 16

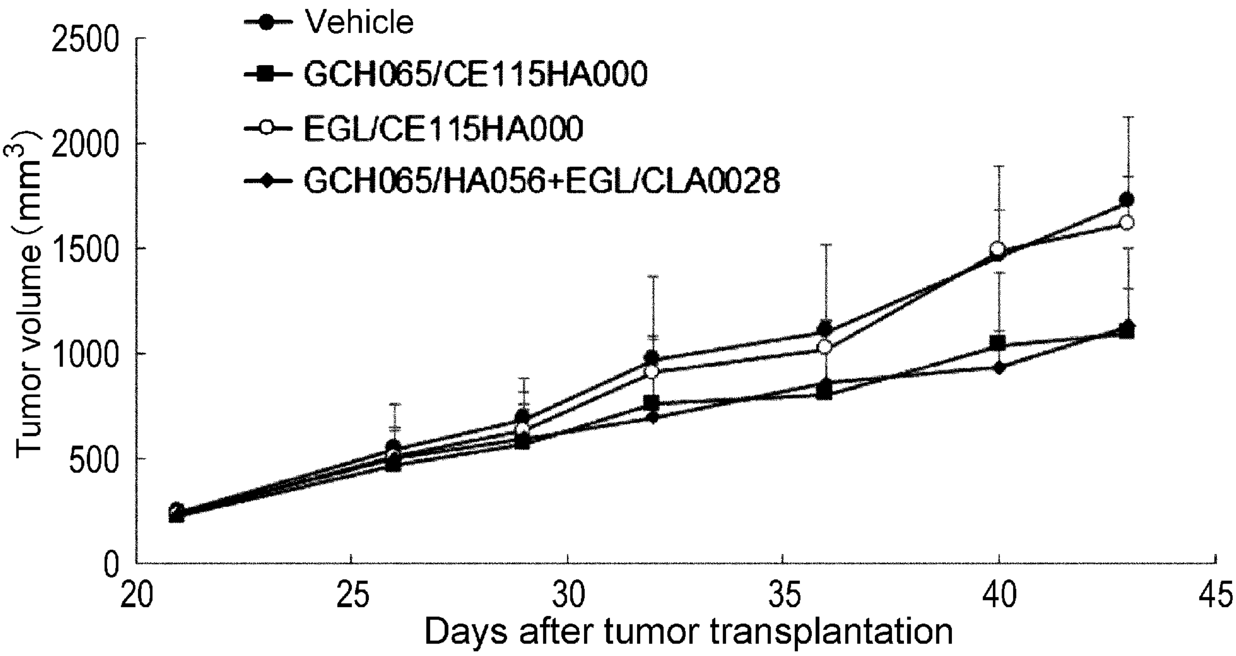
Vehicle
GCH065/CE115HA000
EGL/CE115HA000
GCH065/HA056+EGL/CLA0028
Tumor volume (mm3)
2500
2000
1500
1000
500
0
20
25
30
35
40
45
Days after tumor transplantation

FIG. 17

**INTERNATIONAL SEARCH REPORT**

International application No.
PCT/JP2019/005258

A. CLASSIFICATION OF SUBJECT MATTER
Int.Cl. C07K16/42(2006.01)i, C07K16/00(2006.01)i, C07K16/28(2006.01)i, C12N15/13(2006.01)n

According to International Patent Classification (IPC) or to both national classification and IPC

B. FIELDS SEARCHED

Minimum documentation searched (classification system followed by classification symbols)
Int.Cl. C07K16/42, C07K16/00, C07K16/28, C12N15/13

Documentation searched other than minimum documentation to the extent that such documents are included in the fields searched

| | |
|---|---|
| Published examined utility model applications of Japan | 1922-1996 |
| Published unexamined utility model applications of Japan | 1971-2019 |
| Registered utility model specifications of Japan | 1996-2019 |
| Published registered utility model applications of Japan | 1994-2019 |

Electronic data base consulted during the international search (name of data base and, where practicable, search terms used)
JSTPlus/JMEDPlus/JST7580 (JDreamIII), CAplus/MEDLINE/EMBASE/BIOSIS (STN)

C. DOCUMENTS CONSIDERED TO BE RELEVANT

| Category* | Citation of document, with indication, where appropriate, of the relevant passages | Relevant to claim No. |
|---|---|---|
| X<br>-<br>Y | TOWBIN, H. et al., "Sandwich immunoassay for the hapten angiotensin II A novel assay principle based on antibodies against immune complexes", Journal of Immunological Methods, 1995, vol. 181, pp. 167-176, abstract, fig. 4, 5, etc. | 1-3, 15<br>-<br>1-15 |
| Y | WO 2017/159287 A1 (CHUGAI PHARMACEUTICAL CO., LTD.) 21 September 2017, claims, paragraph [0016], examples 3, 4, etc. & JP 2017-214405 A & JP 6175590 B1 & EP 3431102 A1 examples 3, 4 & KR 10-2018-0116215 A & CN 109069640 A | 1-15 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 26 April 2019 (26.04.2019) | 14 May 2019 (14.05.2019) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**

International application No.
PCT/JP2019/005258

C (Continuation). DOCUMENTS CONSIDERED TO BE RELEVANT

| Category* | Citation of document, with indication, where appropriate, of the relevant passages | Relevant to claim No. |
|---|---|---|
| Y | WO 2015/083764 A1 (CHUGAI PHARMACEUTICAL CO., LTD.) 11 June 2015, paragraph [0012], examples 1-6, etc. & US 2016/0304862 A1 examples 1-6 & EP 3078744 A1 & KR 10-2016-0093666 A & CN 105980557 A | 1-15 |
| A | WO 2009/142221 A1 (OTSUKA PHARMACEUTICAL CO., LTD.) 26 November 2009, claim 2, examples, etc. & US 2010/0040605 A1 claim 2 & EP 2289943 A1 & KR 10-2011-0007621 A & CN 102037018 A | 1-15 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009142221 A
- WO 2013180200 A
- WO 2009025846 A
- WO 2015156268 A
- WO 2012073985 A
- WO 2006106905 A
- WO 2009089004 A
- WO 2010129304 A
- WO 2014084607 A
- WO 96027011 A
- WO 2012058768 A
- WO 2007110205 A
- WO 2011143545 A
- WO 2014104165 A
- WO 2016020444 A
- WO 2008119565 A
- WO 2007042261 A
- WO 2015083764 A
- WO 2015046554 A


### Non-patent literature cited in the description


- **RALPH, A.** *Scientific Reports,* 2017, vol. 7, 1-15
- **PAUL J. CARTER ; GREG A. LAZAR.** Next generation antibody drugs: pursuit of the 'high-hanging fruit. *Nature Reviews Drug Discovery,* 01 December 2017, https://www.nature.com/articles/nrd.2017.227
- **PEGGS, K. S.** *Clinical and Experimental Immunology,* 2009, vol. 157, 9-19
- **MIMOTO, F.** *Current Pharmaceutical Biotechnology,* 2016, vol. 17, 1298-1314
- **IGAWA, T.** *Nature Biotechnology,* 2010, vol. 28, 1203-1208
- *MAbs,* 01 March 2012, vol. 4 (2
- **NAZARIAN, A. A.** *Journal of Biomolecular Screening,* 2015, vol. 20, 519-27
- **CHAN, A. C. ; CARTER P. J.** *Nature Review,* 2010, vol. 10, 301-316
- **WOZNIAK-KNOPP, G.** *Peds,* 2010, vol. 23 (4), 289-297
- **HILL Z. B.** *Nature Chemical Biology,* 2018, vol. 14, 112-117
- **PAUL J. CARTER ; GREG A. LAZAR.** Next generation antibody drugs: pursuit of the 'high-hanging fruit', [online. *Nature Reviews Drug Discovery,* 01 December 2017, https://www.nature.com/articles/nrd.2017.227
- **KONTERMANN, R. E.** *MAbs,* 2012, vol. 4, 182-197
- **HILL Z. B.** *Nature Chemical Biology,* 2018, vol. 14, 112-117
- **NAZARIAN, A. A.** *Journal of Biomolecular Screening,* 2015, vol. 20, 519-527
- **KABAT et al.** Sequences of Proteins of Immunological Interest. Public Health Service, National Institutes of Health, 1991
- **DAERON.** *Annu. Rev. Immunol.,* 1997, vol. 15, 203-234
- **RAVETCH ; KINET.** *Annu. Rev. Immunol,* 1991, vol. 9, 457-92
- **CAPEL et al.** *Immunomethods,* 1994, vol. 4, 25-34
- **DE HAAS et al.** *J. Lab. Clin. Med.,* 1995, vol. 126, 330-41
- **MARGARET MERCHANT et al.** *Nature Biotechnology,* 1998, vol. 16, 677-681
- *Protein Engineering Design & Selection,* 2010, vol. 23, 195-202
- *Biochemistry,* 1993, vol. 32 (27), 6828-6835
- **BAEUERLE PA.** *Curr. Opin. Mol. Ther.,* 2009, vol. 11, 22-30
- *Protein Science,* 1995, vol. 4, 2411-2423
- *Methods Mol. Biol.,* 2002, vol. 178, 133-145
- *Protein Science,* 1995, vol. 4, 2411-2423
- *J. Appl. Cryst.,* 2007, vol. 40, 658-674
- *Acta Cryst.,* 2010, vol. D66, 486-501
- *Acta Cryst.,* 2011, vol. D67, 355-367
- *Acta Cryst.,* 2012, vol. D68, 352-367
- *Journal for ImmunoTherapy of Cancer,* 2018, vol. 6, 56
- *Immunology.,* 2017, vol. 152 (3), 425-438